# EUROPEAN PATENT SPECIFICATION

(11) **EP 4 568 664 B1**
(45) Date of publication and mention of the grant of the patent: **01.04.2026**
(21) Application number: 24724715.8
(22) Date of filing: 08.04.2024
(51) Int. Cl.: A61K 31/351, A61K 31/4166, A61K 31/4184, A61K 31/4245, A61K 31/427, A61K 31/53, A61K 38/17, A61K 45/06, A61P 1/16, A61P 3/04, A61K 38/26

(54) **COMBINATION COMPRISING A THR-BETA AGONIST AND A GLP-1R AGONIST FOR USE IN TREATING OBESITY**
KOMBINATION AUS EINEM THR-BETA-AGONISTEN UND EINEM GLP-1R-AGONISTEN ZUR VERWENDUNG BEI DER BEHANDLUNG VON FETTLEIBIGKEIT
COMBINAISON COMPRENANT UN AGONISTE THR-BETA ET UN AGONISTE GLP-1R POUR SON UTILISATION DANS LE TRAITEMENT DE L'OBÉSITÉ

(30) Priority: 07.04.2023 US 202363495049 P; 12.07.2023 US 202363526362 P; 29.12.2023 US 202363615985 P
(43) Date of publication of application: 18.06.2025
(73) Proprietor: Terns Pharmaceuticals, Inc., Foster City, California 94404 (US)
(72) Inventor: TEAL, Olivia D., Foster City, California 94404 (US); QUINN, Kevin P., Foster City, California 94404 (US); JONES, Christopher T., Foster City, California 94404 (US); JASPER, Jeffrey R., Foster City, California 94404 (US)
(74) Representative: Cooley (UK) LLP
(86) International application number: PCT/US2024/023598
(87) International publication number: WO 2024/211898

(56) References cited:
- WO-A1-2019/240938
- WO-A1-2021/231646
- WO-A1-2022/040600
- WO-A1-2023/049518
- WO-A1-2023/086561
- WO-A1-2023/164050

## Description

### CROSS-REFERENCE TO RELATED APPLICATIONS

This application claims priority to and the benefit of U.S. Provisional Application Nos. 63/495,049, filed on April 7, 2023, 63/526,362, filed on July 12, 2023, and 63/615,985, filed on December 29, 2023.

### BACKGROUND

GLP-1 is a 30 amino acid long incretin hormone secreted by the L-cells in the intestine in response to ingestion of food. GLP-1 has been shown to stimulate insulin secretion in a physiological and glucose-dependent manner, decrease glucagon secretion, inhibit gastric emptying, decrease appetite, and stimulate proliferation of beta-cells. Marketed GLP-1R agonists are peptides that are typically administered by subcutaneous injection. Liraglutide and semaglutide were the first GLP-1 peptides to be approved for both the treatment of type II diabetes mellitus (T2DM) and obesity. Semaglutide has also been approved for the treatment of T2DM as a bioavailable oral formulation.

In a healthy subject, GLP-1 plays an important role regulating post-prandial blood glucose levels by stimulating glucose-dependent insulin secretion by the pancreas resulting in increased glucose absorption in the periphery. GLP-1 also suppresses glucagon secretion, leading to reduced hepatic glucose output. In addition, GLP-1 delays gastric emptying and slows small bowel motility delaying food absorption.

Thyroid hormone (TH) is synthesized in the thyroid gland in response to the thyroid stimulating hormone (TSH) secreted by the pituitary. Thyroid hormones function by binding to the thyroid hormone receptors (THR). The thyroid hormone receptor belongs to a family of nuclear receptors and regulates the target gene expressions. Thyroid hormone receptors include two different subtypes, i.e., THRα and THRβ. THRα is mainly distributed in cardiac tissues and plays an important role in regulating heart function. The THRβ subtype is mainly expressed in the liver and the pituitary, and regulates cholesterol metabolism and thyrotropin secretion.

At normal levels, thyroid hormones THs maintain body weight, metabolic rate, body temperature, and mood, and are responsible for regulating serum cholesterol. Attempts have been made to use thyroid hormones to regulate serum cholesterol. However, given the possible side effects on the heart from taking natural thyroid hormone (e.g., tachycardia and arrhythmia, heart failure, and thyroid axis function, muscle metabolism, and osteoporosis,) they are unsuitable for treating high cholesterol and obesity. Research results regarding the study of animals with selective knock-out of the THR gene, and research results of some selective THR ligands show that the side effects on the heart caused by these thyroid hormones can be attributed to THRα but not THRβ. Therefore, THRβ-seletive ligands may provide the benefits of THR agonism (e.g., cholesterol lowering) without the undesirable effects associated with THRα.

Combination therapies of known agonists can yield unexpected synergies and improved effects to a significantly greater extent than administration of either agent alone but effects of combination therapies are highly unpredictable. Developing new combination therapies for the treatment of various liver disorders and cardiometabolic diseases, including obesity, remains a critical unmet clinical need.

GLP-1R regulates post prandial blood glucose and satiety, which can induce weight loss but efficacy is limited by metabolic adaptation, a compensatory process that lowers energy expenditure (EE). Moreover, the higher dose levels of GLP-1R needed to achieve clinical meaningful weight loss, are associated with more severe and frequent GI-related side effects including nausea and emesis, which can negatively impact patient compliance. In a meta-analysis evaluating the efficacy and safety of orforglipron as an anti-obesity medication it has been shown that the total adverse events were significantly higher with all the doses of orforglipron compared to placebo, with the hazard ratios being higher with higher doses. Gastrointestinal side-effects were predominant side effects, being dose-dependent, with nausea, vomiting, constipation, and gastroesophageal reflux being the predominant ones (Dutta et al., Obes Sci Pract. 2024 Apr; 10(2):e743 PMID: 38414573). Compared to placebo, the occurrence of total adverse events was significantly higher with orforglipron 12 mg , 24 mg , 36 mg and 45 mg daily doses. (Frias et al., Lancet. 2023;402(10400):472-483, Wharton et al., N Engl J Med. 2023;389(10):877-888, Pratt et al, Diabetes Obes Metabol. 2023;25:2642-2649). Therefore, dose dependent gastrointestinal side effects are a persistent challenge in the field of GLP-1 therapeutics.

Mechanisms to improve the effectiveness of GLP-1 based therapies at lower doses, could mitigate GI-related side effects and improve patient compliance and outcomes.

WO 2019/240938 A1 discloses thyroid hormone receptor agonists for use in the treatment of various metabolic diseases.This document does not disclose a combination of one of the four THR-β agonists claimed and a GLP-1R agonist for use in treating obesity.

WO 2021/231646 A1 refers to a method of treating liver diseases such as non-alcoholic steatohepatitis (NASH) comprising administering to the patient an SSAO inhibitor and a THR-β agonist.

WO 2023/164050 A1 discloses GLP-1R agonists for use in the treatment of obesity.

WO 2023/086561 A1 reports on a method of treating a liver disorder by administering a Farnesoid X Receptor (FXR) agonist and a THR-β agonist.

### SUMMARY

In one aspect, the present disclosure is directed to a combination of a THRβ agonist which is: 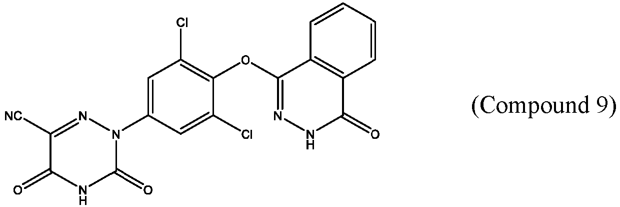 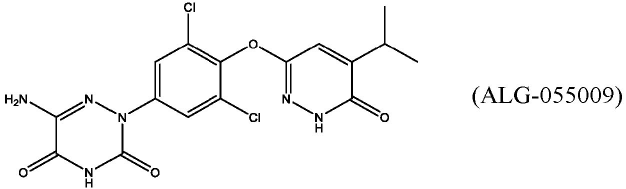 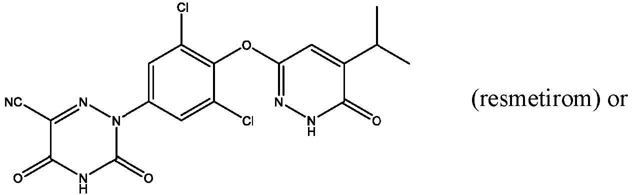 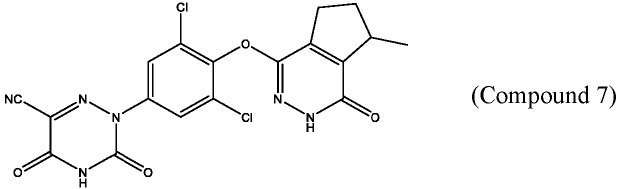 or a pharmaceutically acceptable salt thereof, and a GLP-1R agonist, or a pharmaceutically acceptable salt thereof, for use in
(i) effectuating weight loss in a patient having a BMI or 25 kg/m² or greater, or
(ii) the prevention or treatment of obesity.

In another aspect, the present disclosure is directed to the non-therapeutic use of a combination of a THRβ agonist which is: 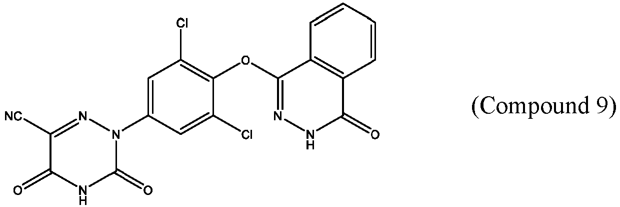 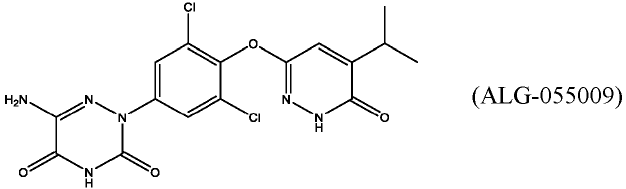 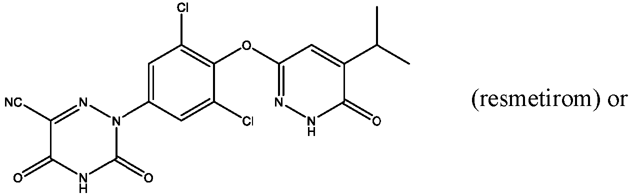 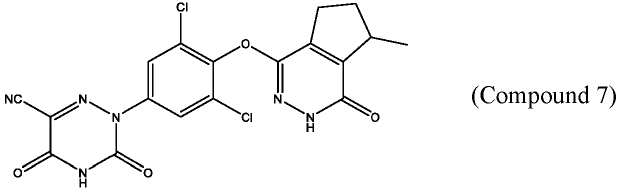 or a pharmaceutically acceptable salt thereof, and a GLP-1R agonist, or a pharmaceutically acceptable salt thereof, for effectuating weight loss.

In some embodiments, the THRβ agonist of the combination of the present disclosure is Compound 9: 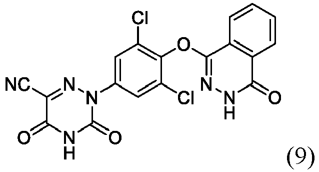 or a pharmaceutically acceptable salt thereof.

In some embodiments, the THRβ agonist of the combination of the present disclosure is a potassium salt of Compound 9.

In some embodiments, the GLP-1R agonist of the combination of the present disclosure is a compound of Formula (I-1): 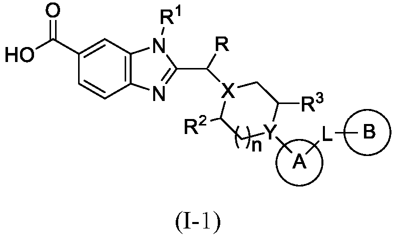 or a pharmaceutically acceptable salt thereof, wherein:
X is N or CH;
Y is N or CR⁴;
n is 0 or 1;
R is hydrogen;
R¹ is -C₁-C₆ alkylene-R⁵;
R² is hydrogen, oxo, or C₁-C₆ alkyl;
R³ is hydrogen, oxo, or C₁-C₆ alkyl and R⁴ is hydrogen, OH, or C₁-C₆ alkyl;
or R³ and R⁴ are taken together with the carbon atoms to which they are attached to form C₃-C₆ cycloalkyl optionally substituted by halo or C₁-C₃ alkyl;
R⁵ is 5-membered heterocyclyl or 5-membered heteroaryl, each of which comprises 1, 2, or 3 heteroatoms independently selected from O, N, and S, wherein at least one heteroatom of R⁵ is S, and further wherein R⁵ is optionally substituted by halo, -O-C₁₋₆ alkyl, C₁₋₆ alkyl, C₁₋₆ alkenyl, or C₁-C₆ haloalkyl;
Ring A is 5- to 12-membered heterocyclene or 5- to 12-membered heteroarylene, each of which is independently optionally substituted by halo, CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein
* represents the point of attachment to ring A and ** represents the point of attachment to ring B;
when L is *-O-C₁-C₆ alkylene-**, the C₁-C₆ alkylene of L is optionally substituted by R^{L}, wherein each R^{L} is independently C₁-C₆ alkyl or halo, or two R^{L} are taken together with the carbon atom or atoms to which they are attached to form C₃-C₆ cycloalkyl or 3- to 6-membered heterocyclyl; and
when L is C₁-C₆ alkylene, the C₁-C₆ alkylene is optionally substituted by R^{L1} wherein each R^{L1} is independently halo, OH, oxo, or C₁-C₆ alkyl, or two R^{L1} are taken together with the carbon atom or atoms to which they are attached to form C₃-C₆ cycloalkyl or 3- to 6-membered heterocyclyl;
R⁶ is hydrogen or C₁-C₆ alkyl; and
Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, C₁-C₆ haloalkyl, -COCH₃, -CONH₂, -S(O)₂CH₃, and phenyl.

In some embodiments, the GLP-1R agonist of the combination of the present disclosure is a compound of Formula (I-1a): 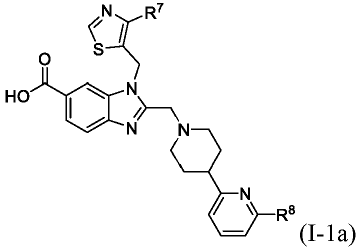 wherein R⁷ is hydrogen, chloro, bromo, fluoro, methyl, or vinyl; and

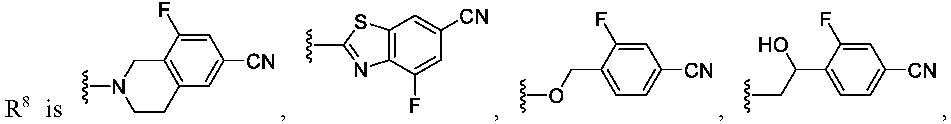

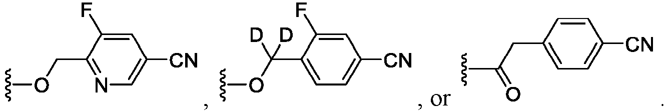

In some embodiments, the GLP-1R agonist is a compound of Formula (I**): 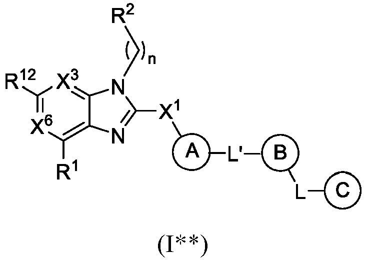 or a pharmaceutically acceptable salt thereof; wherein:
X³ is CR⁶ or N;
X⁶ is CR⁴ or N;
R¹ is -C₁₋₆ haloalkyl, halogen, -O-X⁴, or -NR⁸R⁹, or R¹ and R⁴, together with the atoms
to which they are attached, combine to form a 5- or 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃,-(CH₂-CH(-OCH₃)-CH₂-O)₁₋₅-CH₃, C₃₋₁₀ cycloalkyl, or C₆₋₁₀ aryl, wherein the alkyl, alkylene, heteroalkyl, or haloalkyl group is optionally substituted with one or more deuterium, C₁₋₆ alkoxy, hydroxyl, -CN, or oxo, and the cycloalkyl, heterocyclyl, or aryl group is optionally substituted with one or more halogen, C₁₋₆ alkoxy, or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocycyl; wherein the C₁₋₆ alkyl is optionally substituted by one or more oxo;
n is 0, 1, 2, 3, 4, 5, or 6;
R² is:
   hydrogen;
   C₁₋₆ alkyl optionally substituted with deuterium;
   C₁₋₆ haloalkyl;
   -(O)-C₁₋₆ alkyl;
   -CN;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 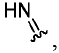 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a 5- or 6-membered heterocyclyl;
   R⁴ is halogen, hydrogen, -C(O)OH, or -O-R⁸
      wherein R⁸ and R¹, together with the atoms to which they are attached, combine to form a 6-membered heterocyclyl;
   R¹² is hydrogen, -C(O)OH,-C(O)NR^{N12}R^{N12'}, -C(O)NR¹²S(O)₂R^{12'}, -(C₂₋₆ alkynylene)-C(O)OH, -(C₁₋₆ alkylene)-C(O)OH, -NR^{N12}-(C₁₋₆ alkylene)-C(O)OH, 5-10 membered heteroaryl or 5- to 10-membered heterocyclyl optionally substituted with one or more oxo, C₁₋₆ alkyl or C₁₋₆ haloalkyl;
   R^{N12} and R^{N12'} independently are H or C₁₋₆ alkyl;
   X¹ is 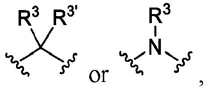
   wherein R³ and R^{3'} independently are H, D or C₁₋₆ alkyl, wherein the C₁₋₆ alkyl is optionally substituted with detuerium;
   Ring A is 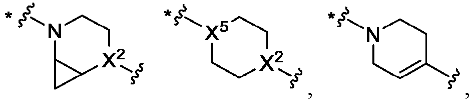 phenylene optionally substituted with one or more halo or C₁₋₆ alkyl, or 6-membered heteroarylene optionally substituted with one or more halo or C₁₋₆ alkyl;
   wherein * indicates attachment to X¹,
      X⁵ is CR³ or N, and
      X² is CR³ or N;
   L' is a bond or -O-;
   Ring B is a C₆₋₁₀ arylene, a 5-10 membered heteroarylene, or a 3-10 membered heterocycylene, wherein the C₆₋₁₀ arylene, 5-10 membered heteroarylene, or 3-10 membered heterocyclene is optionally substituted with one or more oxo, C₁₋₆ alkyl, C₁₋₆ alkoxy, or halogen;
   L is a bond, *-(C₁₋₆ alkylene)-, *-NR^{L}-(C₁₋₆ alkylene)-, *-O-(C₁₋₆ alkylene)-, or *-(C₁₋₆ alkylene)-O-, wherein * indicates attachment to Ring B and the C₁₋₆ alkylene is optionally substituted with deuterium;
      wherein R^{L} is H or C₁₋₆ alkyl; and
   Ring C is:
      a 6-membered aryl optionally substituted with one or more C₃₋₁₀ cycloalkyl, C₁₋₆ alkyl, C₁₋₆ haloalkyl, 3-10 membered heterocyclyl, halogen, C₁₋₆ alkoxy, C₁₋₆ haloalkoxy, -CN, C₃₋₁₀ cycloalkyl, or -C(O)NR'₂;
         wherein R' is H or C₁₋₆ alkyl
      a 6-membered heteroaryl comprising a nitrogen atom optionally substituted with one or more halogen, -CN, C₁₋₆ haloalkyl, -O-C₁₋₆ alkyl, C₃₋₁₀ cycloalkyl, -C(=O)-(C₃₋₁₀ cycloalkyl), 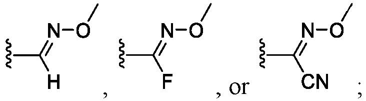 or
      a bicylic 9- or 10-membered heteroaryl or heterocyclyl optionally substituted with one or more C₁₋₆ alkyl, halogen, -CN, or oxo.

In some embodiments, the GLP-1R agonist of the combination of the present disclosure is a compound of Formula (I-5) 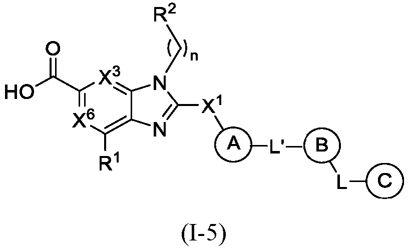 or a pharmaceutically acceptable salt thereof; wherein:
X³ is CR⁶ or N;
X⁶ is CR⁴ or N;
R¹ is -C₁₋₆ haloalkyl, -O-X⁴, or -NR⁸R⁹, or R¹ and R⁴, together with the atoms to which they are attached, combine to form a 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, hydroxyl, -CN, or oxo, and the cycloalkyl or heterocyclyl group is optionally substituted with one or more halogen or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocyclyl; wherein the C₁₋₆ alkyl is optionally substituted by one or more oxo;
n is 0, 1, 2, 3, 4, 5, or 6;
R² is:
   hydrogen;
   branched C₁₋₆ alkyl;
   C₁₋₆ haloalkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 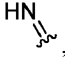 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a 5- or 6-membered heterocyclyl;
   R⁴ is hydrogen or -O-R⁸
      wherein R⁸ and R¹, together with the atoms to which they are attached, combine to form a 6-membered heterocyclyl;
   X¹ is 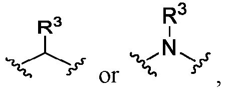
   wherein R³ is H or C₁₋₆ alkyl;
   Ring A is 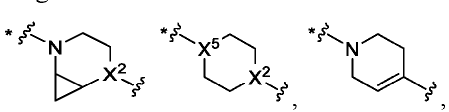 phenylene optionally substituted with one or more halo or C₁₋₆ alkyl, or 6-membered heteroarylene optionally substituted with one or more halo or C₁₋₆ alkyl;
   wherein * indicates attachment to X¹,
      X⁵ is CR³ or N, and
      X² is CR³ or N;
   L' is a bond or -O-;
   Ring B is a C₆₋₁₀ arylene, a 6-10 membered heteroarylene, or a 3-10 membered heterocyclene, wherein the C₆₋₁₀ arylene, 6-10 heteroarylene, or 3-10 membered heterocyclene is optionally substituted with one or more C₁₋₆ alkyl, C₁₋₆ alkoxy, or halogen;
   L is a bond, *-CH₂-, *-O-(C₁₋₆ alkylene)-, or *-(C₁₋₆ alkylene)-O-, wherein * indicates attachment to Ring B and the C₁₋₆ alkylene is optionally substituted with deuterium; and
   Ring C is:
      a 6-membered aryl optionally substituted with one or more C₁₋₆ alkyl, 3-10 membered heterocyclyl, halogen, -OCH₃, -CN, or C₃₋₁₀ cycloalkyl;
      a 6-membered heteroaryl comprising a nitrogen atom optionally substitut or more halogen, -CN, -O-C₁₋₆ alkyl, C₃₋₁₀ cycloalkyl, -C(=O)-(C₃₋₁₀ cycloalkyl), 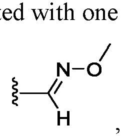 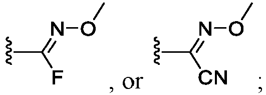 or
         a bicylic 9- or 10-membered heteroaryl comprising two nitrogen atoms optionally substituted with one or more C₁₋₆ alkyl, or oxo.

In some embodiments, the GLP-1R agonist of the combination of the present disclosure is Compound 1-2: 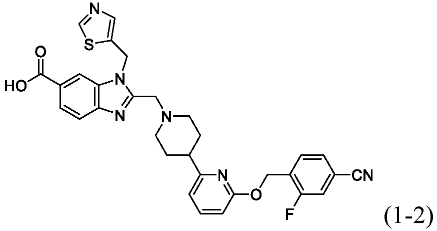 or a pharmaceutically acceptable salt thereof.

In some embodiments, the GLP-1R agonist of the combination of the present disclosure is a meglumine salt of Compound 1-2.

In some embodiments, the GLP-1R agonist of the combination of the present disclosure is selected from those listed in Tables 1-5.

In some embodiments, the GLP-1R agonist of the combination of the present disclosure is selected from those listed in Table 5A.

In some embodiments, the THRβ agonist of the combination of the present disclosure is compound 9 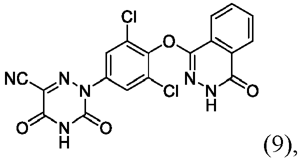 and the GLP-1R agonist is orforglipron, danuglipron, liraglutide, exenatide, dulaglutide, albiglutide, lixisenatide, tirzepatide, or semaglutide, or a pharmaceutically acceptable salt thereof.

In some embodiments, the THRβ agonist of the combination of the present disclosure is compound 9 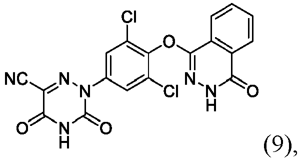 and the GLP-1R agonist is orforglipron, or a pharmaceutically acceptable salt thereof.

In some embodiments, the THRβ agonist of the combination of the present disclosure is resmetirom, ALG-055009 or a pharmaceutically acceptable salt thereof and the GLP-1R agonist is compound 1-2 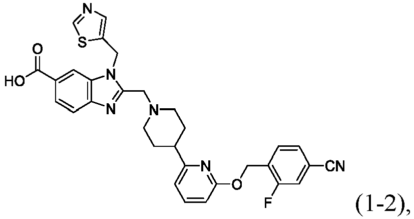 or a pharmaceutically acceptable salt thereof.

In some embodiments, the THRβ agonist of the combination of the present disclosure is compound 9 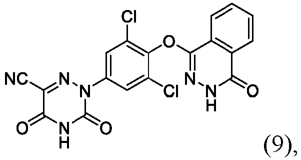 or a pharmaceutically acceptable salt thereof, and the GLP-1R agonist is compound 1-2 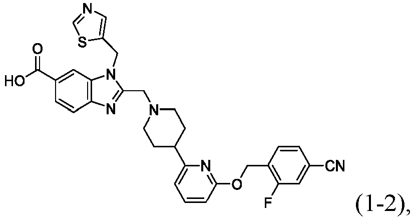 or a pharmaceutically acceptable salt thereof.

In one aspect, the present disclosure is directed to the combination for use in increasing the proportion of lean body mass relative to total body mass in a patient in need thereof, comprising administering to the patient a combination disclosed herein.

In one aspect, the present disclosure is directed to the combination for use in decreasing the proportion of fat mass relative to total body mass in a patient in need thereof, comprising administering to the patient a combination disclosed herein.

In one aspect, the present disclosure is directed to the combination for use in effectuating weight loss in a patient in need thereof, comprising administering to the patient a combination disclosed herein.

In some embodiments of the non-therapeutic use, the patient has a Body Mass Index (BMI) of 20 kg/m² to 25 kg/m².

In some embodiments, the patient has a BMI of 25 kg/m² to 30 kg/m².

In some embodiments of the non-therapeutic use, the patient has a BMI of 20 kg/m² or greater.

In some embodiments, the patient has a BMI of 25 kg/m² or greater.

In some embodiments, the patient has a BMI of 27 kg/m² or greater.

In some embodiments, the patient has a BMI or 30 kg/m² or greater.

In some embodiments, the cardiometabolic disease is obesity.

In some embodiments, the THRβ agonist is administered at substantially the same time as the GLP-1R agonist.

In some embodiments, the THRβ agonist is administered after the GLP-1R agonist.

In some embodiments, the THRβ agonist is administered prior to the GLP-1R agonist.

In some embodiments, the THRβ agonist is selective to THRβ.

### BRIEF DESCRIPTION OF THE FIGURES

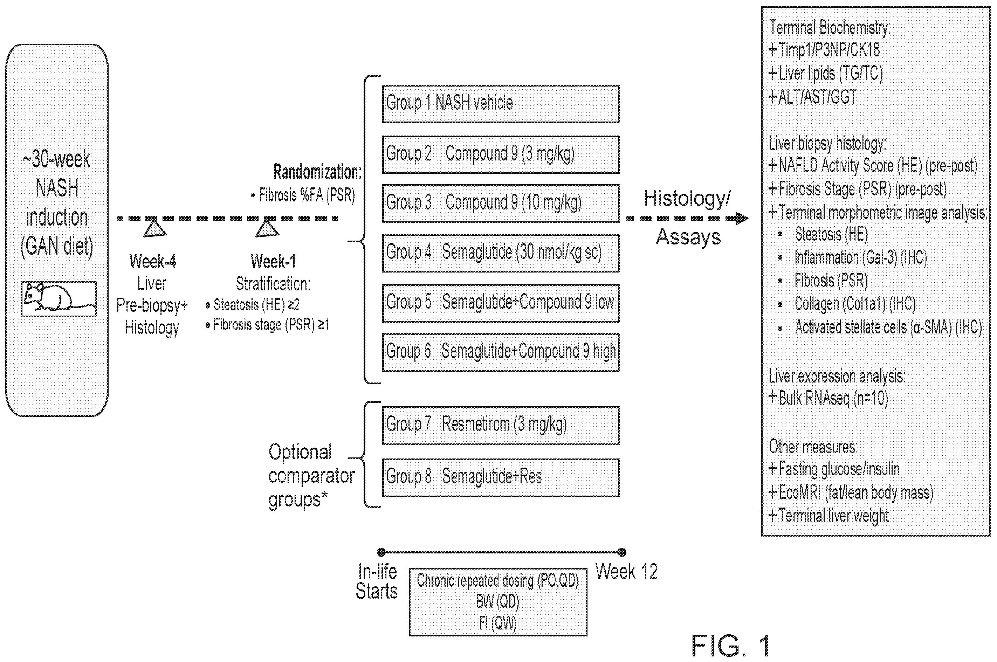

FIG. 1 depicts the study design for usage of a THRβ agonist (Compound 9) and a GLP-1R agonist (semaglutide) in combination to treat NASH.

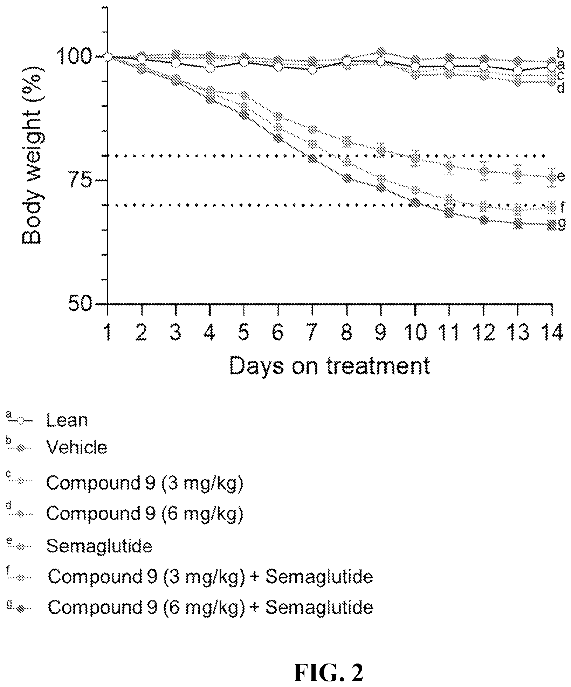

FIG. 2 depicts a graph showing the change from baseline in the body weight (%) of DIO-NASH mice over days of treatment with Compound 9 alone and in combination with semaglutide.

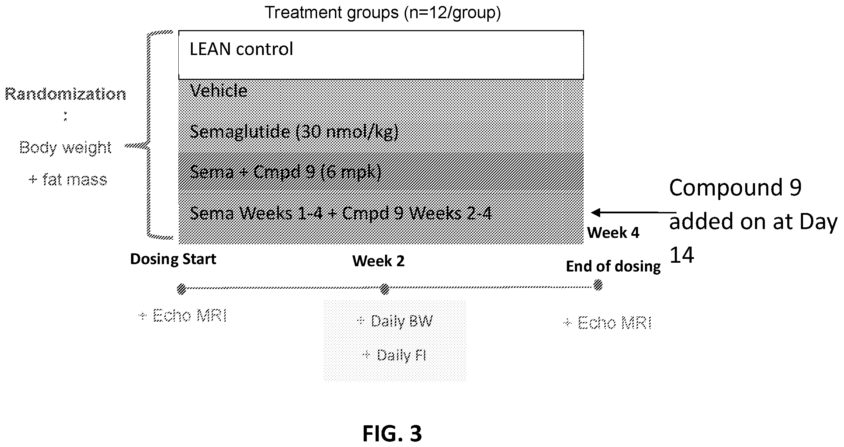

FIG. 3 depicts the study design for useage of a THRβ agonist (Compound 9) and a GLP-1R agonist (semaglutide) in combination for chronic weight management.

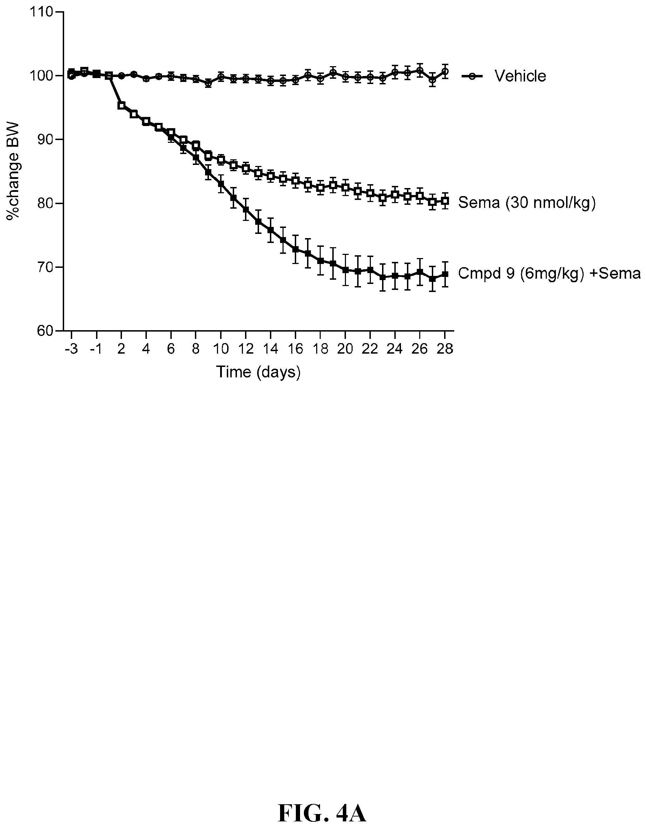

FIG. 4A depicts a graph showing the change in weight loss in Diet Induced Obese (DIO) mice over days of treatment with semaglutide alone or in combination with Compound 9.

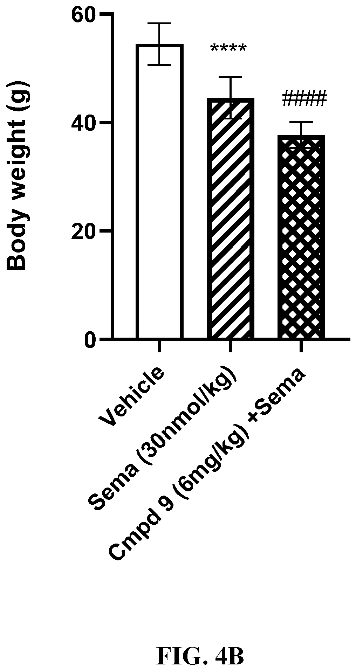

FIG. 4B depicts a graph showing the change in weight loss in DIO mice following treatment with semaglutide alone or in combination with Compound 9.

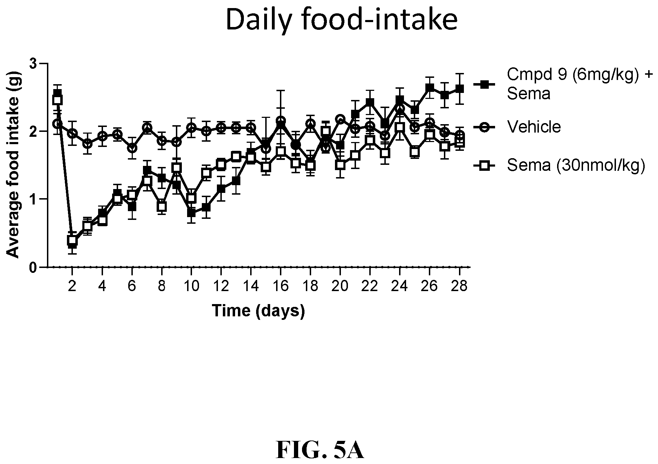

FIG. 5A depicts a graph showing the daily food intake in DIO mice over days of treatment with semaglutide alone or in combination with Compound 9.

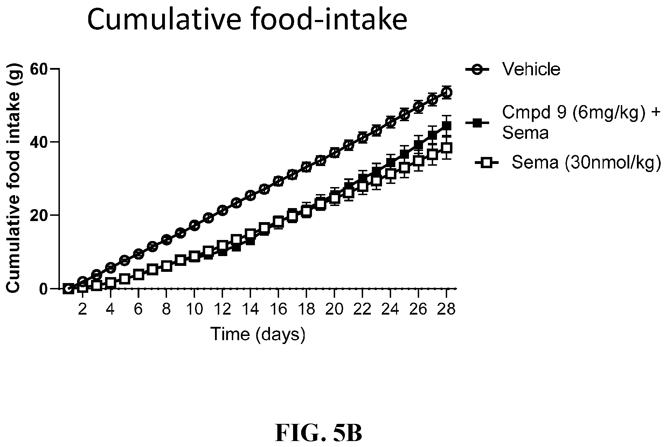

FIG. 5B depicts a graph showing the cumulative food intake in DIO mice over days of treatment with semaglutide alone or in combination with Compound 9.

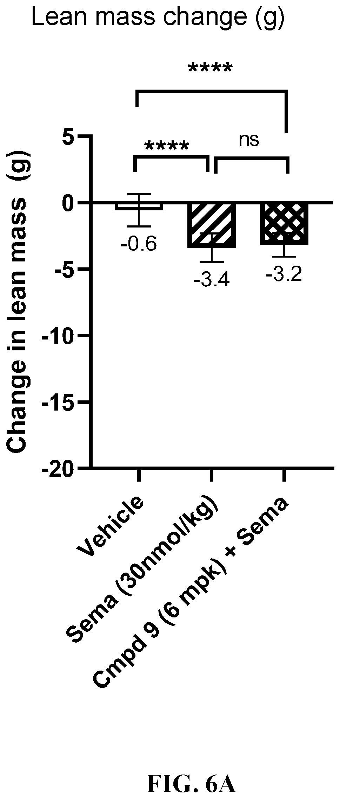

FIG. 6A depicts a graph showing the change in lean mass in DIO mice following treatment with semaglutide alone or in combination with Compound 9. **** Indicates p-value <0.0001 vs. Vehicle

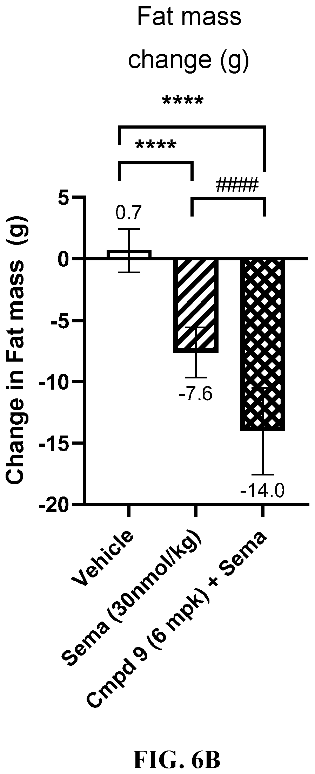

FIG. 6B depicts a graph showing the change in fat mass in DIO mice following treatment with semaglutide alone or in combination with Compound 9. **** Indicates p-value <0.0001 vs. Vehicle; ^{####}indicates p-value <0.001 vs. Semaglutide.

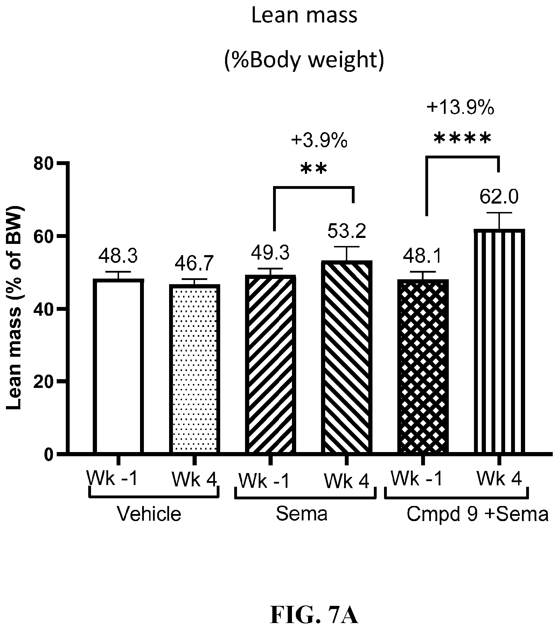

FIG. 7A depicts a graph showing the change in lean mass as a percent of bodyweight in DIO mice following treatment with semaglutide alone or in combination with Compound 9. The proportion of lean mass to total body mass increased by 3.9% following treatment by semaglutide alone and by 13.9% following treatment by semaglutide in combination with Compound 9. ** indicates p-value of 0.0028 and **** indicates p-value <0.0001.

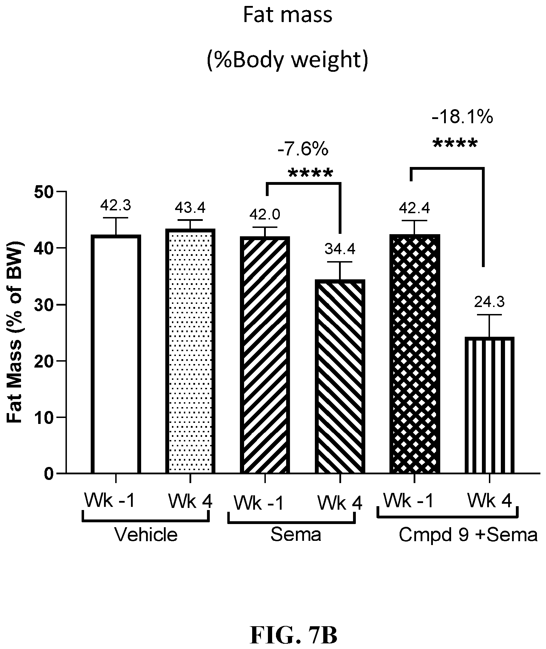

FIG. 7B depicts a graph showing the change in fat mass as a percent of bodyweight in DIO mice following treatment with semaglutide alone or in combination with Compound 9.

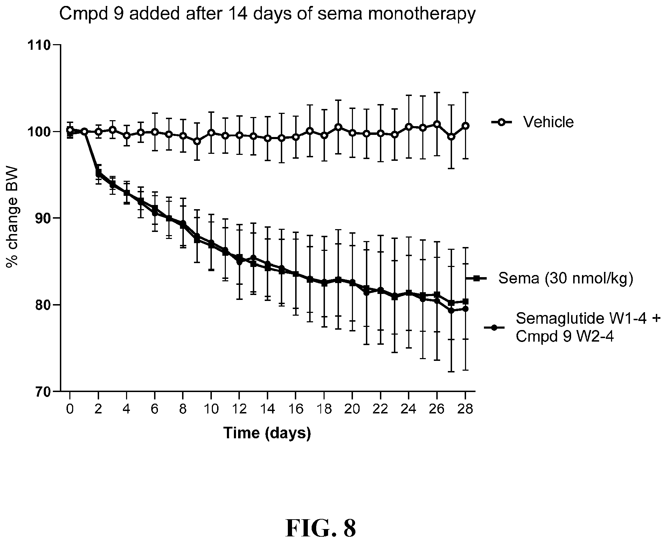

FIG. 8 depicts a graph showing the percent change from baseline in bodyweight in DIO mice following treatment with semaglutide alone or in combination with Compound 9, wherein Compound 9 is added after 14 days of semaglutide monotherapy.

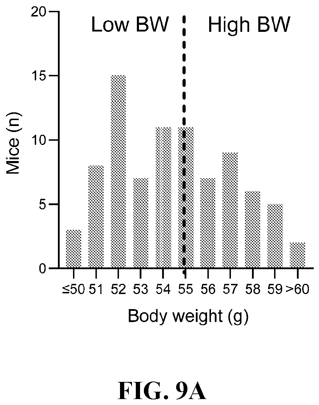

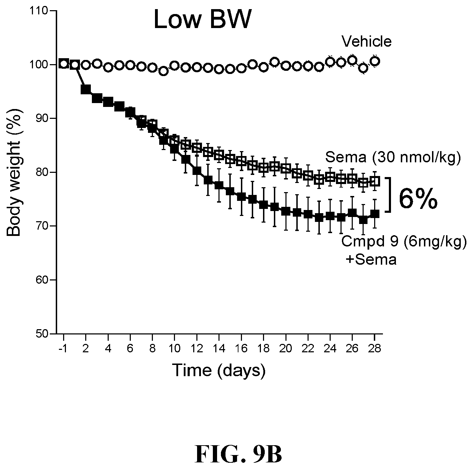

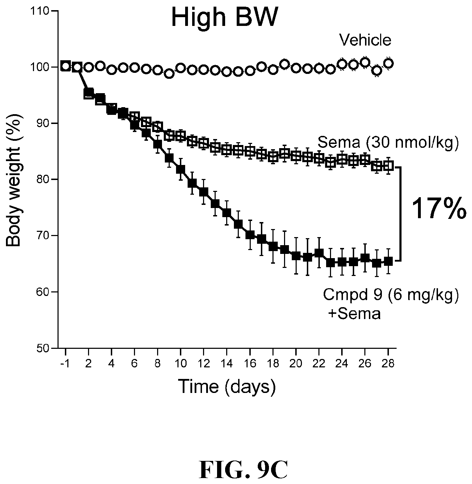

FIGs. 9A-C depict a graph showing the percent change from baseline in bodweight (BW) in DIO mice following treatment with semaglutide alone or semaglutide in combination with Compound 9. In a Post hoc analysis mice were divided into two subgroups based on their initial starting body weight (FIG. 9A). Body weights ranged from 50-54.9g (Low BW) and 55-60g (High BW). Compound 9 + Semaglutide has greater efficacy in mice with higher initial body weight (FIG. 9C) compared to those with lower initial body weight (FIG 9B).

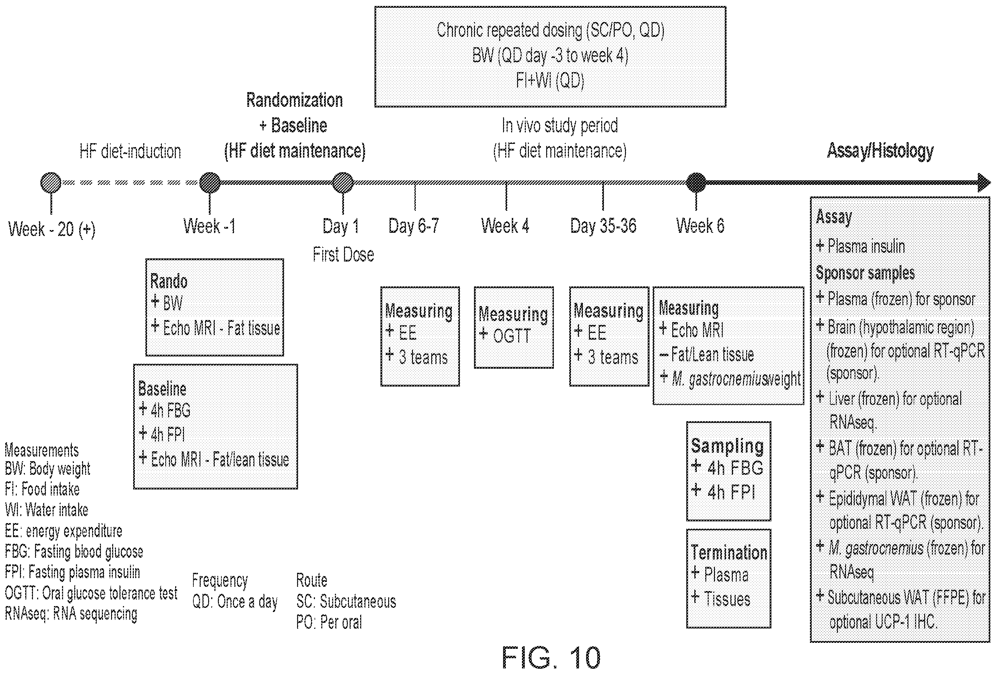

FIG. 10 depicts the design of a study to evaluate the effects of 6 weeks treatment with Compound 9 alone and in combination with Semaglutide on metabolic parameters, energy expenditure and glycemic control in male DIO mice at thermoneutrality.

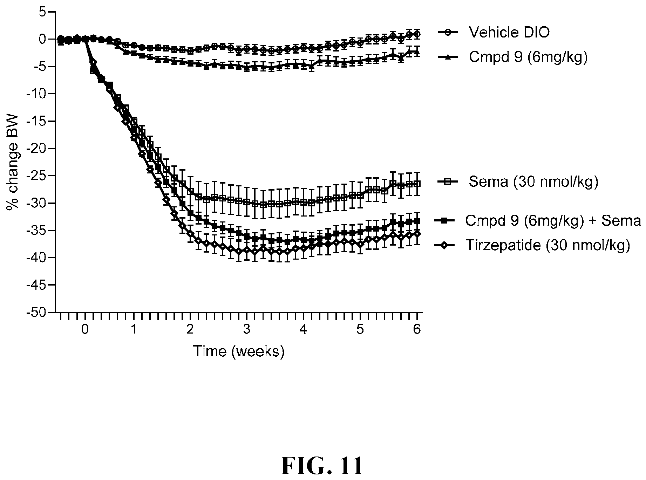

FIG. 11 depicts a graph showing the percent change from baseline in body weight in DIO mice following treatment with semaglutide alone, Compound 9 alone, semaglutide in combination with Compound 9, or tirzepatide alone. After 6 weeks of treatment, Compound 9+ Semaglutide induced additional weight loss that was comparable to tirzepatide. ****p-value <0.0001;*p-value <0.05.

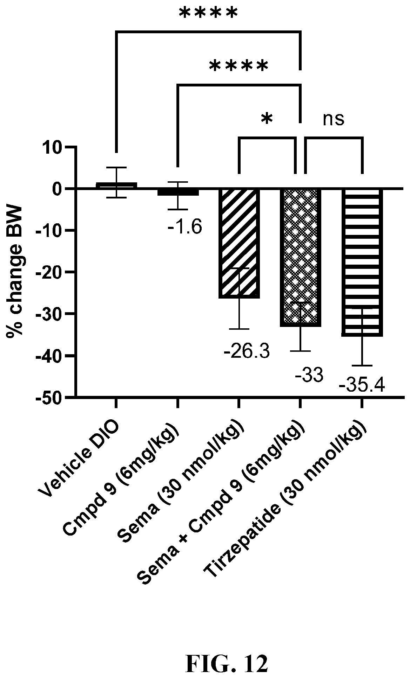

FIG. 12 depicts a graph showing the percent change from baseline in body weight in DIO mice following treatment with semaglutide alone, Compound 9 alone, semaglutide in combination with Compound 9, or tirzepatide alone. Compound 9 + Semaglutide induced an additional ~7% weight loss compared with semaglutide treatment alone.

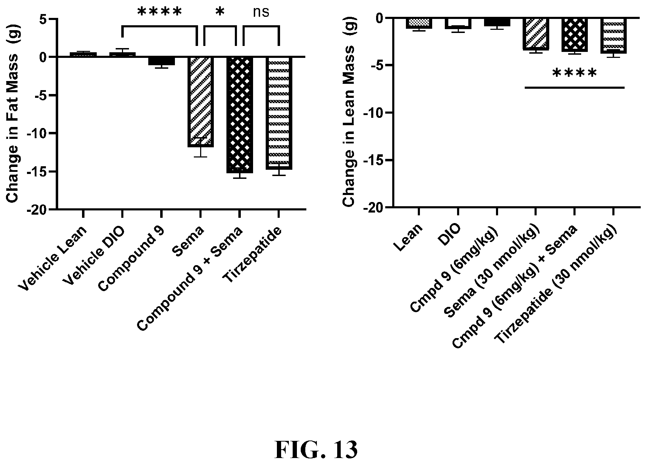

FIG. 13 depicts a pair of graphs showing the percent change in fat mass and lean mass following treatment with semaglutide alone, Compound 9 alone, semaglutide in combination with Compound 9, or tirzepatide. The body composition of mice was assessed on weeks 1 and 6 of the study using an EchoMRI 3-1 Body composition analyzer. Compound 9 + Semaglutide enhanced fat loss compared with Semaglutide treatment alone while lean mass loss was similar between semaglutide alone and the combination of Compound 9 + Semaglutide. ****p-value <0.0001;*p-value <0.05.

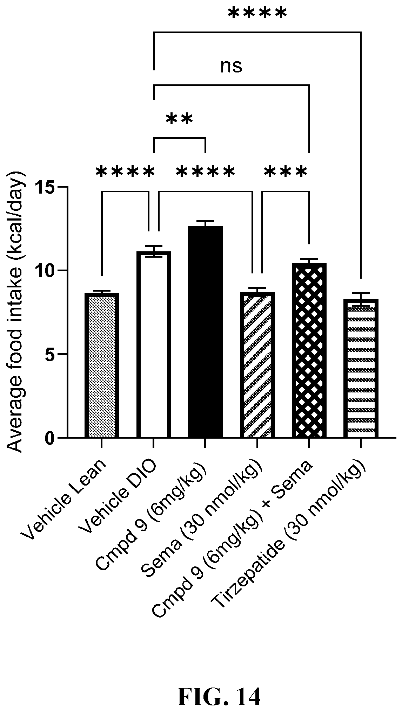

FIG. 14 depicts a graph showing the average food intake in DIO mice following treatment with semaglutide alone, Compound 9 alone, semaglutide in combination with Compound 9, or tirzepatide. Compound 9 normalized semaglutide-induced reduction in food intake. ****p-value <0.0001;***p-value <0.001; **p-value <0.01.

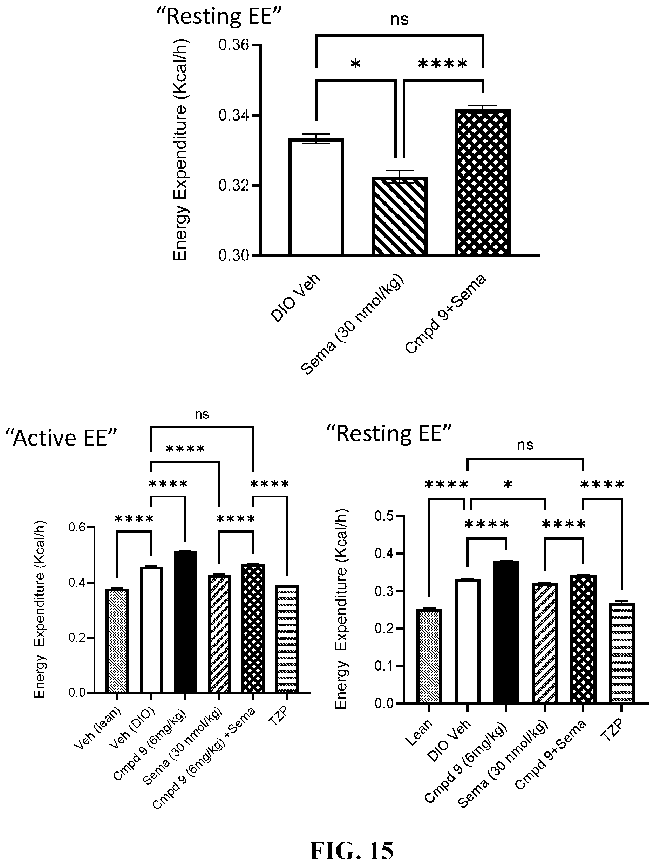

FIG. 15 depicts three graphs showing the energy expenditure (EE) assessed after week 4 in DIO mice following treatment with semaglutide alone, Compound 9 alone, semaglutide in combination with Compound 9, or tirzepatide. Compound 9+semaglutide prevented lowering of energy expenditure (EE) induced by weight Loss. Compound 9 restored energy expenditure to normal (DIO Veh) levels when combined with semaglutide. ****p-value <0.0001;*p-value <0.05.

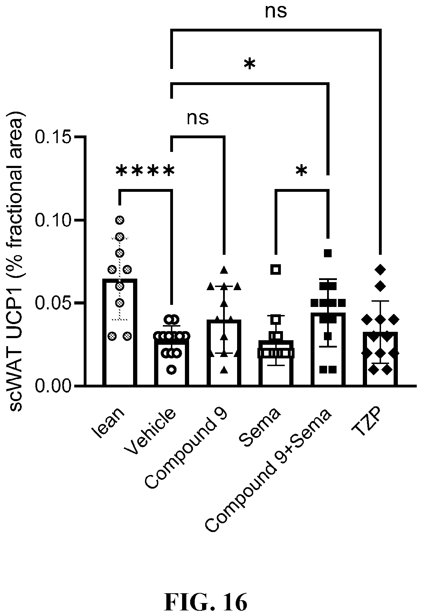

FIG. 16 depicts a graph showing uncoupling protein 1 (UCP-1) expression in DIO mice following treatment with semaglutide alone, Compound 9 alone, semaglutide in combination with Compound 9, or tirzepatide. Obesity lowered uncoupling protein 1 (UCP-1) expression in subcutaneous adipose tissue. Compound 9 partially restored the obesity induced lowering of UCP-1. *p< 0.05, one way ANOVA followed by Fisher's LSD test.

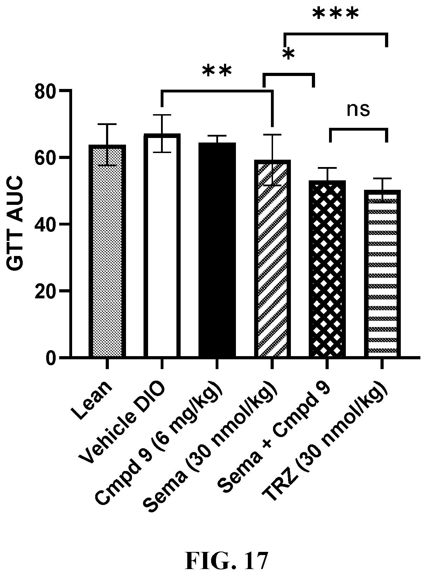

FIG. 17 depicts a graph showing the results of a glucose tolerance test (GTT) administered at week 4 to DIO mice following treatment with semaglutide alone, Compound 9 alone, semaglutide in combination with Compound 9, or tirzepatide. Compound 9+semaglutide further enhanced glucose tolerance compared with semaglutide treatment alone. ***p-value <0.001; **p-value <0.01; *p-value <0.05.

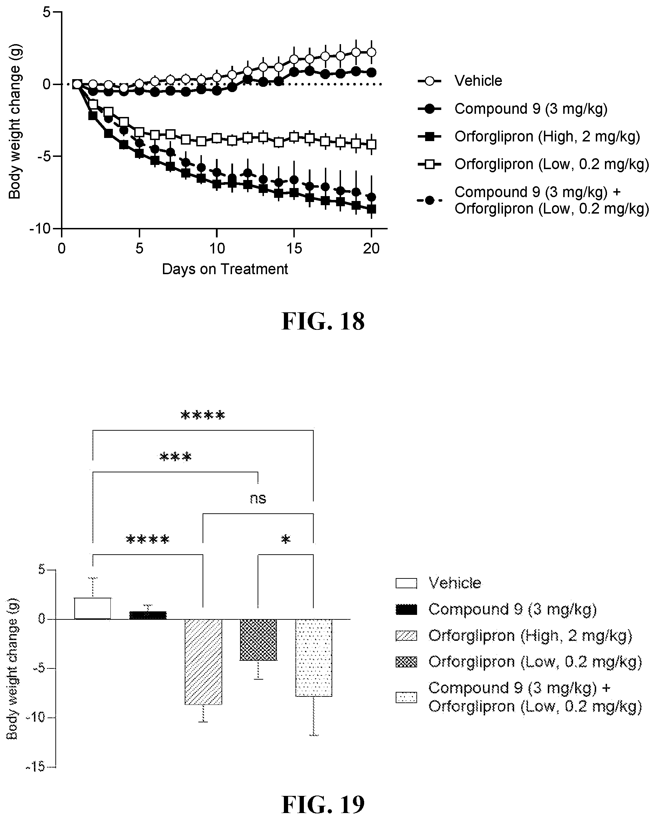

FIG. 18 depicts a graph showing body weight changes from baseline over time in DIO hGLP-1R mice following treatment with Compound 9 alone, orforglipron alone, or orforglipron in combination with Compound 9. Daily change from baseline in body weight (g) shown as mean (SE) with n = 6-7 mice per group. Vehicle, Compound 9 (3 mg/kg), and orforglipron (0.2 mg/kg and 2 mg/kg) were dosed once daily by oral gavage.
FIG. 19 depicts a graph showing body weight change from baseline in DIO hGLP-1R mice following treatment with Compound 9 alone, orforglipron alone, or orforglipron in combination with Compound 9. Change from baseline at 20 day in body weight (g) shown as mean (SD) with n = 6-7 mice per group. Statistical significance determined by ordinary one-way ANOVA with correction for multiple comparisons. *p-value <0.05, ***p-value <0.001, ****p-value <0.0001. ns = not significant.

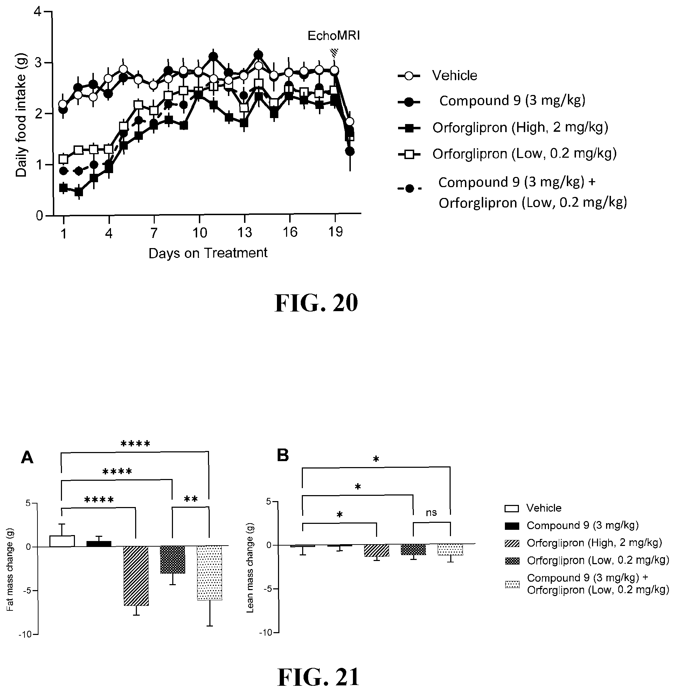

FIG. 20 depicts a graph showing daily food intake over time in DIO hGLP-1R mice following treatment with Compound 9 alone, orforglipron alone, or orforglipron in combination with Compound 9. Daily food intake (g) shown as mean (SE) with n = 6-7 mice per group. Measurement of body mass composition on Day 19 leading to acute food intake decrease is denoted.
FIGs. 21A-B depict two graphs showing body mass composition change from baseline in DIO hGLP-1R mice following treatment with Compound 9 alone, orforglipron alone, or orforglipron in combination with Compound 9. Day 19 change from baseline in fat (FIG. 21A) and lean (FIG. 21B) mass (g) shown as mean (SD) with n = 6-7 mice per group. Statistical significance determined by ordinary one-way ANOVA with correction for multiple comparisons. *p-value <0.05, **p-value <0.01, ***p-value <0.001, ****p-value <0.0001. ns = not significant.

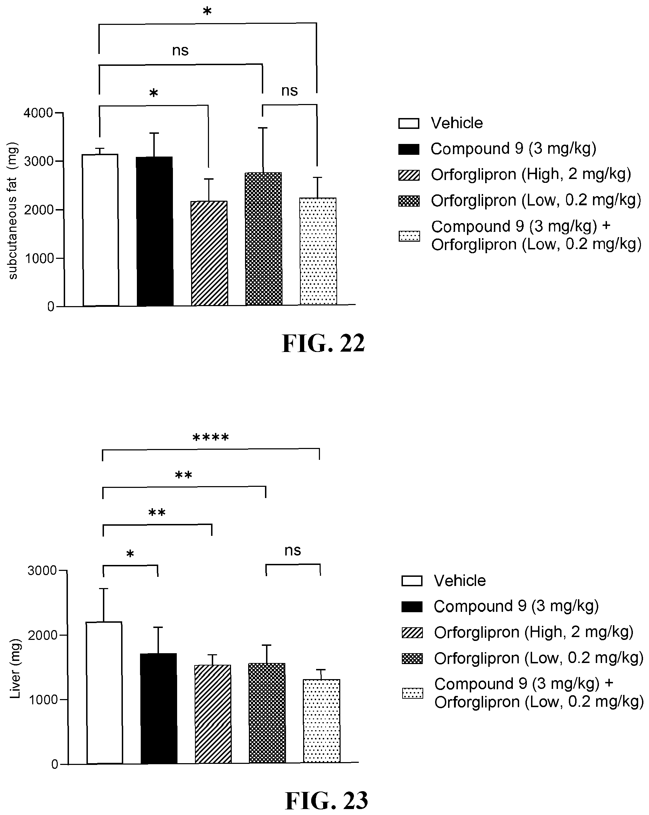

FIG. 22 depicts a graph showing terminal subcutaneous fat in DIO hGLP-1R mice following treatment with Compound 9 alone, orforglipron alone, or orforglipron in combination with Compound 9. Data represents mean (SD) subcutaneous fat (mg) collected at study termination with n = 6-7 mice per group. Statistical significance determined by ordinary one-way ANOVA with correction for multiple comparisons. *p-value <0.05. ns = not significant.
FIG. 23 depicts a graph showing terminal liver weights in DIO hGLP-1R mice following treatment with Compound 9 alone, orforglipron alone, or orforglipron in combination with Compound 9. Data represents mean (SD) liver weight (mg) collected at study termination with n = 6-7 mice per group. Statistical significance determined by ordinary one-way ANOVA with correction for multiple comparisons. *p-value <0.05, **p-value <0.01, **p-value <0.01, ****p-value <0.0001. ns = not significant.

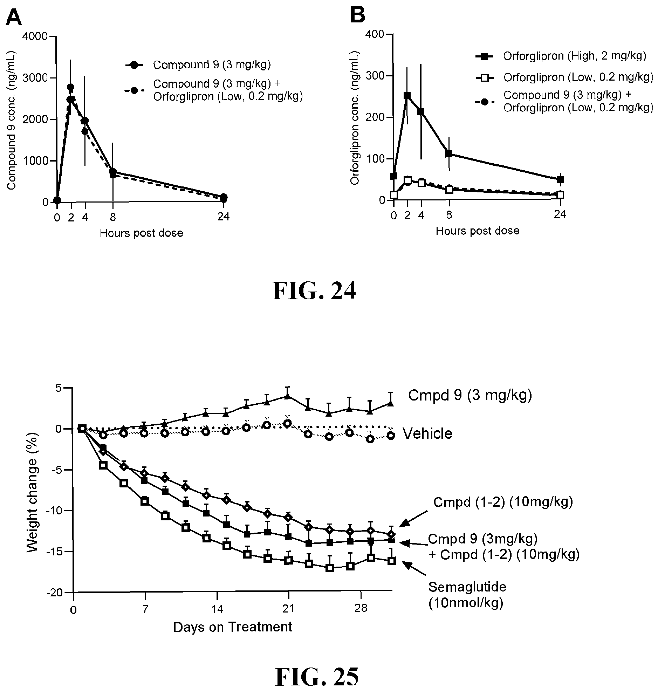

FIGs. 24A-B depict two graphs showing concentration-time profiles of Compound 9 and orforglipron in DIO hGLP-1R mice. Compound concentration shown as mean (SD) over time for Compound 9 (FIG. 24A) or orforglipron (FIG. 24B) alone and in combination. Data represent sparse sampling across each treatment cohort as follows: predose (0-hour post dose), n = 3; 2-hour post dose, n = 3; 4-hour post dose, n = 4; 8-hour post dose, n = 4; 24-hour post dose, n = 7. Nominal doses and times are shown.
FIG. 25 depicts a graph showing weight loss induced by combination treatment of Compound 9 + Compound (1-2) compared to monotherapy in diet induced obese transgenic hGLP1R mice.

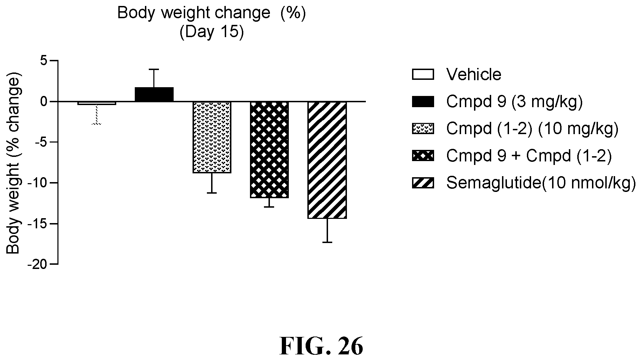

FIG. 26 depicts a graph showing the weight loss induced by combination treatment of Compound 9 + Compound (1-2) compared to monotherapy in diet induced obese transgenic hGLP1R mice. Percent change in body weight after 15 days of treatment.

### DETAILED DESCRIPTION

### Definitions

As used herein, the following definitions shall apply unless otherwise indicated. Further, if any term or symbol used herein is not defined as set forth below, it shall have its ordinary meaning in the art.

"Comprising" is intended to mean that the compositions and methods include the recited elements, but not exclude others. "Consisting essentially of" when used to define compositions and methods, shall mean excluding other elements of any essential significance to the combination. For example, a composition consisting essentially of the elements as defined herein would not exclude other elements that do not materially affect the basic and novel characteristic(s) of the claimed invention. "Consisting of" shall mean excluding more than trace amount of, e.g., other ingredients and substantial method steps recited. Embodiments defined by each of these transition terms are within the scope of this invention.

"Combination therapy" or "combination treatment" (also referred to herein as a combinational thereapy) refers to the use of two or more drugs or agents in treatment, e.g., the use of a compound of formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I"), (I‴*), (I‴), (I*), (I'), (I), (I-P01), (II**), or (II*) as utilized herein together with another agent is a combination therapy.

Administration in "combination" refers to the administration of two agents (e.g., a compound of formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I"), (I‴*), (I‴), (I*), (I'), (I), (I-P01), (II**), or (II*) as utilized herein, and another agent) in any manner in which the pharmacological effects of both manifest in the patient at the same time. Thus, administration in combination does not require that a single pharmaceutical composition, the same dosage form, or even the same route of administration be used for administration of both agents or that the two agents be administered at precisely the same time. The agents can be formulated in two separate pharmaceutically acceptable compositions. Both agents can also be formulated in a single pharmaceutically acceptable composition. A non-limiting example of such a single composition is an oral composition or an oral dosage form. For example, and without limitation, it is contemplated that a compound of formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I"), (I‴*), (I"'), (I*), (I'), (I), (I-P01), (II**) or (II*) can be administered in combination therapy with another agent in accordance with the present invention.

The term "excipient" as used herein means an inert or inactive substance that may be used in the production of a drug or pharmaceutical, such as a tablet containing a compound of the invention as an active ingredient. Various substances may be embraced by the term excipient, including without limitation any substance used as a binder, disintegrant, coating, compression/encapsulation aid, cream or lotion, lubricant, solutions for parenteral administration, materials for chewable tablets, sweetener or flavoring, suspending/gelling agent, or wet granulation agent. Binders include, *e.g*., carbomers, povidone, xanthan gum, etc.; coatings include, *e.g*., cellulose acetate phthalate, ethylcellulose, gellan gum, maltodextrin, enteric coatings, etc.; compression/encapsulation aids include, *e.g*., calcium carbonate, dextrose, fructose dc (dc = "directly compressible"), honey dc, lactose (anhydrate or monohydrate; optionally in combination with aspartame, cellulose, or microcrystalline cellulose), starch dc, sucrose, etc.; disintegrants include, *e.g*., croscarmellose sodium, gellan gum, sodium starch glycolate, etc.; creams or lotions include, *e.g*., maltodextrin, carrageenans, etc.; lubricants include, *e.g*., magnesium stearate, stearic acid, sodium stearyl fumarate, etc.; materials for chewable tablets include, *e.g*., dextrose, fructose dc, lactose (monohydrate, optionally in combination with aspartame or cellulose), etc.; suspending/gelling agents include, *e.g.*, carrageenan, sodium starch glycolate, xanthan gum, etc.; sweeteners include, *e.g.*, aspartame, dextrose, fructose dc, sorbitol, sucrose dc, etc.; and wet granulation agents include, *e.g*., calcium carbonate, maltodextrin, microcrystalline cellulose, etc.

"Patient" refers to mammals and includes humans and non-human mammals. Examples of patients include, but are not limited to mice, rats, hamsters, guinea pigs, pigs, rabbits, cats, dogs, goats, sheep, cows, and humans. In some embodiments, patient refers to a human.

"Patient's response" as used herein refers to magnitude of treatment efficacy (e.g., amount of weight loss, percentage of fat loss, etc.). Improving a "patient's response" to a treatment can include, but are not limited to, increasing the magnitude of efficacy of the treatment (e.g. increasing weight loss, increasing fat loss, etc.). In some embodiments, improving a patient's response to a treatment (e.g. the combinations and methods of the present disclosure) comprises maintaining the efficacy while decreasing side effects.

"Pharmaceutically acceptable" refers to safe and non-toxic, preferably for *in vivo*, more preferably, for human administration.

"Pharmaceutically acceptable salt" refers to a salt that is pharmaceutically acceptable. A compound described herein may be administered as a pharmaceutically acceptable salt.

"Salt" refers to an ionic compound formed between an acid and a base. When the compound provided herein contains an acidic functionality, such salts include, without limitation, alkali metal, alkaline earth metal, and ammonium salts. As used herein, ammonium salts include, salts containing protonated nitrogen bases and alkylated nitrogen bases. Exemplary and non-limiting cations useful in pharmaceutically acceptable salts include Na, K, Rb, Cs, NH₄, Ca, Ba, imidazolium, and ammonium cations based on naturally occurring amino acids. When the compounds utilized herein contain basic functionality, such salts include, without limitation, salts of organic acids, such as carboxylic acids and sulfonic acids, and mineral acids, such as hydrogen halides, sulfuric acid, phosphoric acid, and the likes. Exemplary and non-limiting anions useful in pharmaceutically acceptable salts include oxalate, fumarate, maleate, acetate, propionate, succinate, tartrate, chloride, sulfate, bisulfate, mono-, di-, and tribasic phosphate, mesylate, tosylate, and the likes.

"Therapeutically effective amount" or dose of a compound or a composition refers to that amount of the compound or the composition that results in reduction or inhibition of symptoms or a prolongation of survival in a patient. The results may require multiple doses of the compound or the composition.

"Treatment" or "treating" refers to an approach for obtaining beneficial or desired results including clinical results. For purposes of this invention, beneficial or desired results include, but are not limited to, one or more of the following: decreasing one or more symptoms resulting from the disease or disorder, diminishing the extent of the disease or disorder, stabilizing the disease or disorder (*e.g*., preventing or delaying the worsening of the disease or disorder), delaying the occurrence or recurrence of the disease or disorder, delaying or slowing the progression of the disease or disorder, ameliorating the disease or disorder state, providing a remission (whether partial or total) of the disease or disorder, decreasing the dose of one or more other medications required to treat the disease or disorder, enhancing the effect of another medication used to treat the disease or disorder, delaying the progression of the disease or disorder, increasing the quality of life, and/or prolonging survival of a patient. Also encompassed by "treatment" is a reduction of pathological consequence of the disease or disorder. The methods of the invention contemplate any one or more of these aspects of treatment.

As used herein, "delaying" development of a disease means to defer, hinder, slow, retard, stabilize and/or postpone development of the disease and/or slowing the progression or altering the underlying disease process and/or course once it has developed. This delay can be of varying lengths of time, depending on the history of the disease and/or subject being treated. As is evident to one skilled in the art, a sufficient or significant delay can, in effect, encompass prevention, in that the subject does not develop clinical symptoms associated with the disease. A method that "delays" development of a disease is a method that reduces probability of disease development in a given time frame and/or reduces extent of the disease in a given time frame, when compared to not using the method, including stabilizing one or more symptoms resulting from the disease.

A subject who is "at risk" of developing a disease may or may not have detectable disease, and may or may not have displayed detectable disease prior to the treatment methods described herein. "At risk" denotes that a subject has one or more so-called risk factors, which are measurable parameters that correlate with development of a disease. A subject having one or more of these risk factors has a higher probability of developing the disease than a subject without these risk factor(s). These risk factors include, but are not limited to, age, sex, race, diet, history of previous disease, presence of precursor disease and genetic (*i.e.*, hereditary) considerations. Compounds may, in some embodiments, be administered to a subject (including a human) who is at risk or has a family history of the disease or condition.

"Stereoisomer" or "stereoisomers" refer to compounds that differ in the stereogenicity of the constituent atoms such as, without limitation, in the chirality of one or more stereocenters or related to the cis or trans configuration of a carbon-carbon or carbon-nitrogen double bond. Stereoisomers include enantiomers and diastereomers.

"Alkyl" refers to monovalent saturated aliphatic hydrocarbyl groups having from 1 to 12 carbon atoms, from 1 to 10 carbon atoms, or from 1 to 6 carbon atoms. This term includes, by way of example, linear and branched hydrocarbyl groups such as methyl (CH₃-), ethyl (CH₃CH₂-), *n*-propyl (CH₃CH₂CH₂-), isopropyl ((CH₃)₂CH-), *n*-butyl (CH₃CH₂CH₂CH₂-), isobutyl ((CH₃)₂CHCH₂-), *sec*-butyl ((CH₃)(CH₃CH₂)CH-), *t*-butyl ((CH₃)₃C-), n-pentyl (CH₃CH₂CH₂CH₂CH₂-), and neopentyl ((CH₃)₃CCH₂-). Cₓ alkyl refers to an alkyl group having x number of carbon atoms.

"Alkylene" refers to a divalent saturated aliphatic hydrocarbyl group having from 1 to 12 carbon atoms, for example from 1 to 10 carbon atoms, and from 1 to 6 carbon atoms. This term includes, by way of example, linear and branched hydrocarbyl groups such as methylene (-CH₂-), ethylene (-CH₂CH₂- or -CH(Me)-), propylene (-CH₂CH₂CH₂- or -CH(Me)CH₂-, or - CH(Et)-) and the like.

"Alkenyl" refers to straight or branched monovalent hydrocarbyl groups having from 2 to 6 carbon atoms, for example2 to 4 carbon atoms and having at least 1, orfrom 1 to 2 sites of vinyl (>C=C<) unsaturation. Such groups are exemplified, for example, by vinyl, allyl, and but-3-en-1-yl. Included within this term are the *cis* and *trans* isomers or mixtures of these isomers. Cₓ alkenyl refers to an alkenyl group having x number of carbon atoms.

"Alkynyl" refers to straight or branched monovalent hydrocarbyl groups having from 2 to 6 carbon atoms, for example 2 to 3 carbon atoms and having at least 1 or from 1 to 2 sites of acetylenic (-C≡C-) unsaturation. Examples of such alkynyl groups include acetylenyl (-C≡CH), and propargyl (-CH₂C≡CH). Cₓ alkynyl refers to an alkynyl group having x number of carbon atoms.

"Alkoxy" refers to the group -O-alkyl wherein alkyl is defined herein. Alkoxy includes, by way of example, methoxy, ethoxy, *n*-propoxy, isopropoxy, *n*-butoxy, *t*-butoxy, *sec*-butoxy, and n-pentoxy.

"Aryl" refers to a monovalent aromatic carbocyclic group of from 6 to 14 carbon atoms having a single ring (*e.g.*, phenyl (Ph)) or multiple condensed rings (*e.g.*, naphthyl or anthryl) which condensed rings may or may not be aromatic (*e.g*., 2-benzoxazolinone, 2H-1,4-benzoxazin-3(4H)-one-7-yl, and the like) provided that the point of attachment is at an aromatic carbon atom. Examplary aryl groups include phenyl and naphthyl. A divalent aryl group is referred to herein as "arylene." A divalent phenyl group is referred to herein as "phenylene".

"Cyano" refers to the group -C≡N.

"Cycloalkyl" refers to saturated or unsaturated but nonaromatic cyclic alkyl groups of, for example,from 3 to 10 carbon atoms, from 3 to 8 carbon atoms, orfrom 3 to 6 carbon atoms, having single or multiple cyclic rings including fused, bridged, and spiro ring systems. Cₓ cycloalkyl refers to a cycloalkyl group having x number of ring carbon atoms. Examples of suitable cycloalkyl groups include, for instance, adamantyl, cyclopropyl, cyclobutyl, cyclopentyl, and cyclooctyl. One or more the rings can be aryl, heteroaryl, or heterocyclic provided that the point of attachment is through the non-aromatic, non-heterocyclic ring saturated carbocyclic ring. "Substituted cycloalkyl" refers to a cycloalkyl group having from 1 to 5 or 1 to 3 substituents selected from the group consisting of oxo, thione, alkyl, substituted alkyl, alkenyl, substituted alkenyl, alkynyl, substituted alkynyl, alkoxy, substituted alkoxy, acyl, acylamino, acyloxy, amino, substituted amino, aminocarbonyl, aminothiocarbonyl, aminocarbonylamino, aminothiocarbonylamino, aminocarbonyloxy, aminosulfonyl, aminosulfonyloxy, aminosulfonylamino, amidino, aryl, substituted aryl, aryloxy, substituted aryloxy, arylthio, substituted arylthio, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkylthio, substituted cycloalkylthio, guanidino, substituted guanidino, halo, hydroxy, heteroaryl, substituted heteroaryl, heteroaryloxy, substituted heteroaryloxy, heteroarylthio, substituted heteroarylthio, heterocyclic, substituted heterocyclic, heterocyclyloxy, substituted heterocyclyloxy, heterocyclylthio, substituted heterocyclylthio, nitro, SO₃H, substituted sulfonyl, sulfonyloxy, thioacyl, thiol, alkylthio, and substituted alkylthio, wherein said substituents are defined herein.

"Halo" or "halogen" refers to fluoro, chloro, bromo and iodo and in some embodiments is fluoro or chloro.

"Hydroxy" or "hydroxyl" refers to the group -OH.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring (*e.g*., pyridinyl or furyl) or multiple condensed rings (*e.g*., indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide (N→O), sulfinyl, or sulfonyl moieties. Exemplary heteroaryls include 5 or 6 membered heteroaryls such as pyridinyl, pyrrolyl, thiophenyl, and furanyl. Other exemplary heteroaryls include 9 or 10 membered heteroaryls, such as indolyl, quinolinyl, quinolonyl, isoquinolinyl, and isoquinolonyl. A divalent heteroaryl group is referred to herein as "heteroarylene."

"Heterocycle" or "heterocyclic" or "heterocycloalkyl" or "heterocyclyl" refers to a saturated or partially saturated, but not aromatic, group having from 1 to 10 ring carbon atoms, from 1 to 8 carbon atoms, and from 1 to 6 carbon atoms, from 1 to 4 ring heteroatoms, from 1 to 3 heteroatoms, and from 1 to 2 heteroatoms selected from the group consisting of nitrogen, sulfur, or oxygen. Cₓ heterocycloalkyl refers to a heterocycloalkyl group having x number of ring atoms including the ring heteroatoms. Heterocycle encompasses single ring or multiple condensed rings, including fused bridged and spiro ring systems. In fused ring systems, one or more the rings can be cycloalkyl, aryl or heteroaryl provided that the point of attachment is through the non-aromatic ring. In one embodiment, the nitrogen and/or sulfur atom(s) of the heterocyclic group are optionally oxidized to provide for the N-oxide, sulfinyl, sulfonyl moieties. A divalent heterocyclyl group is referred to herein as "heterocyclene."

Examples of heterocyclyl and heteroaryl include, but are not limited to, azetidinyl, pyrrolyl, imidazolyl, pyrazolyl, pyridyl, pyrazyl, pyrimidyl, pyridazyl, indolizyl, isoindolyl, indolyl, dihydroindolyl, indazolyl, purinyl, quinolizinyl, isoquinolinyl, quinolinyl, phthalazinyl, naphthylpyridinyl, quinoxalinyl, quinazolinyl, cinnolinyl, pteridinyl, carbazolyl, carbolinyl, phenanthridinyl, acridinyl, phenanthrolinyl, isothiazolyl, phenazinyl, isoxazolyl, phenoxazinyl, phenothiazinyl, imidazolidinyl, imidazolinyl, piperidinyl, piperazinyl, indolinyl, phthalimidyl, 1,2,3,4-tetrahydroisoquinolinyl, 4,5,6,7-tetrahydrobenzo[b]thiophenyl, thiazolyl, thiazolidinyl, thiophenyl, benzo[b]thiophenyl, morpholinyl, thiomorpholinyl (also referred to as thiamorpholinyl), 1,1-dioxothiomorpholinyl, piperidinyl, pyrrolidinyl, and tetrahydrofuranyl.

"Oxo" refers to the atom (=O) or (O).

The terms "optional" or "optionally" as used throughout the specification means that the subsequently described event or circumstance may but need not occur, and that the description includes instances where the event or circumstance occurs and instances in which it does not. For example, "the nitrogen atom is optionally oxidized to provide for the N-oxide (N→O) moiety" means that the nitrogen atom may but need not be oxidized, and the description includes situations where the nitrogen atom is not oxidized and situations where the nitrogen atom is oxidized.

The dosage amount of a compound as described herein is determined based on the free acid or free base of a compound, as appropriate.

Provided herein are compositions and the use of such compositions for treating obesity and/or related comorbidities. The methods comprise administering to the patient a glucagon-like peptide-1 receptors (GLP-1R) agonist and a thyroid hormone receptor beta (THRβ) agonist, as described herein.

### GLP-1R Agonists

Suitable GLP-1R agonists that can be used in accordance with the combinations and/or methods described herein include, but are not limited to orforglipron, danuglipron, liraglutide, exenatide, dulaglutide, albiglutide, lixisenatide, tirzepatide, orforglipron, or semaglutide, or a pharmaceutically acceptable salt of the GLP-1R agonist, or a pharmaceutically acceptable solvates of either the GLP-1R agonist or the salt of the GLP-1R agonist. Suitable GLP-1R agonists that can be used in accordance with the combinations and/or methods described herein also include danuglipron tromethamine, SAL-0112, exenatide biobetter, E-2HSA, ECC-5004, dapiglutide, HDM-1002, AZD-9550, BGM-0504, VK2735, AMG-133, HL-08, HZ-010, exenatide SR, DD-01, CT-388, CT-868, CT-996, GL-0034, GMA-105, GMA-106, GLP-06, SCO-094, CagriSema, amycretin (e.g., oral amycretin), ZT-002, DR-10624, DR-10627, retatrutide, NN-6177, NN-9490, NN-9847, NN-9904, efocipegtrutide, GSBR-1290, HB-1085, 4P-004, HM15211, survodutide, froniglutide, efinopegdutide, PF-06954522, YH-25724, YN-012, YN-015, mazdutide, MDR-001, KN-056, MWN-101, emvidutide ALT-801, AP-026, PEG-loxenatide, PEGylated exenatide, ITCA 650, XW-004, XW-014, or efpeglenatide, or a pharmaceutically acceptable salt of the GLP-1R agonist, or a pharmaceutically acceptable solvates of either the GLP-1R agonist or the salt of the GLP-1R agonist.

Suitable GLP-1R agonists that can be used in accordance with the combinations described herein are also described in, e.g., PCT Pub. Nos. WO/2023/049518, WO/2022/040600, WO/2023/076237, WO/2023/164050, PCT Application Nos. PCT/US2022/047687, PCT/US2023/013700, PCT/US2024/022311, or US Provisional Application No. 63/492 895.

In some embodiments, the GLP-1R agonist is a compound of Formula (I-1) 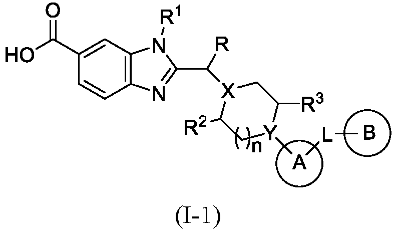 or a pharmaceutically acceptable salt thereof, wherein:
X is N or CH;
Y is N or CR⁴;
n is 0 or 1;
R is hydrogen;
R¹ is -C₁-C₆ alkylene-R⁵;
R² is hydrogen, oxo, or C₁-C₆ alkyl;
R³ is hydrogen, oxo, or C₁-C₆ alkyl and R⁴ is hydrogen, OH, or C₁-C₆ alkyl;
or R³ and R⁴ are taken together with the carbon atoms to which they are attached to form C₃-C₆ cycloalkyl optionally substituted by halo or C₁-C₃ alkyl;
R⁵ is 5-membered heterocyclyl or 5-membered heteroaryl, each of which comprises 1, 2, or 3 heteroatoms independently selected from O, N, and S, wherein at least one heteroatom of R⁵ is S, and further wherein R⁵ is optionally substituted by halo, -O-C₁₋₆ alkyl, C₁₋₆ alkyl, C₁₋₆ alkenyl, or C₁-C₆ haloalkyl;
Ring A is 5- to 12-membered heterocyclene or 5- to 12-membered heteroarylene, each of which is independently optionally substituted by halo, CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein
* represents the point of attachment to ring A and ** represents the point of attachment to ring B;
when L is *-O-C₁-C₆ alkylene-**, the C₁-C₆ alkylene of L is optionally substituted by R^{L}, wherein each R^{L} is independently C₁-C₆ alkyl or halo, or two R^{L} are taken together with the carbon atom or atoms to which they are attached to form C₃-C₆ cycloalkyl or 3- to 6-membered heterocyclyl; and
when L is C₁-C₆ alkylene, the C₁-C₆ alkylene is optionally substituted by R^{L1} wherein each R^{L1} is independently halo, OH, oxo, or C₁-C₆ alkyl, or two R^{L1} are taken together with the carbon atom or atoms to which they are attached to form C₃-C₆ cycloalkyl or 3- to 6-membered heterocyclyl;
R⁶ is hydrogen or C₁-C₆ alkyl; and
Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, C₁-C₆ haloalkyl, -COCH₃, -CONH₂, -S(O)₂CH₃, and phenyl.

In some embodiments, the GLP-1R agonist is a compound of Formula (I-1a) 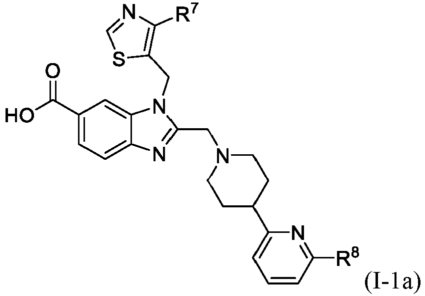 wherein R⁷ is hydrogen, chloro, bromo, fluoro, methyl, or vinyl; and
R⁸ is

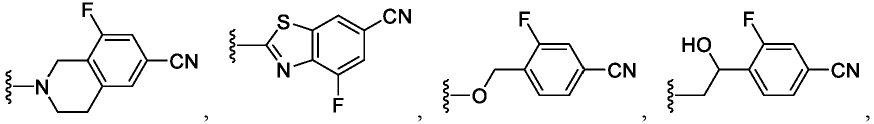

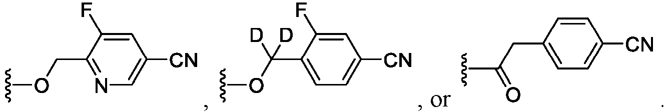

In some embodiments, the GLP-1R agonist is a compound of Formula (I-2) 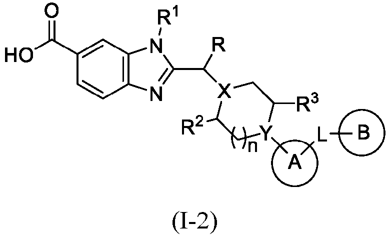 or a stereoisomer, tautomer, or a pharmaceutically acceptable salt of any of the foregoing,
wherein:
X is N or CH;
Y is N or CR⁴, wherein R⁴ is hydrogen, OH or C₁-C₆ alkyl;
n is 0 or 1;
R is hydrogen;
R¹ is -C₁-C₆ alkylene-R⁵, wherein R⁵ is 3- to 6-membered heterocyclyl or 5- to 6-membered heteroaryl, each of which is independently optionally substituted by C₁-C₆ alkyl, or
   R¹ is taken together with R and the intervening atoms to form a Ring C, wherein Ring C is a 5- to 7-membered heterocyclyl optionally substituted by C₁-C₆ alkyl;
R² and R³ are independently hydrogen, oxo, or C₁-C₆ alkyl, wherein when Y is CR⁴, R³ and
R⁴ are optionally taken together with the carbon atoms to which they are attached to form C₃-C₆ cycloalkyl;
Ring A is 5- to 12-membered heterocyclene or 5- to 12-membered heteroarylene, each of which is independently optionally substituted by halo, CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein
   * represents the point of attachment to ring A and ** represents the point of attachment to ring B,
   when L is *-O-C₁-C₆ alkylene-**, the C₁-C₆ alkylene is optionally substituted by R^{L},
wherein:
   each R^{L} is independently C₁-C₆ alkyl or halo, or
      two R^{L} are taken together with the carbon atom or atoms to which they are attached to form C₃-C₆ cycloalkyl or 3- to 6-membered heterocyclyl,
   when L is C₁-C₆ alkylene, the C₁-C₆ alkylene is optionally substituted by R^{L1}, wherein:
      each R^{L1} is independently halo, OH, or C₁-C₆ alkyl; or
      two R^{L1} are taken together with the carbon atom or atoms to which they are attached to form C₃-C₆ cycloalkyl or 3- to 6-membered heterocyclyl; and
   R⁶ is hydrogen or C₁-C₆ alkyl; and
      Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, C₁-C₆ haloalkyl, -COCH₃, -CONH₂, -S(O)₂CH₃, and phenyl,
      with the proviso that
   when R¹ is -C₁-C₆ alkylene-R⁵, wherein R⁵ is 3- to 6-membered heterocyclyl or 3- to 6-membered heteroaryl, each of which is optionally substituted by C₁-C₆ alkyl, Y is N or CH, n is 1, R² and R³ are independently hydrogen or C₁-C₆ alkyl, ring A is 6-membered heteroaryl optionally substituted one or two substituents each independently selected from the group consisting of F, Cl and CN, and L is *-OCH₂-**, then ring B is not phenyl optionally substituted by one or two substituents each independently selected from the group consisting of halo, CN, and C₁-C₆ alkyl;
   when R¹ is -C₁-C₆ alkylene-R⁵, wherein R⁵ is 3- to 6-membered heterocyclyl or 3- to 6-membered heteroaryl, each of which is optionally substituted by C₁-C₆ alkyl, Y is N or CH, n is 1, R² and R³ are independently hydrogen or C₁-C₆ alkyl, ring A is 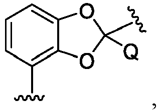 wherein Q is H or CH₃, and L is a bond, then ring B is neither phenyl or pyridinyl, each of which is optionally substituted by one or two substituents each independently selected from the group consisting of halo, CN, and C₁-C₆ alkyl; and
   when R¹ is -C₁-C₆ alkylene-R⁵, wherein R⁵ is 4-membered heterocyclyl or 5-membered heteroaryl, each of which is optionally substituted by C₁-C₆ alkyl, X is N, Y is N or CH, n is 1, and R² and R³ are independently hydrogen or oxo, then ring B is not 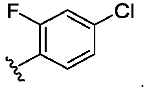 In some such embodiments of Formula (I-2), Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, -COCH₃, -CONH₂, -S(O)₂CH₃, and phenyl. In some such embodiments of Formula (I-2), when L is *-O-C₁-C₆ alkylene-**, the C₁-C₆ alkylene is optionally substituted by R^{L}, wherein each R^{L} is independently C₁-C₆ alkyl, or two R^{L} are taken together with the carbon atom or atoms to which they are attached to form C₃-C₆ cycloalkyl or 3- to 6-membered heterocyclyl. In some such embodiments of Formula (I-2), when L is C₁-C₆ alkylene, the C₁-C₆ alkylene is unsubstituted.

In some embodiments, the GLP-1R agonist is a compound of Formula (I-3) 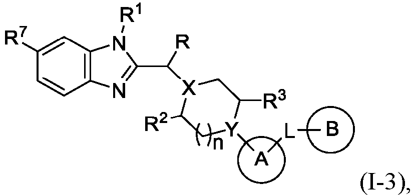 or a pharmaceutically acceptable salt thereof, wherein:
X is N or CH;
Y is N or CR⁴;
n is 0 or 1;
R is hydrogen;
R¹ is -C₁-C₆ alkylene-R⁵;
R² is hydrogen, oxo, or C₁-C₆ alkyl;
R³ is hydrogen, oxo, or C₁-C₆ alkyl and R⁴ is hydrogen, OH or C₁-C₆ alkyl,
or R³ and R⁴ are taken together with the carbon atoms to which they are attached to form C₃₋C₆ cycloalkyl optionally substituted by halo or C₁-C₃ alkyl;
R⁵ is 3- to 6-membered heterocyclyl or 5- to 6-membered heteroaryl, wherein the 3- to 6-membered heterocyclyl or 5- to 6-membered heteroaryl of R⁵ is independently optionally substituted by halo, C₁-C₆ alkyl, C₁-C₆ alkoxy, C₁-C₆ alkenyl, or C₁-C₆ haloalkyl;
R⁷ is selected from the group consisting of 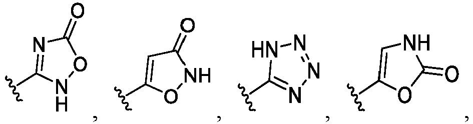 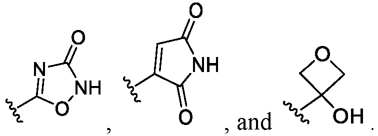
or R⁷ is -C(O)NH-R⁸, wherein R⁸ is hydrogen, -OH, -S(O)₂-C₁-C₆ alkyl, or -C₁-C₆ alkyl optionally substituted by halo;
Ring A is 5- to 12-membered heterocyclene, 5- to 12-membered heteroarylene, or C₆-C₁₄ arylene, each of which is independently optionally substituted by halo, oxo, -CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein:
   * represents the point of attachment to ring A and ** represents the point of attachment to ring B;
   when L is *-O-C₁-C₆ alkylene-**, the C₁-C₆ alkylene is optionally substituted by R^{L}, wherein each R^{L} is independently C₁-C₆ alkyl or halo, or two R^{L} are taken together with the carbon atom or atoms to which they are attached to form C₃-C₆ cycloalkyl or 3- to 6-membered heterocyclyl; and
   when L is C₁-C₆ alkylene, the C₁-C₆ alkylene is optionally substituted by R^{L1} wherein each R^{L1} is independently halo, OH, oxo, or C₁-C₆ alkyl, or two R^{L1} are taken together with the carbon atom or atoms to which they are attached to form C₃-C₆ cycloalkyl or 3- to 6-membered heterocyclyl;
   R⁶ is hydrogen or C₁-C₆ alkyl; and
   Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, C₁-C₆ haloalkyl, -C(O)CH₃, -C(O)NH₂, -S(O)₂CH₃, cyclopropyl, and phenyl,
   with the proviso that:
      when R⁷ is -C(O)NH-R⁸, R¹ is 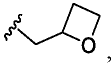 X is N, Y is CH, n is 1, R² and R³ are each hydrogen,
      ring A is 6-membered heteroaryl, and L is *-OCH₂-**, then ring B is not

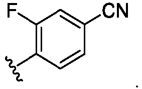

In some embodiments, the GLP-1R agonist is a compound of Formula (I-4) 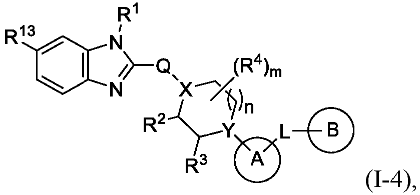 or a pharmaceutically acceptable salt, wherein:
R¹³ is -C(O)OH or 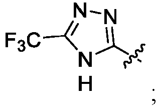
X is N or CR^{x}, wherein R^{x} is hydrogen, OH or C₁-C₆ alkyl;
Y is N or CR^{y}, wherein R^{y} is hydrogen, OH or C₁-C₆ alkyl;
n is 0 or 1;
Q is selected from the group consisting of -C(R⁷)(R⁸)-, -O-, -N(R⁹)-, and -S-, wherein
R⁷ and R⁸ are independently hydrogen, halogen, or C₁-C₆ alkyl; and
R⁹ is hydrogen or C₁-C₆ alkyl;
R¹ is optionally substituted -C₁-C₆ alkyl or -C₁-C₆ alkylene-R⁵, wherein R⁵ is C₃-C₆ cycloalkyl, 3- to 6-membered heterocyclyl, or 5- to 6-membered heteroaryl, each of which is independently optionally substituted by one to three substituents independently selected from the group consisting of halo, oxo, CN, C₁-C₆ alkyl, C₃-C₆ cycloalkyl, and -OC₁-C₆ alkyl, wherein each C₁-C₆ alkyl, C₃-C₆ cycloalkyl, and -OC₁-C₆ alkyl substituent is independently optionally substituted by halo or CN;
R² and R³ are taken together with the carbon atoms to which they are attached to form an optionally substituted C₃-C₄ cycloalkyl ring; or
R² and R^{x}, when present, are taken together with the carbon atoms to which they are attached to form an optionally substituted C₃-C₅ cycloalkyl ring, and R³ is hydrogen, oxo, or C₁-C₆ alkyl;
m is 0, 1, 2, or 3;
R⁴ is oxo or C₁-C₆ alkyl;
Ring A is C₆-C₁₄ arylene, 5- to 12-membered heterocyclene or 5- to 12-membered heteroarylene, each of which is independently optionally substituted by halo, OH, CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein * represents the point of attachment to ring A and ** represents the point of attachment to ring B, and wherein:
   when L is C₁-C₆ alkylene, *-O-C₁-C₆ alkylene-**, *-C₁-C₆ alkylene-O-**, or *-NR⁶-C₁-C₆ alkylene-**, L is optionally substituted by one to three R^{L} substituents, wherein each R^{L} is independently halo, OH, or C₁-C₆ alkyl; or two R^{L} are taken together with the carbon atom or atoms to which they are attached to form C₃-C₆ cycloalkyl or 3- to 6-membered heterocyclyl;
R⁶, when present, is hydrogen or C₁-C₆ alkyl; and
Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl optionally substituted by halo or CN, -OC₁-C₆ alkyl optionally substituted by halo or CN, - COCH₃, -CONH₂, -S(O)₂CH₃, and phenyl optionally substituted by halo or CN.

In some embodiments, the GLP-1R agonist is a compound of Formula (I**): 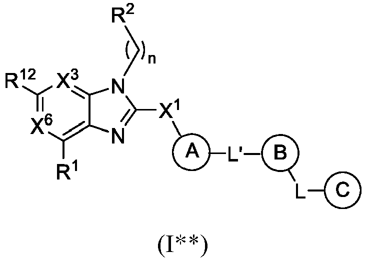 or a pharmaceutically acceptable salt thereof; wherein:
X³ is CR⁶ or N;
X⁶ is CR⁴ or N;
R¹ is -C₁₋₆ haloalkyl, halogen, -O-X⁴, or -NR⁸R⁹, or R¹ and R⁴, together with the atoms to which they are attached, combine to form a 5- or 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃,-(CH₂-CH(-OCH₃)-CH₂-O)₁₋₅-CH₃, C₃₋₁₀ cycloalkyl, or C₆₋₁₀ aryl, wherein the alkyl, heteroalkyl, alkylene, or haloalkyl group is optionally substituted with one or more deuterium, C₁₋₆ alkoxy, hydroxyl, -CN, or oxo, and the cycloalkyl, heterocyclyl, or aryl group is optionally substituted with one or more halogen, C₁₋₆ alkoxy, or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocycyl; wherein the C₁₋₆ alkyl is optionally substituted by one or more oxo;
n is 0, 1, 2, 3, 4, 5, or 6;
R² is:
hydrogen;
C₁₋₆ alkyl optionally substituted with deuterium;
C₁₋₆ haloalkyl;
-(O)-C₁₋₆ alkyl;
-CN;
a C₃₋₁₀ cycloalkyl optionally substituted with one or more -CN, C₁₋₆ haloalkyl, or C₁₋₆ alkyl optionally substituted with one or more -CN;
a 4 or 5-membered heterocyclyl comprising at least one oxygen or at least one sulfur atom, wherein the 4- or 5-membered heterocyclyl is optionally substituted with one or more oxo, 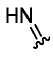 , or C₁₋₆ alkyl;
a 5-membered heteroaryl, comprising 1 or 2 heteroatoms independently selected from N, and S, wherein at least one heteroatom of R⁵ is S; or
R² and R⁷, together with the atoms to which they are attached, combine to form a 5- or 6-membered heterocyclyl;
R⁴ is halogen, hydrogen, -C(O)OH, or -O-R⁸,
   wherein R⁸ and R¹, together with the atoms to which they are attached, combine to form a 6-membered heterocyclyl;
R¹² is hydrogen, -C(O)OH,-C(O)NR^{N12}R^{N12'}, -C(O)NR¹² S(O)₂R^{12'}, -(C₂₋₆ alkynylene)-C(O)OH, -(C₁₋₆ alkylene)-C(O)OH, -NR^{N12}-(C₁₋₆ alkylene)-C(O)OH, 5-10 membered heteroaryl or 5- to 10-membered heterocyclyl optionally substituted with one or more oxo, C₁₋₆ alkyl or C₁₋₆ haloalkyl;
R^{N12} and R^{N12'} independently are H or C₁₋₆ alkyl;
X¹ is 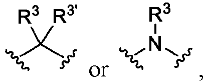
wherein R³ and R^{3'} independently are H, D or C₁₋₆ alkyl, wherein the C₁₋₆ alkyl is optionally substituted with detuerium;
Ring A is 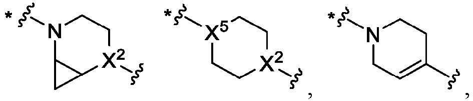 phenylene optionally substituted with one or more halo or C₁₋₆ alkyl, or 6-membered heteroarylene optionally substituted with one or more halo or C₁₋₆ alkyl;
wherein * indicates attachment to X¹,
   X⁵ is CR³ or N, and
   X² is CR³ or N;
L' is a bond or -O-;
Ring B is a C₆₋₁₀ arylene, a 5-10 membered heteroarylene, or a 3-10 membered heterocycylene, wherein the C₆₋₁₀ arylene, 5-10 membered heteroarylene, or 3-10 membered heterocyclene is optionally substituted with one or more oxo, C₁₋₆ alkyl, C₁₋₆ alkoxy, or halogen;
L is a bond, *-(C₁₋₆ alkylene)-, *-NR^{L}-(C₁₋₆ alkylene)-, *-O-(C₁₋₆ alkylene)-, or *-(C₁₋₆ alkylene)-O-, wherein * indicates attachment to Ring B and the C₁₋₆ alkylene is optionally substituted with deuterium;
   wherein R^{L} is H or C₁₋₆ alkyl; and
Ring C is:
   a 6-membered aryl optionally substituted with one or more C₃₋₁₀ cycloalkyl, C₁₋₆ alkyl, C₁₋₆ haloalkyl, 3-10 membered heterocyclyl, halogen, C₁₋₆ alkoxy, C₁₋₆ haloalkoxy, -CN, C₃₋₁₀ cycloalkyl, or -C(O)NR'₂;
      wherein R' is H or C₁₋₆ alkyl
   a 6-membered heteroaryl comprising a nitrogen atom optionally substituted with one or more halogen, -CN, C₁₋₆ haloalkyl, -O-C₁₋₆ alkyl, C₃₋₁₀ cycloalkyl, -C(=O)-(C₃₋₁₀ cycloalkyl), 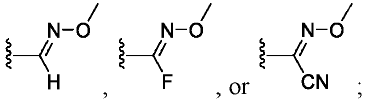 or
   a bicylic 9- or 10-membered heteroaryl or heterocyclyl optionally substituted with one or more C₁₋₆ alkyl, halogen, -CN, or oxo.

In some embodiments, the GLP-1R agonist is a compound of Formula (I**): 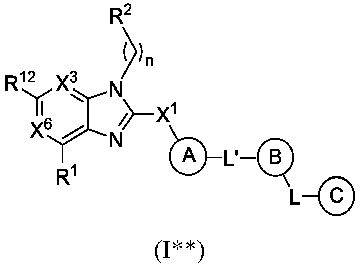 or a pharmaceutically acceptable salt thereof; wherein:
X³ is CR⁶ or N;
X⁶ is CR⁴ or N;
R¹ is -C₁₋₆ haloalkyl, halogen, -O-X⁴, or -NR⁸R⁹, or R¹ and R⁴, together with the atoms to which they are attached, combine to form a 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, C₁₋₆ alkoxy, hydroxyl, -CN, or oxo, and the cycloalkyl or heterocyclyl group is optionally substituted with one or more halogen or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocyclyl; wherein the C₁₋₆ alkyl is optionally substituted by one or more oxo;
n is 0, 1, 2, 3, 4, 5, or 6;
R² is:
   hydrogen;
   C₁₋₆ alkyl optionally substituted with deuterium;
   C₁₋₆ haloalkyl;
   -(O)-C₁₋₆ alkyl;
   -CN;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 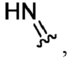 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a 5- or 6-membered heterocyclyl;
R⁴ is hydrogen, -C(O)OH, or -O-R⁸
   wherein R⁸ and R¹, together with the atoms to which they are attached, combine to form a 6-membered heterocyclyl;
R¹² is hydrogen, -C(O)OH or 5-10 membered heteroaryl optionally substituted with one or more oxo, C₁₋₆ alkyl or C₁₋₆ haloalkyl;
X¹ is 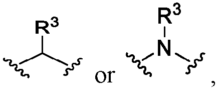
wherein R³ is H or C₁₋₆ alkyl;
Ring A is 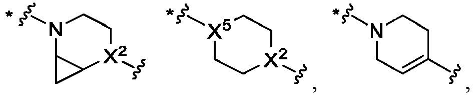 phenylene optionally substituted with one or more halo or C₁₋₆ alkyl, or 6-membered heteroaryl optionally substituted with one or more halo or C₁₋₆ alkyl;
wherein * indicates attachment to X¹,
   X⁵ is CR³ or N, and
   X² is CR³ or N;
L' is a bond or -O-;
Ring B is a C₆₋₁₀ arylene, a 5-10 membered heteroarylene, or a 3-10 membered heterocyclene, wherein the C₆₋₁₀ arylene, 5-10 membered heteroarylene, or 3-10 membered heterocyclene is optionally substituted with one or more oxo, C₁₋₆ alkyl, C₁₋₆ alkoxy, or halogen;
L is a bond, *-(C₁₋₆ alkylene)-, *-NR^{L}-(C₁₋₆ alkylene)-, *-O-(C₁₋₆ alkylene)-, or *-(C₁₋₆ alkylene)-O-, wherein * indicates attachment to Ring B and the C₁₋₆ alkylene is optionally substituted with deuterium;
   wherein R^{L} is H or C₁₋₆ alkyl; and
Ring C is:
   a 6-membered aryl optionally substituted with one or more C₁₋₆ alkyl, C₁₋₆ haloalkyl, 3-10 membered heterocyclyl, halogen, C₁₋₆ alkoxy, C₁₋₆ haloalkoxy, -CN, C₃₋₁₀ cycloalkyl, or -C(O)NR'₂;
      wherein R' is H or C₁₋₆ alkyl
   a 6-membered heteroaryl comprising a nitrogen atom optionally substituted with one or more halogen, -CN, C₁₋₆ haloalkyl, -O-C₁₋₆ alkyl, C₃₋₁₀ cycloalkyl, -C(=O)-(C₃₋₁₀ cycloalkyl), 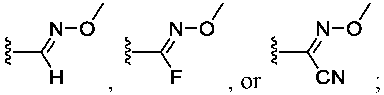 or a bicylic 9- or 10-membered heteroaryl optionally substituted with one or more C₁₋₆ alkyl, halogen, or oxo.

In some embodiments, the GLP-1R agonist is a compound of Formula I*: 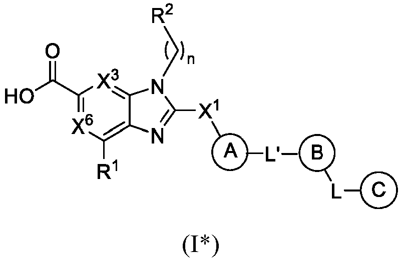 or a pharmaceutically acceptable salt thereof; wherein:
X³ is CR⁶ or N;
X⁶ is CR⁴ or N;
R¹ is -C₁₋₆ haloalkyl, -O-X⁴, or -NR⁸R⁹, or R¹ and R⁴, together with the atoms to which they are attached, combine to form a 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, hydroxyl, -CN, or oxo, and the cycloalkyl or heterocyclyl group is optionally substituted with one or more halogen or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocycyl; wherein the C₁₋₆ alkyl is optionally substituted by one or more oxo;
n is 0, 1, 2, 3, 4, 5, or 6;
R² is:
   hydrogen;
   C₁₋₆ alkyl;
   C₁₋₆ haloalkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 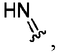 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a 5- or 6-membered heterocyclyl;
   R⁴ is hydrogen or -O-R⁸
      wherein R⁸ and R¹, together with the atoms to which they are attached, combine to form a 6-membered heterocyclyl;
   X¹ is 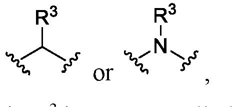
   wherein R³ is H or C₁₋₆ alkyl;
   Ring A is 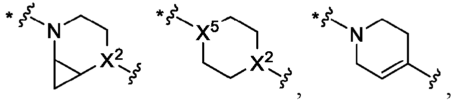 phenylene optionally substituted with one or more halo or C₁₋₆ alkyl, or 6-membered heteroarylene optionally substituted with one or more halo or C₁₋₆ alkyl;
   wherein * indicates attachment to X¹,
      X⁵ is CR³ or N, and
      X² is CR³ or N;
   L' is a bond or -O-;
   Ring B is a C₆₋₁₀ arylene, a 6-10 membered heteroarylene, or a 3-10 membered heterocycylene, wherein the C₆₋₁₀ arylene, 6-10 heteroarylene, or 3-10 membered heterocyclene is optionally substituted with one or more C₁₋₆ alkyl, C₁₋₆ alkoxy, or halogen;
   L is a bond, *-CH₂-, *-O-(C₁₋₆ alkylene)-, or *-(C₁₋₆ alkylene)-O-, wherein * indicates attachment to Ring B and the C₁₋₆ alkylene is optionally substituted with deuterium; and
   Ring C is:
      a 6-membered aryl optionally substituted with one or more C₁₋₆ alkyl, 3-10 membered heterocyclyl, halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 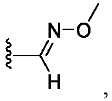 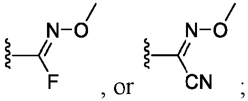 or
      a bicylic 9- or 10-membered heteroaryl comprising two nitrogen atoms optionally substituted with one or more C₁₋₆ alkyl, or oxo.

In some embodiments, the compound of Formula (I*) is of Formula (I'): 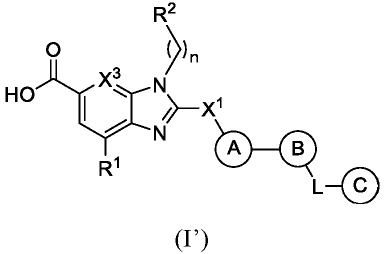 or a pharmaceutically acceptable salt thereof; wherein:
X³ is CH or N;
R¹ is -C₁₋₆ haloalkyl or -O-X⁴;
wherein X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more -CN, and the cycloalkyl or heterocyclyl group is optionally substituted with one or more halogen or -CN;
n is 0, 1, 2, 3, 4, 5, or 6;
R² is hydrogen;
-(O)-C₁₋₆ alkyl;
a C₃₋₁₀ cycloalkyl optionally substituted with one or more -CN, C₁₋₆ haloalkyl, or C₁₋₆ alkyl optionally substituted with one or more -CN;
a 4 or 5-membered heterocyclyl comprising at least one oxygen or at least one sulfur atom, wherein the 4- or 5-membered heterocyclyl is optionally substituted with one or more oxo, 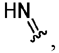 or C₁₋₆ alkyl; or
a 5-membered heteroaryl, comprising 1 or 2 heteroatoms independently selected from N, and S, wherein at least one heteroatom of R⁵ is S;
X¹ is 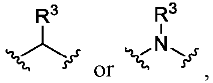
wherein R³ is H or C₁₋₆ alkyl;
Ring A is 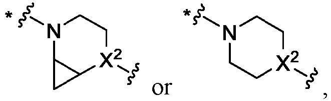
wherein * indicates attachment to X¹, and
wherein X² is CH or N;
Ring B is a 6-10 membered heteroarylene, or a 3-10 membered heterocycylene optionally substituted with one or more C₁₋₆ alkyl;
L is a bond or *-O-(C₁₋₆ alkylene)-, wherein * indicates attachment to Ring B;
Ring C is:
   a 6-membered aryl optionally substituted with one or more halogen, -OCH₃, -CN, or C₃₋₁₀ cycloalkyl;
   a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 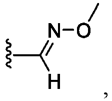 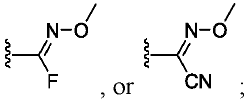
   a bicylic 9- or 10-membered heteroaryl comprising two nitrogen atoms optionally substituted with one or more C₁₋₆ alkyl, or oxo.

In some embodiments, the GLP-1R agonist is a compound of Formula I" : 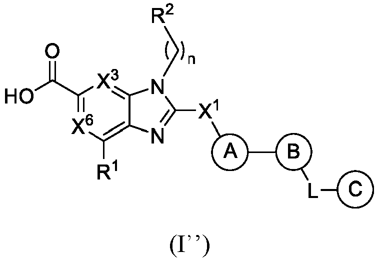 or a pharmaceutically acceptable salt thereof; wherein:
X³ is CR⁶ or N;
X⁶ is CR⁴ or N;
R¹ is -C₁₋₆ haloalkyl, -O-X⁴, or -NR⁸R⁹, or R¹ and R⁴, together with the atoms to which they are attached, combine to form a 6-membered heterocyclyl;
wherein X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, hydroxyl, -CN, or oxo, and the cycloalkyl or heterocyclyl group is optionally substituted with one or more halogen or -CN;
R⁶ is hydrogen, halogen, or -O-R⁷;
   wherein R⁷ and R², together with the atoms to which they are attached, combine to form a 5- or 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocycyl; wherein the C₁₋₆ alkyl is optionally substituted by one or more oxo;
n is 0, 1, 2, 3, 4, 5, or 6;
R² is:
   hydrogen;
   C₁₋₆ alkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 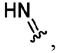 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a C₂₋₉ heterocyclyl;
   R⁴ is hydrogen or -O-R⁸
      wherein R⁸ and R¹, together with the atoms to which they are attached, combine to form a 6-membered heterocyclyl;;
   X¹ is 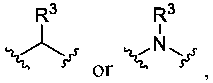
   wherein R³ is H or C₁₋₆ alkyl;
   Ring A is 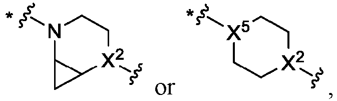
   wherein * indicates attachment to X¹,
      X⁵ is CH or N, and
      X² is CH or N;
   Ring B is a 6-10 membered heteroarylene, or a 3-10 membered heterocycylene optionally substituted with one or more C₁₋₆ alkyl;
   L is a bond or *-O-(C₁₋₆ alkylene)-, wherein * indicates attachment to Ring B; and
   Ring C is:
      a 6-membered aryl optionally substituted with one or more halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 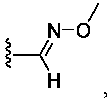 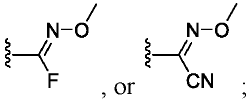 or
      a bicylic 9- or 10-membered heteroaryl comprising two nitrogen atoms optionally substituted with one or more C₁₋₆ alkyl, or oxo.

In some embodiments, the GLP-1R agonist is a compound of Formula (I‴*): 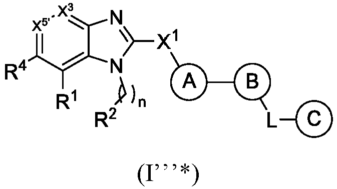 or a pharmaceutically acceptable salt thereof; wherein:
X³ is CR^{X3} or N;
   wherein R^{X3} is H, halogen, C₁₋₆ alkyl, C₁₋₆ haloalkoxy, -O-(CH₂CH₂-O)₁₋₅-CH₃, or C₁₋₆ alkoxy;
X^{5'} is CR^{5'}
R¹ is -C₁₋₆ haloalkyl or -O-X⁴, or R¹ and R⁴, together with the atoms to which they are attached, combine to form a 6-membered heterocyclyl;
wherein X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, halogen, or -CN, and the cycloalkyl or heterocyclyl group is optionally substituted with one or more halogen or -CN;
n is 0, 1, 2, 3, 4, 5, or 6;
R² is:
   hydrogen;
   halogen;
   branched C₃₋₆ alkyl;
   -(O)-C₁₋₆ alkyl;
   C₃₋₁₀ cycloalkyl optionally substituted with one or more -CN, C₁₋₆ haloalkyl, or C₁₋₆ alkyl optionally substituted with one or more -CN;
   4 or 5-membered heterocyclyl comprising at least one oxygen or at least one sulfur atom, wherein the 4- or 5-membered heterocyclyl is optionally substituted with one or more oxo, 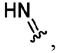 or C₁₋₆ alkyl; or
   5-membered heteroaryl, comprising 1 or 2 heteroatoms independently selected from N, and S, wherein at least one heteroatom of R⁵ is S; or
   R² and R¹, together with the atoms to which they are attached, combine to form a 6-membered heterocyclyl;
   R^{5'} is hydrogen or -C(O)-OH;
   R⁴ is hydrogen, halogen, -C(O)-OH, or -O-R⁸,
      wherein R⁸ and R¹, together with the atoms to which they are attached, combine to form a 6-membered heterocyclyl;
   X¹ is 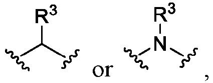
   wherein R³ is H or C₁₋₆ alkyl;
   Ring A is 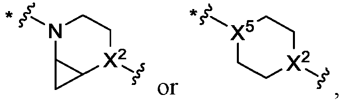
   wherein * indicates attachment to X¹,
   wherein X⁵ is CH or N, and
      X² is CH or N;
   Ring B is a 6-10 membered heteroarylene, or a 3-10 membered heterocycylene optionally substituted with one or more C₁₋₆ alkyl;
   L is a bond or *-O-(C₁₋₆ alkylene)-, wherein * indicates attachment to Ring B;
   Ring C is:
      a 6-membered aryl optionally substituted with one or more halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 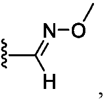 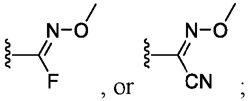 or
      a bicylic 9- or 10-membered heteroaryl comprising two nitrogen atoms optionally substituted with one or more C₁₋₆ alkyl, or oxo.

In some embodiments, the GLP-1R agonist is a compound of Formula (I‴) 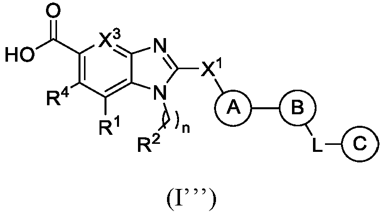 or a pharmaceutically acceptable salt thereof; wherein:
X³ is CR^{X3} or N;
   wherein R^{X3} is H, halogen, C₁₋₆ alkyl, C₁₋₆ haloalkoxy, or C₁₋₆ alkoxy;
R¹ is -C₁₋₆ haloalkyl or -O-X⁴, or R¹ and R⁴, together with the atoms to which they are attached, combine to form a 6-membered heterocyclyl;
wherein X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, -CN, and the cycloalkyl or heterocyclyl group is optionally substituted with one or more halogen or -CN;
n is 0, 1, 2, 3, 4, 5, or 6;
R² is:
   hydrogen;
   halogen;
   C₃₋₆ alkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 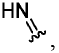 or C₁₋₆ alkyl;
   a 5-membered heteroaryl, comprising 1 or 2 heteroatoms independently selected from N, and S, wherein at least one heteroatom of R⁵ is S; or
   R² and R¹, together with the atoms to which they are attached, combine to form a 6-membered heterocyclyl;
   R⁴ is hydrogen or -O-R⁸
      wherein R⁸ and R¹, together with the atoms to which they are attached, combine to form a 6-membered heterocyclyl;;
   X¹ is 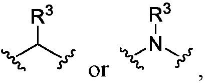
   wherein R³ is H or C₁₋₆ alkyl;
   Ring A is 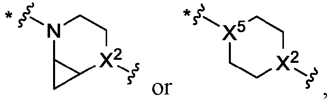
   wherein * indicates attachment to X¹,
   wherein X⁵ is CH or N, and
      X² is CH or N;
   Ring B is a 6-10 membered heteroarylene, or a 3-10 membered heterocycylene optionally substituted with one or more C₁₋₆ alkyl;
   L is a bond or *-O-(C₁₋₆ alkylene)-, wherein * indicates attachment to Ring B;
   Ring C is:
      a 6-membered aryl optionally substituted with one or more halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 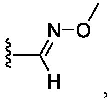 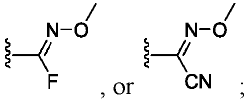
      a bicylic 9- or 10-membered heteroaryl comprising two nitrogen atoms optionally substituted with one or more C₁₋₆ alkyl, or oxo.

In some embodiments of Formula (I‴), or a pharmaceutically acceptable salt thereof,
X³ is CH or N;
R¹ is -C₁₋₆ haloalkyl or -O-X⁴, or R¹ and R⁴, together with the atoms to which they are attached, combine to form a 6-membered heterocyclyl;
wherein X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, -CN, and the cycloalkyl or heterocyclyl group is optionally substituted with one or more halogen or -CN;
n is 0, 1, 2, 3, 4, 5, or 6;
R² is:
   hydrogen;
   C₃₋₆ alkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 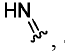 or C₁₋₆ alkyl;
   a 5-membered heteroaryl, comprising 1 or 2 heteroatoms independently selected from N, and S, wherein at least one heteroatom of R⁵ is S; or
   R² and R¹, together with the atoms to which they are attached, combine to form a 6-membered heterocyclyl;
   R⁴ is hydrogen or -O-R⁸
      wherein R⁸ and R¹, together with the atoms to which they are attached, combine to form a 6-membered heterocyclyl;;
   X¹ is 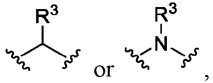
   wherein R³ is H or C₁₋₆ alkyl;
   Ring A is 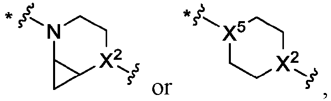
   wherein * indicates attachment to X¹,
   wherein X⁵ is CH or N, and
      X² is CH or N;
   Ring B is a 6-10 membered heteroarylene, or a 3-10 membered heterocycylene optionally substituted with one or more C₁₋₆ alkyl;
   L is a bond or *-O-(C₁₋₆ alkylene)-, wherein * indicates attachment to Ring B;
   Ring C is:
      a 6-membered aryl optionally substituted with one or more halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 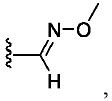 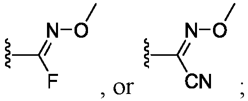 a bicylic 9- or 10-membered heteroaryl comprising two nitrogen atoms optionally substituted with one or more C₁₋₆ alkyl, or oxo.

In some embodiments, the compound of Formula (I‴) is of Formula (I): 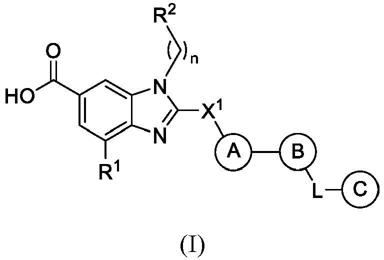 or a pharmaceutically acceptable salt thereof; wherein
R¹ is -C₁₋₆ haloalkyl or -O-X⁴;
wherein X⁴ is C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), -(-CH₂CH₂-O)₁₋₅-CH₃, or - (CH₂-CH(-OCH₃)-CH₂-O)₁₋₅-CH₃, wherein the alkyl, heteroalkyl, alkylene, or haloalkyl group is optionally substituted with one or more -CN, and the cycloalkyl or heterocyclyl group is optionally substituted with one or more halogen or -CN;
n is 0, 1, 2, 3, 4, 5, or 6;
R² is:
   hydrogen;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 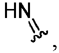 or C₁₋₆ alkyl; or
   a 5-membered heteroaryl, comprising 1 or 2 heteroatoms independently selected from N, and S, wherein at least one heteroatom of R⁵ is S;
   X¹ is 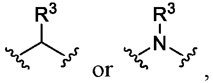
   wherein R³ is H or C₁₋₆ alkyl;
   Ring A is 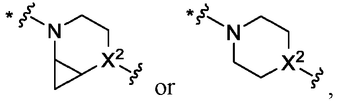
   wherein * indicates attachment to X¹, and
   wherein X² is CH or N;
   Ring B is:
      a 6-membered heteroarylene comprising nitrogen;
      a 9-membered heterocycylene comprising two oxygen atoms optionally substituted with one or more C₁₋₆ alkyl; or
      a 10-membered heterocycylene comprising two oxygen atoms;
      L is a bond or *-O-(C₁₋₆ alkylene)-, wherein * indicates attachment to Ring B;
      Ring C is:
         a 6-membered aryl optionally substituted with one or more halogen, -OCH₃, -CN, or C₃₋₁₀ cycloalkyl;
         a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 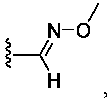 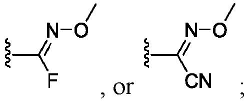
         a bicylic 9- or 10-membered heteroaryl comprising two nitrogen atoms optionally substituted with one or more C₁₋₆ alkyl, or oxo.

In some embodiments, the GLP-1R agonist is a compound of Formula (I-5) 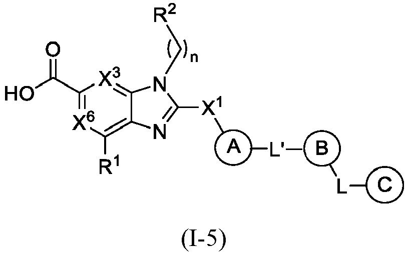 or a pharmaceutically acceptable salt thereof; wherein:
X³ is CR⁶ or N;
X⁶ is CR⁴ or N;
R¹ is -C₁₋₆ haloalkyl, -O-X⁴, or -NR⁸R⁹, or R¹ and R⁴, together with the atoms to which they are attached, combine to form a 6-membered heterocyclyl;
X⁴ is hydrogen, C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more deuterium, hydroxyl, -CN, or oxo, and the cycloalkyl or heterocyclyl group is optionally substituted with one or more halogen or -CN;
R⁶ is hydrogen, halogen, or -O-R⁷;
   wherein R⁷ and R², together with the atoms to which they are attached, combine to form a 6-membered heterocyclyl;
R⁸ and R⁹ each independently are selected from hydrogen, C₁₋₆ alkyl, or -S(O)₂-C₁₋₆ alkyl, or R⁸ and R⁹, together with the atoms to which they are attached, combine to form a 6-membered heterocyclyl; wherein the C₁₋₆ alkyl is optionally substituted by one or more oxo;
n is 0, 1, 2, 3, 4, 5, or 6;
R² is:
   hydrogen;
   branched C₁₋₆ alkyl;
   C₁₋₆ haloalkyl;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 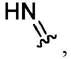 or C₁₋₆ alkyl;
   a 5-membered heteroaryl, comprising 1 or 2 heteroatoms independently selected from N, and S, wherein at least one heteroatom of R⁵ is S; or
   R² and R⁷, together with the atoms to which they are attached, combine to form a 5- or 6-membered heterocyclyl;
   R⁴ is hydrogen or -O-R⁸,
      wherein R⁸ and R¹, together with the atoms to which they are attached, combine to form a 6-membered heterocyclyl;
   X¹ is 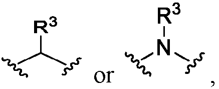
   wherein R³ is H or C₁₋₆ alkyl;
   Ring A is 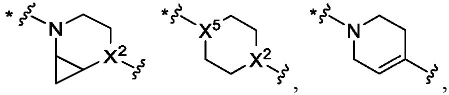 phenylene optionally substituted with one or more halo or C₁₋₆ alkyl, or 6-membered heteroarylene optionally substituted with one or more halo or C₁₋₆ alkyl;
   wherein * indicates attachment to X¹,
      X⁵ is CR³ or N, and
      X² is CR³ or N;
   L' is a bond or -O-;
   Ring B is a C₆₋₁₀ arylene, a 6-10 membered heteroarylene, or a 3-10 membered heterocyclene, wherein the C₆₋₁₀ arylene, 6-10 heteroarylene, or 3-10 membered heterocyclene is optionally substituted with one or more C₁₋₆ alkyl, C₁₋₆ alkoxy, or halogen;
   L is a bond, *-CH₂-, *-O-(C₁₋₆ alkylene)-, or *-(C₁₋₆ alkylene)-O-, wherein * indicates attachment to Ring B and the C₁₋₆ alkylene is optionally substituted with deuterium; and
   Ring C is:
      a 6-membered aryl optionally substituted with one or more C₁₋₆ alkyl, 3-10 membered heterocyclyl, halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 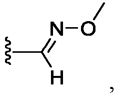 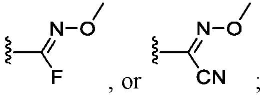 or a bicylic 9- or 10-membered heteroaryl comprising two nitrogen atoms optionally substituted with one or more C₁₋₆ alkyl, or oxo.

In some embodiments, the GLP-1R agonist is a compound of Formula (I-P01): 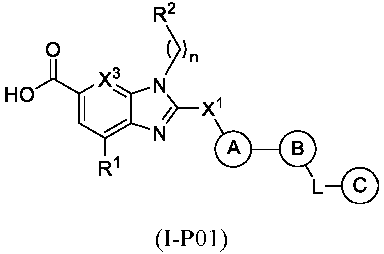 or a pharmaceutically acceptable salt thereof; wherein:
X³ is CH or N;
R¹ is -C₁₋₆ haloalkyl or -O-X⁴;
wherein X⁴ is C₁₋₆ alkyl, C₁₋₆ heteroalkyl, C₁₋₆ haloalkyl, -(C₁₋₆ alkylene)-(C₃₋₁₀ cycloalkyl), or -(C₁₋₆ alkylene)-(3- to 8-membered heterocyclyl), 3- to 8-membered heterocyclyl, -(CH₂CH₂-O)₁₋₅-CH₃, or -(CH₂-CH(-OCH₃)-CH₂-O)₁₋₅-CH₃, wherein the alkyl, heteroalkyl, alkylene, or haloalkyl group is optionally substituted with one or more -CN, and the cycloalkyl or heterocyclyl group is optionally substituted with one or more halogen or -CN;
n is 0, 1, 2, 3, 4, 5, or 6;
R² is:
   hydrogen;
   -(O)-C₁₋₆ alkyl;
   a C₃₋₁₀ cycloalkyl optionally substituted with one or more -CN, C₁₋₆ haloalkyl, or C₁₋₆ alkyl optionally substituted with one or more -CN;
   a 4 or 5-membered heterocyclyl comprising at least one oxygen or at least one sulfur atom, wherein the 4- or 5-membered heterocyclyl is optionally substituted with one or more oxo, 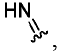 or C₁₋₆ alkyl; or
   a 5-membered heteroaryl, comprising 1 or 2 heteroatoms independently selected from N, and S, wherein at least one heteroatom of R⁵ is S;
   X¹ is 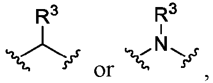
   wherein R³ is H or C₁₋₆ alkyl;
   Ring A is 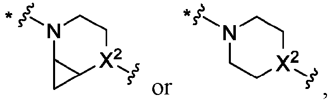
   wherein * indicates attachment to X¹, and
   wherein X² is CH or N;
   Ring B is a 6-10 membered heteroarylene, or a 3-10 membered heterocycylene optionally substituted with one or more C₁₋₆ alkyl;
   L is a bond or *-O-(C₁₋₆ alkylene)-, wherein * indicates attachment to Ring B;
   Ring C is:
      a 6-membered aryl optionally substituted with one or more halogen, -OCH₃, -CN, or C₃₋₁₀ cycloalkyl;
      a 6-membered heteroaryl comprising a nitrogen atom optionally substituted with one or more halogen, -CN, -O-C₁₋₆ alkyl, C₃₋₁₀ cycloalkyl, -C(=O)-(C₃₋₁₀ cycloalkyl), 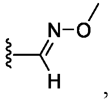 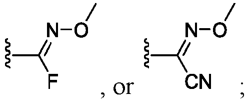
      a bicylic 9- or 10-membered heteroaryl comprising two nitrogen atoms optionally substituted with one or more C₁₋₆ alkyl, or oxo.

In some embodiments the compound of Formula (I-5) is of Formula (I-5a): 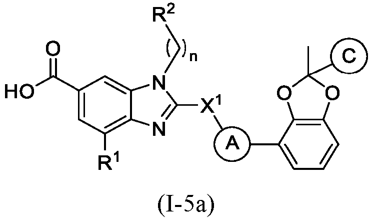
or a pharmaceutically acceptable salt thereof;
wherein R¹, n, R², X¹, Ring A, and Ring C are as defined for Formula (I-5).

In some embodiments, the compound of Formula (I-5) is of Formula (I-5b): 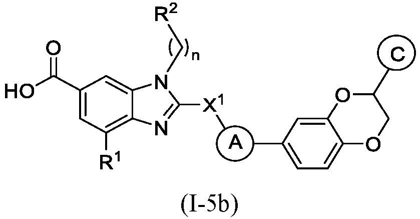
or a pharmaceutically acceptable salt thereof;
wherein R¹, n, R², X¹, Ring A, and Ring C are as defined for Formula (I-5).

In some embodiments, the compound of Formula (I-5) is of Formula (I-5c): 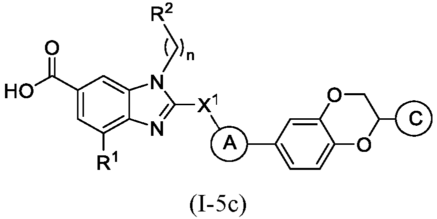
or a pharmaceutically acceptable salt thereof;
wherein R¹, n, R², X¹, Ring A, and Ring C are as defined for Formula (I-5).

In some embodiments, the compound of Formula (I-5) is of Formula (I-5d): 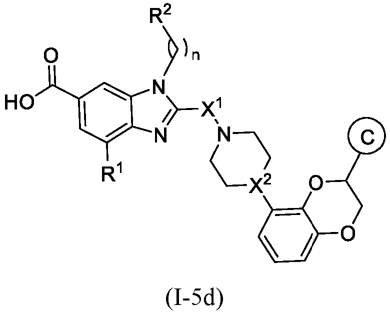
or a pharmaceutically acceptable salt thereof;
wherein R¹, n, R², X¹, X², and Ring C are as defined for Formula (I-5).

In some embodiments, the compound of Formula (I-5) is of Formula (I-5e): 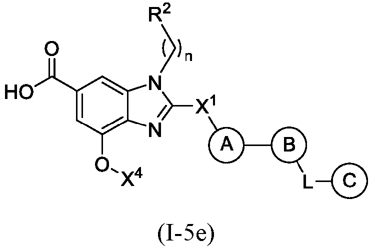
or a pharmaceutically acceptable salt thereof;
wherein X⁴, n, R², X¹, Ring A, Ring B, and Ring C are as defined for Formula (I-5).

In some embodiments, the compound of Formula (I-5) is of Formula (I-5f): 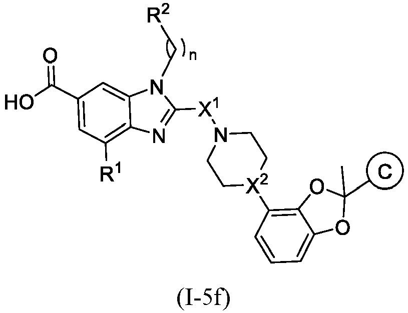
or a pharmaceutically acceptable salt thereof;
wherein R¹, n, R², X¹, and Ring C are as defined for Formula (I-5).

In some embodiments, the compound of Formula (I-5) is of Formula (I-5g): 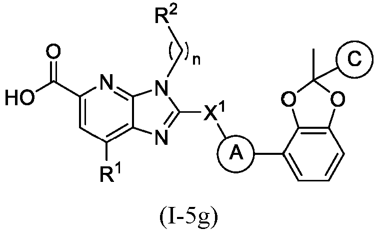
or a pharmaceutically acceptable salt thereof;
wherein R¹, n, R², X¹, Ring A, and Ring C are as defined for Formula (I-5).

In some embodiments, the compound of Formula (I-5) is of Formula (I-5h): 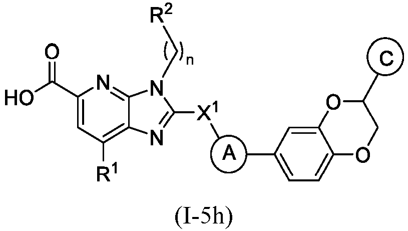
or a pharmaceutically acceptable salt thereof;
wherein R¹, n, R², X¹, Ring A, and Ring C are as defined for Formula (I-5).

In some embodiments, the compound of Formula (I-5) is of Formula (I-5i): 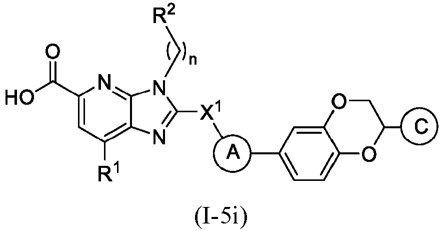
or a pharmaceutically acceptable salt thereof;
wherein R¹, n, R², X¹, and Ring A, Ring C are as defined for Formula (I-5).

In some embodiments, the compound of Formula (I-5) is of Formula (I-5j) 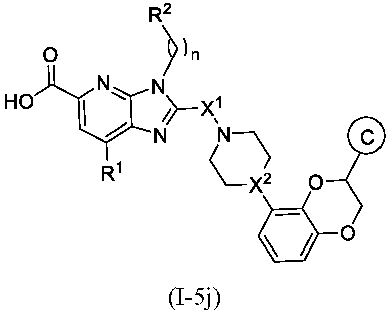
or a pharmaceutically acceptable salt thereof;
wherein R¹, n, R², X¹, and Ring C are as defined for Formula (I-5).

In some embodiments, the compound of Formula (I-5) is of Formula (I-5k) 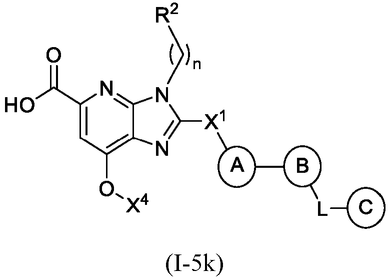
or a pharmaceutically acceptable salt thereof;
wherein X⁴, R², X¹, n Ring A, Ring B, L, and Ring C are as defined for Formula (I-5).

In some embodiments, the compound of Formula (I-5) is of Formula (I-5l) 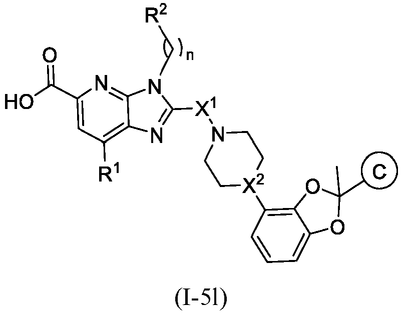
or a pharmaceutically acceptable salt thereof;
wherein R¹, R², X¹, n, and Ring C are as defined for Formula (I-5).

In some embodiments, the compound of Formula (I-5) is of Formula (I-5m) 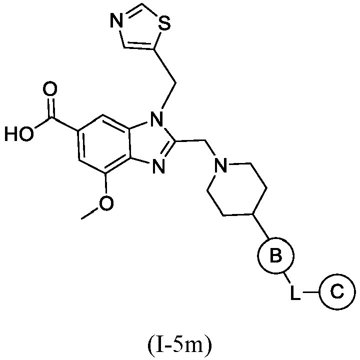
or a pharmaceutically acceptable salt thereof;
wherein Ring B, L, and Ring C are as defined for Formula (I-5).

In some embodiments, the compound of Formula (I-5) is of Formula (I-5n) 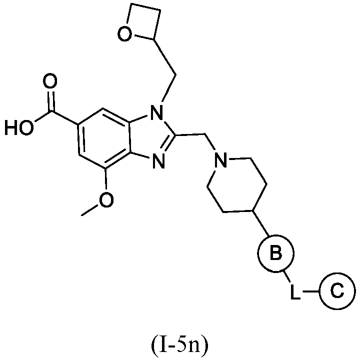
or a pharmaceutically acceptable salt thereof;
wherein Ring B, L, and Ring C are as defined for Formula (I-5).

In some embodiments, the compound of Formula (I-5) is of Formula (I-5o) 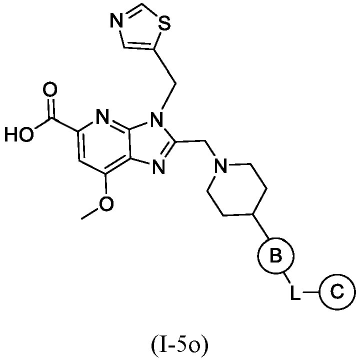
or a pharmaceutically acceptable salt thereof;
wherein Ring B, L, and Ring C are as defined for Formula (I-5).

In some embodiments, the compound of Formula (I-5) is of Formula (I-5p) 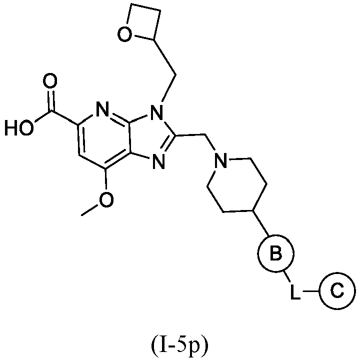
or a pharmaceutically acceptable salt thereof;
wherein Ring B, L, and Ring C are as defined for Formula (I-5).

In some embodiments, the compound of Formula (I-5) is of Formula (IQ) 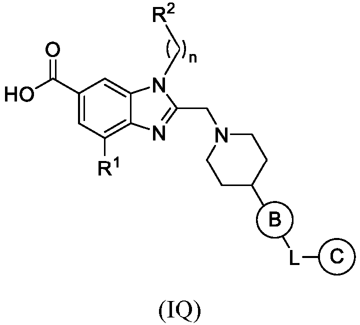
or a pharmaceutically acceptable salt thereof;
wherein R¹, R², n, Ring B, L, and Ring C are as defined for Formula (I-5).

In some embodiments, the compound of Formula (I-5) is of Formula (IR) 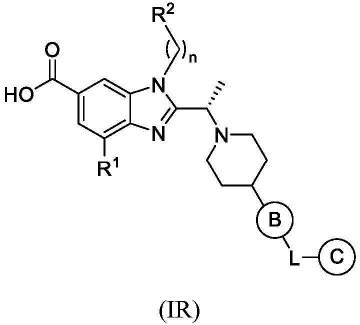
or a pharmaceutically acceptable salt thereof;
wherein R¹, R², n, Ring B, L, and Ring C are as defined for Formula (I-5).

In some embodiments, the compound of Formula (I-5) is of Formula (IS) 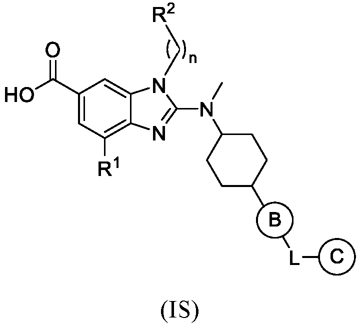 Wherein R¹, R², n, Ring B, L, and Ring C are as defined for Formula (I-5).

In some embodiments, the compound of Formula (I-5) is of Formula (IT) 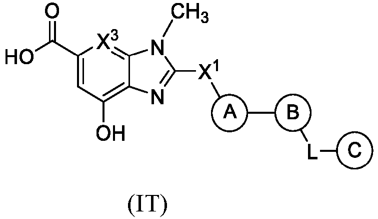
or a pharmaceutically acceptable salt thereof;
wherein X¹, X³, Ring A, Ring B, L, and Ring C are as defined for Formula (I-5).

In some embodiments, the compound of Formula (I-5) is of Formula (IU) 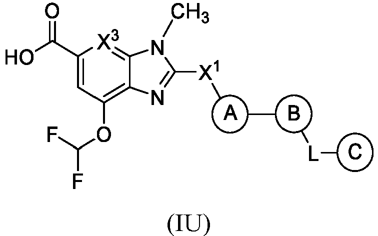
or a pharmaceutically acceptable salt thereof;
wherein X³, X¹, Ring A, Ring B, L, and Ring C are as defined for Formula (I-5).
or a pharmaceutically acceptable salt thereof;
wherein X³, R⁴, R¹, n, and R² are as defined for Formula (I-5).

In some embodiments, the compound of Formula (I-5) is of Formula (IX) 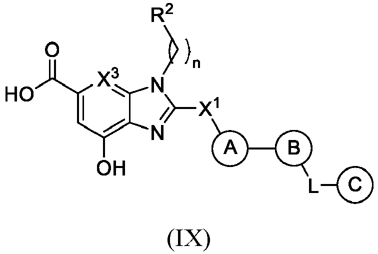
or a pharmaceutically acceptable salt thereof;
wherein X³, n, R², X¹, Ring A, Ring B, L, and Ring C are as defined for Formula (I-5).

In some embodiments, the compound of Formula (I-5) is of Formula (IY) 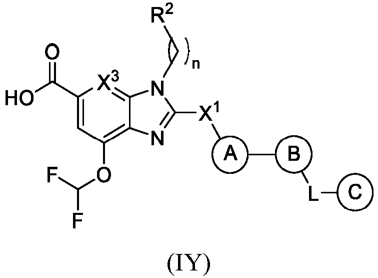 or a pharmaceutically acceptable salt thereof;
wherein X³, n, R², X¹, Ring A, Ring B, L, and Ring C are as defined for Formula (I-5).

In some embodiments, the compound of Formula (I-5) is of Formula (IZ) 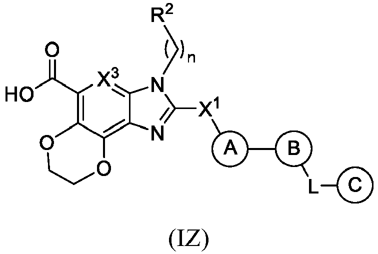
or a pharmaceutically acceptable salt thereof;
wherein X³, n, R², X¹, Ring A, Ring B, L, and Ring C are as defined for Formula (I-5).

In some embodiments, the compound of Formula (I‴) is of Formula (IAA) 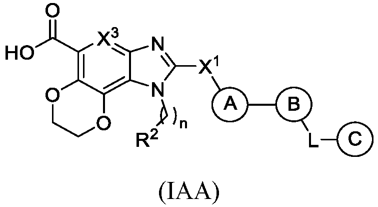
or a pharmaceutically acceptable salt thereof;
wherein X³, n, R², X¹, Ring A, Ring B, L, and Ring C are as defined for Formula (I-5).

In some embodiments, the compound of Formula (I-5) is of Formula (IAB) 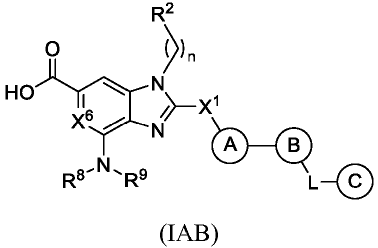
or a pharmaceutically acceptable salt thereof;
wherein X6, R8, R9, n, R2, X1, Ring A, Ring B, L, and Ring C are as defined for Formula (I-5).

In some embodiments, the compound of Formula (I-5) is of Formula (IAC) 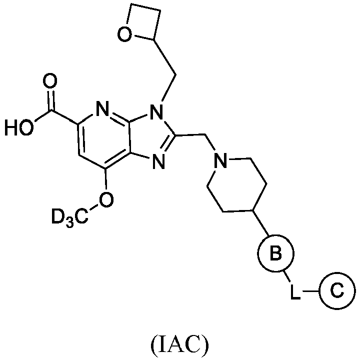
or a pharmaceutically acceptable salt thereof;
wherein Ring B, L, and Ring C are as defined for Formula (I-5).

In some embodiments, the compound of Formula (I-5) is of Formula (IAD) 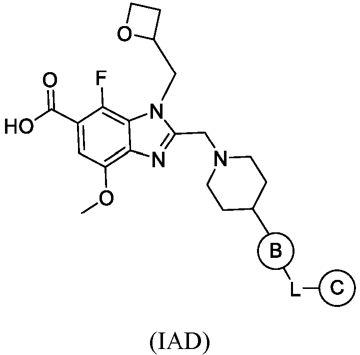
or a pharmaceutically acceptable salt thereof;
wherein Ring B, L, and Ring C are as defined for Formula (I-5).

In some embodiments, the compound of Formula (I-5) is of Formula (IAE) 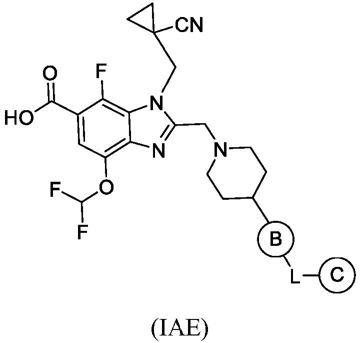
or a pharmaceutically acceptable salt thereof;
wherein Ring B, L, and Ring C are as defined for Formula (I-5).

In some embodiments, the compound of Formula (I-5) is of Formula (IAG) 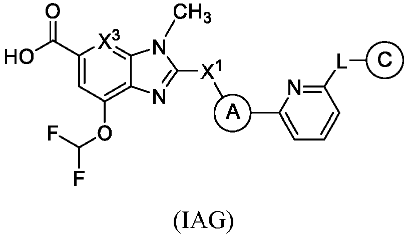
or a pharmaceutically acceptable salt thereof;
wherein X3, X1, Ring A, L, and Ring C are as defined for Formula (I-5).

In some embodiments, the compound of Formula (I-5) is of Formula (IAH): 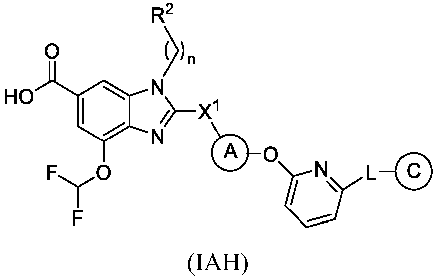
or a pharmaceutically acceptable salt thereof;
wherein n, R2, X1, Ring A, L, and Ring C are as defined for Formula (I-5).

In some embodiments, the compound of Formula (I-5) is of Formula (IAI): 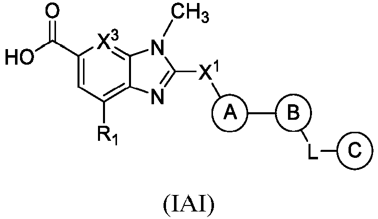
wherein R¹ is -O-(C₁₋₆ haloalkyl);
X3, X1, Ring A, Ring B, L, and Ring C are as defined for Formula (I-5);
or a pharmaceutically acceptable salt thereof.

In some embodiments, the compound of Formula (I-5) is of Formula (IAJ): 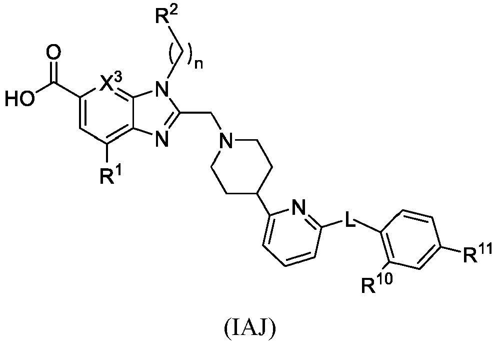
wherein R² is hydrogen, thiazolyl, oxetanyl, cyclopropyl optionally substituted with cyano, or methoxy;
n is 1 or 2;
R¹ is -O-C₁₋₆ alkyl, -OH, or -NH₂, wherein the C₁₋₆ alkyl is optionally substituted with one or more halo or deuterium;
X³ is CR⁶, wherein R⁶ is halo or hydrogen;
L is *-O-(C₁₋₆ alkylene)-, wherein * indicates attachment to Ring B and the C₁₋₆ alkylene is optionally substituted with deuterium;
R¹⁰ is halo; and
R¹¹ is halo or cyano;
or a pharmaceutically acceptable salt thereof.

In some embodiments, the compound of Formula (I**) is of Formula (II**): 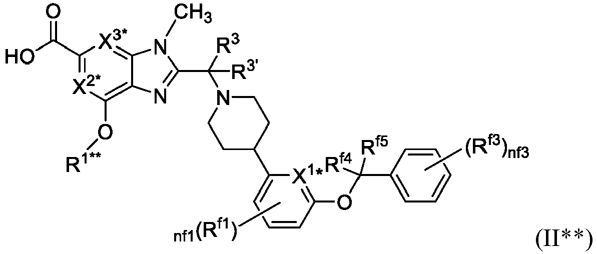 or a pharmaceutically acceptable salt thereof; wherein
R^{f4} and R^{f5} are each independently selected from C₁₋₆ alkyl, H and D
nf1 is 0, 1, 2, 3, or 4;
nf3 is 0, 1, 2, 3, 4, or 5;
each R^{f1} is halogen;
R³ and R^{3'} independently are H or D;
X¹* is N or CR^{f1};
X²* and X³* independently are CH or CF;
each R^{f3} is independently selected from halogen, -CN, C₁₋₆ alkyl, C₁₋₆ haloalkyl, C₁₋₆ alkoxy, or -C(O)N(R^{f3'})₂,
   each R^{f3'} is indepdendently selected from H or C₁₋₆ alkyl; and
R^{1**} is H or C₁₋₂ alkyl optionally substituted with one or more deuterium or halogen.

In some embodiments, the compound of Formula (I**) is of Formula (II*): 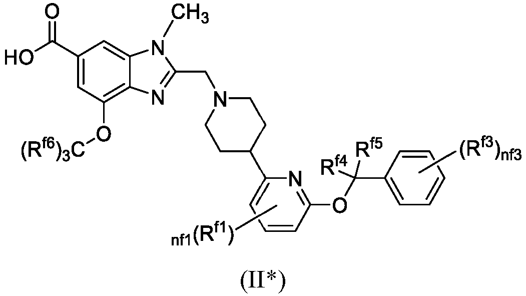 or a pharmaceutically acceptable salt thereof;
each R^{f1} is independently selected from halogen
R^{f4} and R^{f5} are each independently selected from C₁₋₆ alkyl, H and D
nf1 is 0, 1, 2, or 3;
nf3 is 0, 1, 2, 3, 4, or 5;
each R^{f1} is halogen;
each R^{f3} is independently selected from halogen, -CN, C₁₋₆ alkyl, C₁₋₆ haloalkyl, C₁₋₆ alkoxy, or -C(O)N(R^{f3'})₂,
   each R^{f3'} is independently selected from H or C₁₋₆ alkyl;
each R^{f6} is independently selected from H, D or F.

In some embodiments, R^{f4} and R^{f5} are each independently selected from CH₃, H, and D.

In some embodiments, each R^{f1} is fluorine.

In some embodiments, X³ is N. In some embodiments, X³ is CH.

In some embodiments, X⁶ is N. In some embodiments, X⁶ is CR⁴.

In some embodiments, R¹ is R¹ is -O-C₁₋₆ alkyl. In some embodiments, the alkyl group is linear. In some embodiments, the alkyl group is branched. In some embodiments, the alkyl group is linear and is optionally substituted with one or more deuterium, -CN or -O-C₁₋₆ alkyl. In some embodiments, the alkyl group is unsubstituted. In some embodiments, the alkyl group is substituted with one or more -CN or -O-C₁₋₆ alkyl. In some embodiments, R¹ is -O-CH₂-CH₃, -O-CH₃, -O-CH₂-CN, -O-CH₂-CH₂-O-CH₃, or -O-CH₂CH(-O-CH₃)-CH₃. In some embodiments, R¹ is branched and optionally substituted with one or more -CN. In some embodiments, R¹ is

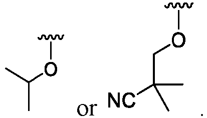

In some embodiments, R¹ is -O-C₁₋₆ haloalkyl, wherein the haloalkyl group is linear. In some embodiments, the haloalkyl group is substituted with one or more fluorine. In some embodiments, R¹ is is -O-CHF₂, -O-CF₃, -O-CH₂-CH₂F, -O-CH₂-CF₃, -O-CH₂-CHF-CH₃, -O-CHF-CH₃, -O-CHF-CH₂F, or -O-CH₂-CH₂F.

In some embodiments, R¹ is -O-C₃₋₁₀ cycloalkyl optionally substituted with C₁₋₆ alkoxy or halogen. In some embodiments, R¹ is -O-cyclopropyl or -O-cyclobutyl, wherein the cyclopropyl or cyclobutyl is optionally substituted with fluorine or methoxy. In some embodiments, R¹ is -O-C₃₋₁₀ cycloalkyl optionally substituted with one or more halogen, e.g., fluorine, -OCH₃, or -CN.

In some embodiments, R¹ is -O-(3- to 8-membered heterocyclyl). In some embodiments, the 3- to 8-membered heterocyclyl comprises one oxygen atom. In some embodiments, the 3- to 8-membered heterocyclyl comprises two oxygen atoms. In some embodiments, the R¹ is -O-CH₂-(1,4-dioxan-2-yl) or -O-CH₂-tetrahydrofuran-2-yl.

In some embodiments, R¹ is -O-(C₁₋₆ alkylene)-(C₃₋₁₀ cycloalkyl). In some embodiments, R¹ is -O-(C₁₋₆ alkylene)-(C₃₋₁₀ cycloalkyl) optionally substituted with one or more halogen, cyano, or -OCH₃. In some embodiments, R¹ is 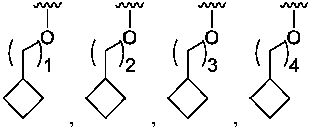 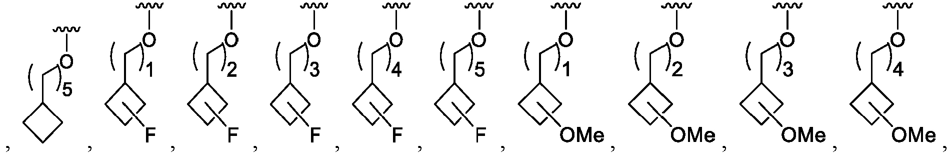 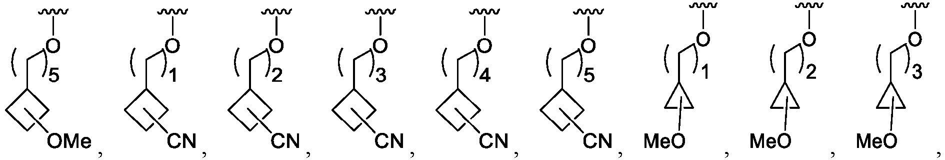 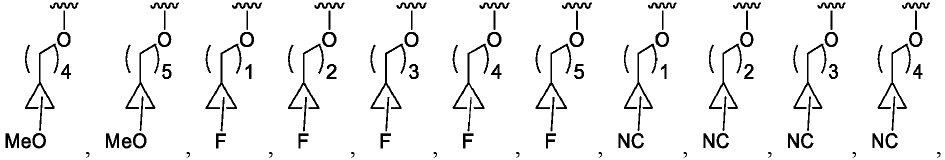 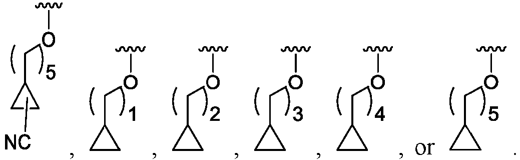 In some embodiments, R¹ is 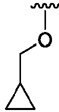 or

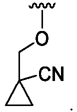

In some embodiments, R¹ is -O-(C₁₋₆ alkylene)-(3- to 8-membered heterocyclyl). In some embodiments, -O-(C₁₋₆ alkylene)-(3- to 8-membered heterocyclyl) optionally substituted with one or more halogen, cyano, or -OCH₃. In some embodiments, R¹ is 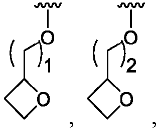 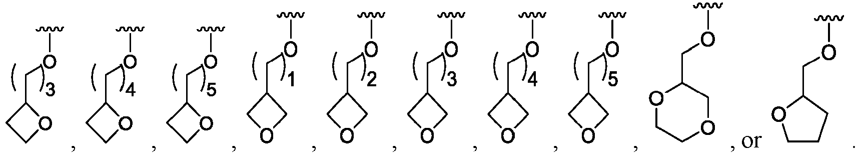 In some embodiments, R¹ is

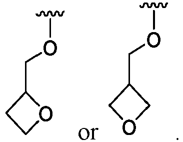

In some embodiments, R¹ is

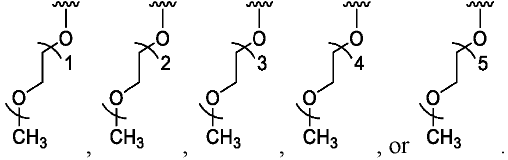

In some embodiments, R¹ is C₁₋₆ haloalkyl. In some embodiments, R¹ is -CF₂-CH₃.

In some embodiments, R¹ is -O-(3- to 8-membered heterocyclyl). In some embodiments, R¹ is oxetanyl. In some embodiments, R¹ is oxetan-3-yl.

In some embodiments, R¹ is -O-(CH₂-CH(-OCH₃)-CH₂-O)₁₋₅-CH₃. In some embodiments, R¹ is

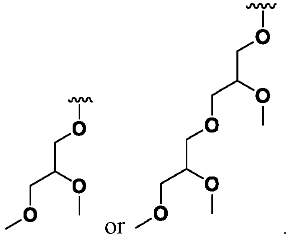

In some embodiments, R¹ is -NR⁸R⁹. In some embodiments, R⁸ and R⁹ combine with the atom to which they are attached to form a 6-membered heterocyclyl. In some embodiments, R⁸ and R⁹ combine with the atom to which they are attached to form morpholine. In some embodiments, R⁸ and R⁹ are hydrogen. In some embodiments, R¹ is NH₂. In some embodiments, R¹ is NR⁸R⁹, wherein R⁸ is H and R⁹ is C₁₋₆ alkyl optionally substituted with oxo.

In some embodiments, R² is C₃₋₁₀ cycloalkyl optionally substituted with one or more - CN, C₁₋₆ haloalkyl, or C₁₋₆ alkyl optionally substituted with one or more -CN. In some embodiments, R² is cyclopropyl optionally substituted with one or more -CN, C₁₋₆ haloalkyl, or C₁₋₆ alkyl optionally substituted with one or more -CN. In some embodiments, R² is

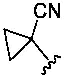

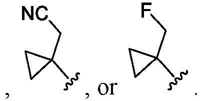

In some embodiments, R² is 4 or 5-membered heterocyclyl comprising at least one oxygen or at least one sulfur atom, wherein the 4- or 5-membered heterocyclyl is optionally substituted with one or more oxo, 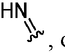 or C₁₋₆ alkyl. In some embodiments, R² is (i) thietane optionally substituted with one or more oxo or 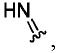 (ii) oxetane, or (iii) tetrahydrofuran optionally substituted with one or more C₁₋₆ alkyl. In some embodiments, R² is

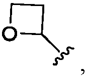

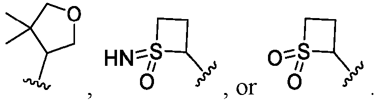

In some embodiments, R² is 5-membered heteroaryl, comprising 1 or 2 heteroatoms independently selected from N, and S, wherein at least one heteroatom of R⁵ is S. In some embodiments, R² is thiazole. In some embodiments, R² is

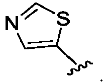

In some embodiments, R² is H. In some embodiments, R² is -OCH₃.

In some embodiments, n is 1.

In some embodiments, X¹ is 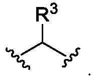 In some embodiments, X¹ is 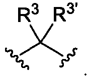 In some embodiments, X¹ is 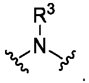 In some embodiments, R³ is methyl. In some embodiments, R³ is hydrogen. In some embodiments, R³ and R^{3'} independently are CH₃, CD₃, deuterium, or hydrogen. In some embodiments, X¹ is 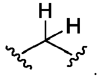 In some embodiments, X¹ is 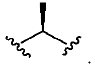 In some embodiments, X¹ is 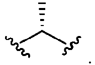 In some embodiments, X¹ is

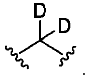

In some embodiments, Ring A is 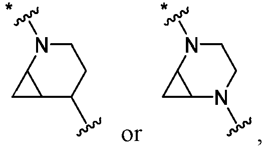 wherein * indicates attachment to X¹. In some embodiments, Ring A is 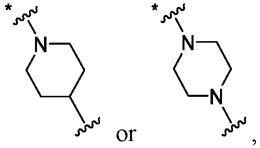 wherein * indicates attachment to X¹. In some embodiments, Ring A is a phenylene ring optionally substituted with one or more halogen or C₁₋₆ alkyl. In some embodiments, Ring A is 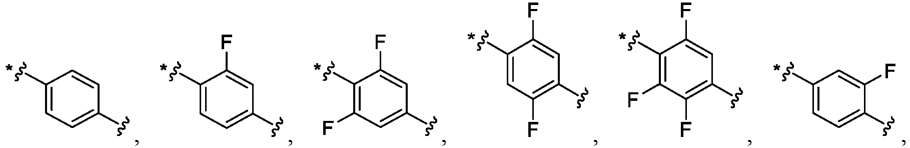 or 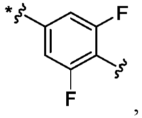 wherein 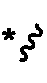 indicates attachment to X¹. In some embodiments, Ring A is a 6-membered heteroarylene ring optionally substituted with one or more halogen. In some embodiments, Ring A is 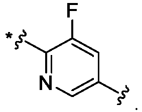 In some embodiments, Ring A is 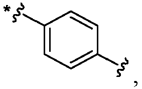 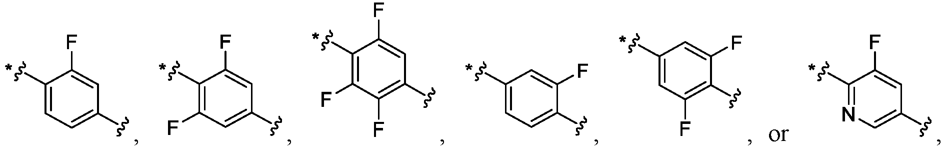 wherein 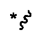 indicates attachment to X¹.

In some embodiments, Ring B is a 6-membered heteroarylene comprising nitrogen. In some embodiments, Ring B is pyridinylene or pyrimidinylene optionally substituted with one or more halogen. In some embodiments, Ring B is pyridinylene. In some embodiments, Ring B is 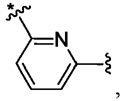 wherein * indicates attachment to Ring A or L'. In some embodiments, Ring B is 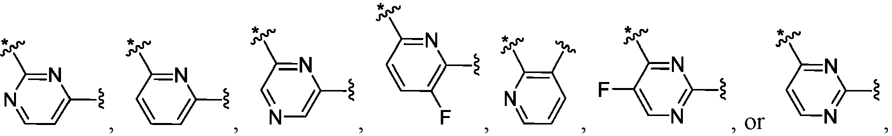 wherein * indicates attachment to Ring A or L'.

In some embodiments, Ring B is a 9-membered heterocycylene comprising two oxygen atoms optionally substituted with one or more C₁₋₆ alkyl. In some embodiments, Ring B is benzoidoxolylene optionally substituted with one or more C₁₋₆ alkyl. In some embodiments, Ring B is 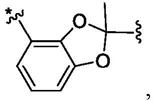 wherein * indicates attachment to Ring A or L'. In some embodiments, Ring B is B is a 10-membered heterocycylene comprising two oxygen atoms. In some embodiments, Ring B is benzodioxanylene. In some embodiments, Ring B is 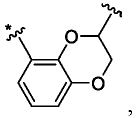 wherein * indicates attachment to Ring A or L'. In some embodmients, Ring B is 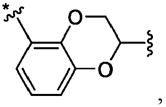 wherein * indicates attachment to Ring A or L'.

In some embodiments, Ring B is a 10-membered heterocyclene comprising one oxygen atom and one nitrogen atom. In some embodiments, Ring B is 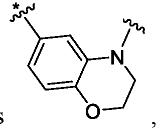 wherein * indicates attachment to Ring A or L'.

In some embodiments, L is a bond. In some embodiments, L is *-O-CH₂-, wherein * indicates attachment to Ring B.

In some embodiments, Ring C is phenyl optionally substituted with one or more halogen, -CN, -OCH₃, 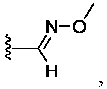 or cyclopropyl. In some embodiments, Ring C is 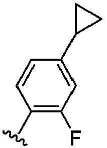 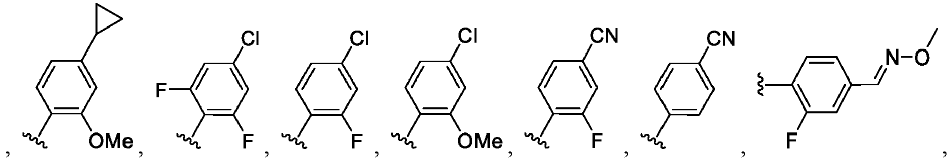 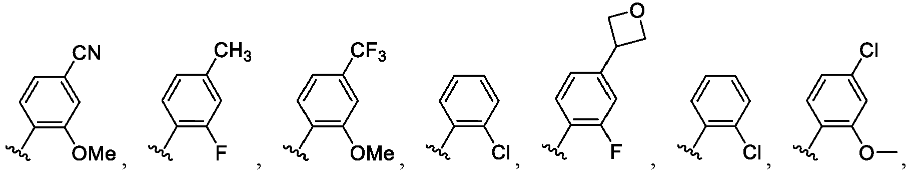 or

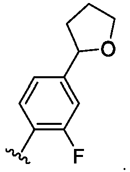

In some embodiments, Ring C is a 6-membered heteroaryl comprising a nitrogen atom optionally substituted with one or more halogen, -CN, -O-C₁₋₆ alkyl, -C(=O)-(C₃₋₁₀ cycloalkyl), or C₃₋₁₀ cycloalkyl. In some embodiments, Ring C is pyridinyl optionally substituted with one or more -Cl, -F, -CN, -OCH₃, cyclopropyl, 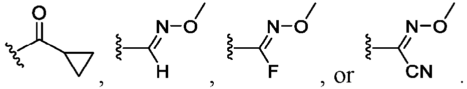 In some embodiments, Ring C is

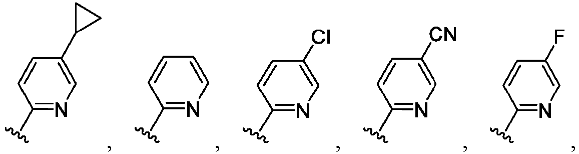

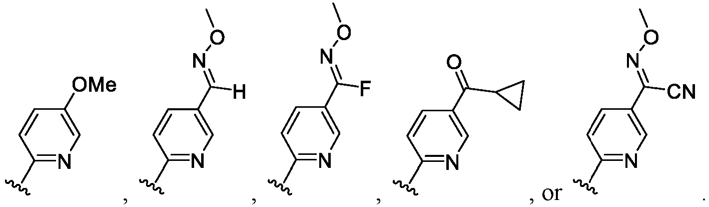

In some embodiments, Ring C is a bicylic 9- or 10-membered heteroaryl comprising two nitrogen atoms optionally substituted with one or more C₁₋₆ alkyl, or oxo. In some embodiments, Ring C is pyrazolopyridine, triazolopyridine, 2,3-dihydro-*1H*-pyrrolopyridine, 1,2-dihydrooxazolopyridine, 1,2,3,4-tetrahydronaphthyridine, 2,3-dihydro-pyridooxazine, 2,3-dihydro-*1H*-pyrrolopyridine, or 2,3-dihydrooxazolopyridine, wherein the pyrazolopyridine, triazolopyridine, 2,3-dihydro-*1H*-pyrrolopyridine, 1,2-dihydrooxazolopyridine, 1,2,3,4-tetrahydronaphthyridine, 2,3-dihydro-pyridooxazine, 2,3-dihydro-*1H*-pyrrolopyridine, or 2,3-dihydrooxazolopyridine is optionally substituted with one or more C₁₋₆ alkyl or oxo. In some embodiments, Ring C is 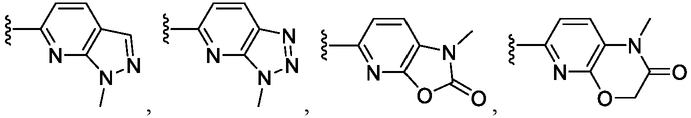 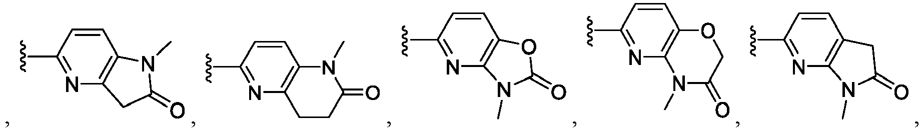 or

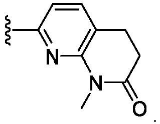

In some embodiments,

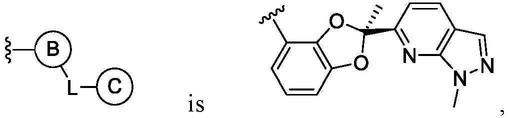

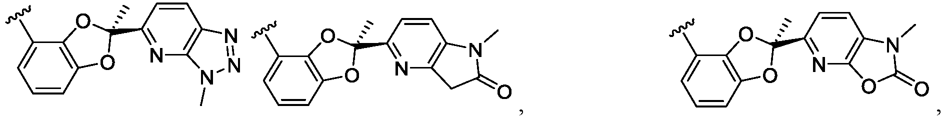

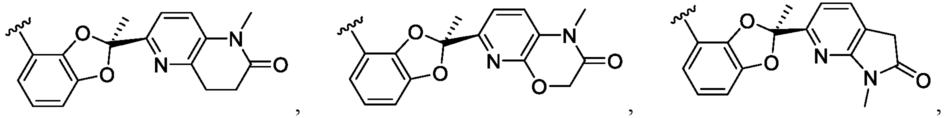

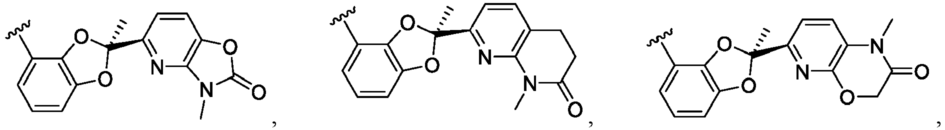

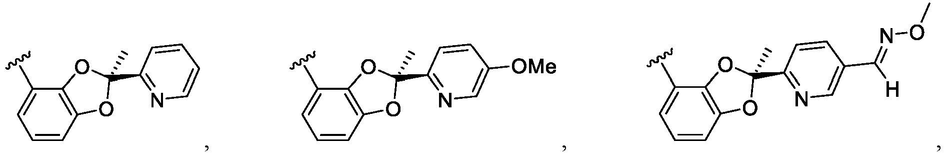

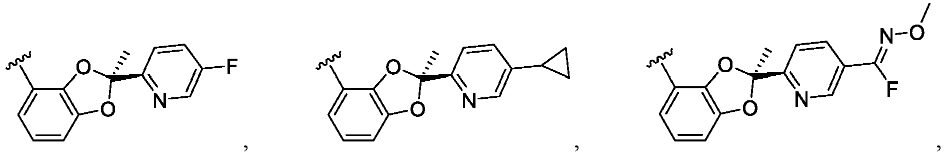

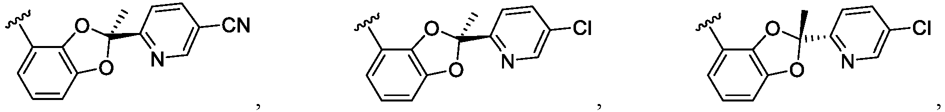

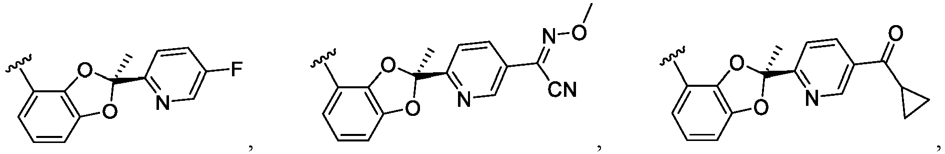

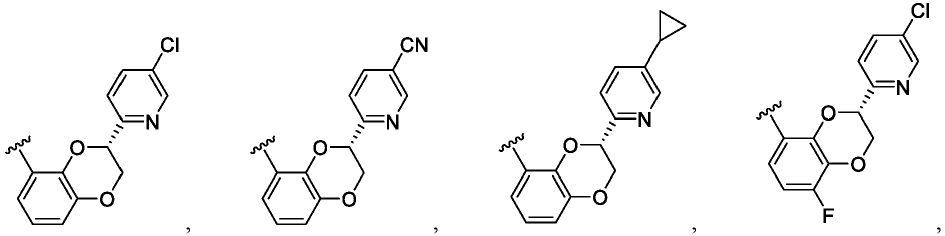

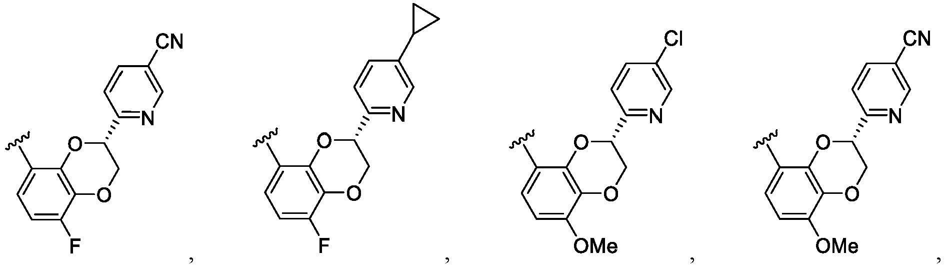

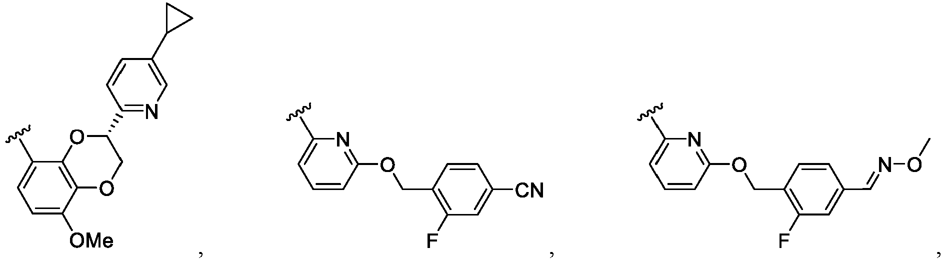

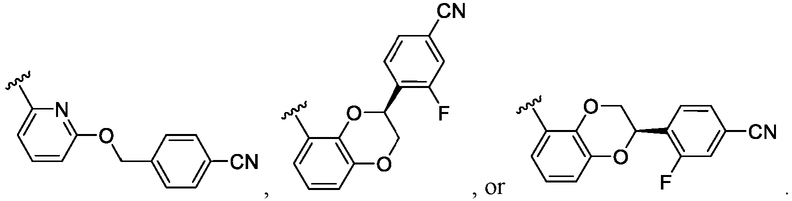

In some embodiments, R¹² is -COOH. In some embodiments, R¹² is

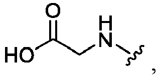

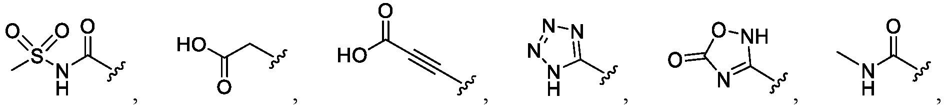

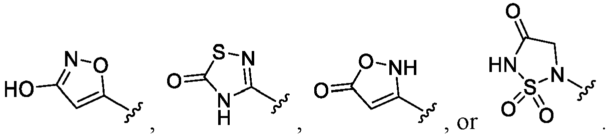

The present disclosure contemplates the combination of any one of Formula (I-5e), (I-5k), (I-5l), (I-5m), (I-5n), (I-5o), (I-5p), (I-5q), (I-5r), (I-5s), (I-5t), (I-5u), (I-5v), (I-5w), (I-5x), (I-5y), (I-5z), (I-5aa), (I-5ab), (I-5ac), or (I-5af) with any one of the following 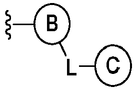 moieties: 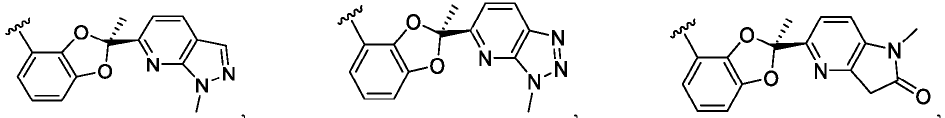 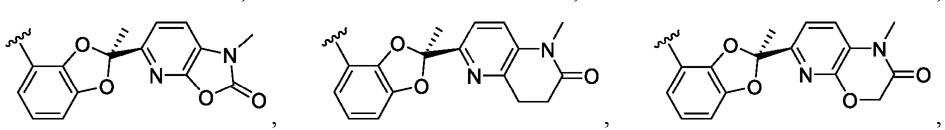 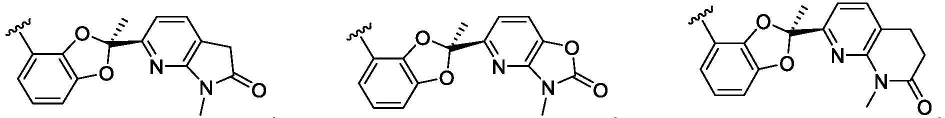 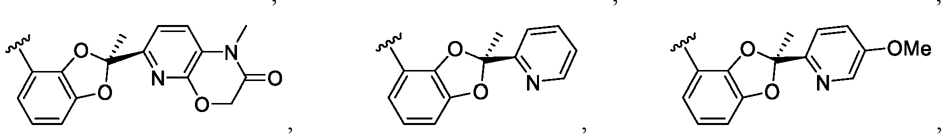 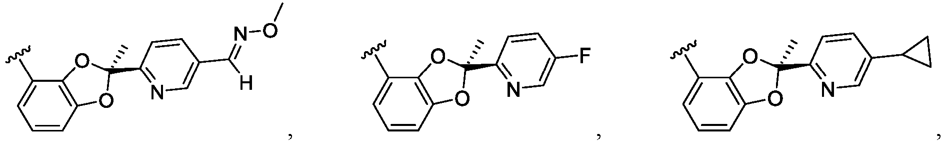 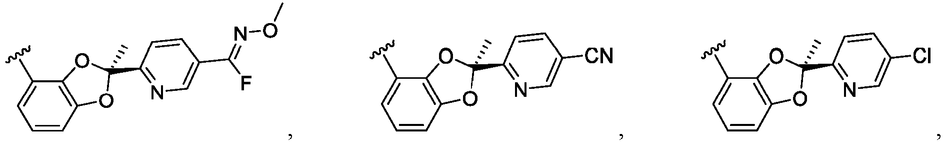 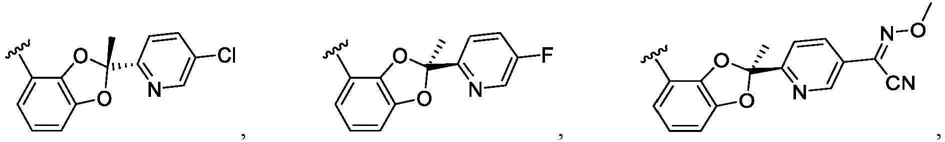 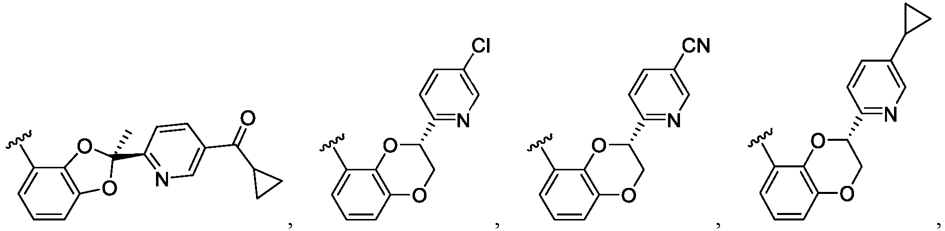 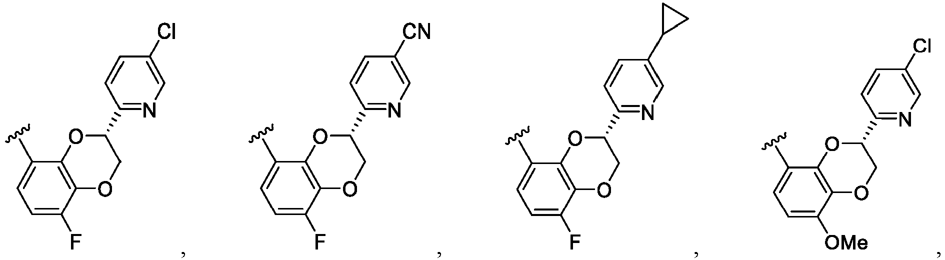 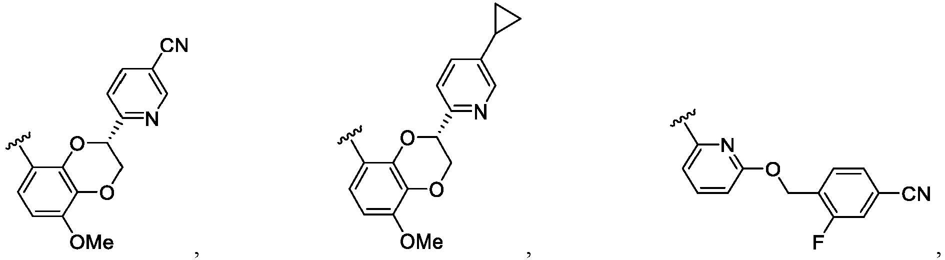 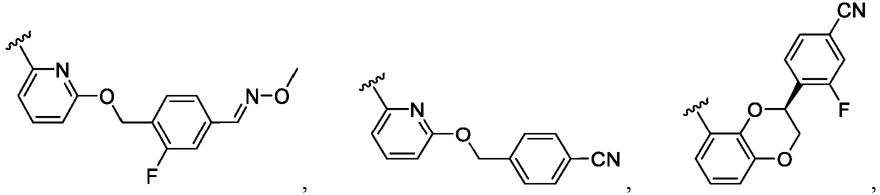 or

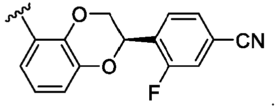

The present disclosure contemplates the combination of any one of Formula (I-5e), (I-5k), (I-5l), (I-5m), (I-5n), (I-5o), (I-5p), (I-5q), (I-5r), (I-5s), (I-5t), (I-5u), (I-5v), (I-5w), (I-5x), (I-5y), (I-5z), (I-5aa), (I-5ab), (I-5ac), or (I-5af) with any one of the following 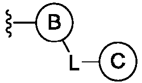 moieties:

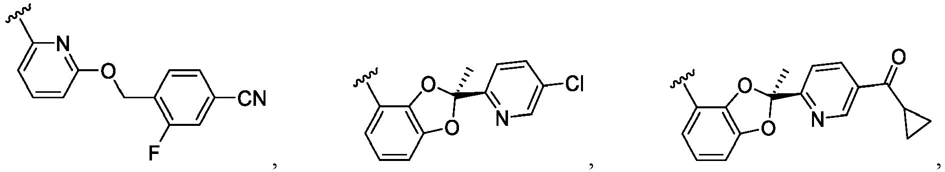

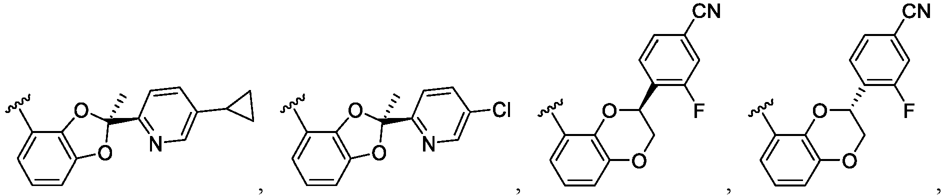

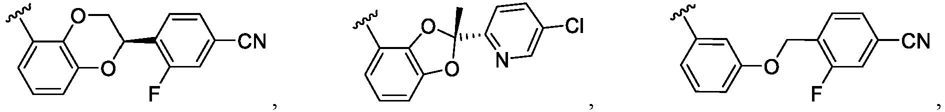

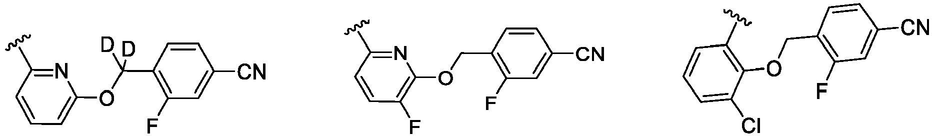

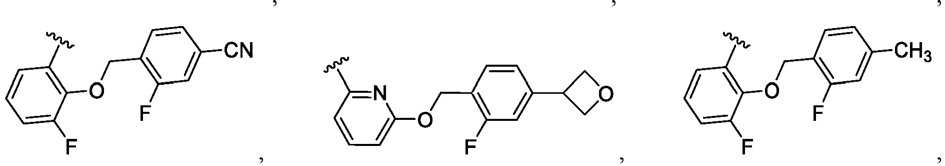

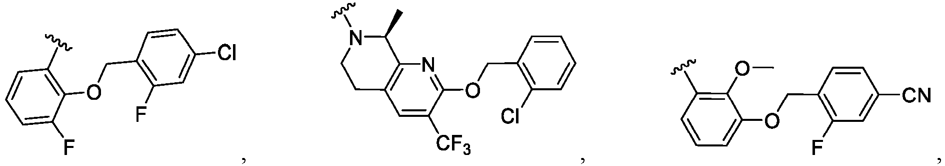

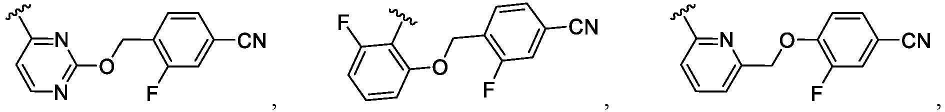

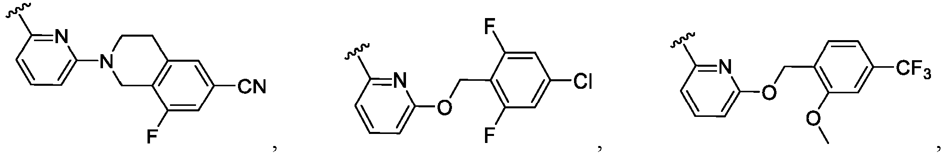

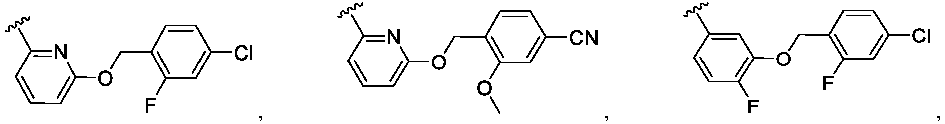

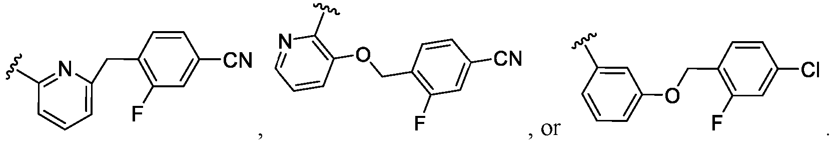

In some embodiments, the GLP-1R agonist is 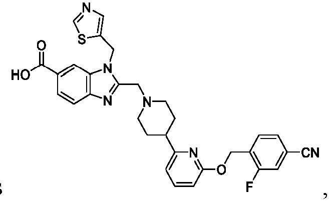 which is 2-((4-(6-((4-cyano-2-fluorobenzyl)oxy)pyridin-2-yl)piperidin-1-yl)methyl)-1-(thiazol-5-ylmethyl)-1H-benzo[d]imidazole-6-carboxylic acid, or a pharmaceutically acceptable salt thereof. In some embodiments, the GLP-1R agonist is 2-((4-(6-((4-cyano-2-fluorobenzyl)oxy)pyridin-2-yl)piperidin-1-yl)methyl)-1-(thiazol-5-ylmethyl)-1H-benzo[d]imidazole-6-carboxylic acid meglumine salt.

In some embodiments, the GLP-1R agonist is selected from those listed in Table 1 below, or a pharmaceutically acceptable salt thereof:

**Table 1**

| ***Compound No.*** | ***Structure*** |
|---|---|
| *1-1* | 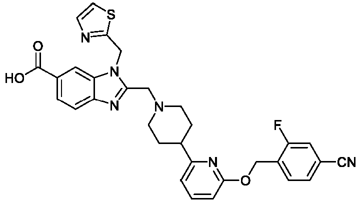 |
| *1-2* | 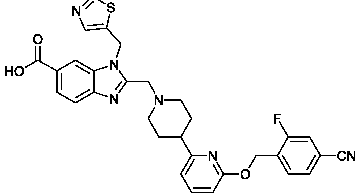 |
| *1-3* | 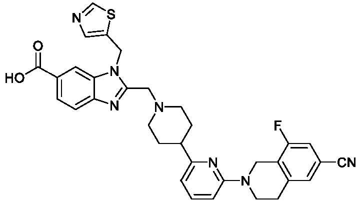 |
| *1-4* | 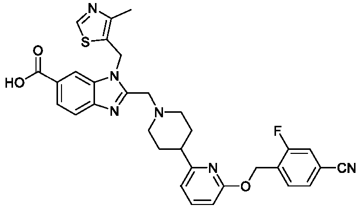 |
| *1-5* | 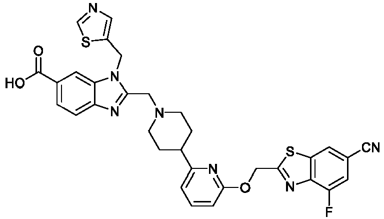 |
| *1-6* | 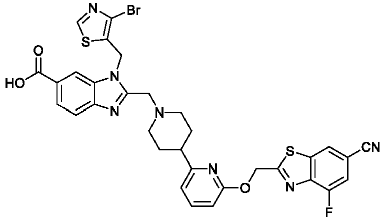 |
| *1-7* | 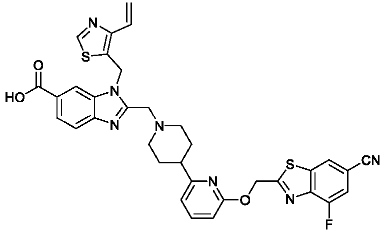 |
| *1-8* | 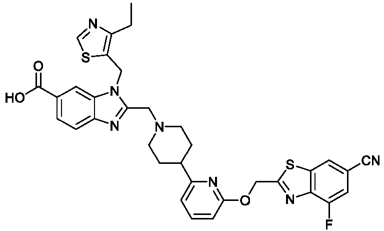 |
| *1-9* | 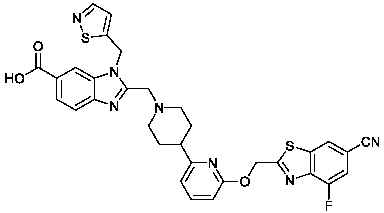 |
| *1-10* | 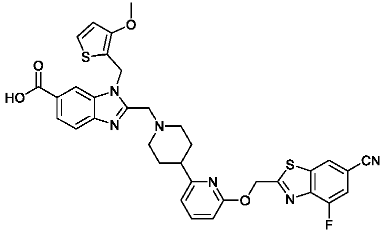 |
| *1-11* | 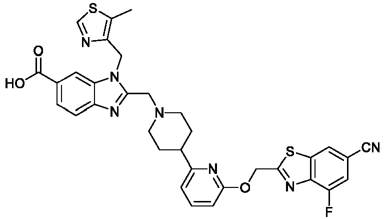 |
| *1-12* | 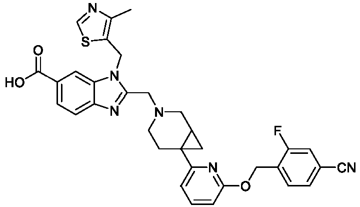 |
| *1-13* | 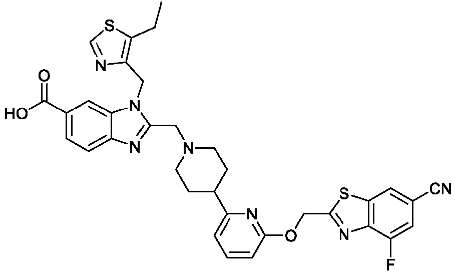 |
| *1-14* | 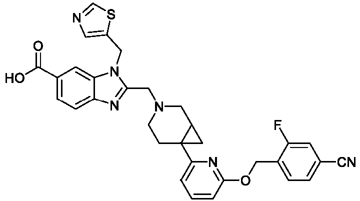 |
| *1-15* | 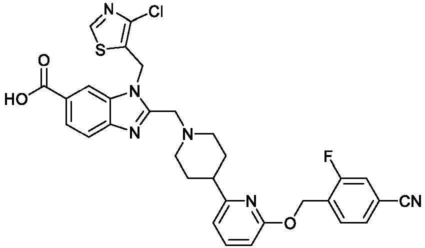 |
| *1-16* | 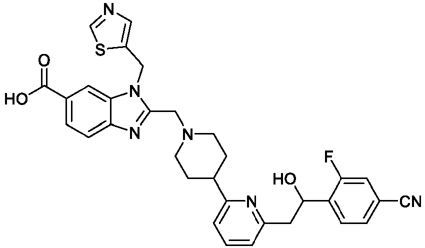 |
| *1-17* | 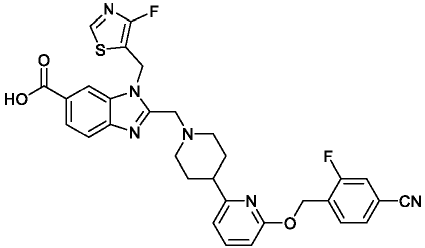 |
| *1-18* | 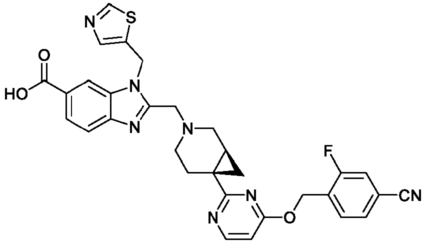 |
| *1-19* | 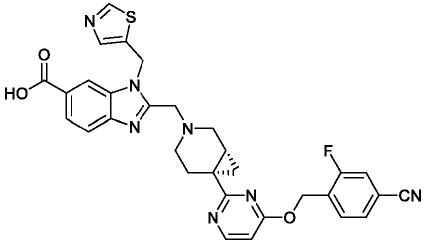 |
| *1-20* | 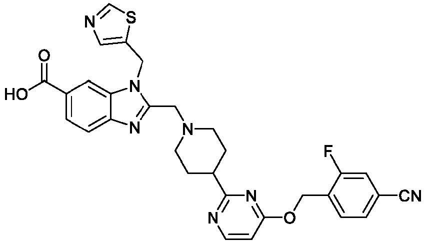 |
| *1-21* | 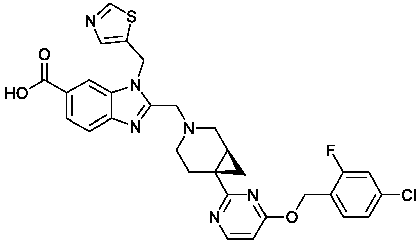 |
| *1-22* | 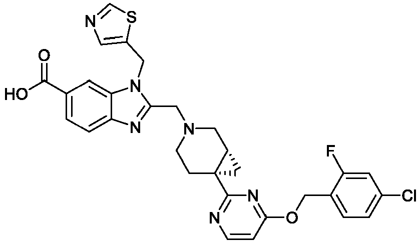 |
| *1-23* | 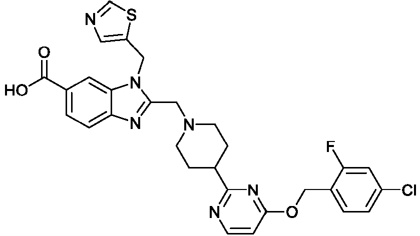 |
| *1-24* | 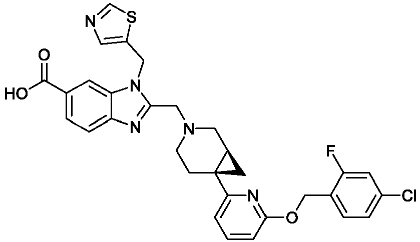 |
| *1-25* | 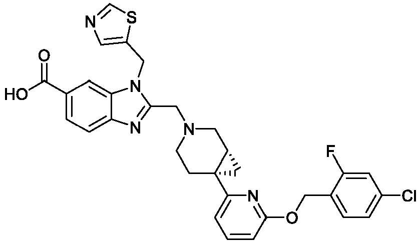 |
| *1-26* | 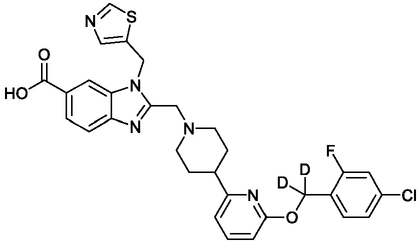 |
| *1-27* | 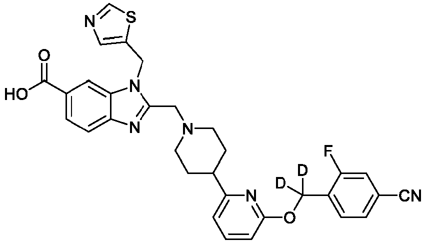 |
| *1-28* | 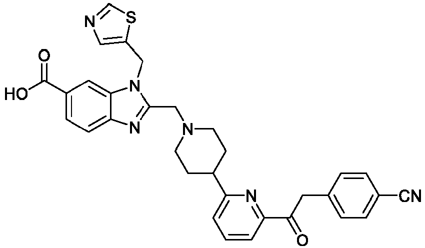 |
| *1-29* | 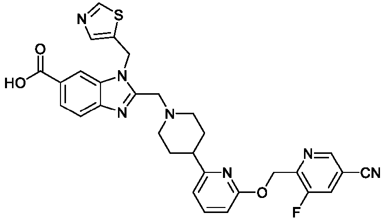 |
| *1-30* | 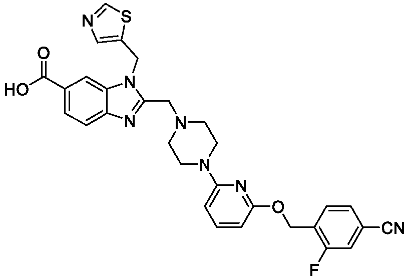 |
| *1-31* | 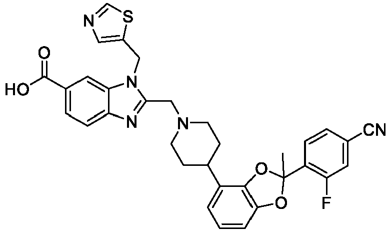 |

In some embodiments, the GLP-1R agonist is selected from those listed in Table 2 below, or a pharmaceutically acceptable salt thereof:

**Table 2**

| **Compound No.** | **Structure** |
|---|---|
| 2-1 | 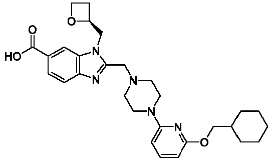 |
| 2-2 | 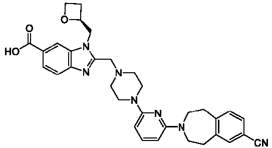 |
| 2-3 | 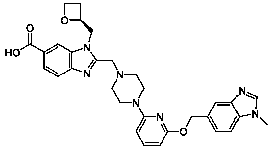 |
| 2-4 | 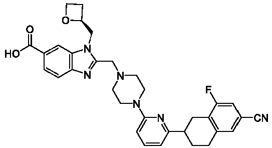 |
| 2-5 | 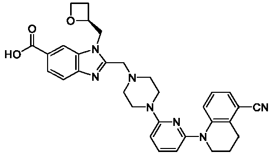 |
| 2-6-P1 | 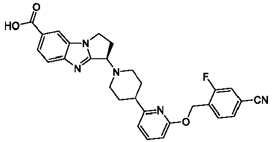 |
| 2-6-P2 | 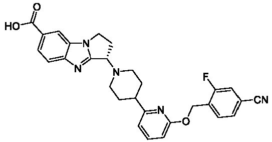 |
| 2-7-P1 | 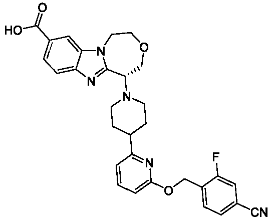 |
| 2-7-P2 | 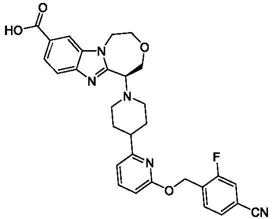 |
| 2-8-P1 | 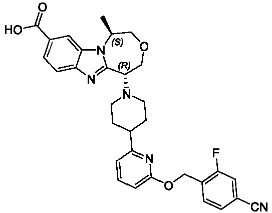 |
| 2-8-P2 | 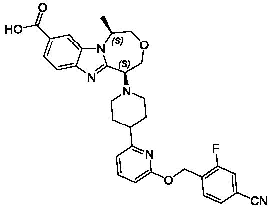 |
| 2-9 | 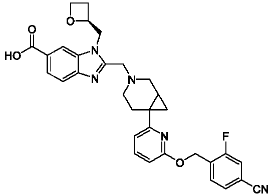 |
| 2-9-P1 | 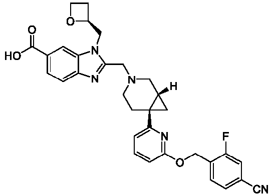 |
| 2-9-P2 | 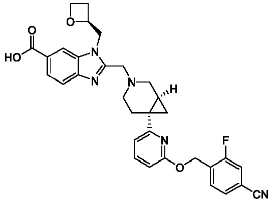 |
| 2-10-P1 | 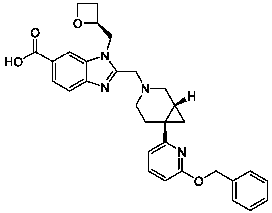 |
| 2-10-P2 | 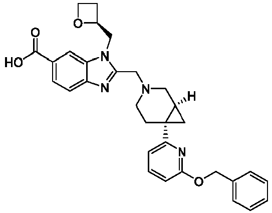 |
| 2-11-P1 | 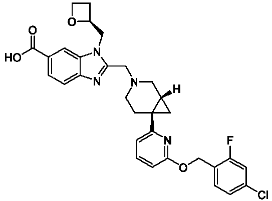 |
| 2-11-P2 | 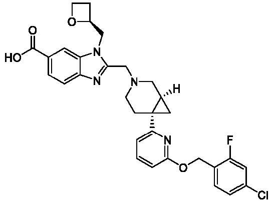 |
| 2-12 | 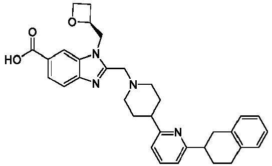 |
| 2-13 | 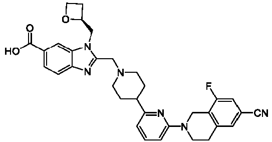 |
| 2-14 | 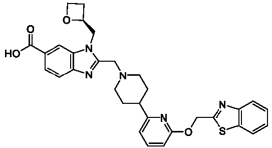 |
| 2-15 | 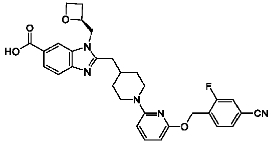 |
| 2-16 | 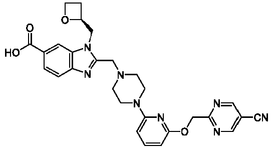 |
| 2-17 | 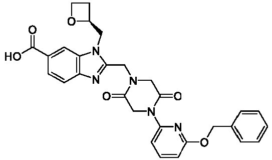 |
| 2-18 | 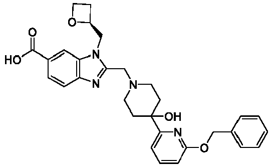 |
| 2-19 | 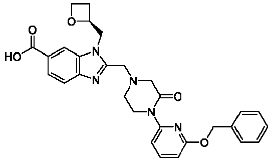 |
| 2-20-P1 | 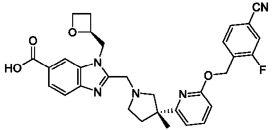 |
| 2-20-P2 | 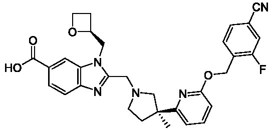 |
| 2-21 | 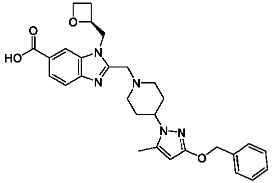 |
| 2-22 | 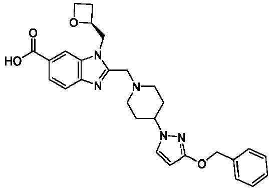 |
| 2-23 | 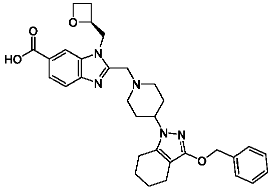 |
| 2-24 | 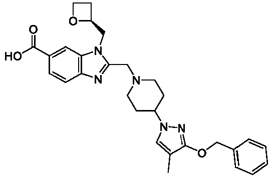 |
| 2-25 | 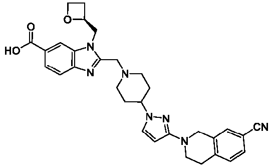 |
| 2-26-P1 | 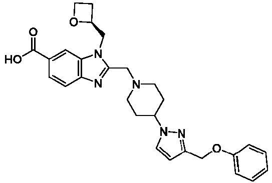 |
| 2-26-P2 | 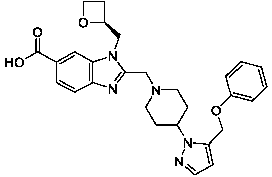 |
| 2-27 | 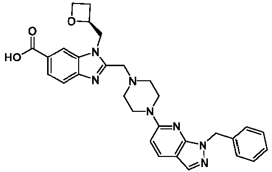 |
| 2-28 | 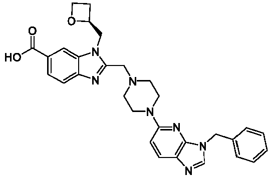 |
| 2-29 | 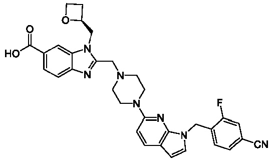 |
| 2-30 | 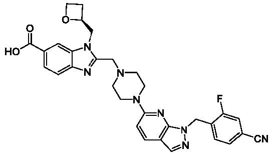 |
| 2-31 | 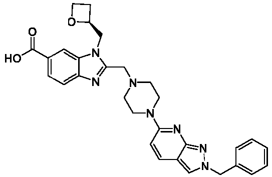 |
| 2-32 | 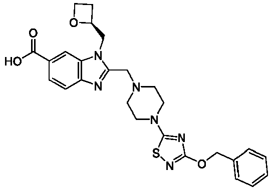 |
| 2-33 | 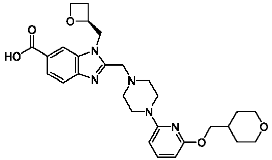 |
| 2-34 | 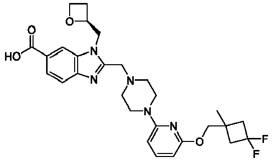 |
| 2-35 | 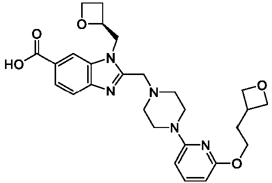 |
| 2-36 | 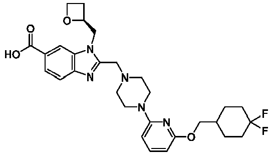 |
| 2-37 | 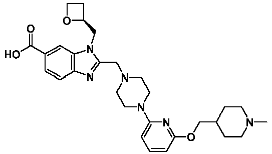 |
| 2-38 | 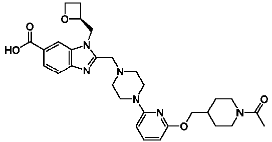 |
| 2-39 | 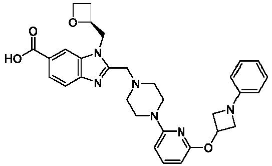 |
| 2-40 | 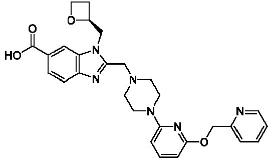 |
| 2-41 | 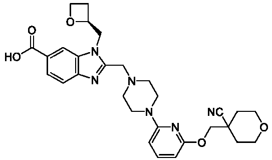 |
| 2-42 | 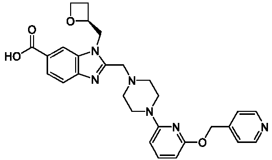 |
| 2-43 | 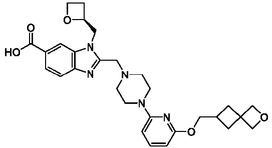 |
| 2-44 | 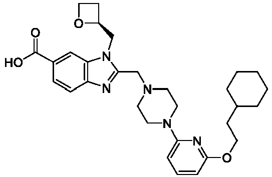 |
| 2-45 | 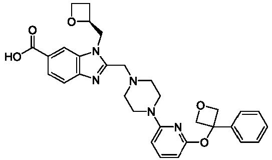 |
| 2-46 | 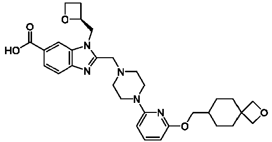 |
| 2-47 | 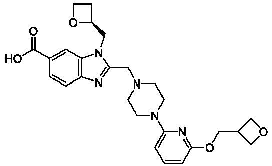 |
| 2-48 | 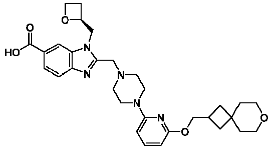 |
| 2-49 | 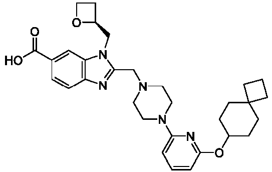 |
| 2-50 | 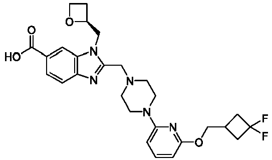 |
| 2-51 | 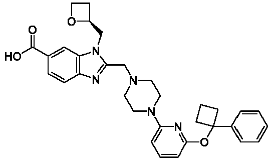 |
| 2-52 | 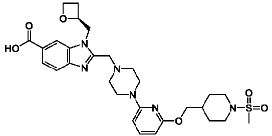 |
| 2-53 | 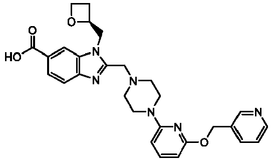 |
| 2-54 | 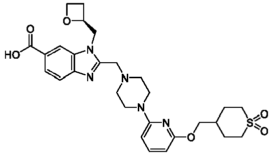 |
| 2-55 | 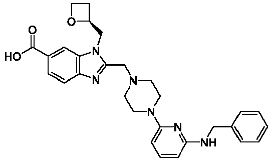 |
| 2-56 | 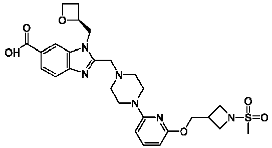 |
| 2-57 | 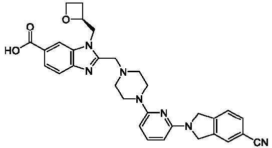 |
| 2-58 | 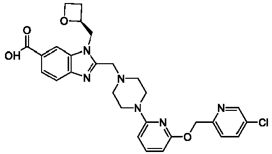 |
| 2-59 | 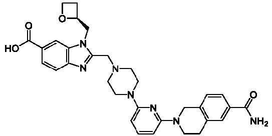 |
| 2-60 | 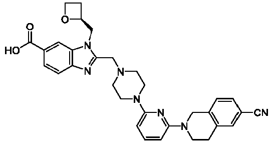 |
| 2-61 | 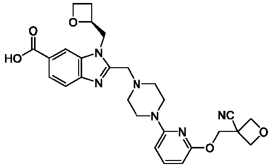 |
| 2-62 | 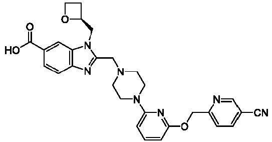 |
| 2-63 | 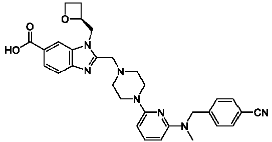 |
| 2-64 | 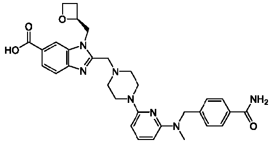 |
| 2-65 | 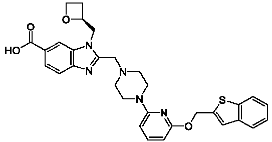 |
| 2-66 | 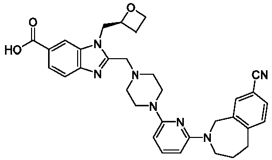 |
| 2-67 | 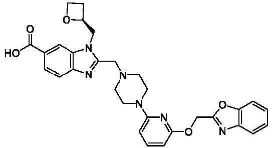 |
| 2-68 | 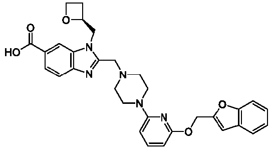 |
| 2-69 | 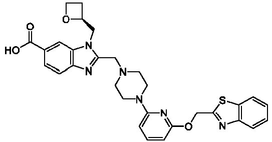 |
| 2-70 | 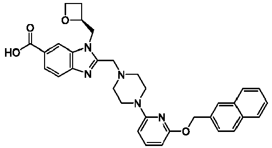 |
| 2-71 | 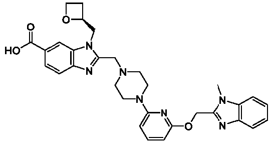 |
| 2-72 | 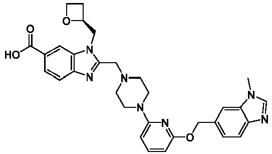 |
| 2-73 | 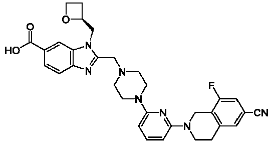 |
| 2-74 | 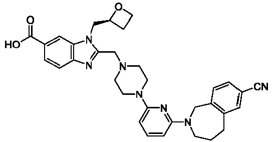 |
| 2-75 | 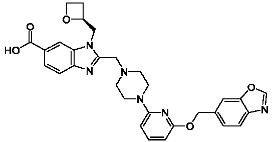 |
| 2-76 | 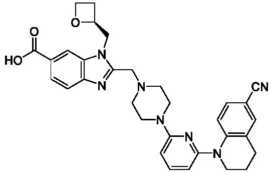 |
| 2-77 | 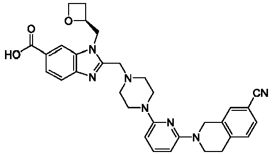 |
| 2-78 | 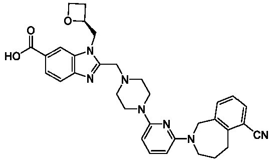 |
| 2-79 | 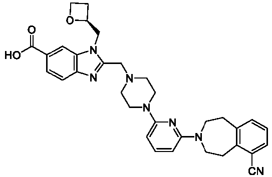 |
| 2-80 | 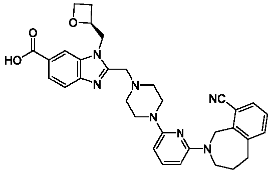 |
| 2-81 | 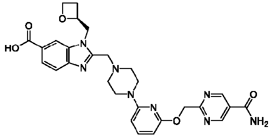 |
| 2-82 | 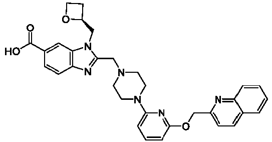 |
| 2-83 | 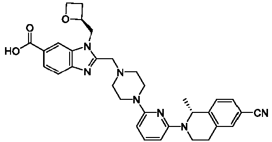 |
| 2-84 | 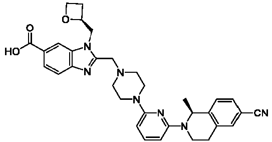 |
| 2-85 | 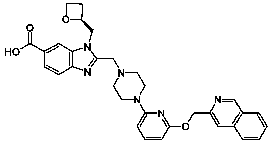 |
| 2-86 | 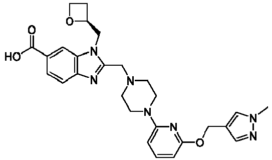 |
| 2-87 | 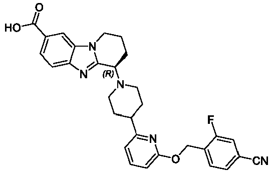 |
| 2-88 | 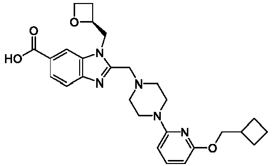 |
| 2-89 | 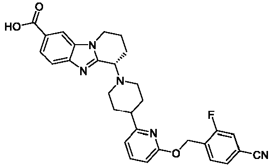 |
| 2-90 | 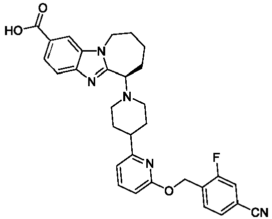 |
| 2-91 | 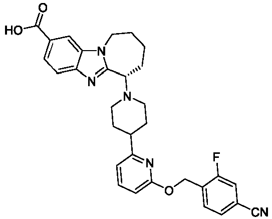 |
| 2-92 | 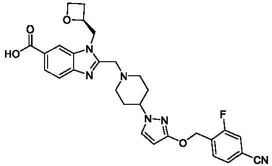 |
| 2-93 | 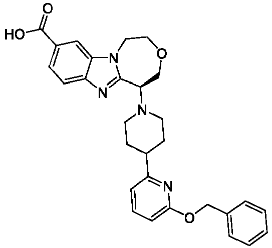 |
| 2-94 | 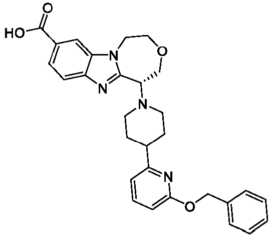 |
| 2-95 | 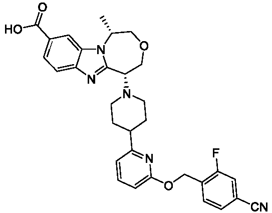 |
| 2-96 | 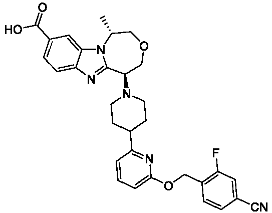 |
| 2-97 | 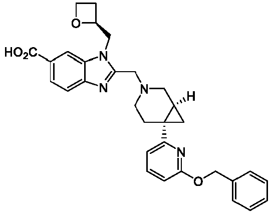 |
| 2-98 | 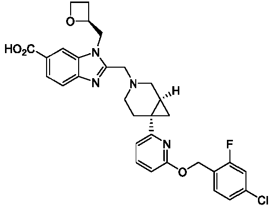 |
| 2-99 | 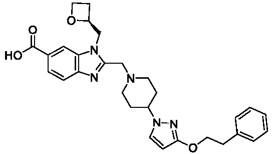 |
| 2-100 | 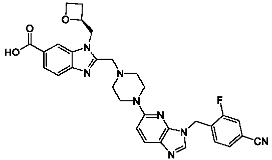 |
| 2-101 | 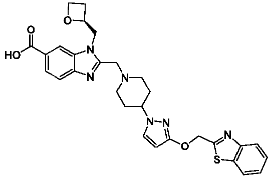 |
| 2-102 | 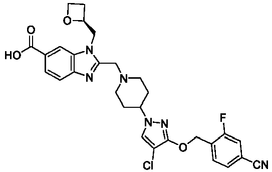 |
| 2-103 | 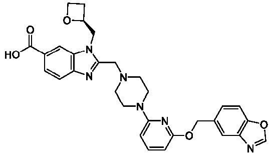 |
| 2-104-P1 | 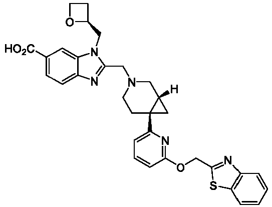 |
| 2-104-P2 | 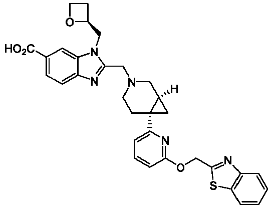 |
| 2-105 | 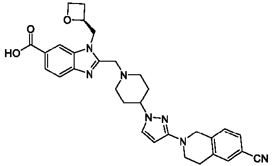 |
| 2-106 | 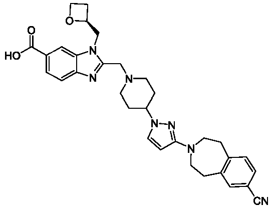 |
| 2-107 | 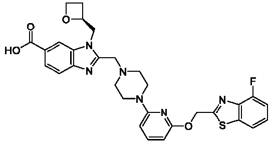 |
| 2-108 | 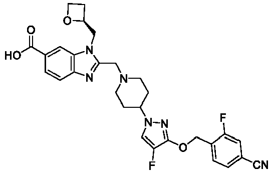 |
| 2-109 | 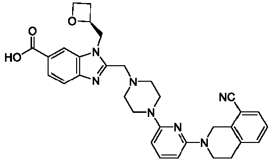 |
| 2-110 | 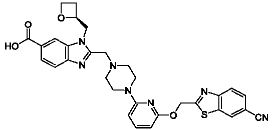 |
| 2-111 | 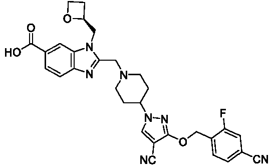 |
| 2-112 | 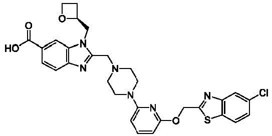 |
| 2-113 | 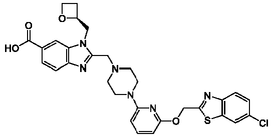 |
| 2-114 | 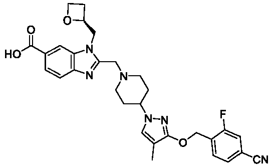 |
| 2-115 | 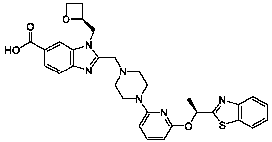 |
| 2-116 | 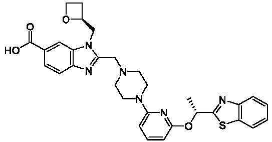 |
| 2-117 | 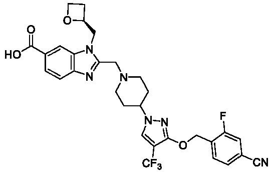 |
| 2-118 | 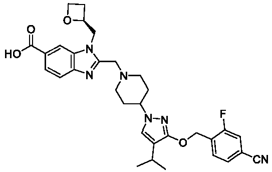 |
| 2-119 | 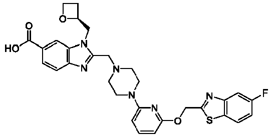 |
| 2-120 | 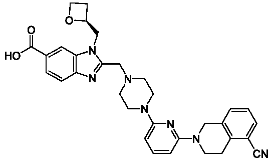 |
| 2-121-P1 | 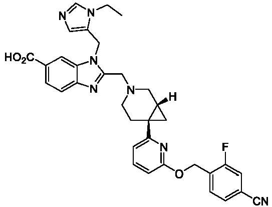 |
| 2-121-P2 | 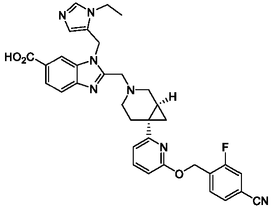 |
| 2-122 | 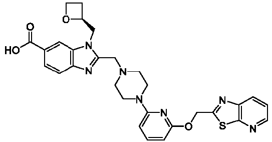 |
| 2-123 | 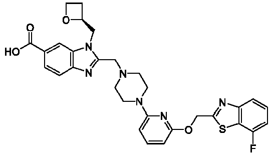 |
| 2-124 | 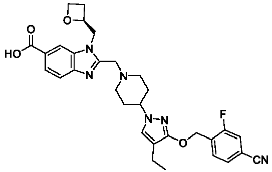 |
| 2-125 | 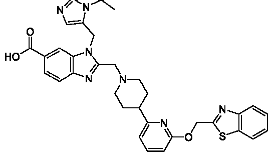 |
| 2-126-P1 | 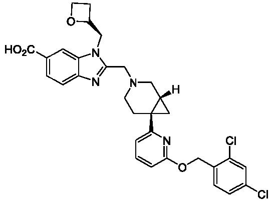 |
| 2-126-P2 | 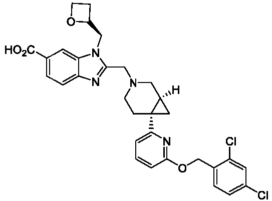 |
| 2-127 | 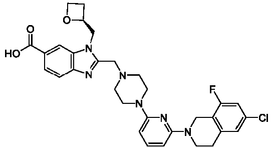 |
| 2-128 | 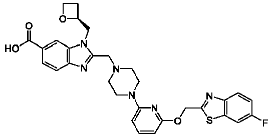 |
| 2-129 | 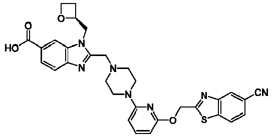 |
| 2-130 | 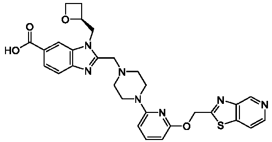 |
| 2-131 | 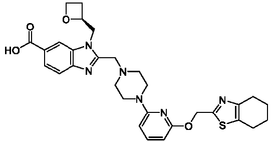 |
| 2-132 | 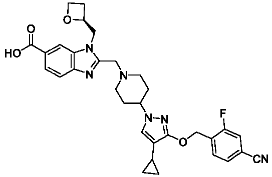 |
| 2-133 | 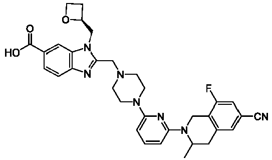 |
| 2-134 | 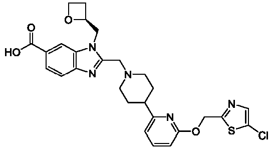 |
| 2-135 | 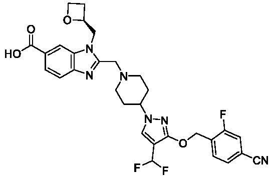 |
| 2-136 | 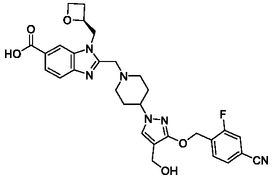 |
| 2-137 | 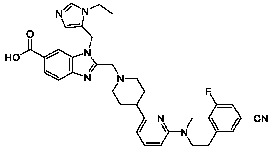 |
| 2-138 | 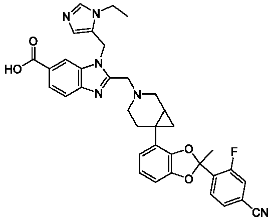 |
| 2-139 | 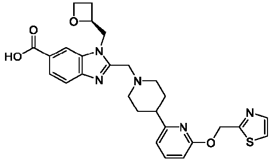 |
| 2-140-P1 | 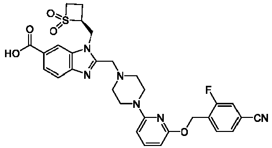 |
| 2-140-P2 | 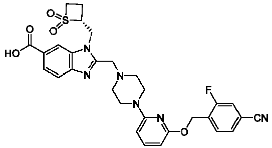 |
| 2-141-P1 | 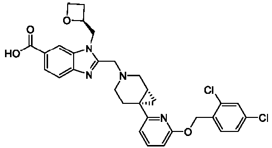 |
| 2-141-P2 | 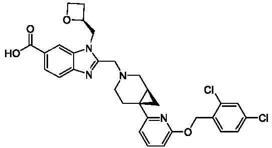 |
| 2-142 | 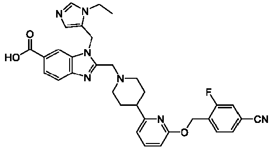 |
| 2-143 | 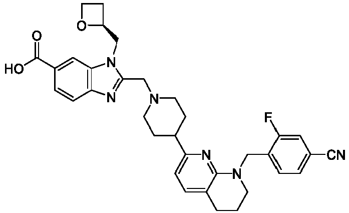 |
| 2-144 | 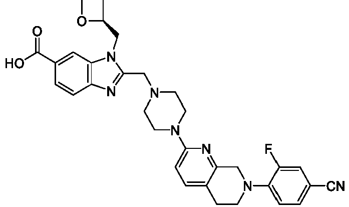 |
| 2-145 | 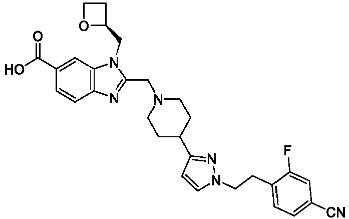 |
| 2-146-P1 | 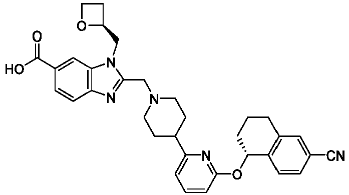 |
| 2-146-P2 | 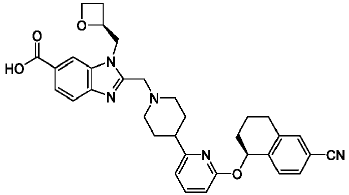 |
| 2-147 | 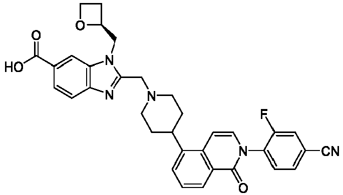 |
| 2-148 | 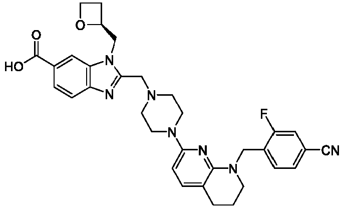 |
| 2-149 | 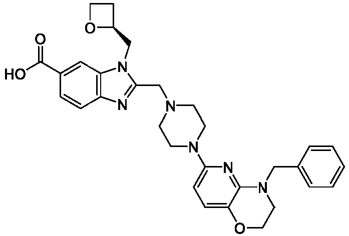 |
| 2-150 | 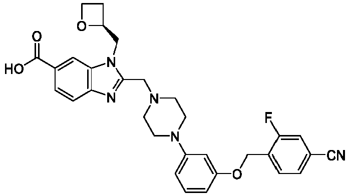 |
| 2-151-P1 | 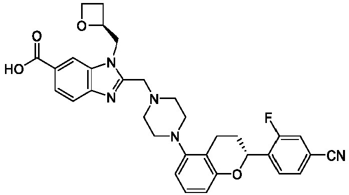 |
| 2-151-P2 | 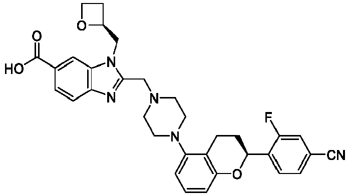 |
| 2-152 | 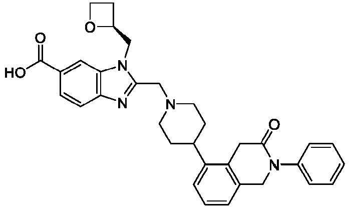 |
| 2-153 | 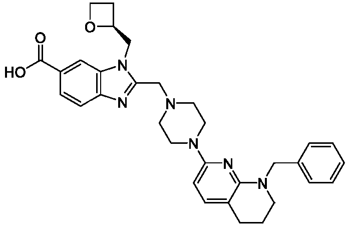 |
| 2-154 | 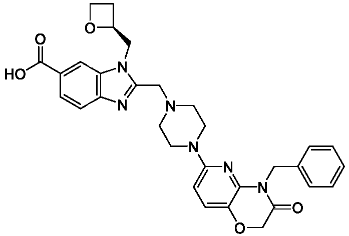 |
| 2-155 | 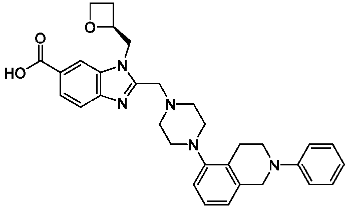 |
| 2-156 | 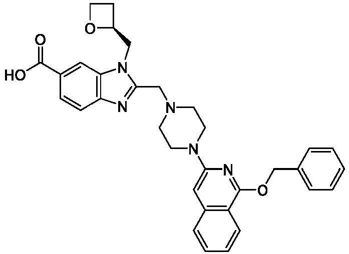 |
| 2-157 | 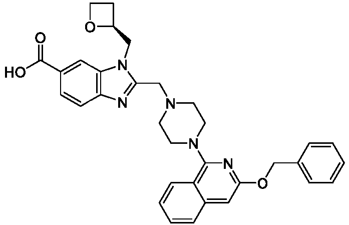 |
| 2-158 | 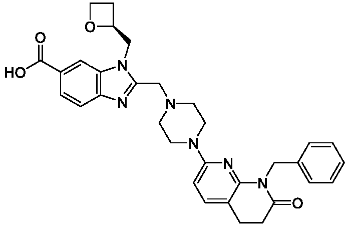 |
| 2-159 | 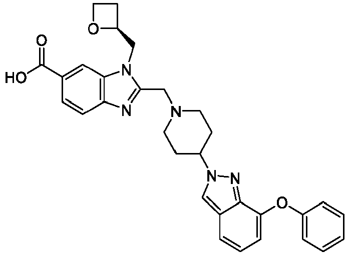 |
| 2-160 | 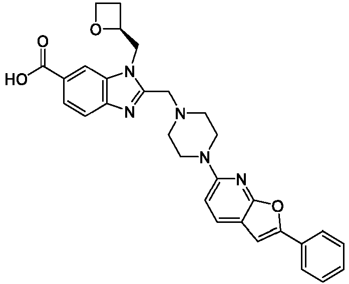 |
| 2-161 | 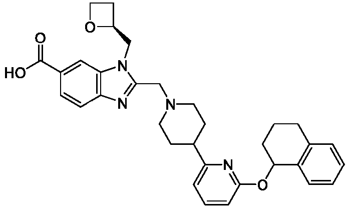 |
| 2-162 | 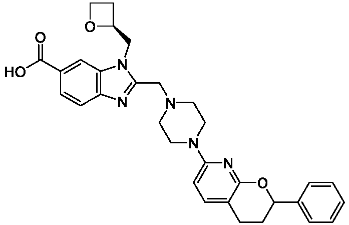 |
| 2-163-P1 | 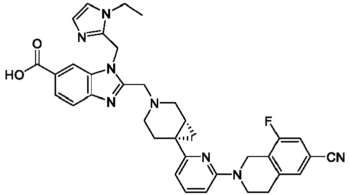 |
| 2-163-P2 | 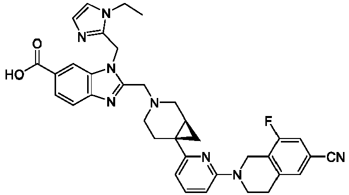 |
| 2-164 | 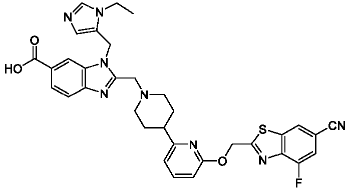 |
| 2-165 | 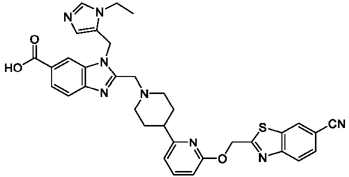 |
| 2-166 | 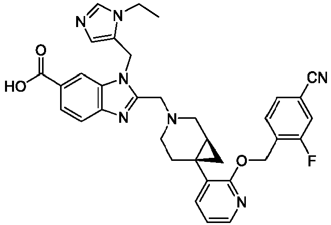 |
| 2-167 | 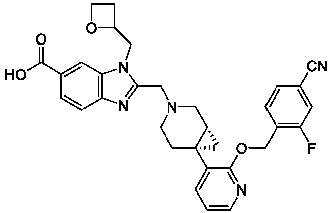 |
| 2-168 | 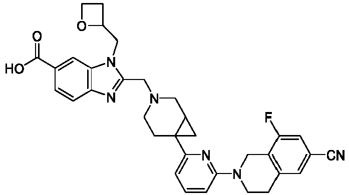 |
| 2-169 | 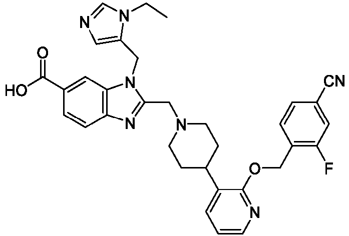 |
| 2-170 | 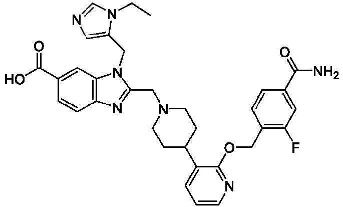 |
| 2-171 | 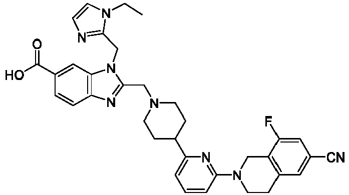 |
| 2-172 | 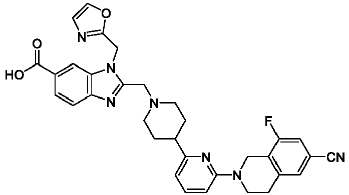 |
| 2-173 | 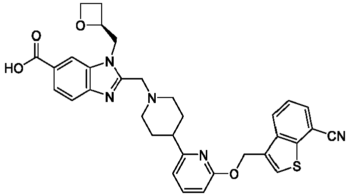 |
| 2-174 | 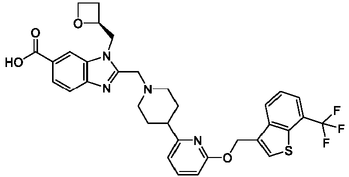 |
| 2-175 | 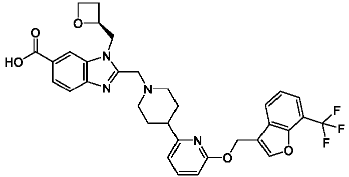 |
| 2-176 | 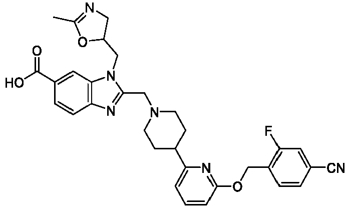 |
| 2-177 | 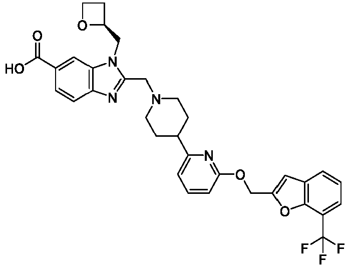 |
| 2-178 | 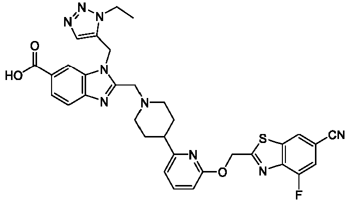 |
| 2-179 | 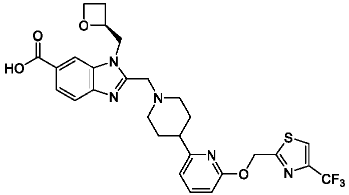 |
| 2-180 | 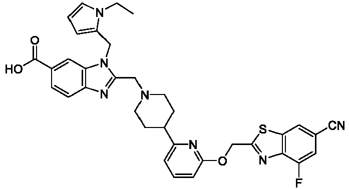 |
| 2-181 | 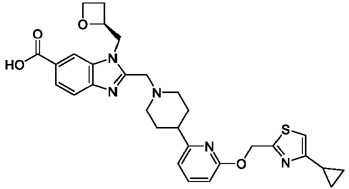 |
| 2-182 | 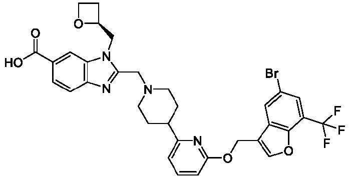 |
| 2-183 | 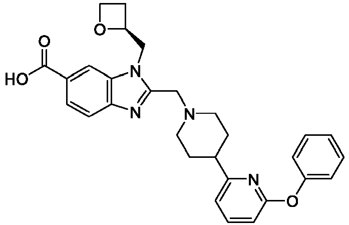 |
| 2-184-P1 | 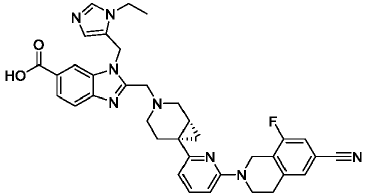 |
| 2-184-P2 | 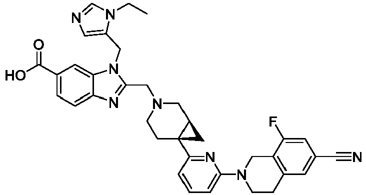 |
| 2-185-P1 | 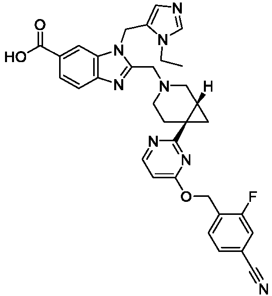 |
| 2-185-P2 | 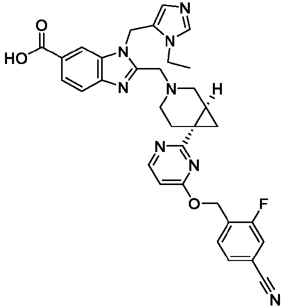 |
| 2-186-P1 | 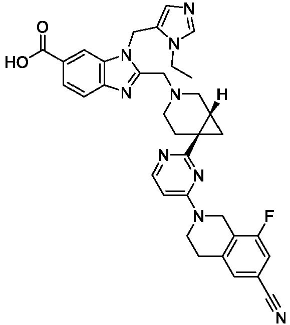 |
| 2-186-P2 | 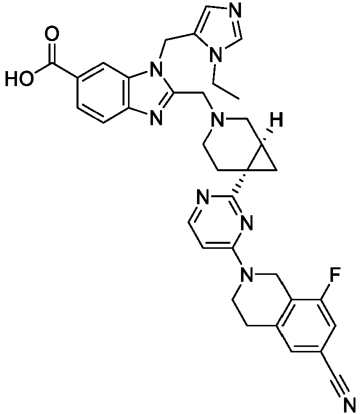 |
| 2-187 | 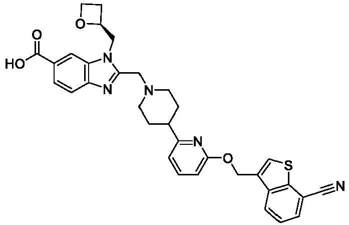 |

In some embodiments, the GLP-1R agonist is selected from those listed in Table 3 below, or a pharmaceutically acceptable salt thereof:

**Table 3**

| **Compound No.** | **Structure** | **Name** |
|---|---|---|
| 3-1 | 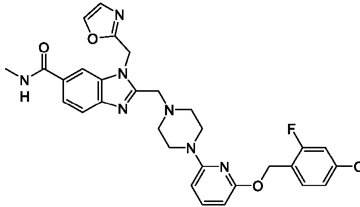 | 2-((4-(6-((4-cyano-2-fluorobenzyl)oxy)pyridin-2-yl)piperazin-1-yl)methyl)-N-methyl-1-(oxazol-2-ylmethyl)-1H-benzo[d]imidazole-6-carboxamide |
| 3-2 | 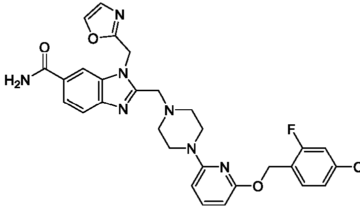 | 2-((4-(6-((4-cyano-2-fluorobenzyl)oxy)pyridin-2-yl)piperazin-1-yl)methyl)-1-(oxazol-2-ylmethyl)-1H-benzo[d]imidazole-6-carboxamide |
| 3-3 | 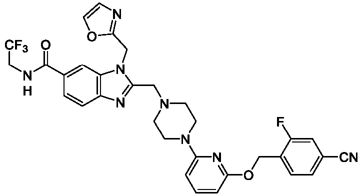 | 2-((4-(6-((4-cyano-2-fluorobenzyl)oxy)pyridin-2-yl)piperazin-1-yl)methyl)-1-(oxazol-2-ylmethyl)-N-(2,2,2-trifluoroethyl)-1H-benzo[d]imidazole-6-carboxamide |
| 3-4 | 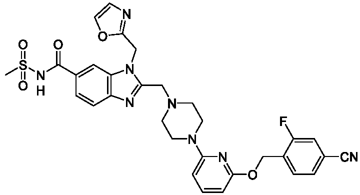 | 2-((4-(6-((4-cyano-2-fluorobenzyl)oxy)pyridin-2-yl)piperazin-1-yl)methyl)-N-(dioxo-15-sulfaneyl)-1-(oxazol-2-ylmethyl)-1H-benzo[d]imidazole-6-carboxamide |
| 3-5 | 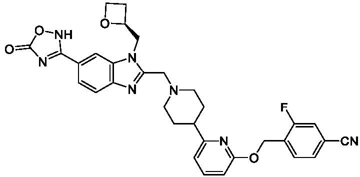 | (S)-3-fluoro-4-(((6-(1-((1-(oxetan-2-ylmethyl)-6-(5-oxo-2,5-dihydro-1,2,4-oxadiazol-3-yl)-1H-benzo[d]imidazol-2-yl)methyl)piperidin-4-yl)pyridin-2-yl)oxy)methyl)benzonitrile |
| 3-6 | 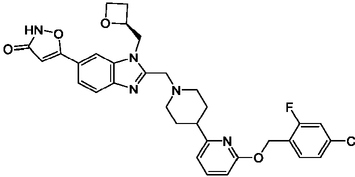 | (S)-5-(2-((4-(6-((4-chloro-2-fluorobenzyl)oxy)pyridin-2-yl)piperidin-1-yl)methyl)-1-(oxetan-2-ylmethyl)-1H-benzo[d]imidazol-6-yl)isoxazol-3(2H)-one |
| 3-7 | 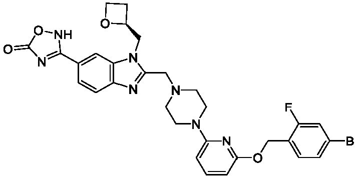 | (S)-3-(2-((4-(6-((4-bromo-2-fluorobenzyl)oxy)pyridin-2-yl)piperazin-1-yl)methyl)-1-(oxetan-2-ylmethyl)-1H-benzo[d]imidazol-6-yl)-1,2,4-oxadiazol-5(2H)-one |
| 3-8 | 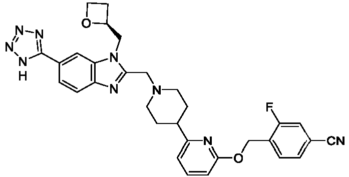 | (S)-3-fluoro-4-(((6-(1-((1-(oxetan-2-ylmethyl)-6-(1H-tetrazol-5-yl)-1H-benzo[d]imidazol-2-yl)methyl)piperidin-4-yl)pyridin-2-yl)oxy)methyl)benzonitrile |
| 3-9 | 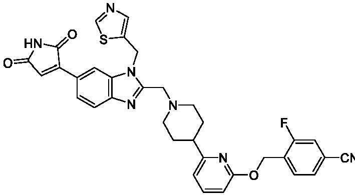 | 4-(((6-(1-((6-(2,5-dioxo-2,5-dihydro-1H-pyrrol-3-yl)-1-(thiazol-5-ylmethyl)-1H-benzo[d]imidazol-2-yl)methyl)piperidin-4-yl)pyridin-2-yl)oxy)methyl)-3-fluorobenzonitrile |
| 3-10 | 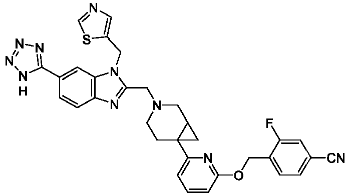 | 4-(((6-(3-((6-(1H-tetrazol-5-yl)-1-(thiazol-5-ylmethyl)-1H-benzo[d]imidazol-2-yl)methyl)-3-azabicyclo[4.1.0]heptan-6-yl)pyridin-2-yl)oxy)methyl)-3-fluorobenzonitrile |
| 3-11 | 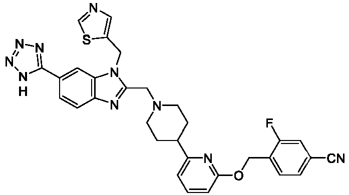 | 4-(((6-(1-((6-(1H-tetrazol-5-yl)-1-(thiazol-5-ylmethyl)-1H-benzo[d]imidazol-2-yl)methyl)piperidin-4-yl)pyridin-2-yl)oxy)methyl)-3-fluorobenzonitrile |
| 3-12 | 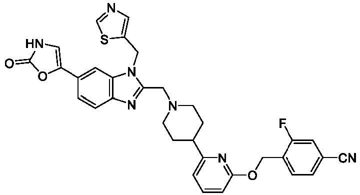 | 3-fluoro-4-(((6-(1-((6-(2-oxo-2,3-dihydrooxazol-5-yl)-1-(thiazol-5-ylmethyl)-1H-benzo[d]imidazol-2-yl)methyl)piperidin-4-yl)pyridin-2-yl)oxy)methyl)benzonitrile |
| 3-13 | 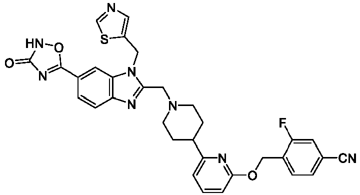 | 3-fluoro-4-(((6-(1-((6-(3-oxo-2,3-dihydro-1,2,4-oxadiazol-5-yl)-1-(thiazol-5-ylmethyl)-1H-benzo[d]imidazol-2-yl)methyl)piperidin-4-yl)pyridin-2-yl)oxy)methyl)benzonitrile |
| 3-14 | 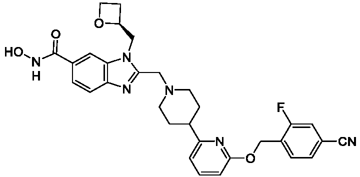 | (S)-2-((4-(6-((4-cyano-2-fluorobenzyl)oxy)pyridin-2-yl)piperidin-1-yl)methyl)-N-hydroxy-1-(oxetan-2-ylmethyl)-1H-benzo[d]imidazole-6-carboxamide |
| 3-15 | 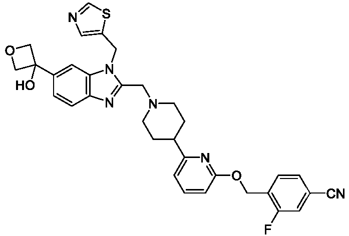 | 3-fluoro-4-(((6-(1-((6-(3-hydroxyoxetan-3-yl)-1-(thiazol-5-ylmethyl)-1H-benzo[d]imidazol-2-yl)methyl)piperidin-4-yl)pyridin-2-yl)oxy)methyl)benzonitrile |
| 3-16 | 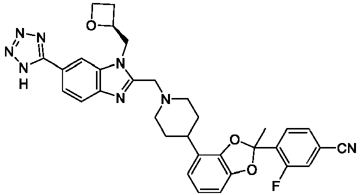 | 3-fluoro-4-(2-methyl-4-(1-((1-(((S)-oxetan-2-yl)methyl)-6-(1H-tetrazol-5-yl)-1H-benzo[d]imidazol-2-yl)methyl)piperidin-4-yl)benzo[d][1,3]dioxol-2-yl)benzonitrile |

In some embodiments, the GLP-1R agonist is selected from those listed in Table 4 below, or a pharmaceutically acceptable salt thereof:

**Table 4**

| **Compound No.** | **Structure** |
|---|---|
| 4-1 | 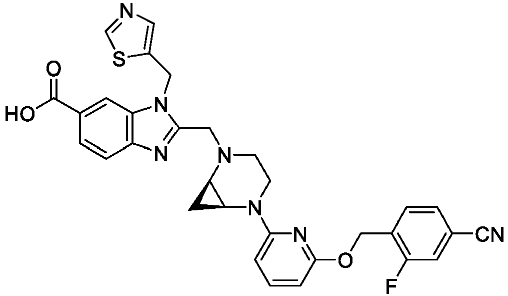 |
| 4-2 | 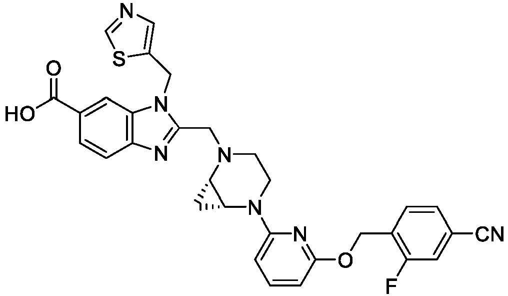 |
| 4-3 | 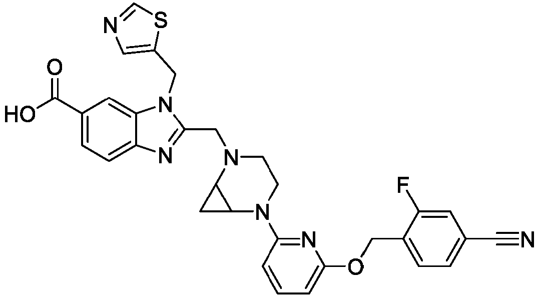 |
| 4-4 | 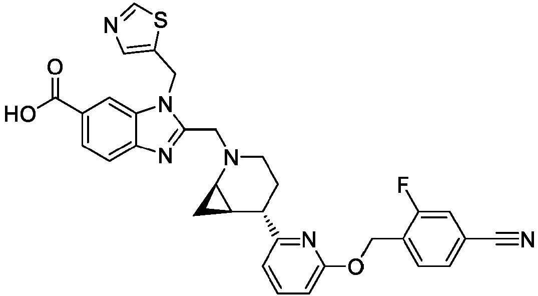 |
| 4-5 | 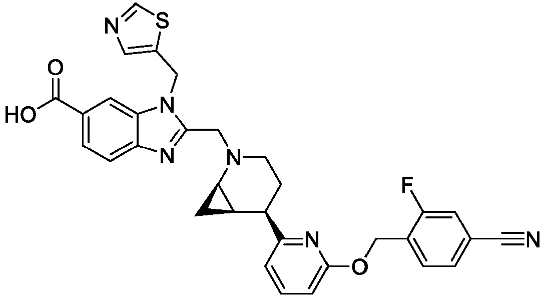 |
| 4-6 | 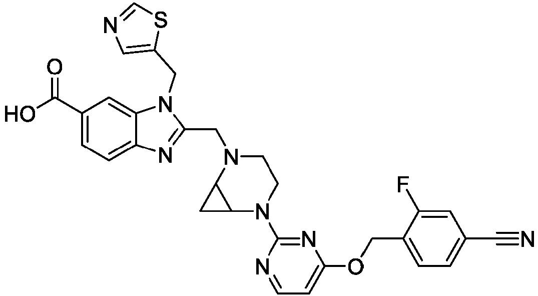 |
| 4-7 | 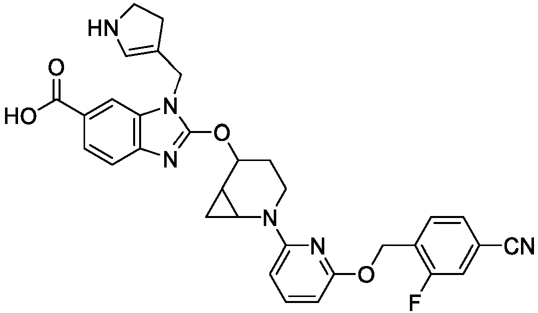 |
| 4-8 | 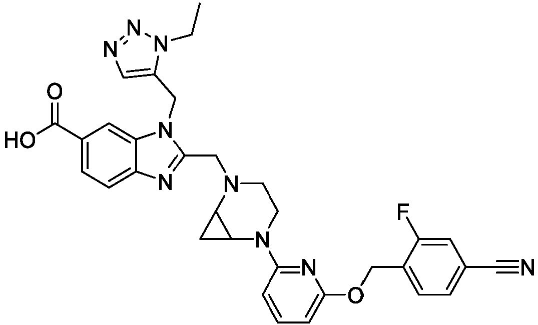 |
| 4-9 | 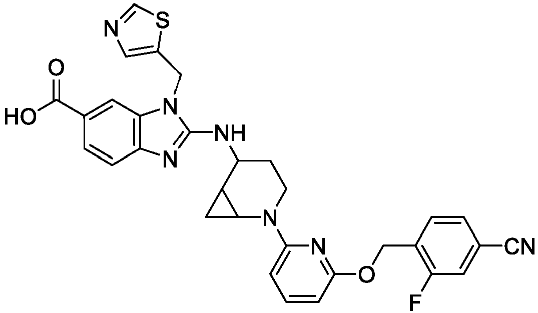 |
| 4-10 | 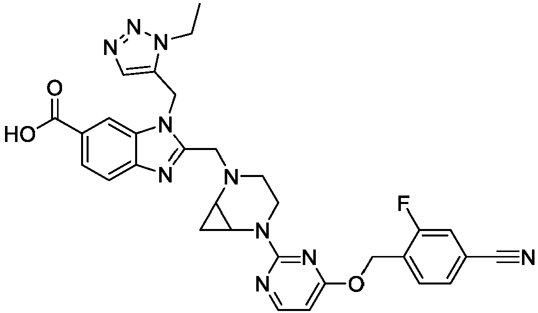 |
| 4-11 | 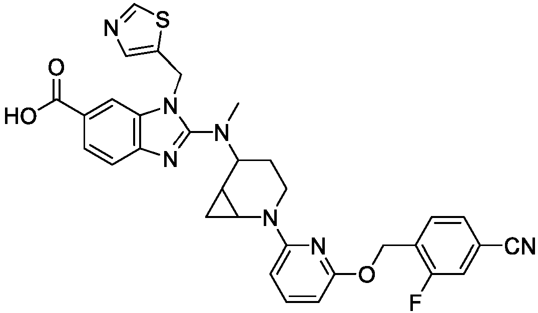 |
| 4-12 | 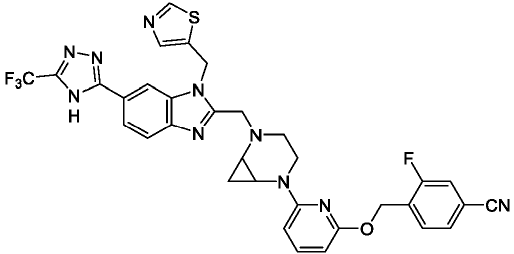 |
| 4-13 | 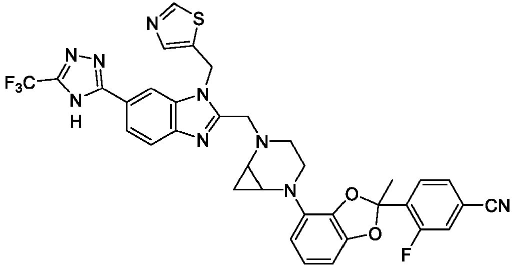 |
| 4-14 | 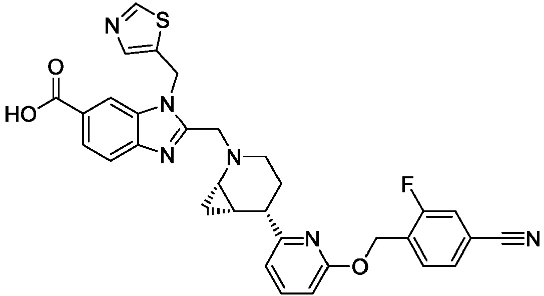 |
| 4-15 | 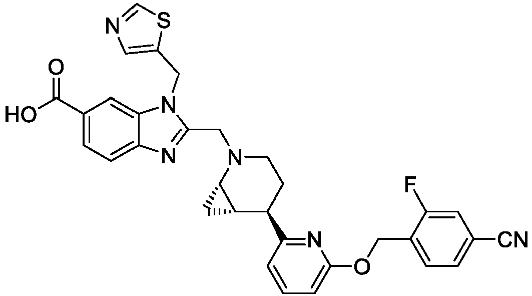 |
| 4-16 | 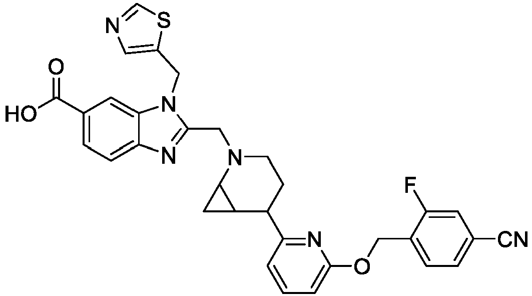 |
| 4-17 | 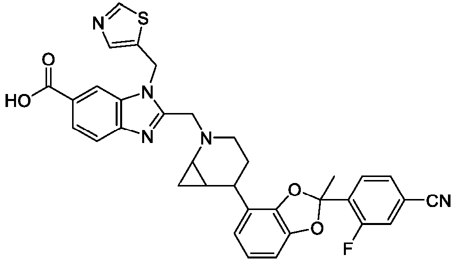 |
| 4-18 | 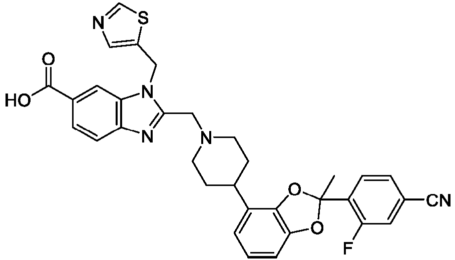 |
| 4-19 | 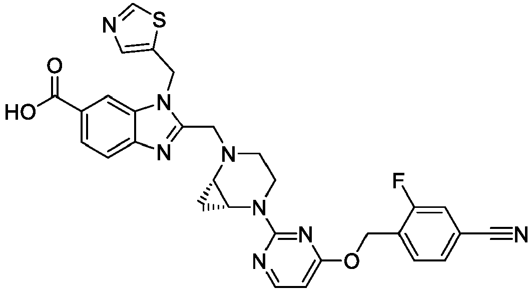 |
| 4-20 | 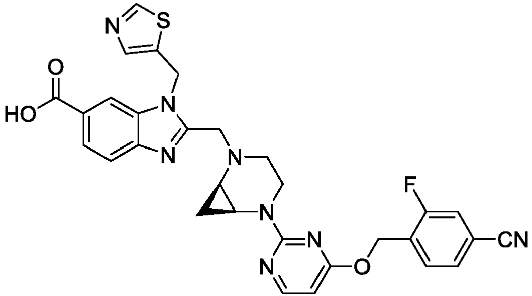 |
| 4-21 | 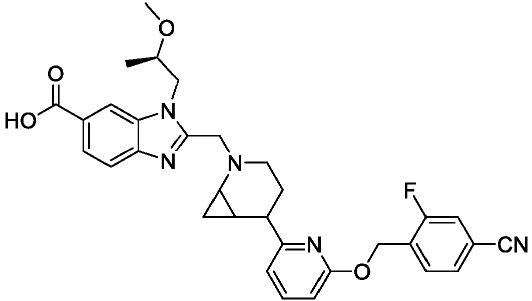 |
| 4-22 | 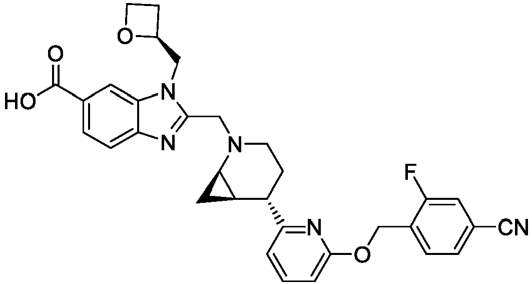 |
| 4-23 | 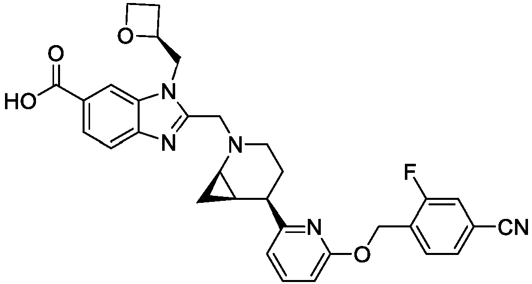 |
| 4-24 | 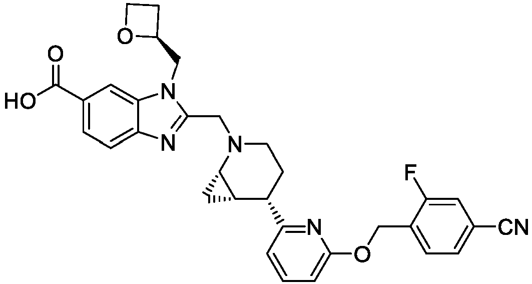 |
| 4-25 | 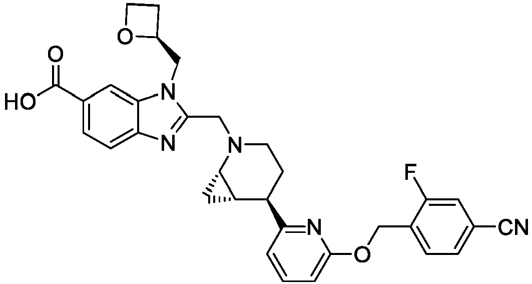 |
| 4-26 | 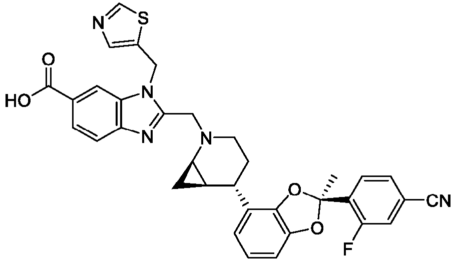 |
| 4-27 | 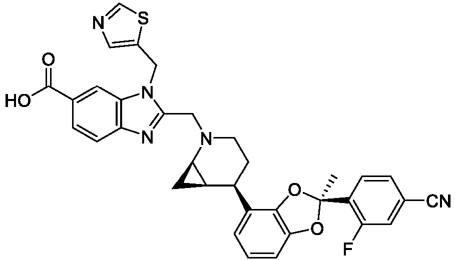 |
| 4-28 | 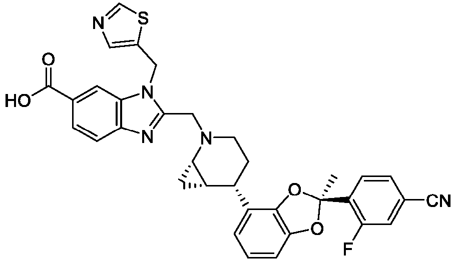 |
| 4-29 | 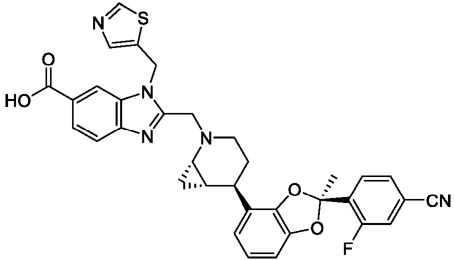 |
| 4-30 | 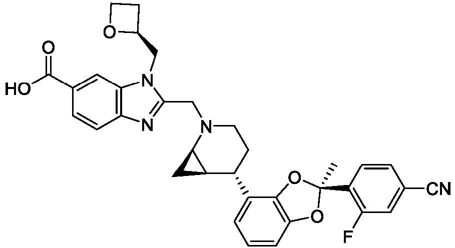 |
| 4-31 | 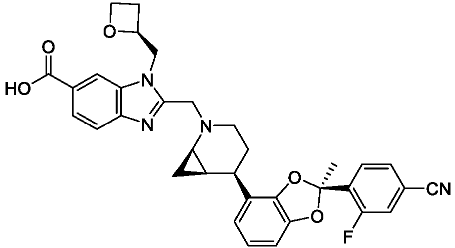 |
| 4-32 | 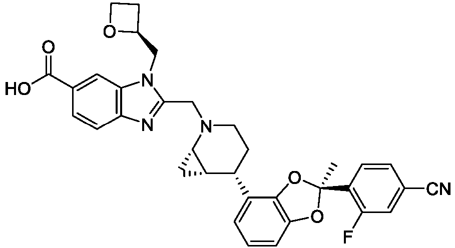 |
| 4-33 | 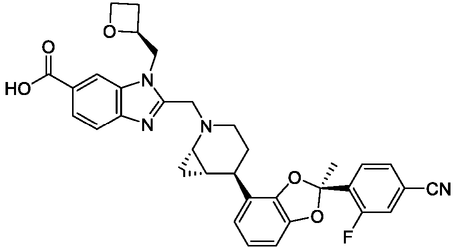 |
| 4-34 | 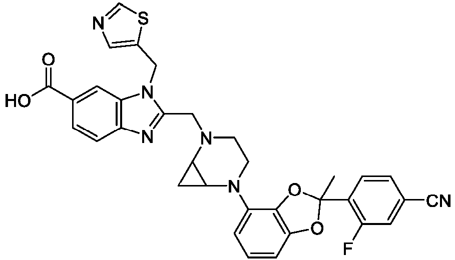 |
| 4-35 | 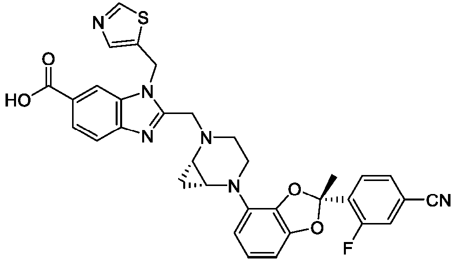 |
| 4-36 | 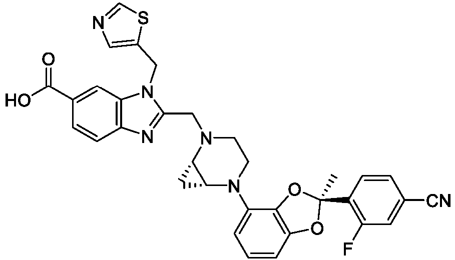 |
| 4-37 | 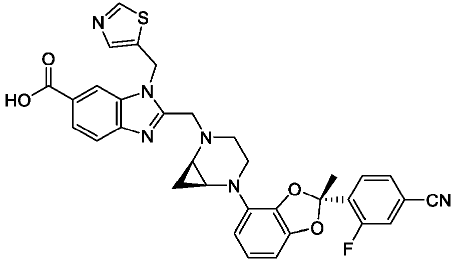 |
| 4-38 | 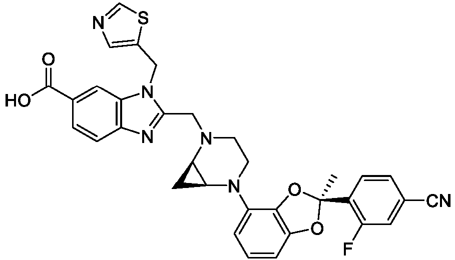 |
| 4-39 | 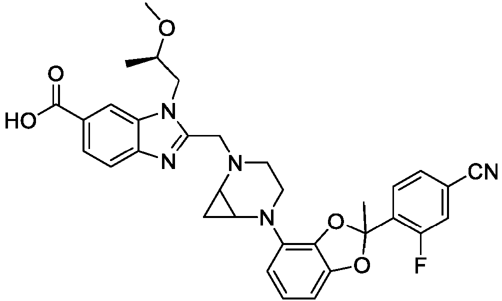 |
| 4-40 | 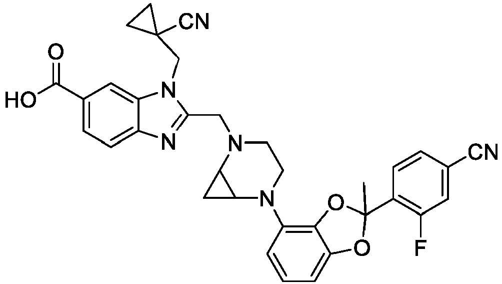 |
| 4-41 | 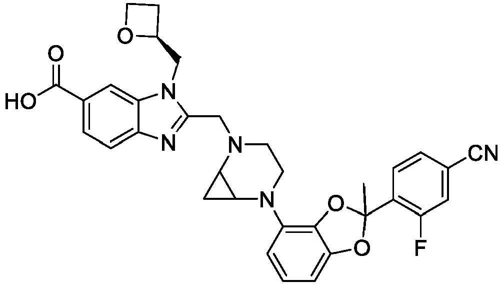 |
| 4-42 | 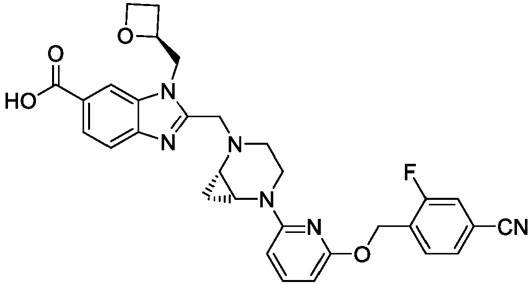 |
| 4-43 | 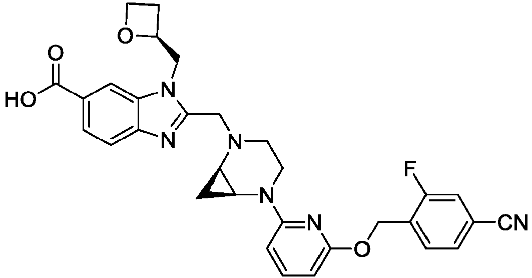 |
| 4-44 | 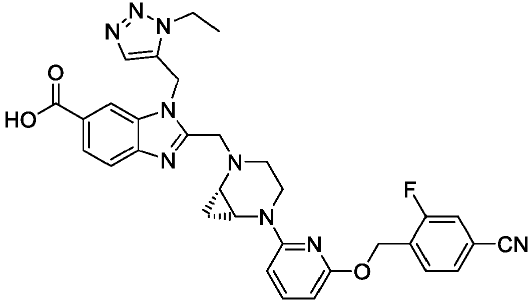 |
| 4-45 | 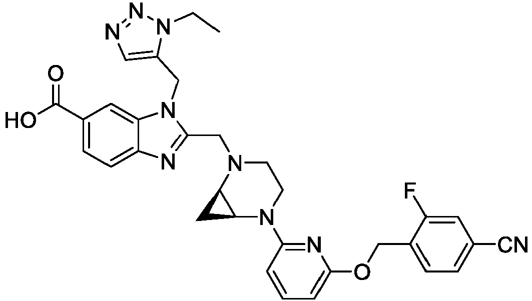 |
| 4-46 | 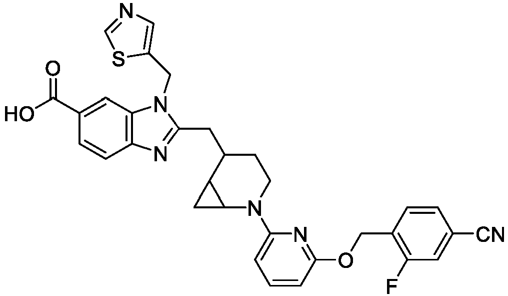 |

In some embodiments, the GLP-1R agonist is selected from those listed in Table 5 below, or a pharmaceutically acceptable salt thereof:

**Table 5**

| Compound No. | Structure |
|---|---|
| 5-1 | 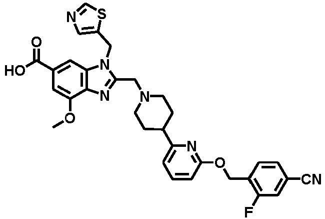 |
| 5-2 | 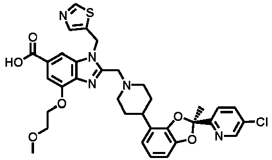 |
| 5-3 | 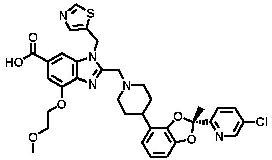 |
| 5-4 | 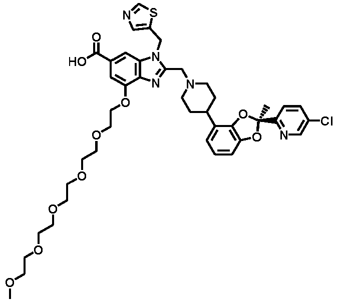 |
| 5-5 | 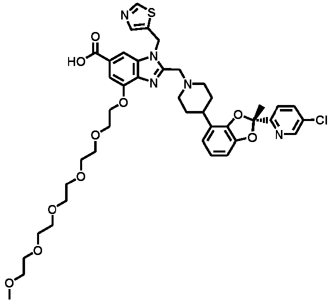 |
| 5-6 | 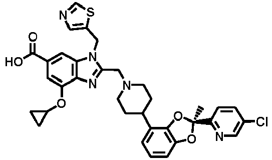 |
| 5-7 | 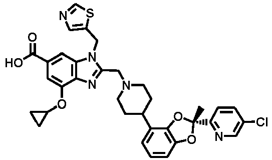 |
| 5-8 | 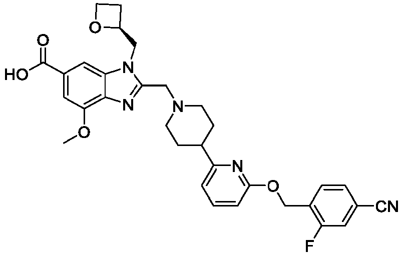 |
| 5-9 | 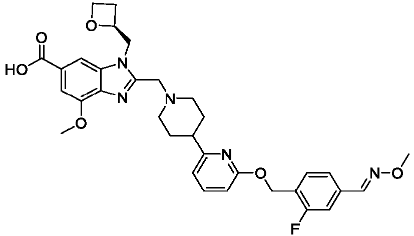 |
| 5-10 | 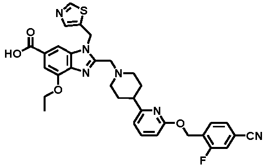 |
| 5-11 | 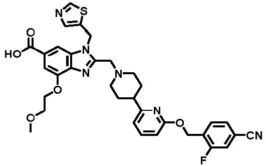 |
| 5-12 | 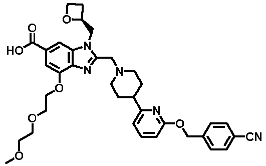 |
| 5-13 | 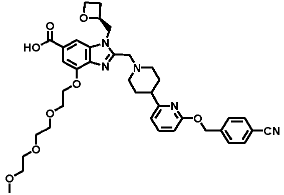 |
| 5-14 | 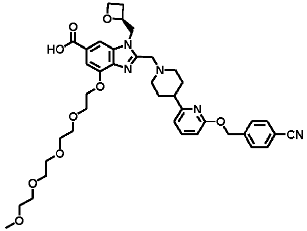 |
| 5-15 | 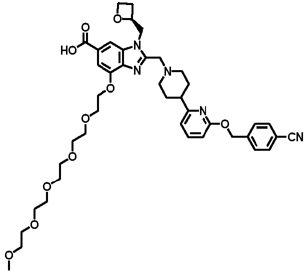 |
| 5-16 | 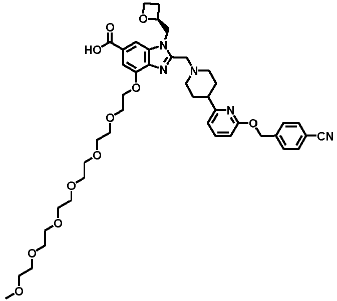 |
| 5-17 | 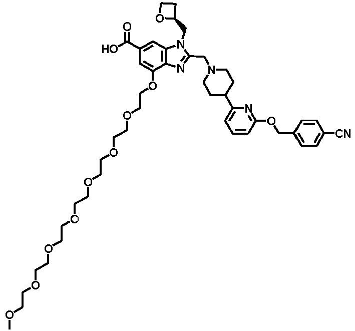 |
| 5-18 | 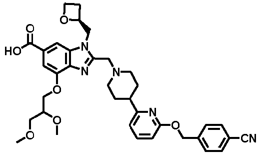 |
| 5-19 | 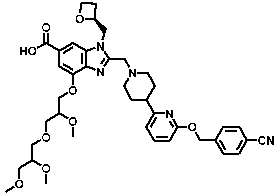 |
| 5-20 | 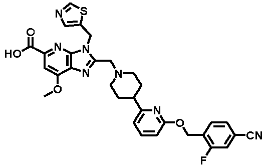 |
| 5-21 | 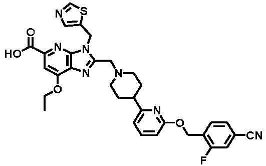 |
| 5-22 | 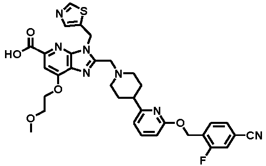 |
| 5-23 | 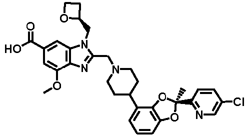 |
| 5-24 | 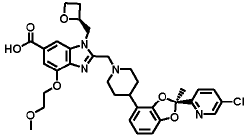 |
| 5-25 | 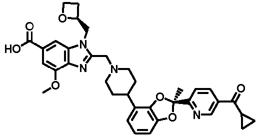 |
| 5-26 | 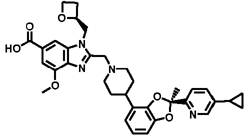 |
| 5-27 | 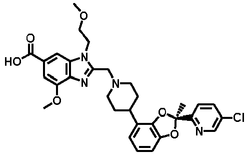 |
| 5-28 | 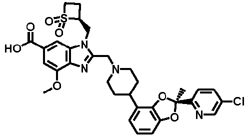 |
| 5-29 | 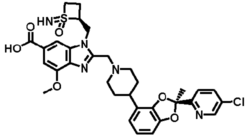 |
| 5-30 | 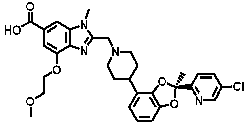 |
| 5-31 | 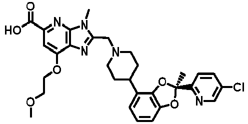 |
| 5-32 | 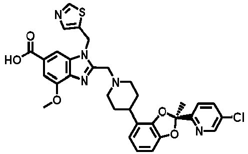 |
| 5-33 | 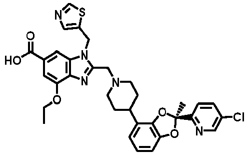 |
| 5-34 | 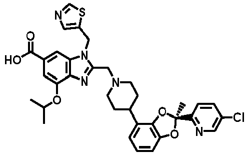 |
| 5-35 | 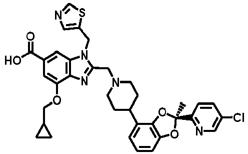 |
| 5-36 | 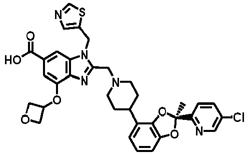 |
| 5-37 | 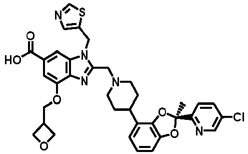 |
| 5-38 | 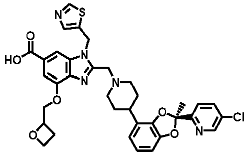 |
| 5-39 | 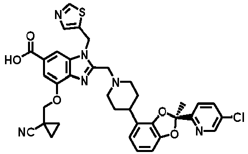 |
| 5-40 | 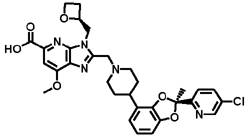 |
| 5-41 | 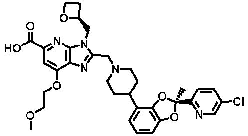 |
| 5-42 | 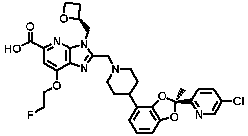 |
| 5-43 | 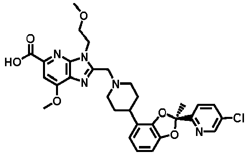 |
| 5-44 | 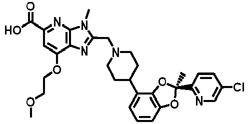 |
| 5-45 | 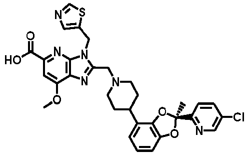 |
| 5-46 | 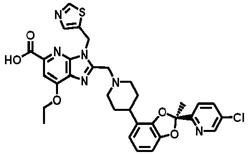 |
| 5-47 | 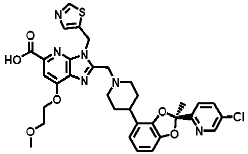 |
| 5-48 | 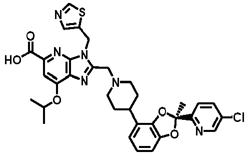 |
| 5-49 | 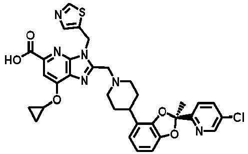 |
| 5-50 | 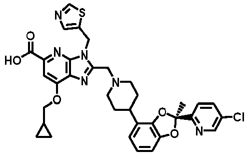 |
| 5-51 | 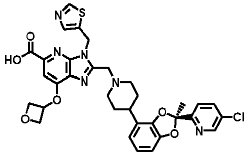 |
| 5-52 | 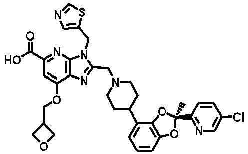 |
| 5-53 | 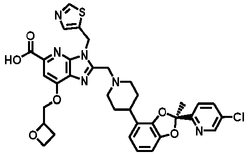 |
| 5-54 | 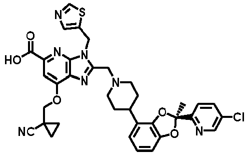 |
| 5-55 | 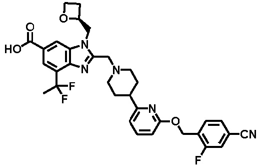 |
| 5-56 | 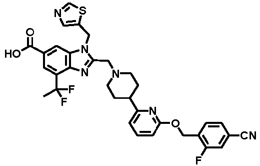 |
| 5-57 | 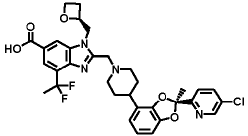 |
| 5-58 | 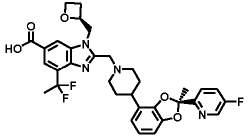 |
| 5-59 | 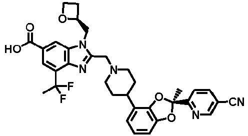 |
| 5-60 | 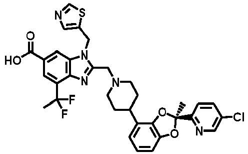 |
| 5-61 | 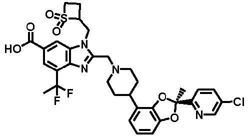 |
| 5-62 | 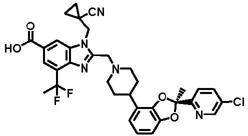 |
| 5-63 | 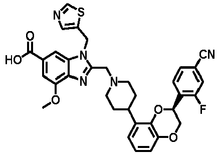 |
| 5-64 | 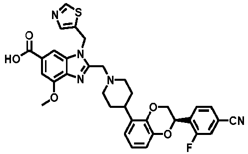 |
| 5-65 | 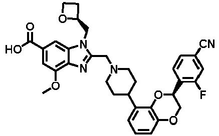 |
| 5-66 | 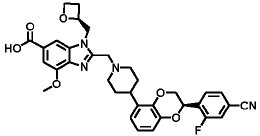 |
| 5-67 | 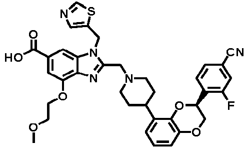 |
| 5-68 | 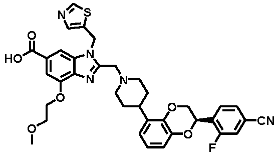 |
| 5-69 | 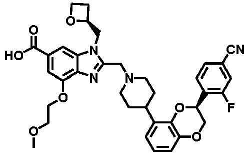 |
| 5-70 | 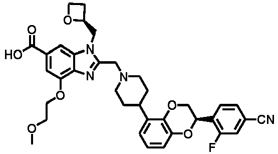 |
| 5-71 | 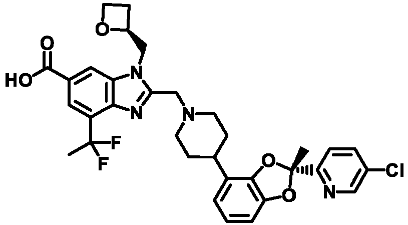 |
| 5-72 | 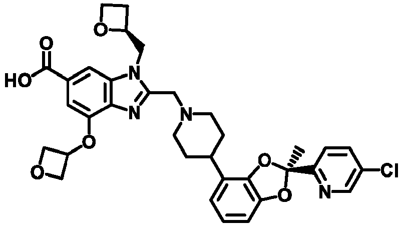 |
| 5-73 | 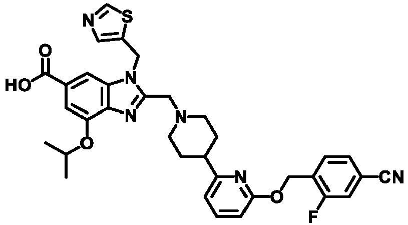 |
| 5-74 | 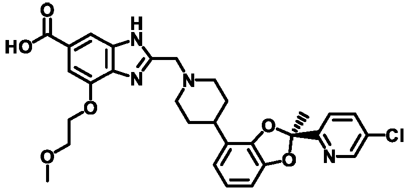 |
| 5-75 | 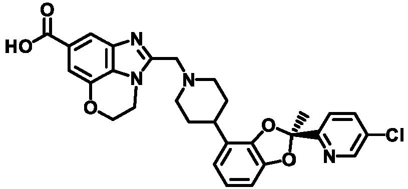 |
| 5-76 | 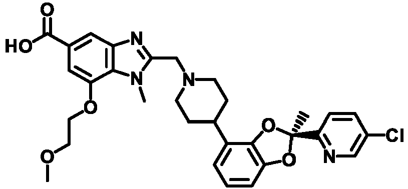 |
| 5-77 | 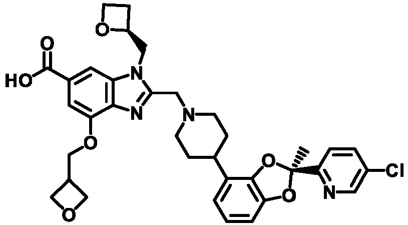 |
| 5-78 | 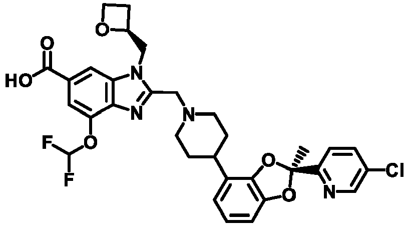 |
| 5-79 | 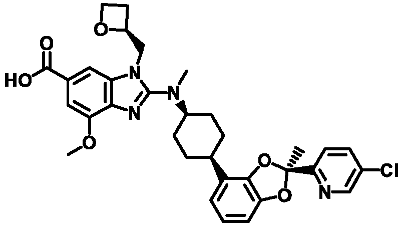 |
| 5-80 | 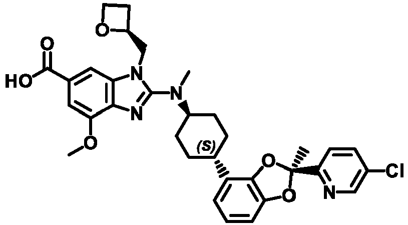 |
| 5-81 | 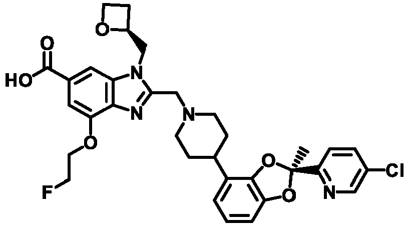 |
| 5-82 | 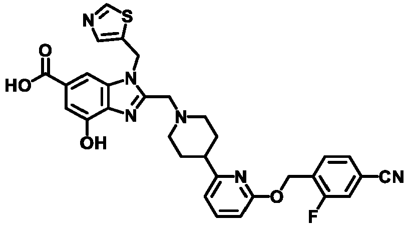 |
| 5-83 | 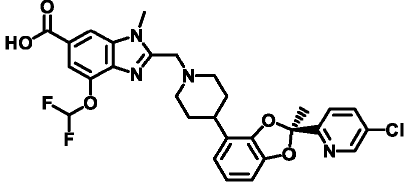 |
| 5-84 | 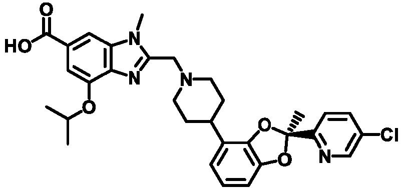 |
| 5-85 | 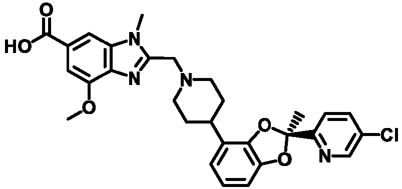 |
| 5-86 | 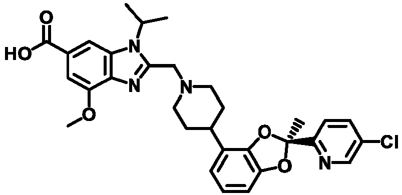 |
| 5-87 | 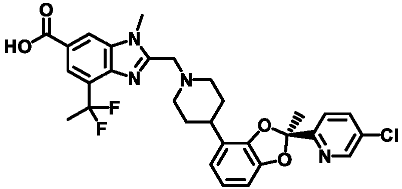 |
| 5-88 | 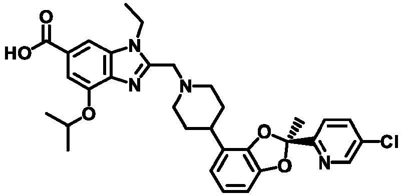 |
| 5-89 | 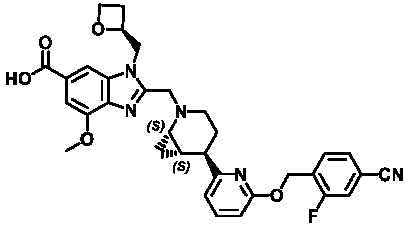 |
| 5-90 | 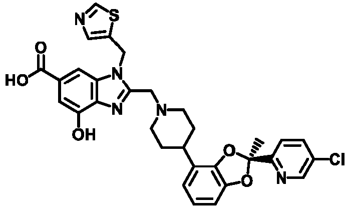 |
| 5-91 | 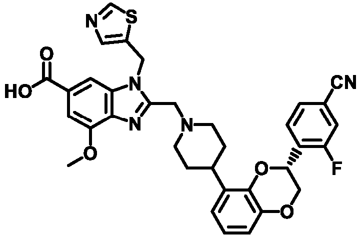 |
| 5-92 | 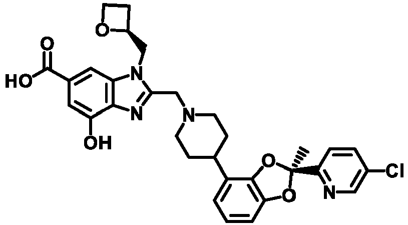 |
| 5-93 | 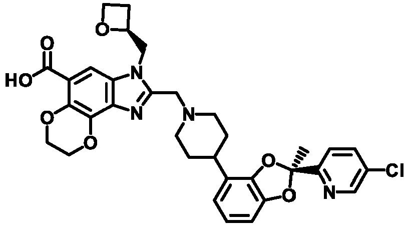 |
| 5-94 | 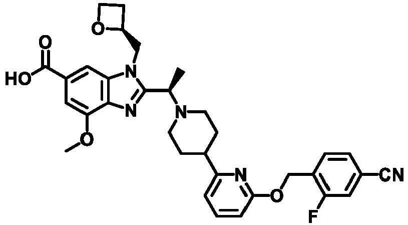 |
| 5-95 | 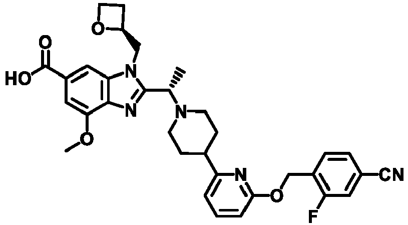 |
| 5-96 | 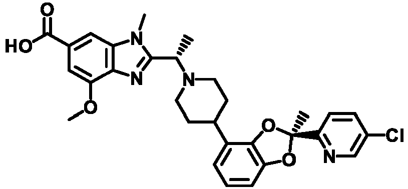 |
| 5-97 | 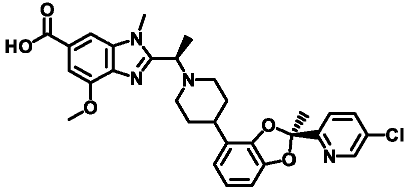 |
| 5-98 | 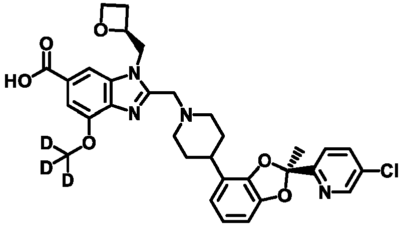 |
| 5-99 | 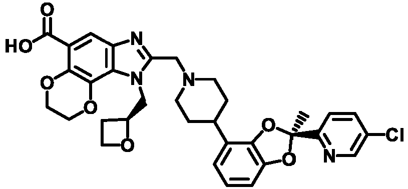 |
| 5-100 | 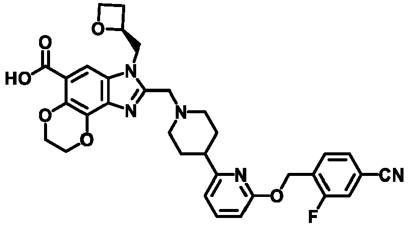 |
| 5-101 | 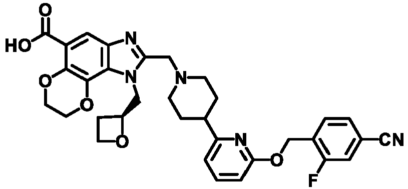 |
| 5-102 | 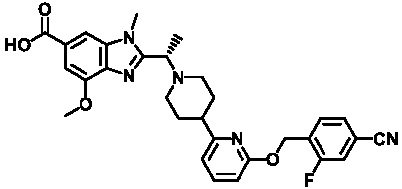 |
| 5-103 | 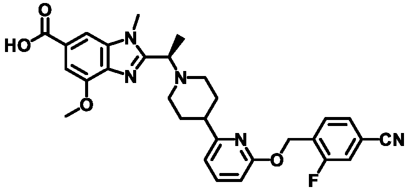 |
| 5-104 | 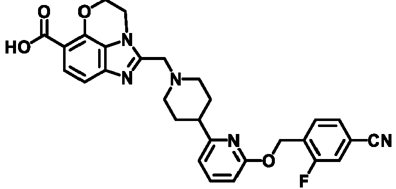 |
| 5-105 | 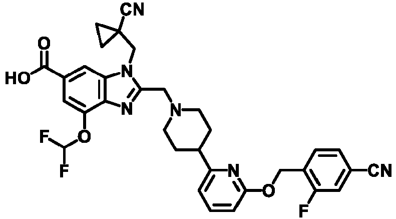 |
| 5-106 | 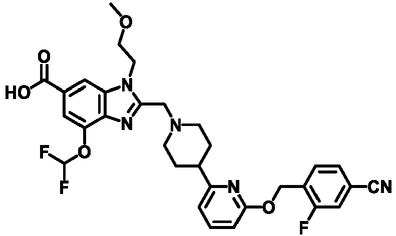 |
| 5-107 | 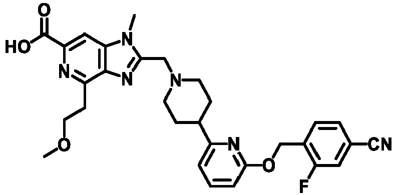 |
| 5-108 | 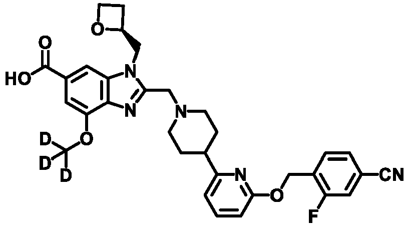 |
| 5-109 | 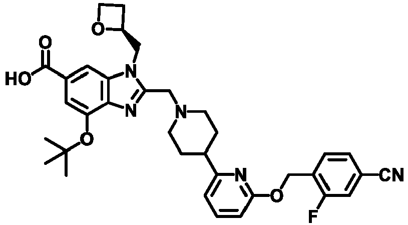 |
| 5-110 | 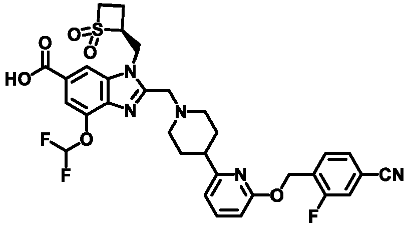 |
| 5-111 | 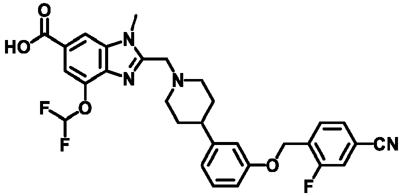 |
| 5-112 | 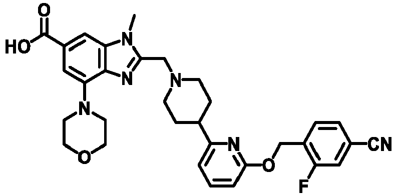 |
| 5-113 | 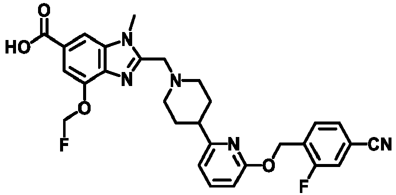 |
| 5-114 | 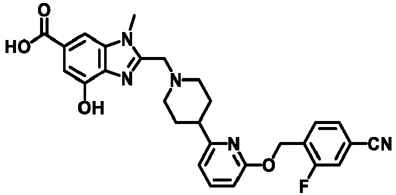 |
| 5-115 | 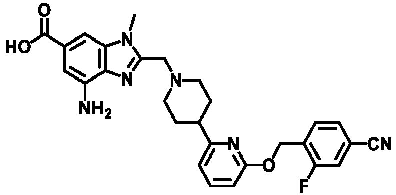 |
| 5-116 | 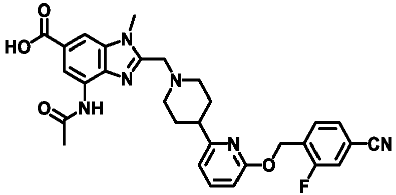 |
| 5-117 | 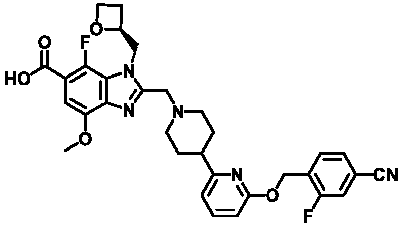 |
| 5-118 | 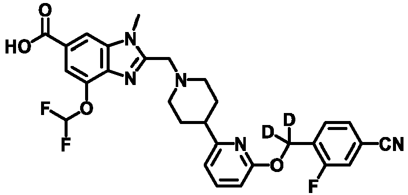 |
| 5-119 | 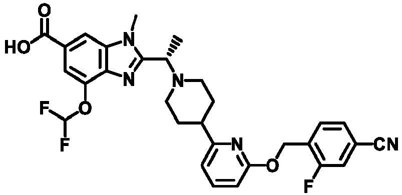 |
| 5-120 | 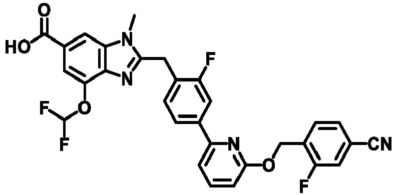 |
| 5-121 | 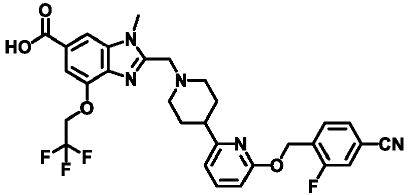 |
| 5-122 | 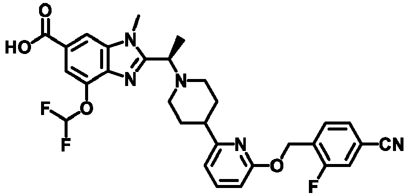 |
| 5-123 | 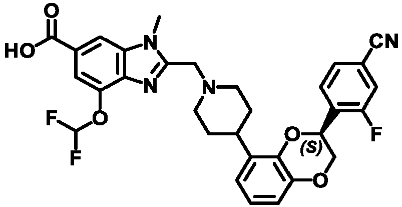 |
| 5-124 | 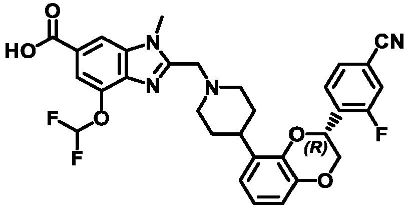 |
| 5-125 | 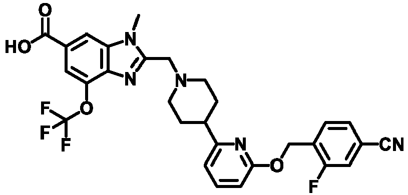 |
| 5-126 | 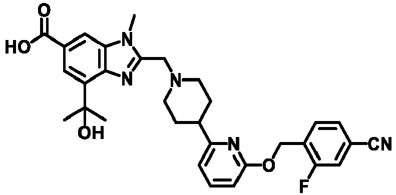 |
| 5-127 | 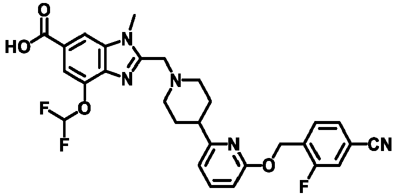 |
| 5-128 | 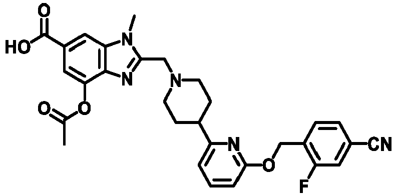 |
| 5-129 | 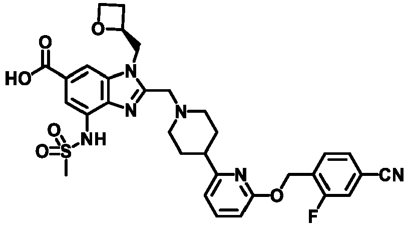 |
| 5-130 | 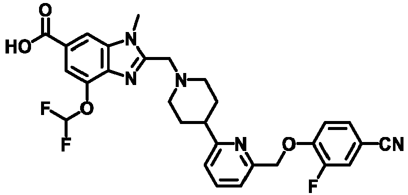 |
| 5-131 | 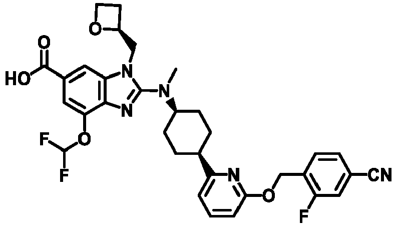 |
| 5-132 | 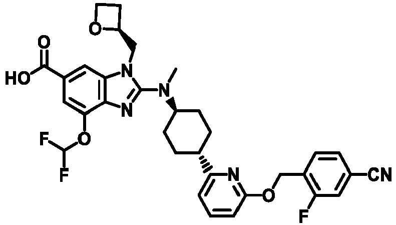 |
| 5-133 | 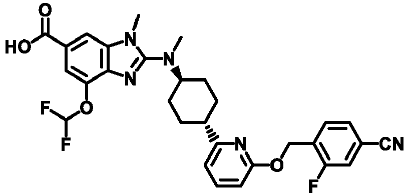 |
| 5-134 | 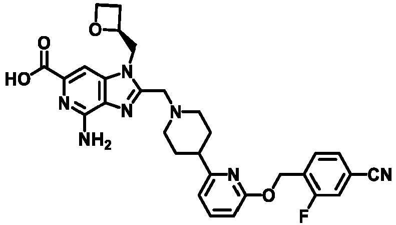 |
| 5-135 | 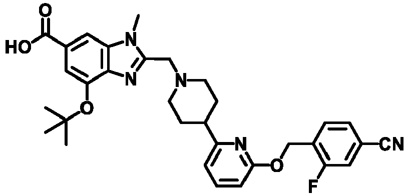 |
| 5-136 | 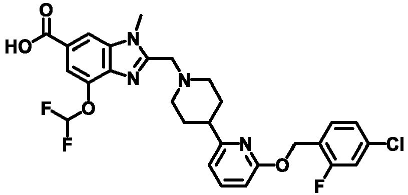 |
| 5-137 | 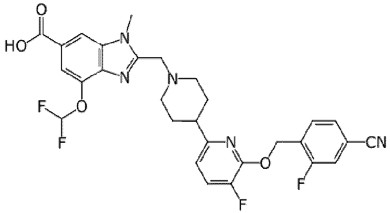 |
| 5-138 | 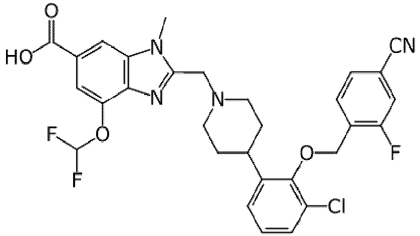 |
| 5-139 | 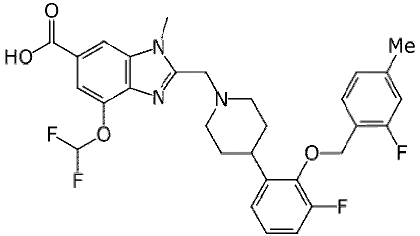 |
| 5-140 | 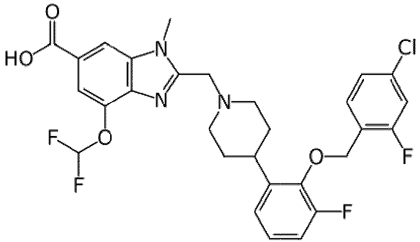 |
| 5-141 | 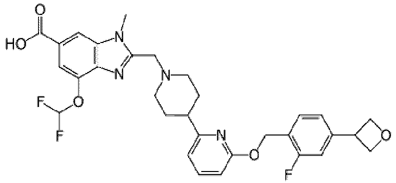 |
| 5-142 | 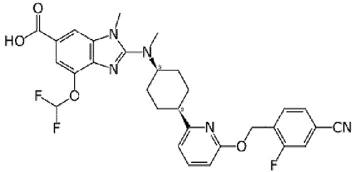 |
| 5-143 | 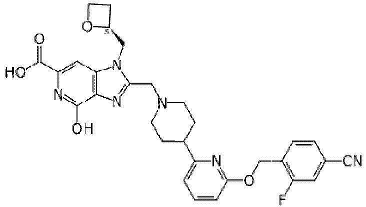 |
| 5-144 | 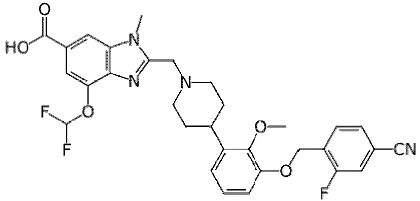 |
| 5-145 | 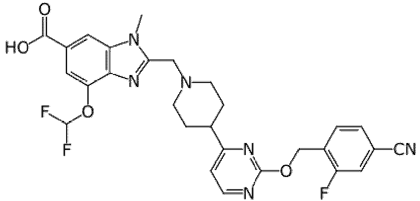 |
| 5-146 | 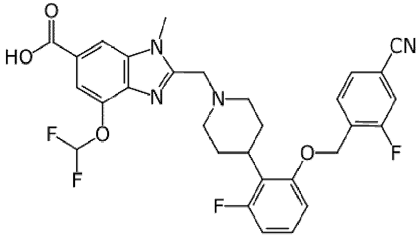 |
| 5-147 | 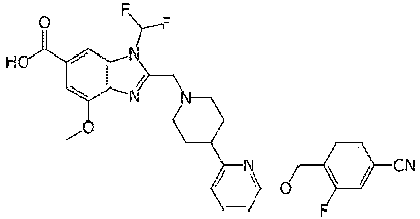 |
| 5-148 | 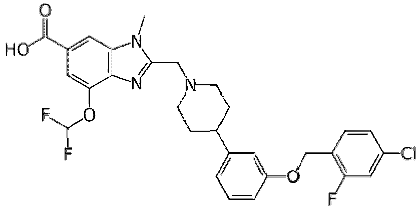 |
| 5-149 | 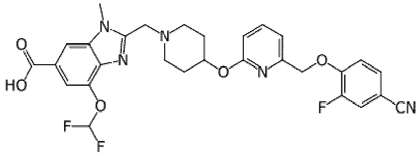 |
| 5-150 | 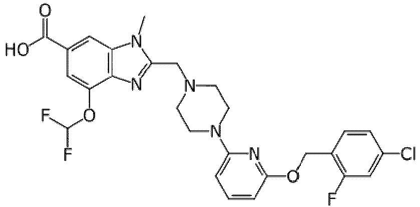 |
| 5-151 | 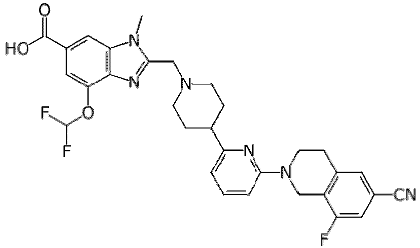 |
| 5-152 | 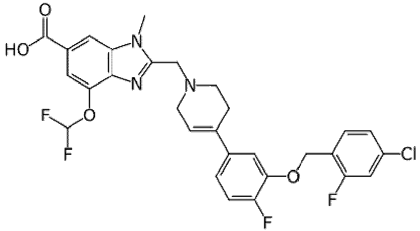 |
| 5-153 | 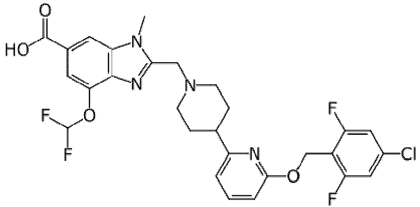 |
| 5-154 | 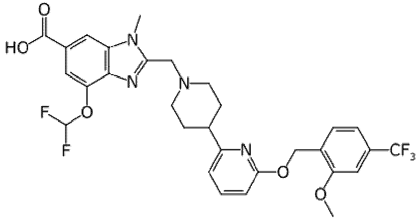 |
| 5-155 | 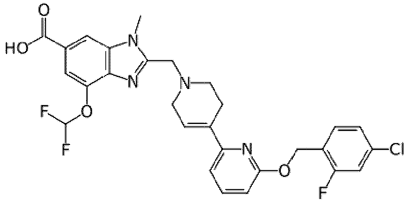 |
| 5-156 | 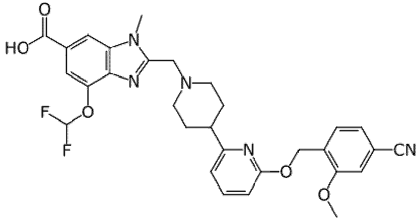 |
| 5-157 | 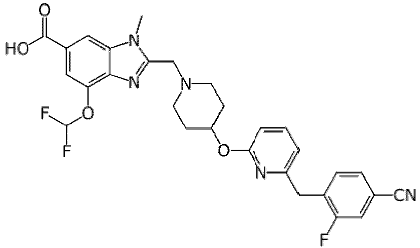 |
| 5-158 | 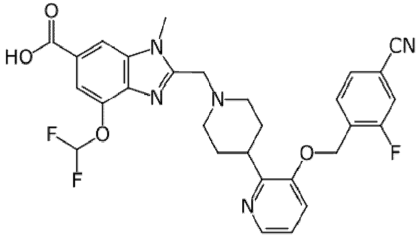 |
| 5-159 | 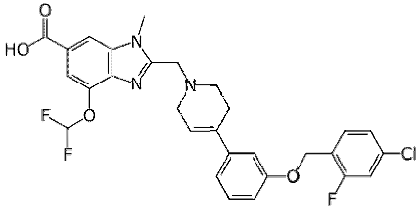 |
| 5-160 | 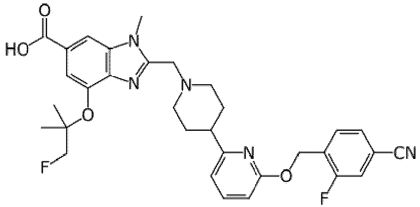 |
| 5-161 | 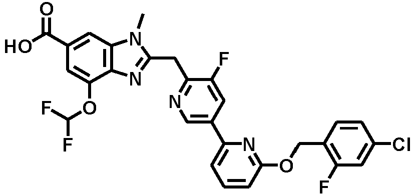 |
| 5-162 | 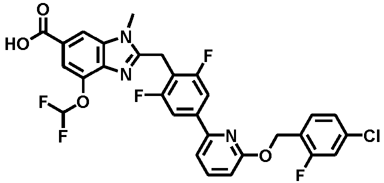 |
| 5-163 | 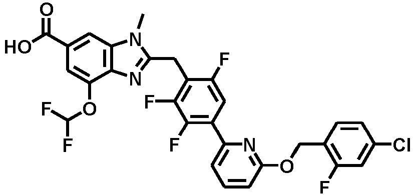 |
| 5-164 | 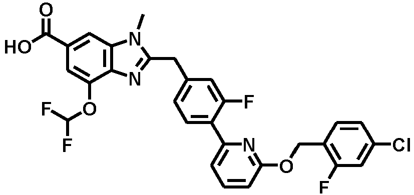 |
| 5-165 | 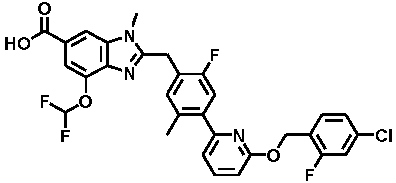 |
| 5-166 | 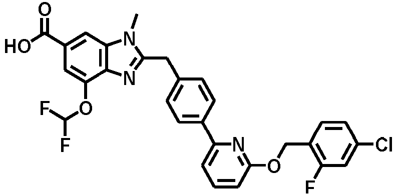 |
| 5-167 | 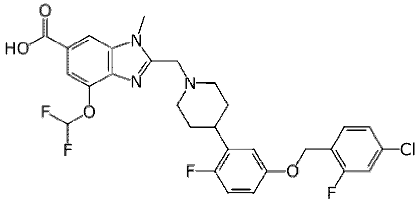 |
| 5-168 | 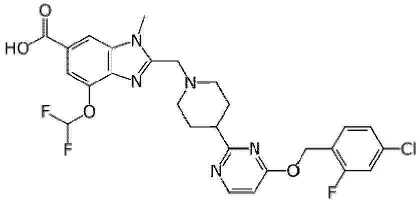 |
| 5-169 | 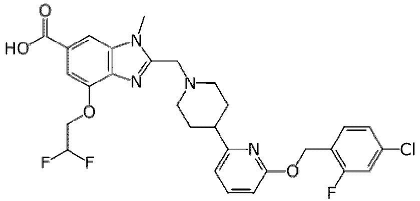 |
| 5-170 | 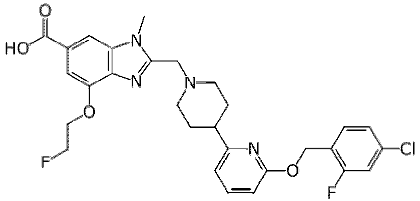 |
| 5-171 | 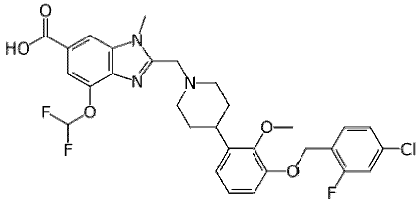 |
| 5-172 | 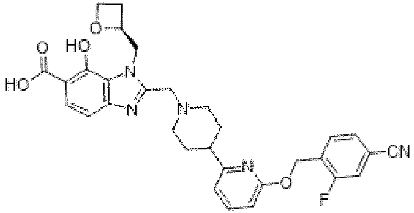 |
| 5-173 | 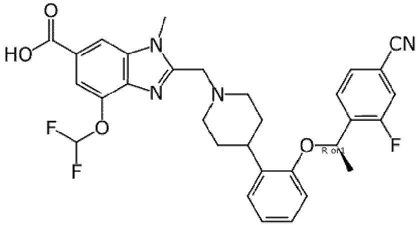 |
| 5-174 | 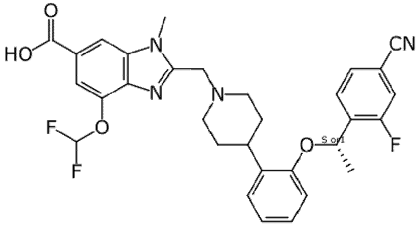 |
| 5-175 | 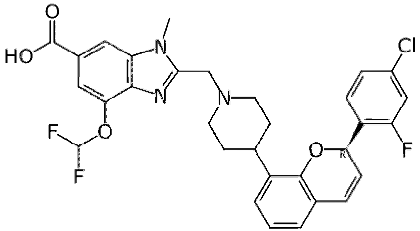 |
| 5-176 | 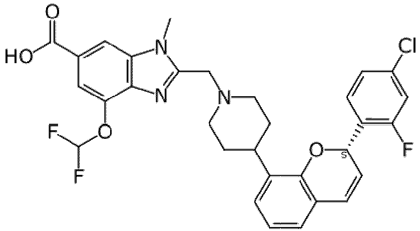 |
| 5-177 | 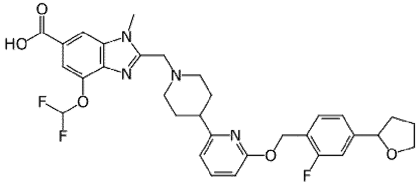 |
| 5-178 | 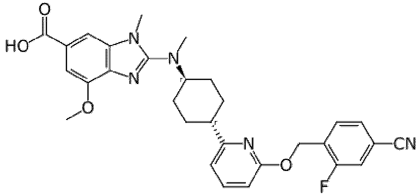 |
| 5-179 | 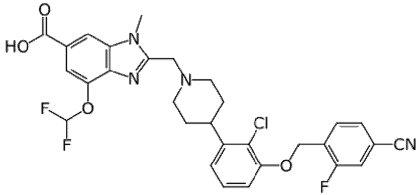 |
| 5-180 | 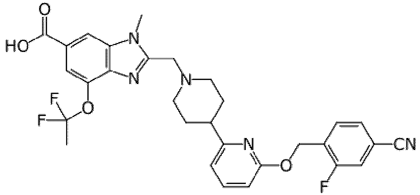 |
| 5-181 | 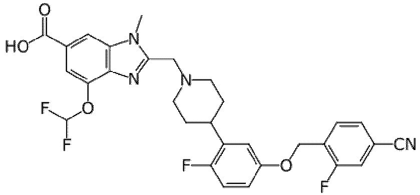 |
| 5-182 | 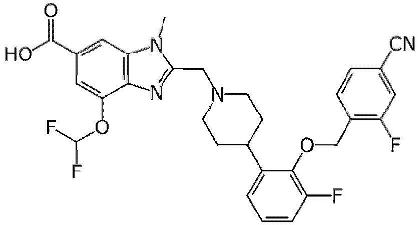 |
| 5-183 | 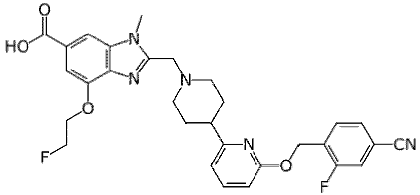 |
| 5-184 | 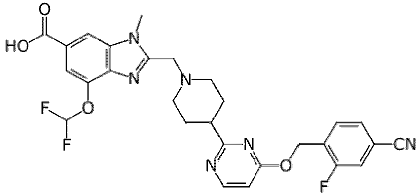 |
| 5-185 | 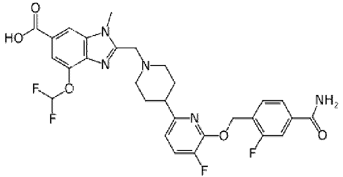 |
| 5-186 | 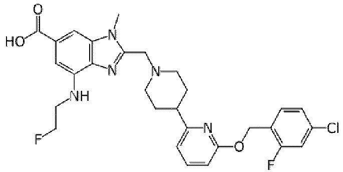 |
| 5-187 | 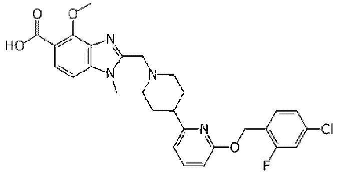 |
| 5-188 | 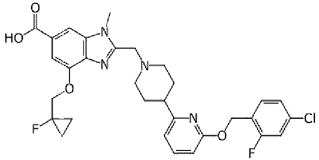 |
| 5-189 | 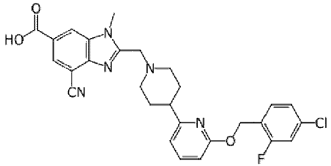 |
| 5-190 | 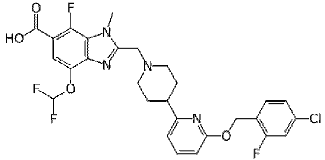 |
| 5-191 | 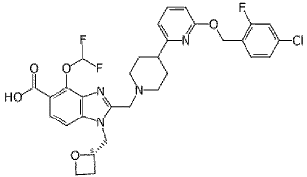 |
| 5-192 | 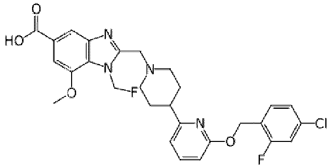 |
| 5-193 | 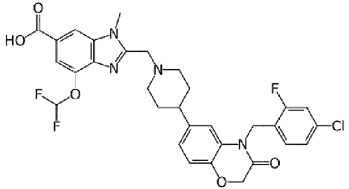 |
| 5-194 | 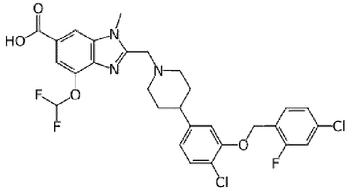 |
| 5-195 | 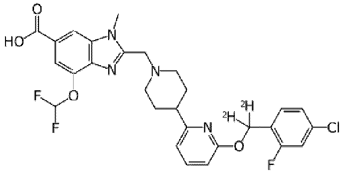 |
| 5-196 | 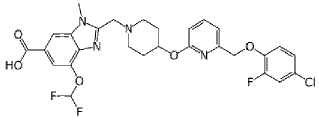 |
| 5-197 | 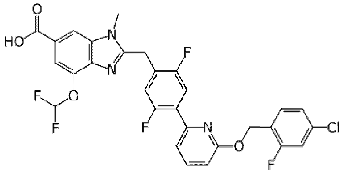 |
| 5-198 | 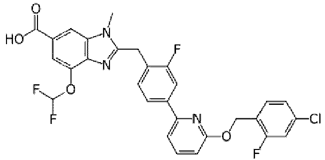 |
| 5-199 | 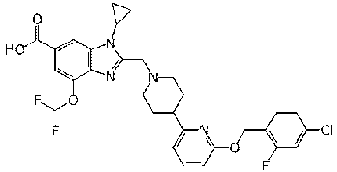 |
| 5-200 | 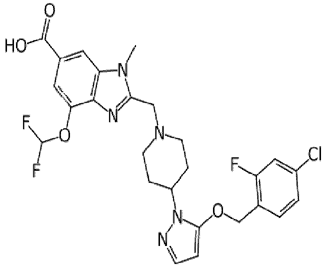 |
| 5-201 | 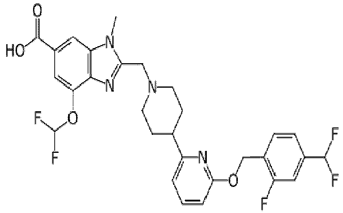 |
| 5-202 | 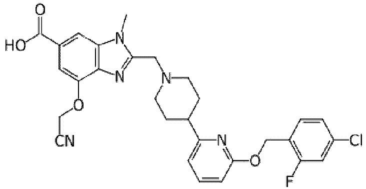 |
| 5-203 | 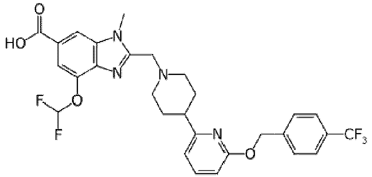 |
| 5-204 | 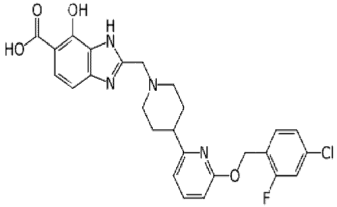 |
| 5-205 | 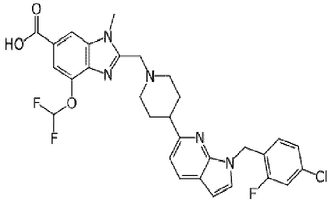 |
| 5-206 | 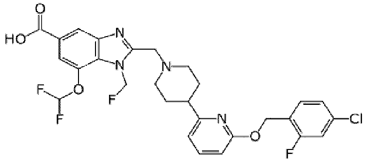 |
| 5-207 | 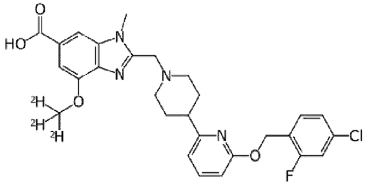 |
| 5-208 | 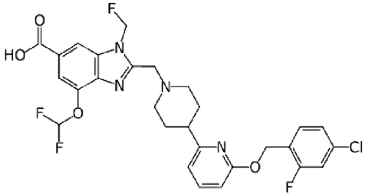 |
| 5-209 | 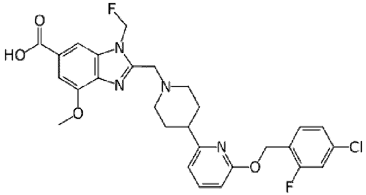 |
| 5-210 | 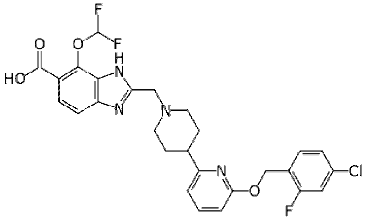 |
| 5-211 | 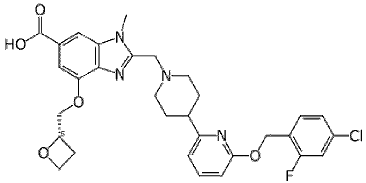 |
| 5-212 | 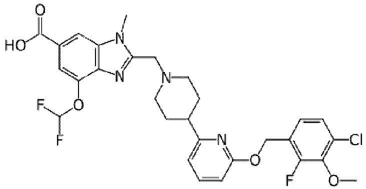 |
| 5-213 | 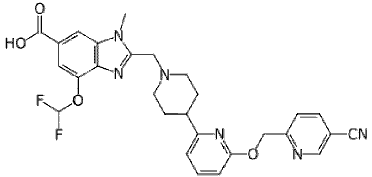 |
| 5-214 | 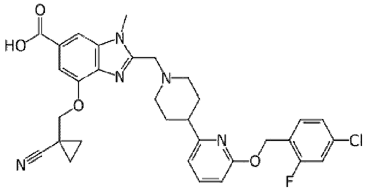 |
| 5-215 | 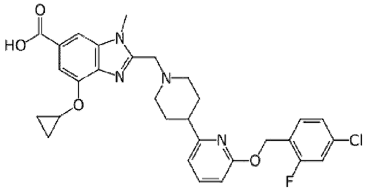 |
| 5-216 | 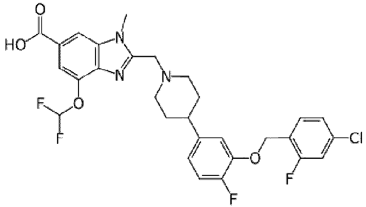 |
| 5-217 | 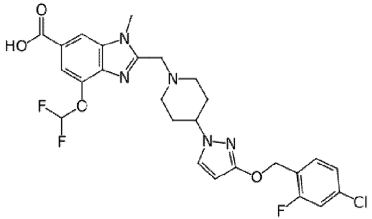 |
| 5-218 | 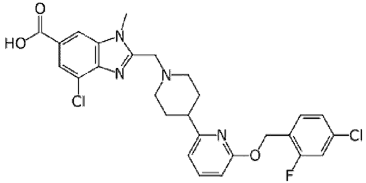 |
| 5-219 | 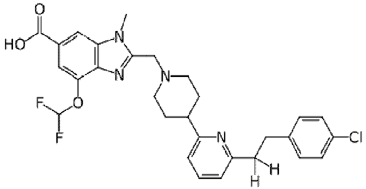 |
| 5-220 | 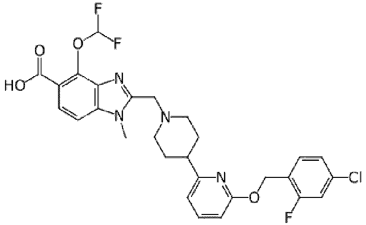 |
| 5-221 | 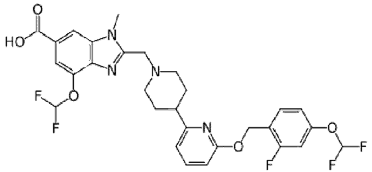 |
| 5-222 | 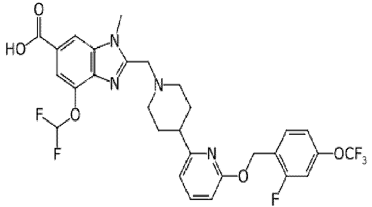 |
| 5-223 | 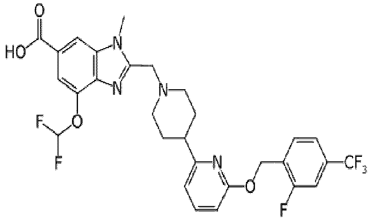 |
| 5-224 | 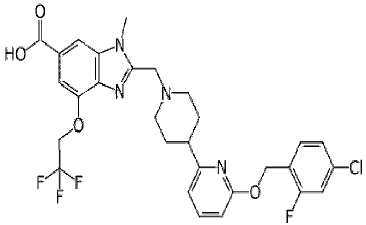 |
| 5-225 | 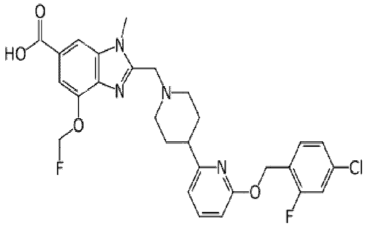 |
| 5-226 | 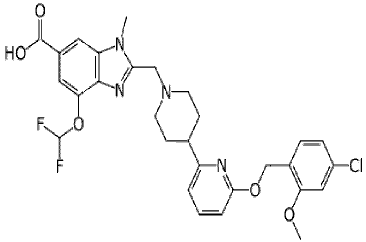 |
| 5-227 | 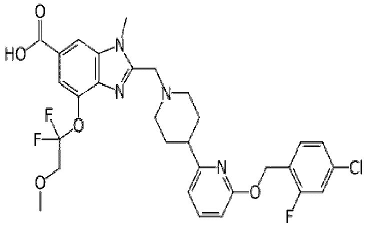 |
| 5-228 | 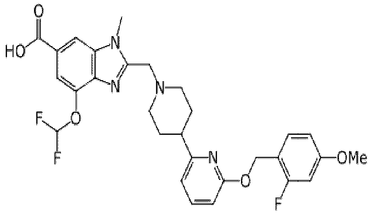 |
| 5-229 | 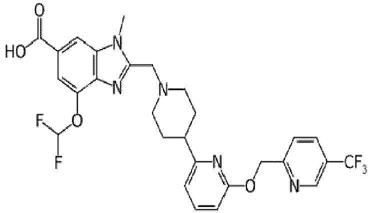 |
| 5-230 | 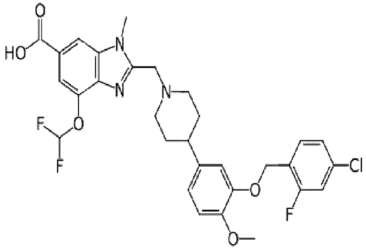 |
| 5-231 | 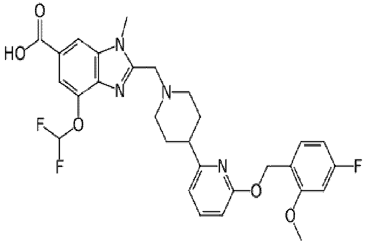 |
| 5-232 | 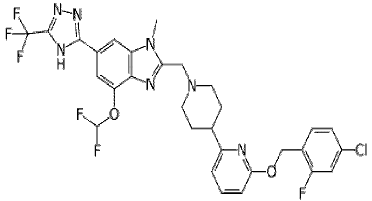 |
| 5-233 | 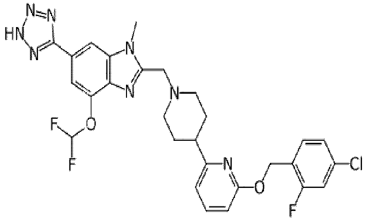 |
| 5-234 | 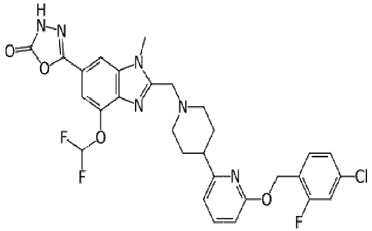 |
| 5-235 | 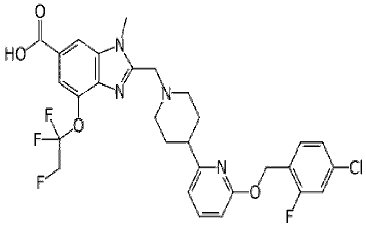 |
| 5-236 | 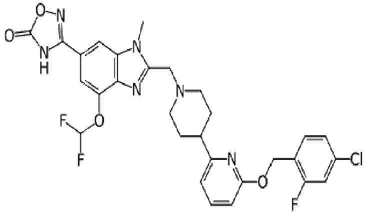 |
| 5-237 | 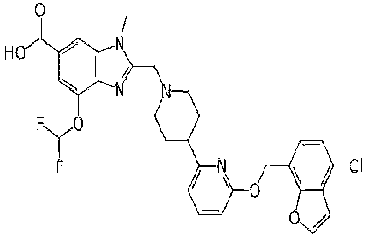 |
| 5-238 | 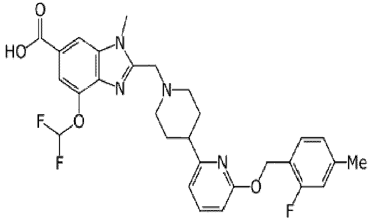 |
| 5-239 | 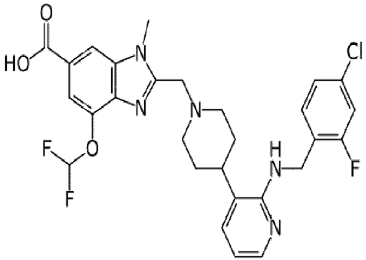 |
| 5-240 | 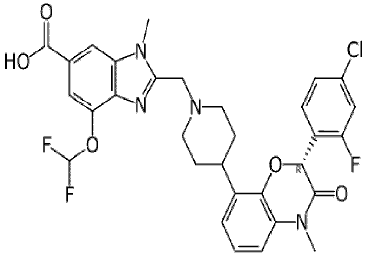 |
| 5-241 | 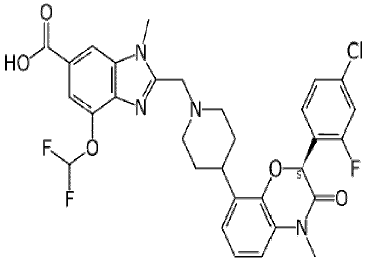 |
| 5-242 | 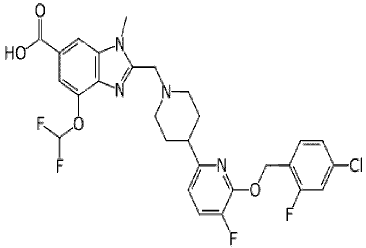 |
| 5-243 | 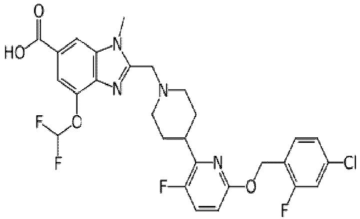 |
| 5-244 | 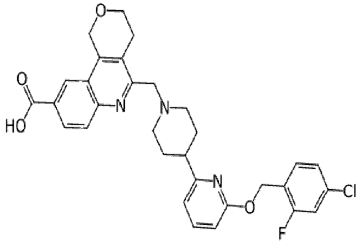 |
| 5-245 | 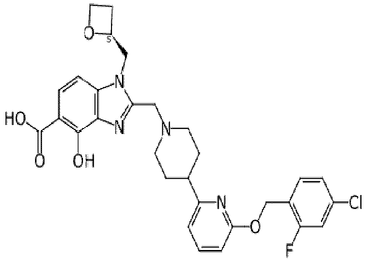 |
| 5-246 | 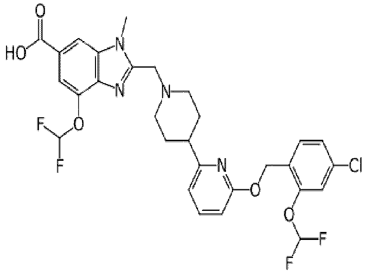 |
| 5-247 | 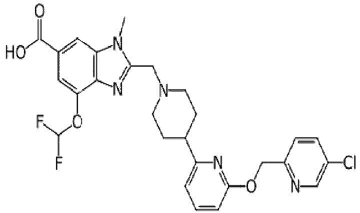 |
| 5-248 | 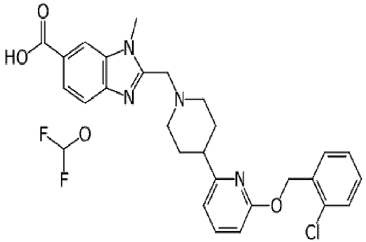 |
| 5-249 | 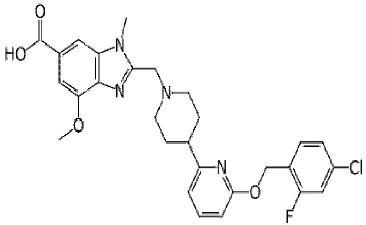 |
| 5-250 | 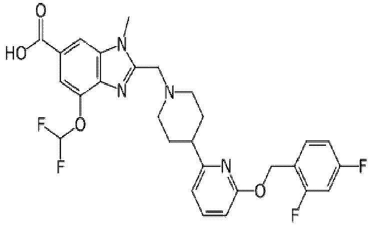 |
| 5-251 | 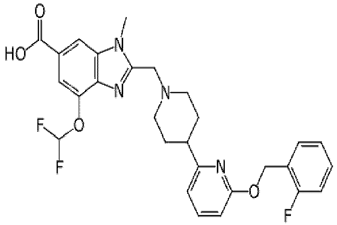 |
| 5-252 | 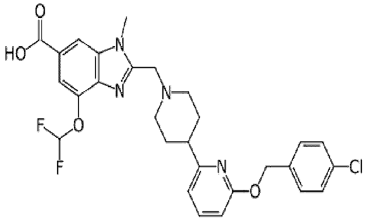 |
| 5-253 | 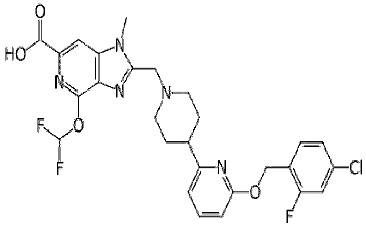 |
| 5-254 | 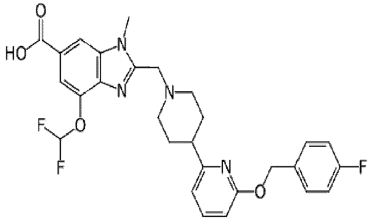 |

In some embodiments, the GLP-1R agonist is selected from those listed in Table 5A below, or a pharmaceutically acceptable salt thereof:

**Table 5A**

| Compound No. | Name | Structure |
|---|---|---|
| 5-255 | (2-((4-(6-((4-chloro-2-fluorobenzyl)oxy)pyridin-2-yl)piperidin-1-yl)methyl)-4-(difluoromethoxy)-1-methyl-1H-benzo[d]imidazol-6-yl)glycine | 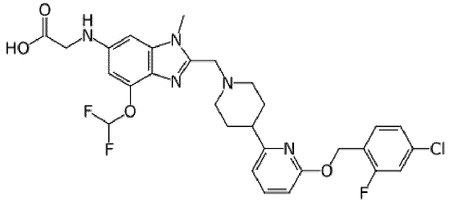 |
| 5-256 | 2-((4-((6-((4-chloro-2-fluorophenoxy)methyl)pyridi n-2-yl)oxy)piperidin-1-yl)methyl)-4-ethoxy-1-methyl-1H-benzo[d]imidazole-6-carboxylic acid | 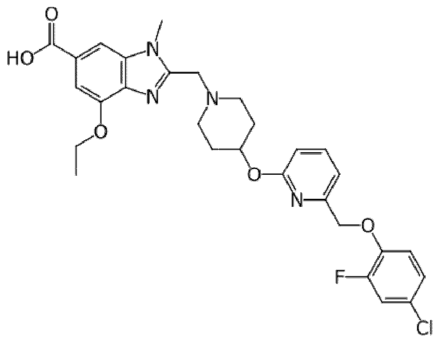 |
| 5-257 | (S)-3-(2-((4-(6-((4-Chloro-2-fluorobenzyl)oxy)pyridin-2-yl)piperidin-1-yl)methyl)-4-methoxy-1-(oxetan-2-ylmethyl)-1H-benzo[d]imidazol-6-yl)propiolic acid | 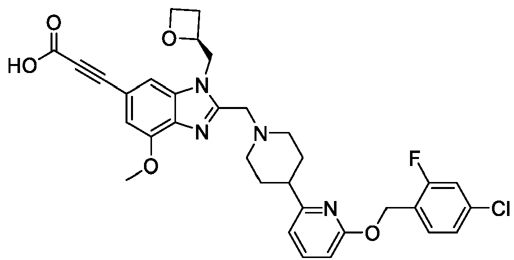 |
| 5-258 | (S)-2-((4-(2-(4-Chloro-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 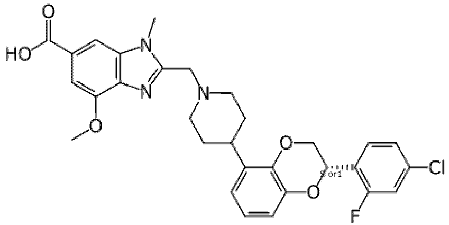 |
| 5-259 | 2-(2-((4-(6-((4-Cyano-2-fluorobenzyl)oxy)pyridin-2-yl)piperidin-1-yl)methyl)-4-ethoxy-1-methyl-1H-benzo[d]imidazol-6-yl)acetic acid | 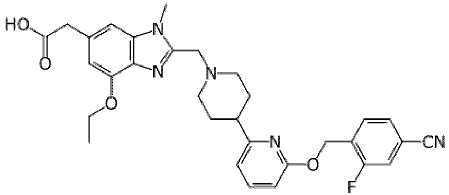 |
| 5-260 | 4-(((6-(1-((4-ethoxy-1-methyl-6-(5-oxo-2,5-dihydro-1,2,4-oxadiazol-3-yl)-1H-benzo[d]imidazol-2-yl)methyl)piperidin-4-yl)pyridin-2-yl)oxy)methyl)-3-fluorobenzonitrile | 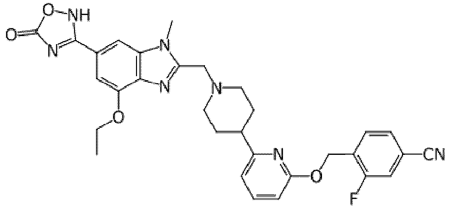 |
| 5-261 | 2-((4-(6-((4-Cyano-2-fluorobenzyl)oxy)pyridin-2-yl)piperidin-1-yl)methyl)-4-ethoxy-1-methyl-N-(methylsulfonyl)-1H-benzo[d]imidazole-6-carboxamide | 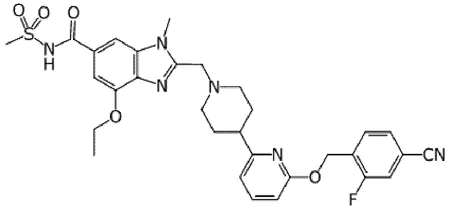 |
| 5-262 | rel-(R)-2-((4-(6-((4-Chloro-2-fluorobenzyl)oxy)pyridin-2-yl)piperidin-1-yl)methyl)-4-((1-fluoropropan-2-yl)oxy)-1-methyl-1H-benzo[d]imidazole-6-carboxylic acid | 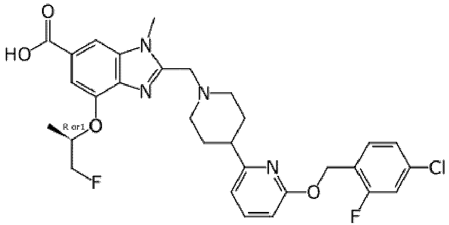 |
| 5-263 | rel-(S)-2-((4-(6-((4-Chloro-2-fluorobenzyl)oxy)pyridin-2-yl)piperidin-1-yl)methyl)-4-((1-fluoropropan-2-yl)oxy)-1-methyl-1H-benzo[d]imidazole-6-carboxylic acid | 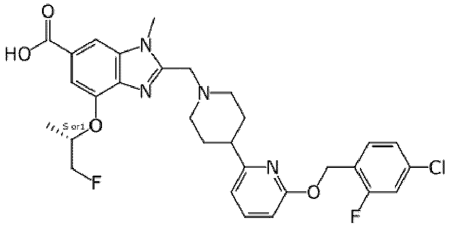 |
| 5-264 | (2-((4-(3-((4-chloro-2-fluorobenzyl)oxy)phenyl)pip eridin-1-yl)methyl)-4-(difluoromethoxy)-1-methyl-1H-benzo[d]imidazol-6-yl)glycine | 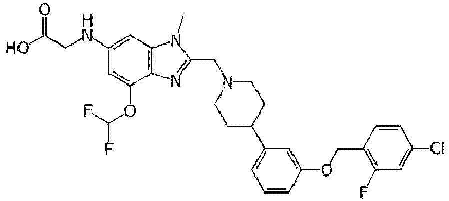 |
| 5-265 | (R)-2-((4-(3-(4-chloro-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 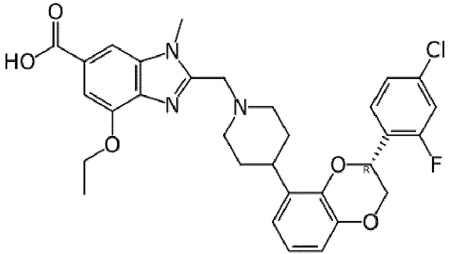 |
| 5-266 | (S)-2-((4-(3-(4-chloro-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 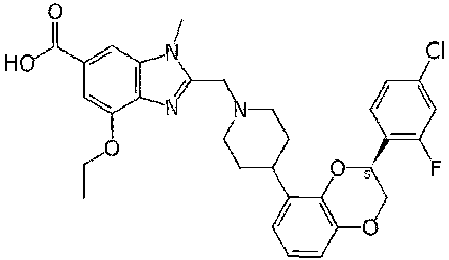 |
| 5-267 | (R)-2-((4-(3-(4-Chloro-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 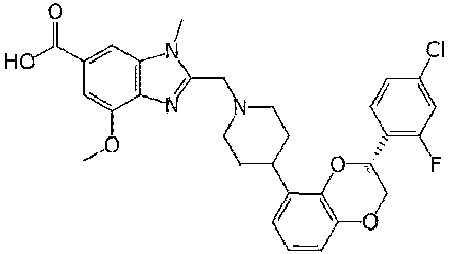 |
| 5-268 | (S)-2-((4-(3-(4-Chloro-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 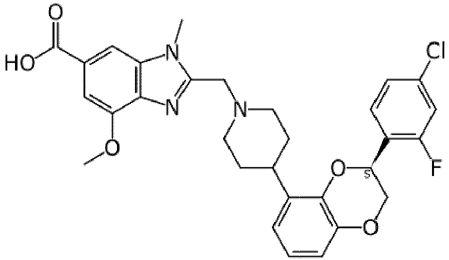 |
| 5-269 | (R)-2-((4-(2-(4-chloro-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 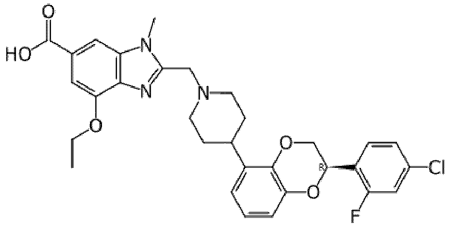 |
| 5-270 | (S)-2-((4-(2-(4-Chloro-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 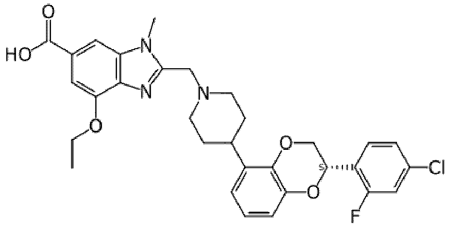 |
| 5-271 | (R)-2-((4-(2-(4-Chloro-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 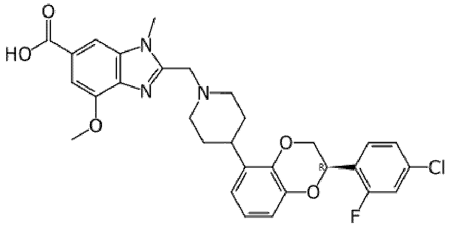 |
| 5-272 | 2-((4-(6-((4-Chloro-2,5-difluorobenzyl)oxy)pyridin-2-yl)piperidin-1-yl)methyl)-4-methoxy-1-methyl-1H-benzo[d]imidazole-6-carboxylic acid | 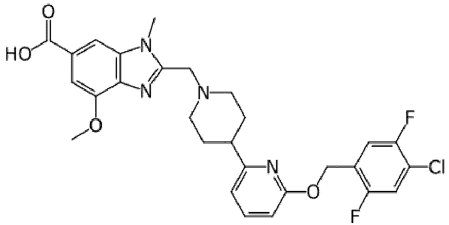 |
| 5-273 | 4-(((6-(1-((4-ethoxy-1-methyl-6-(1H-tetrazol-5-yl)-1H-benzo[d]imidazol-2-yl)methyl)piperidin-4-yl)pyridin-2-yl)oxy)methyl)-3-fluorobenzonitrile | 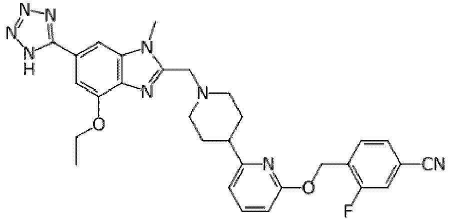 |
| 5-274 | (S)-2-((4-(2-(4-Cyano-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 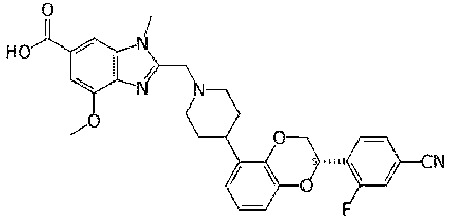 |
| 5-275 | 7-((4-(6-((4-Chloro-2-fluorobenzyl)oxy)pyridin-2-yl)piperidin-1-yl)methyl)-6-methyl-6H-[1,3]dioxolo[4',5':3,4]benzo[ 1,2-d]imidazole-4-carboxylic acid | 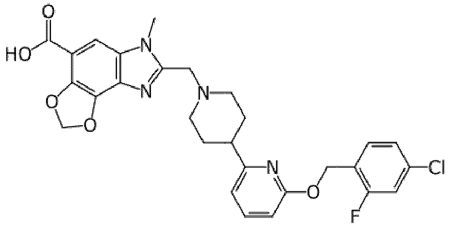 |
| 5-276 | (R)-2-((4-(2-(4-Cyano-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 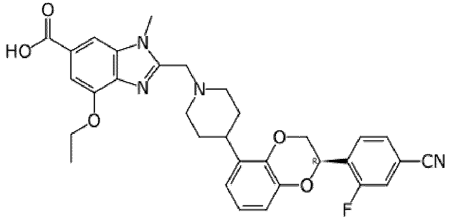 |
| 5-277 | (S)-2-((4-(2-(4-Cyano-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 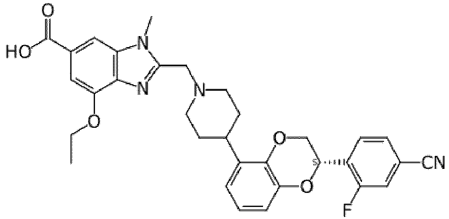 |
| 5-278 | (R)-2-((4-(2-(4-Cyano-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 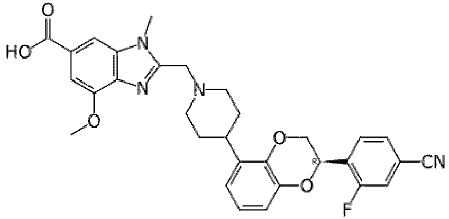 |
| 5-279 | (S)-2-((4-(6-((4-Chloro-2-fluorobenzyl)oxy)pyridin-2-yl)piperidin-1-yl)methyl)-4-methoxy-1-(oxetan-2-ylmethyl)-6-(1H-tetrazol-5-yl)-1H-benzo[d]imidazole | 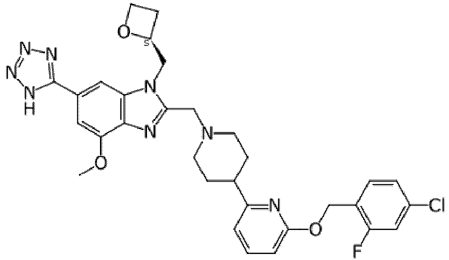 |
| 5-280 | 2-((4-(6-((4-chloro-2-fluorobenzyl)oxy)pyridin-2-yl)piperidin-1-yl)methyl)-4-methoxy-N,1-dimethyl-1H-benzo[d]imidazole-6-carboxamide | 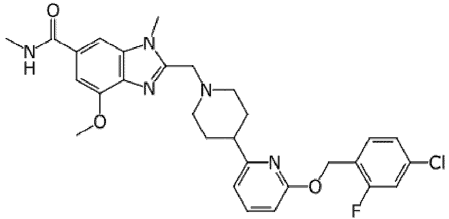 |
| 5-281 | 5-(2-((4-(3-((4-Chloro-2-fluorobenzyl)oxy)phenyl)pip eridin-1-yl)methyl)-4-(difluoromethoxy)-1-methyl-1H-benzo[d]imidazol-6-yl)-1,2,5-thiadiazolidin-3-one 1,1-dioxide | 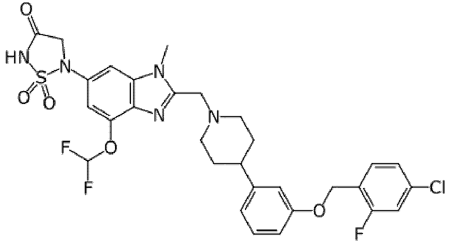 |
| 5-282 | 2-(4-(6-((4-Chloro-2-fluorobenzyl)oxy)pyridin-2-yl)-2,5-difluorobenzyl)-4-methoxy-1-methyl-1H-benzo[d]imidazole-6-carboxylic acid | 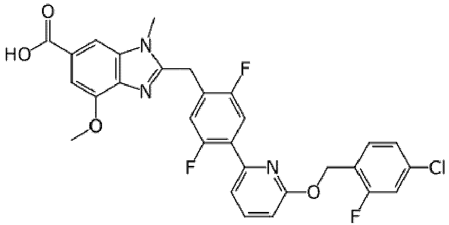 |
| 5-283 | 2-((4-(6-((4-Cyano-2,3-dihydrobenzofuran-7-yl)methoxy)pyridin-2-yl)piperidin-1-yl)methyl)-4-methoxy-1-methyl-1H-benzo[d]imidazole-6-carboxylic acid | 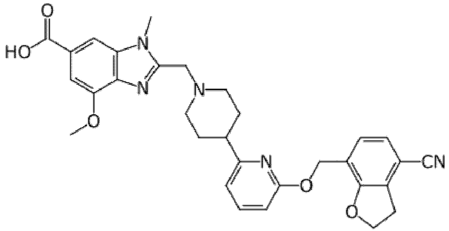 |
| 5-284 | 2-((4-(3-((4-Chloro-2-fluorobenzyl)oxy)phenyl)pip eridin-1-yl)methyl)-4-(ethoxy-1,1-d2)-1-methyl-1H-benzo[d]imidazole-6-carboxylic acid | 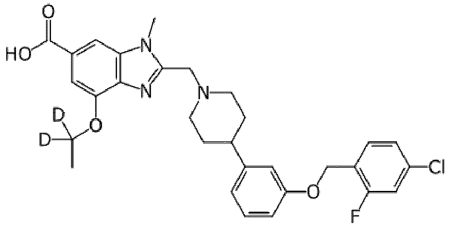 |
| 5-285 | 2-((4-(6-((4-Cyano-2-fluorophenyl)methoxy-d2)pyridin-2-yl)piperidin-1-yl)methyl)-4-(ethoxy-1,1-d2)-1-methyl-1H-benzo[d]imidazole-6-carboxylic acid | 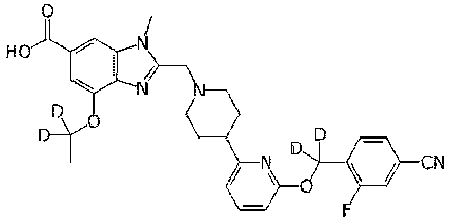 |
| 5-286 | 2-((4-(6-((4-Chloro-2-fluorophenyl)methoxy-d2)pyridin-2-yl)piperidin-1-yl)methyl)-4-(ethoxy-1,1-d2)-1-methyl-1H-benzo[d]imidazole-6-carboxylic acid | 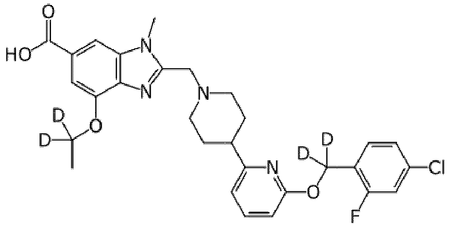 |
| 5-287 | (S)-2-((4-(3-(4-Cyano-2-fluorophenyl)-2,3-dihydrobenzo[b][1,4]dioxin-5-yl)piperidin-1-yl)methyl)-4-ethoxy-1-methyl-1 H-benzo[d]imidazole-6-carboxylic acid | 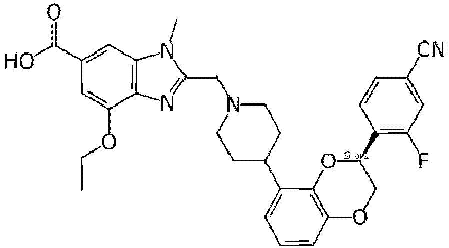 |
| 5-288 | (R)-2-((4-(3-(4-Cyano-2-fluorophenyl)-2,3-dihydrobenzo[b][1,4]dioxin-5-yl)piperidin-1-yl)methyl)-4-ethoxy-1-methyl-1H-benzo[d]imidazole-6-carboxylic acid | 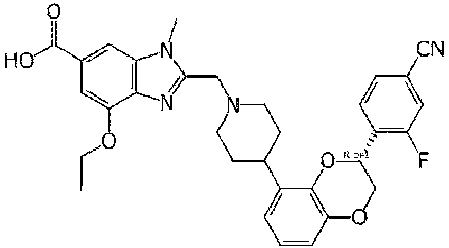 |
| 5-289 | 2-((4-(6-((4-Cyano-2-fluorobenzyl)oxy)pyridin-2-yl)piperidin-1-yl)methyl)-3-methyl-7,8-dihydro-3H-[1,4]dioxino[2',3':3,4]benzo[ 1,2-d]imidazole-5-carboxylic acid | 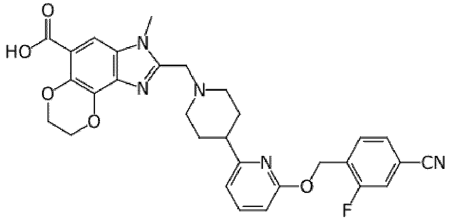 |
| 5-290 | 2-((4-((6-((4-Cyano-2-fluorophenoxy)methyl)pyridi n-2-yl)oxy)piperidin-1-yl)methyl)-4-ethoxy-1-methyl-1H-benzo[d]imidazole-6-carboxylic acid | 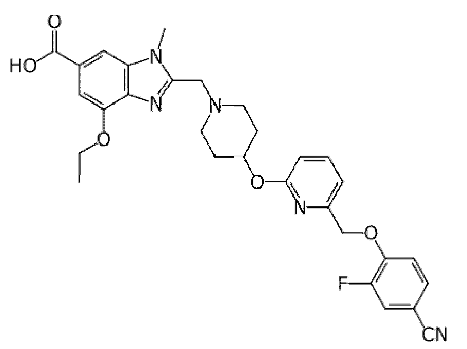 |
| 5-291 | 2-((4-(6-((4-Cyano-2-fluorobenzyl)oxy)pyridin-2-yl)piperidin-1-yl)methyl)-4-(2-hydroxyethoxy)-1-methyl-1H-benzo[d]imidazole-6-carboxylic acid | 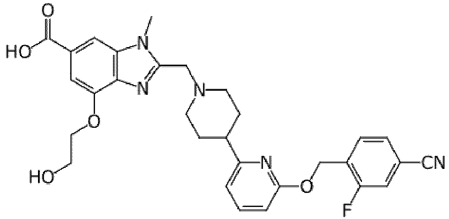 |
| 5-292 | 2-(4-(6-((4-Chloro-2-fluorobenzyl)oxy)pyridin-2-yl)-2-fluorobenzyl)-4-methoxy-1-methyl-1H-benzo[d]imidazole-6-carboxylic acid | 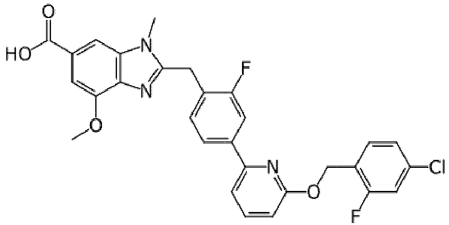 |
| 5-293 | 2-(4-(6-((4-Cyano-2-fluorobenzyl)oxy)pyridin-2-yl)-2-fluorobenzyl)-4-ethoxy-1-methyl-1H-benzo[d]imidazole-6-carboxylic acid | 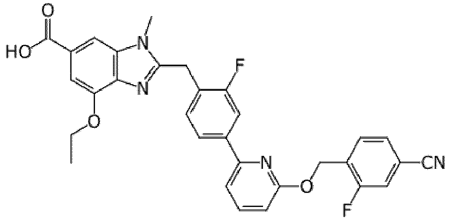 |
| 5-294 | (S)-2-((4-(3-(4-Cyano-2-fluorophenyl)-2,3-dihydrobenzo[b][1,4]dioxin-5-yl)piperidin-1-yl)methyl)-4-(2-fluoroethoxy)-1-methyl-1H-benzo[d]imidazole-6-carboxylic acid | 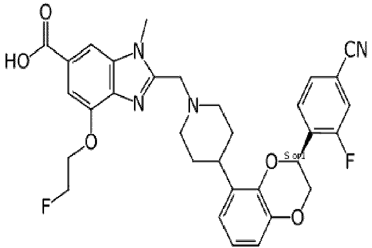 |
| 5-295 | 2-((4-(6-((4-Cyano-2-fluorobenzyl)oxy)pyridin-2-yl)piperidin-1-yl)methyl)-4-(ethoxy-1,1-d2)-1-methyl-1H-benzo[d]imidazole-6-carboxylic acid | 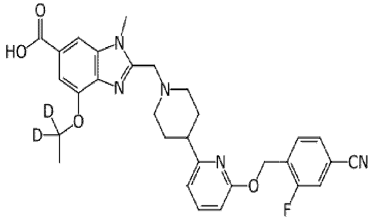 |
| 5-296 | 2-((4-(6-((4-Chloro-2-fluorobenzyl)oxy)pyridin-2-yl)piperidin-1-yl)methyl)-4-(ethoxy-1,1-d2)-1-methyl-1H-benzo[d]imidazole-6-carboxylic acid | 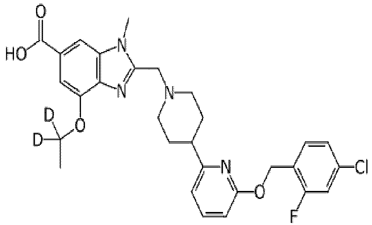 |
| 5-297 | 2-((4-(6-((4-Chloro-2,3-dihydrobenzofuran-7-yl)methoxy)pyridin-2-yl)piperidin-1-yl)methyl)-4-methoxy-1-methyl-1H-benzo[d]imidazole-6-carboxylic acid | 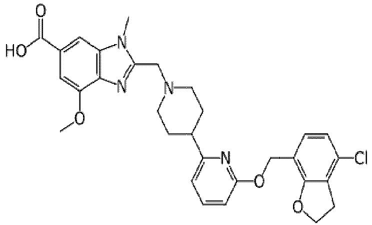 |
| 5-298 | 2-((4-(6-((4-Cyano-2-fluorobenzyl)oxy)pyridin-2-yl)piperidin-1-yl)methyl)-4-ethoxy-1-methyl-1H-benzo[d]imidazole-6-carboxylic acid | 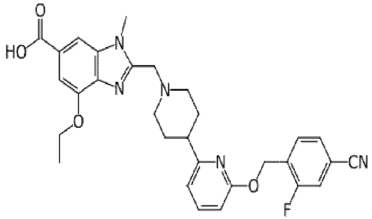 |
| 5-299 | (S)-2-((4-(3-(4-chloro-2-fluorophenyl)-2,3-dihydrobenzo[b][1,4]dioxin-5-yl)piperidin-1-yl)methyl)-4-(2-fluoroethoxy)-1-methyl-1H-benzo[d]imidazole-6-carboxylic acid | 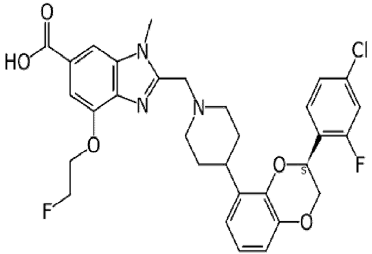 |
| 5-300 | (R)-2-((4-(2-(4-Cyano-2-fluorophenyl)-2,3-dihydrobenzo[b][1,4]dioxin-5-yl)piperidin-1-yl)methyl)-4-(difluoromethoxy)-1-methyl-1H-benzo[d]imidazole-6-carboxylic acid | 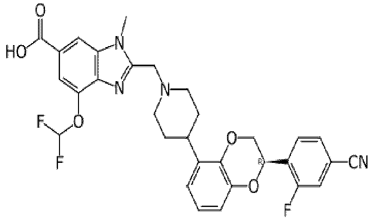 |
| 5-301 | 2-((4-(6-((4-Cyano-2-fluorobenzyl)oxy)pyridin-2-yl)piperidin-1-yl)methyl)-5-fluoro-4-methoxy-1-methyl-1H-benzo[d]imidazole-6-carboxylic acid | 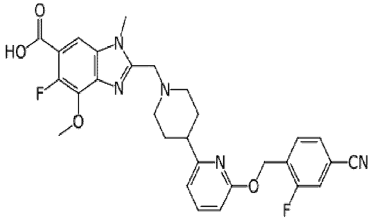 |
| 5-302 | 2-((4-(6-((4-Chloro-2-fluorobenzyl)oxy)pyridin-2-yl)piperidin-1-yl)methyl)-7-fluoro-4-methoxy-1-methyl-1H-benzo[d]imidazole-6-carboxylic acid | 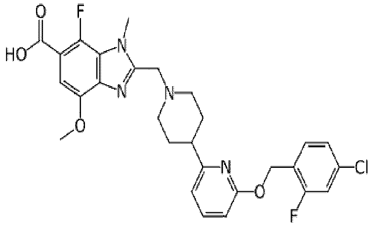 |
| 5-303 | 2-((4-(6-((4-Cyano-2-fluorobenzyl)oxy)pyridin-2-yl)piperidin-1-yl)methyl)-7-fluoro-4-methoxy-1-methyl-1H-benzo[d]imidazole-6-carboxylic acid | 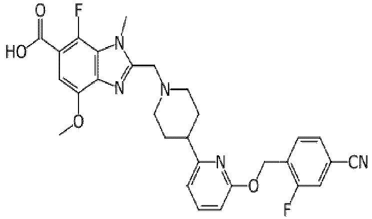 |
| 5-304 | 2-(4-(6-((4-Cyano-2-fluorobenzyl)oxy)pyridin-2-yl)-2,5-difluorobenzyl)-4-methoxy-1-methyl-1H-benzo[d]imidazole-6-carboxylic acid | 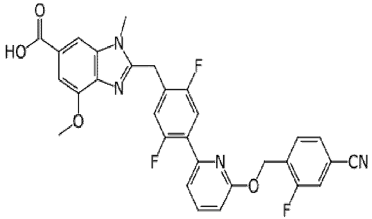 |
| 5-305 | 2-((4-(6-((4-(Difluoromethyl)-2-fluorobenzyl)oxy)pyridin-2-yl)piperidin-1-yl)methyl)-4-methoxy-1-methyl-1H-benzo[d]imidazole-6-carboxylic acid | 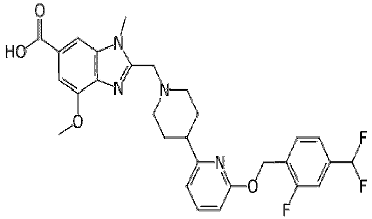 |
| 5-306 | 4-Methoxy-2-((4-(6-((2-methoxy-4-(trifluoromethyl)benzyl)oxy) pyridin-2-yl)piperidin-1-yl)methyl)-1-methyl-1H-benzo[d]imidazole-6-carboxylic acid | 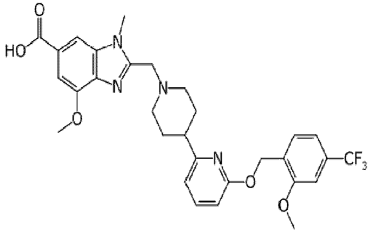 |
| 5-307 | 2-((4-(6-((4-Chloro-2-fluorobenzyl)oxy)pyridin-2-yl)piperidin-1-yl)methyl)-4-hydroxy-1-methyl-1H-benzo[d]imidazole-6-carboxylic acid | 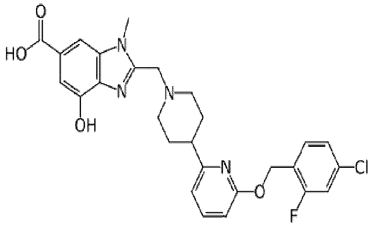 |
| 5-308 | 2-((4-(6-((4-Cyano-2-fluorobenzyl)oxy)pyridin-2-yl)piperidin-1-yl)methyl)-4-methoxy-1-methyl-1H-benzo[d]imidazole-6-carboxylic acid | 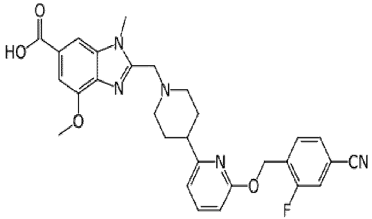 |
| 5-309 | (S)-2-((4-(2-(5-Chloropyridin-2-yl)-2-methylbenzo[d][1,3]dioxol-4-yl)piperidin-1-yl)methyl)-4-(2-methoxyethoxy)-1-methyl-1H-benzo[d]imidazole-6-carboxylic acid | 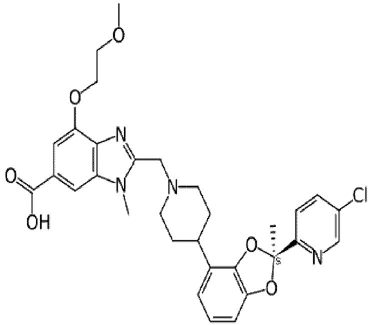 |
| 5-310 | (R)-2-((4-(3-(4-cyano-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 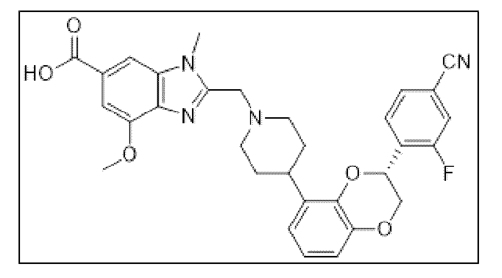 |
| 5-311 | 2-((4-(6-((4-chloro-2-fluorobenzyl)oxy)pyridin-2-yl)piperidin-1-yl)methyl)-3-methyl-7,8-dihydro-3H-[1,4]dioxino[2',3':3,4]benzo[ 1,2-d]imidazole-5-carboxylic acid | 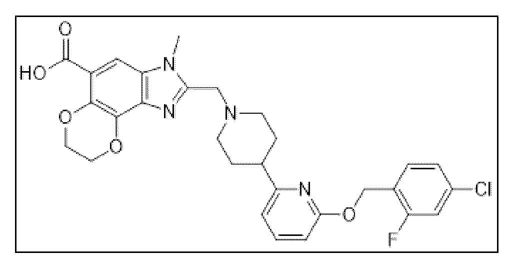 |
| 5-312 | (R)-2-((4-(6-((4-Cyano-2-fluorobenzyl)oxy)pyridin-2-yl)piperidin-1-yl)methyl)-4-(1-fluoroethoxy)-1-methyl-1H-benzo[d]imidazole-6-carboxylic acid | 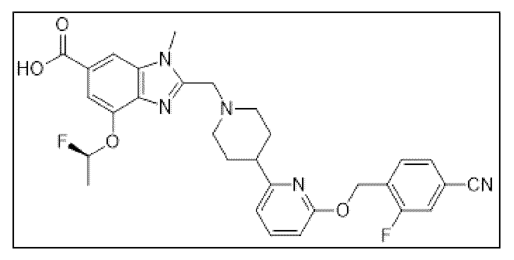 |
| 5-313 | (S)-2-((4-(6-((4-Cyano-2-fluorobenzyl)oxy)pyridin-2-yl)piperidin-1-yl)methyl)-4-(1-fluoroethoxy)-1-methyl-1H-benzo[d]imidazole-6-carboxylic acid | 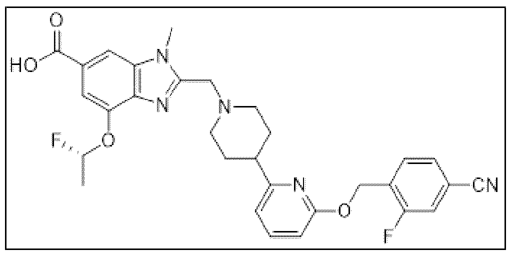 |
| 5-314 | (S)-2-((4-(3-(4-Cyano-2-fluorophenyl)-2,3-dihydrobenzo[b][1,4]dioxin-5-yl)piperidin-1-yl)methyl)-4-methoxy-1-methyl-1H-benzo[d]imidazole-6-carboxylic acid | 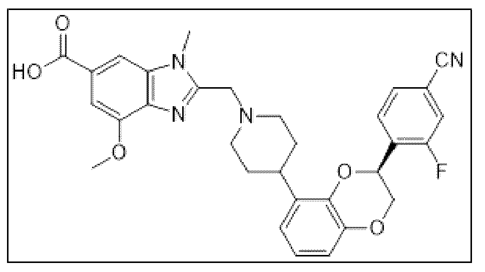 |
| 5-315 | (R)-2-((4-(3-(4-Cyano-2-fluorophenyl)-2,3-dihydrobenzo[b][1,4]dioxin-5-yl)piperidin-1-yl)methyl)-4-(2-fluoroethoxy)-1-methyl-1H-benzo[d]imidazole-6-carboxylic acid | 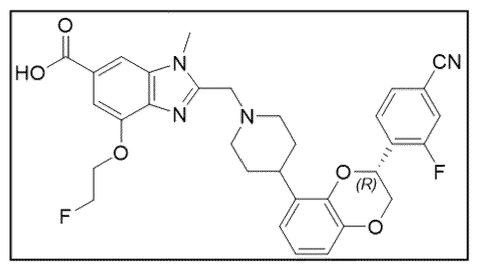 |
| 5-316 | 5-(2-((4-(6-((4-Chloro-2-fluorobenzyl)oxy)pyridin-2-yl)piperidin-1-yl)methyl)-4-(difluoromethoxy)-1-methyl-1H-benzo[d]imidazol-6-yl)isoxazol-3-ol | 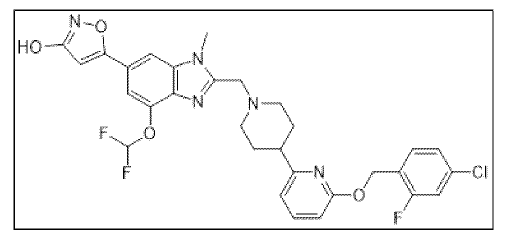 |
| 5-317 | 2-((4-(6-((4-Cyano-2-fluorobenzyl)oxy)pyridin-2-yl)piperidin-1-yl)methyl)-4-((1r,3r)-3-methoxycyclobutoxy)-1-methyl-1H-benzo[d]imidazole-6-carboxylic acid | 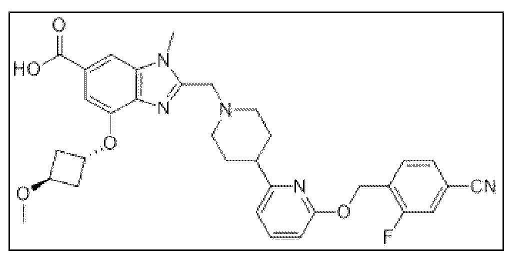 |
| 5-318 | 2-((4-(6-((4-Cyano-2-fluorobenzyl)oxy)pyridin-2-yl)piperidin-1-yl)methyl)-4-((1s,3s)-3-methoxycyclobutoxy)-1-methyl-1H-benzo[d]imidazole-6-carboxylic acid | 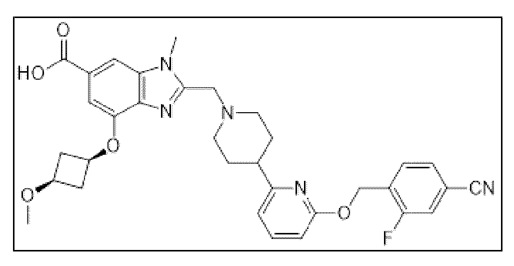 |
| 5-319 | 2-((4-(6-((4-Cyano-2-fluorobenzyl)oxy)pyridin-2-yl)piperidin-1-yl)methyl)-1-methyl-4-propoxy-1H-benzo[d]imidazole-6-carboxylic acid | 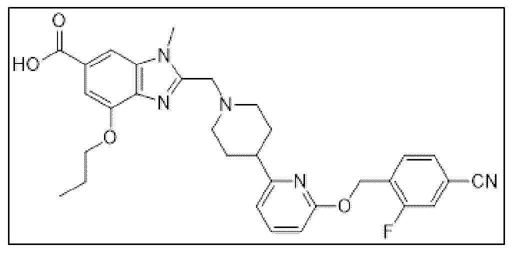 |
| 5-320 | 2-((4-(6-((4-Chloro-2-fluorobenzyl)oxy)pyridin-2-yl)piperidin-1-yl)methyl)-4-((1-hydroxypropan-2-yl)oxy)-1-methyl-1H-benzo[d]imidazole-6-carboxylic acid | 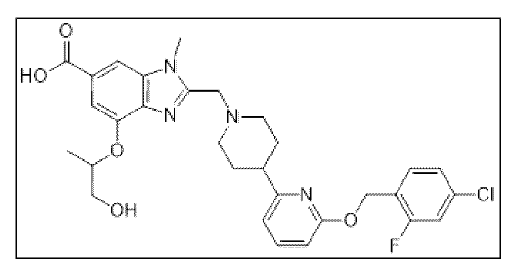 |
| 5-321 | 2-((4-(6-((4-Chloro-2-fluorophenyl)methoxy-d2)pyridin-2-yl)piperidin-1-yl)methyl-d2)-4-(difluoromethoxy)-1-methyl-1H-benzo[d]imidazole-6-carboxylic acid | 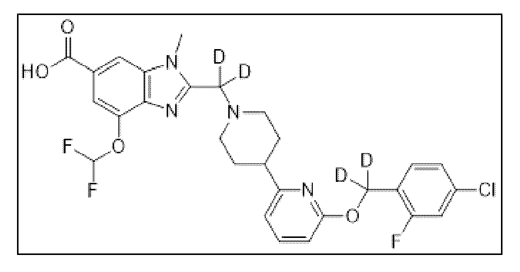 |
| 5-322 | 2-((4-(6-((4-chloro-2-fluorobenzyl)oxy)pyridin-2-yl)piperidin-1-yl)methyl)-1-methyl-4-propoxy-1H-benzo[d]imidazole-6-carboxylic acid | 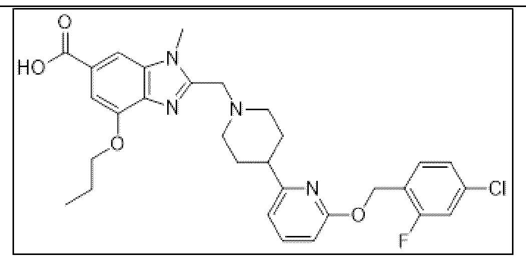 |
| 5-323 | 2-((4-(6-((4-Cyano-2-fluorobenzyl)oxy)pyridin-2-yl)piperidin-1-yl)methyl)-7-methoxy-3-methyl-3H-imidazo[4,5-b]pyridine-5-carboxylic acid | 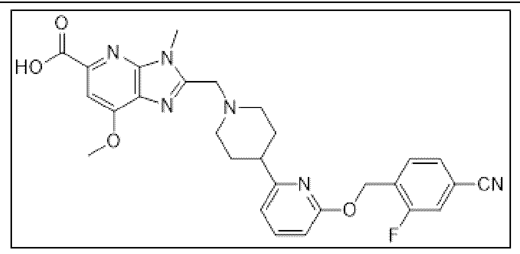 |
| 5-324 | 2-((4-(6-((4-Chloro-2-fluorobenzyl)oxy)pyridin-2-yl)piperidin-1-yl)methyl)-7-methoxy-3-methyl-3H-imidazo[4,5-b]pyridine-5-carboxylic acid | 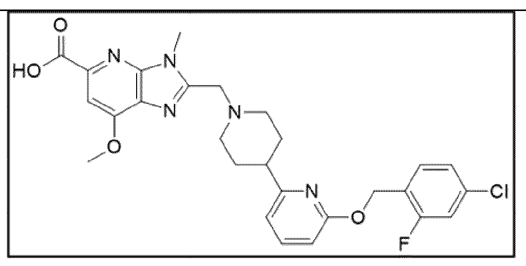 |
| 5-325 | 2-((4-(6-((4-Chloro-2-fluorobenzyl)oxy)pyridin-2-yl)piperidin-1-yl)methyl)-4-((1r,3r)-3-methoxycyclobutoxy)-1-methyl-1H-benzo[d]imidazole-6-carboxylic acid | 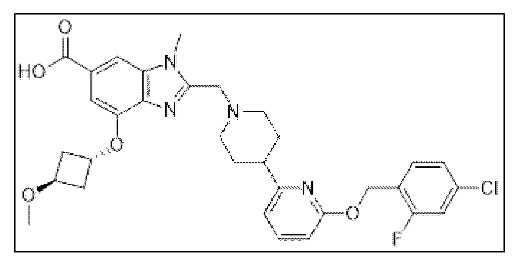 |
| 5-326 | (S)-2-((4-(2-(4-Cyano-2-fluorophenyl)-2,3-dihydrobenzo[b][1,4]dioxin-5-yl)piperidin-1-yl)methyl)-4-(difluoromethoxy)-1-methyl-1H-benzo[d]imidazole-6-carboxylic acid | 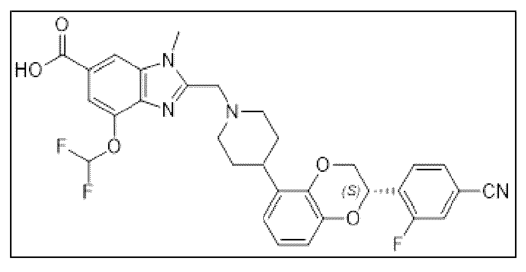 |
| 5-327 | 2-((4-(6-((4-Cyano-2,5-difluorobenzyl)oxy)pyridin-2-yl)piperidin-1-yl)methyl)-4-methoxy-1-methyl-1H-benzo[d]imidazole-6-carboxylic acid | 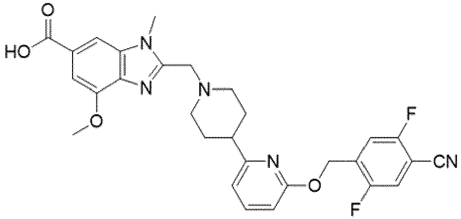 |
| 5-328 | 2-((4-(6-((4-Chloro-2-fluorobenzyl)oxy)pyridin-2-yl)piperidin-1-yl)methyl)-7-(difluoromethoxy)-3-methyl-3H-imidazo[4,5-b]pyridine-5-carboxylic acid | 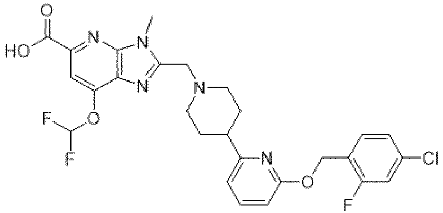 |
| 5-329 | (S)-2-((4-(6-((4-chloro-2-fluorobenzyl)oxy)pyridin-2-yl)piperidin-1-yl)methyl)-4-(1,2-difluoroethoxy)-1-methyl-1H-benzo[d]imidazole-6-carboxylic acid | 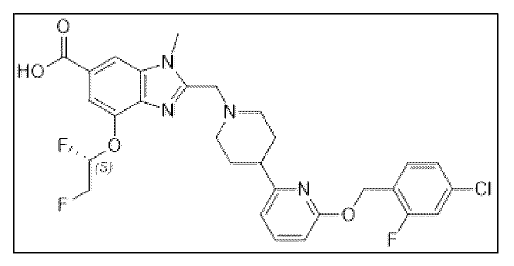 |
| 5-330 | (R)-2-((4-(6-((4-chloro-2-fluorobenzyl)oxy)pyridin-2-yl)piperidin-1-yl)methyl)-4-(1,2-difluoroethoxy)-1-methyl-1H-benzo[d]imidazole-6-carboxylic acid | 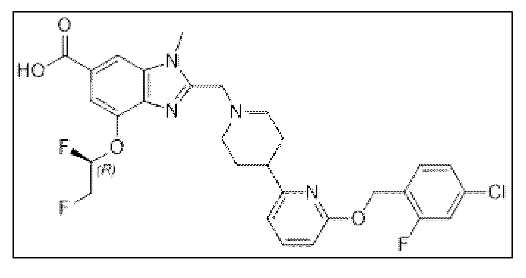 |
| 5-331 | 2-((4-(6-((4-Chloro-2,3-dihydrobenzofuran-7-yl)methoxy)pyridin-2-yl)piperidin-1-yl)methyl)-4-(difluoromethoxy)-1-methyl-1H-benzo[d]imidazole-6-carboxylic acid | 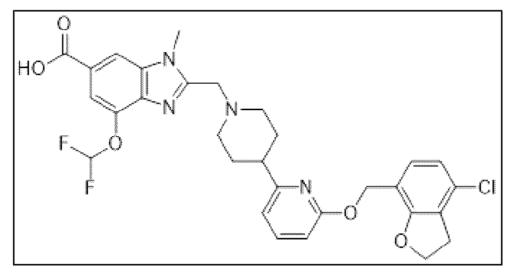 |
| 5-332 | (S)-2-((4-(3-(4-chloro-2-fluorophenyl)-2,3-dihydrobenzo[b][1,4]dioxin-5-yl)piperidin-1-yl)methyl)-4-(2-fluoroethoxy)-1-methyl-1H-benzo[d]imidazole-6-carboxylic acid | 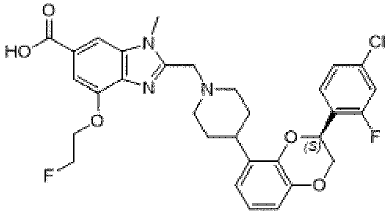 |
| 5-333 | (R)-2-((4-(3-(4-chloro-2-fluorophenyl)-2,3-dihydrobenzo[b][1,4]dioxin-5-yl)piperidin-1-yl)methyl)-4-(2-fluoroethoxy)-1-methyl-1H-benzo[d]imidazole-6-carboxylic acid | 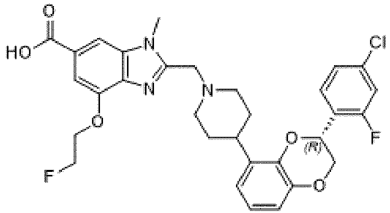 |
| 5-334 | (S)-2-((4-(3-(4-Chloro-2-fluorophenyl)-2,3-dihydrobenzo[b][1,4]dioxin-5-yl)piperidin-1-yl)methyl)-4-(difluoromethoxy)-1-methyl-1H-benzo[d]imidazole-6-carboxylic acid | 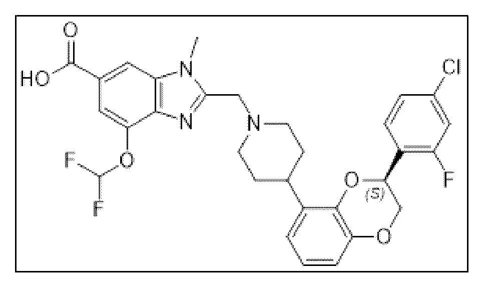 |
| 5-335 | (R)-2-((4-(3-(4-Chloro-2-fluorophenyl)-2,3-dihydrobenzo[b][1,4]dioxin-5-yl)piperidin-1-yl)methyl)-4-(difluoromethoxy)-1-methyl-1H-benzo[d]imidazole-6-carboxylic acid | 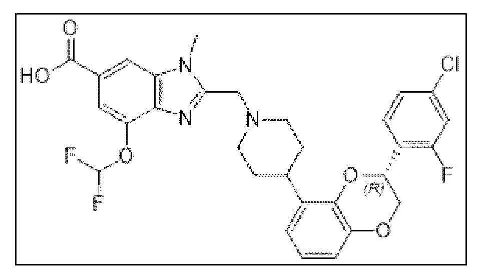 |
| 5-336 | 2-((4-(6-((4-Cyano-2-fluorobenzyl)oxy)pyridin-2-yl)piperidin-1-yl)methyl)-6-fluoro-7-methoxy-1-methyl-1H-benzo[d]imidazole-5-carboxylic acid | 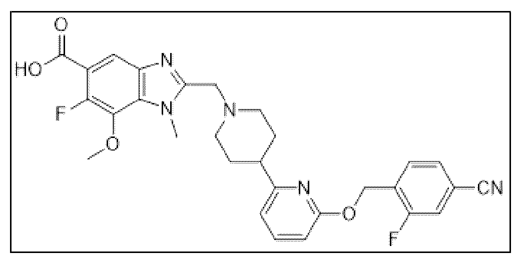 |
| 5-337 | 2-((4-(6-((4-Chloro-2-fluorobenzyl)oxy)pyridin-2-yl)piperidin-1-yl)methyl)-5-fluoro-4-methoxy-1-methyl-1H-benzo[d]imidazole-6-carboxylic acid | 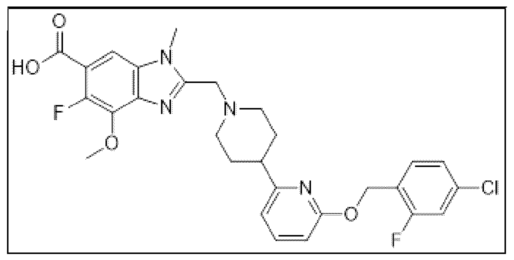 |
| 5-338 | 2-((4-(6-((4-chloro-2-fluorobenzyl)oxy)pyridin-2-yl)piperidin-1-yl)methyl)-6-fluoro-7-methoxy-1-methyl-1H-benzo[d]imidazole-5-carboxylic acid | 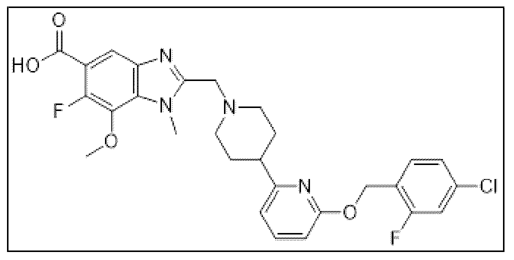 |
| 5-339 | 2-((4-(3-((4-Cyano-2-fluorobenzyl)oxy)-4-fluorophenyl)piperidin-1-yl)methyl)-7-fluoro-4-methoxy-1-methyl-1H-benzo[d]imidazole-6-carboxylic acid | 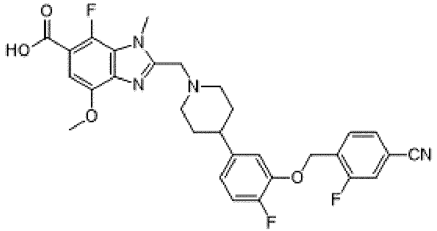 |
| 5-340 | 2-((4-(3-((4-Chloro-2-fluorobenzyl)oxy)-4-fluorophenyl)piperidin-1-yl)methyl)-7-fluoro-4-methoxy-1-methyl-1H-benzo[d]imidazole-6-carboxylic acid | 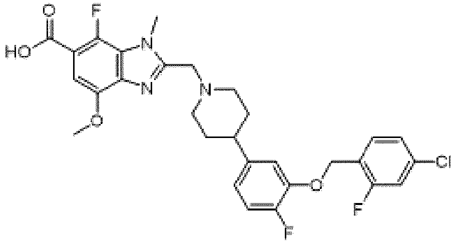 |
| 5-341 | 2-((4-(6-((4-Chloro-2-fluorobenzyl)oxy)pyridin-2-yl)piperidin-1-yl)methyl)-7-hydroxy-1-methyl-1H-benzo[d]imidazole-6-carboxylic acid | 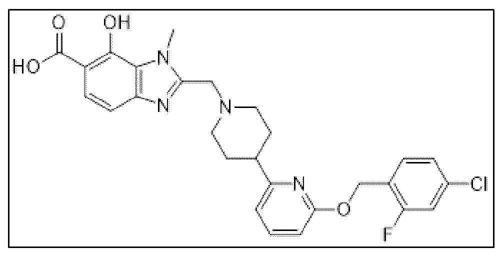 |
| 5-342 | 2-(4-(6-((4-cyano-2-fluorobenzyl)oxy)pyridin-2-yl)-2-fluorobenzyl)-4-methoxy-1-methyl-1H-benzo[d]imidazole-6-carboxylic acid | 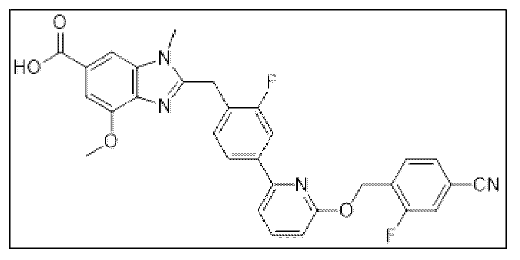 |
| 5-343 | 2-((4-(6-((4-Chloro-2-fluorobenzyl)oxy)pyridin-2-yl)piperidin-1-yl)methyl)-4-(2-hydroxyethoxy)-1-methyl-1H-benzo[d]imidazole-6-carboxylic acid | 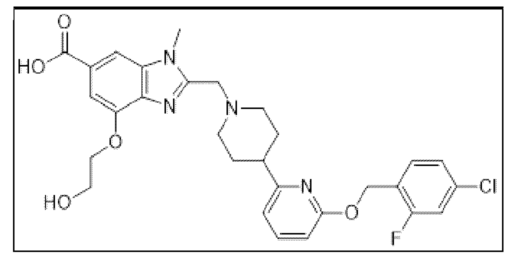 |
| 5-344 | 2-((4-(6-((4-Cyano-2-methoxybenzyl)oxy)pyridin-2-yl)piperidin-1-yl)methyl)-4-methoxy-1-methyl-1H-benzo[d]imidazole-6-carboxylic acid | 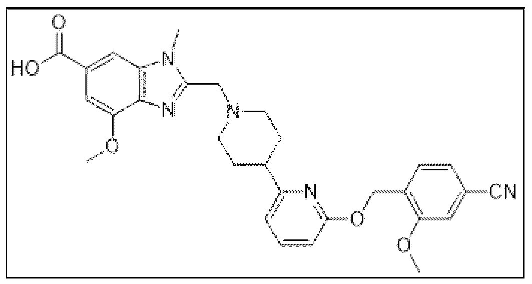 |
| 5-345 | 2-((4-(6-((4-Chloro-2-fluorobenzyl)oxy)pyridin-2-yl)piperidin-1-yl)methyl)-4-hydroxy-1-methyl-1H-benzo[d]imidazole-5-carboxylic acid | 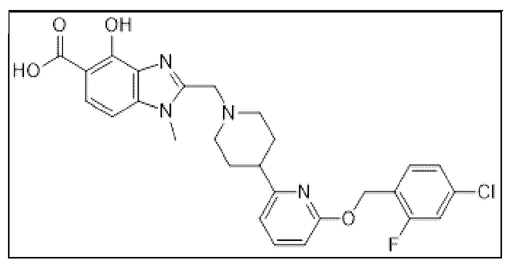 |
| 5-346 | 2-((4-(6-((4-Chloro-2-fluorobenzyl)oxy)pyridin-2-yl)piperidin-1-yl)methyl)-4-(fluoromethoxy)-1-(fluoromethyl)-1H-benzo[d]imidazole-6-carboxylic acid | 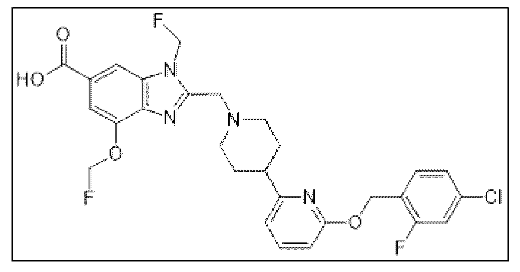 |
| 5-347 | 2-((4-(6-((4-Chloro-2-fluorobenzyl)oxy)pyridin-2-yl)piperidin-1-yl)methyl)-7-(fluoromethoxy)-1-(fluoromethyl)-1H-benzo[d]imidazole-5-carboxylic acid | 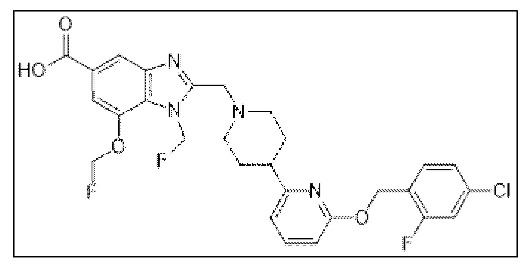 |
| 5-348 | 2-((4-(6-((4-Chloro-2-methoxybenzyl)oxy)pyridin-2-yl)piperidin-1-yl)methyl)-4-methoxy-1-methyl-1H-benzo[d]imidazole-6-carboxylic acid | 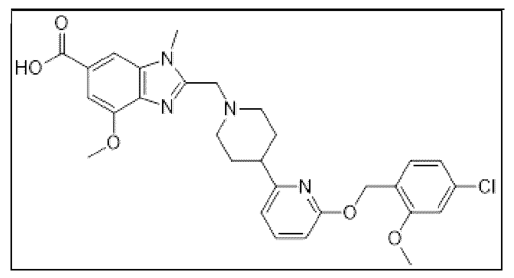 |
| 5-349 | 2-((4-(6-((4-Cyano-2-fluorobenzyl)oxy)pyridin-2-yl)piperidin-1-yl)methyl)-4-(methoxy-d3)-1-methyl-1H-benzo[d]imidazole-6-carboxylic acid | 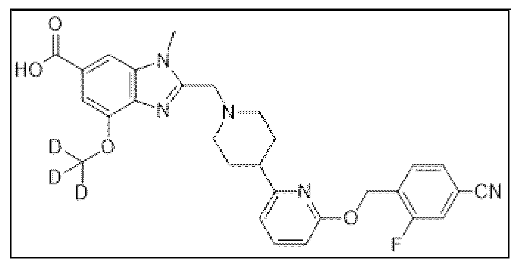 |
| 5-350 | 2-((4-(6-((6-Cyano-4-fluoropyridin-3-yl)methoxy)pyridin-2-yl)piperidin-1-yl)methyl)-4-(difluoromethoxy)-1-methyl-1H-benzo[d]imidazole-6-carboxylic acid | 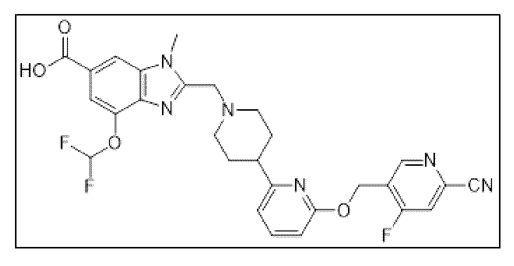 |
| 5-351 | 2-((4-(6-((4-Chloro-2-fluorobenzyl)oxy)pyridin-2-yl)piperidin-1-yl)methyl)-4-(1-fluoropropoxy)-1-methyl-1H-benzo[d]imidazole-6-carboxylic acid | 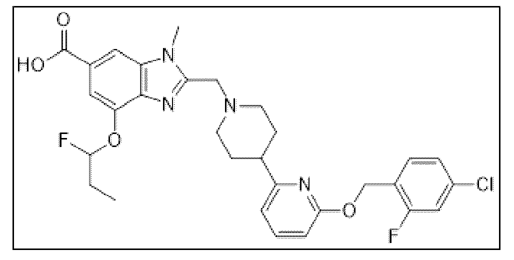 |
| 5-352 | 2-((4-(6-((4-Cyano-2-fluorobenzyl)oxy)pyridin-2-yl)piperidin-1-yl)methyl)-4-(4-fluorophenoxy)-1-methyl-1H-benzo[d]imidazole-6-carboxylic acid | 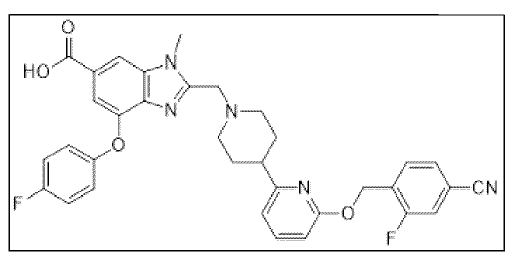 |
| 5-353 | 2-((4-(6-((4-Chloro-2-fluorobenzyl)oxy)pyridin-2-yl)piperidin-1-yl)methyl)-4-((1r,3r)-3-fluorocyclobutoxy)-1-methyl-1H-benzo[d]imidazole-6-carboxylic acid | 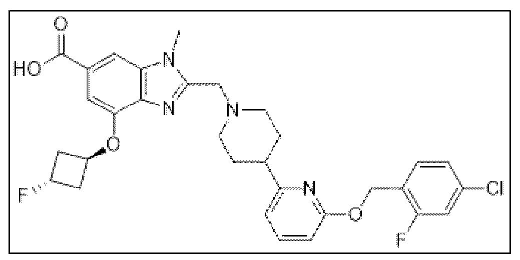 |
| 5-354 | 2-((4-(2-((4-Chloro-2-fluorobenzyl)oxy)-5-fluoropyrimidin-4-yl)piperidin-1-yl)methyl)-4-(difluoromethoxy)-1-methyl-1H-benzo[d]imidazole-6-carboxylic acid | 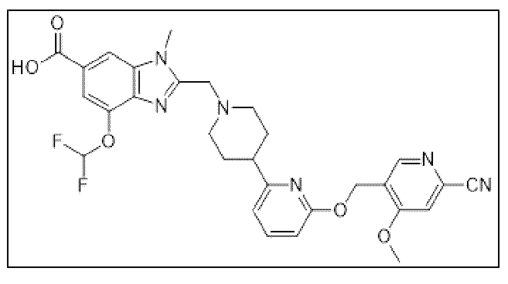 |
| 5-355 | 2-((4-(3-((4-Chloro-2-fluorobenzyl)oxy)pyridazin-4-yl)piperidin-1-yl)methyl)-4-(difluoromethoxy)-1-methyl-1H-benzo[d]imidazole-6-carboxylic acid | 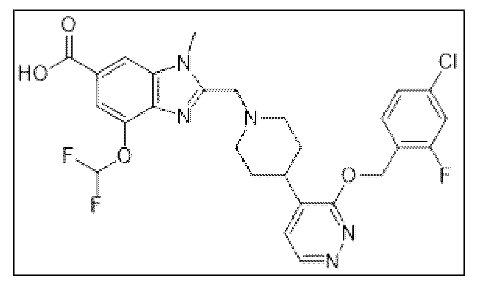 |
| 5-356 | 2-((4-(4-((4-Chloro-2-fluorobenzyl)oxy)-5-fluoropyrimidin-2-yl)piperidin-1-yl)methyl)-4-(difluoromethoxy)-1-methyl-1H-benzo[d]imidazole-6-carboxylic acid | 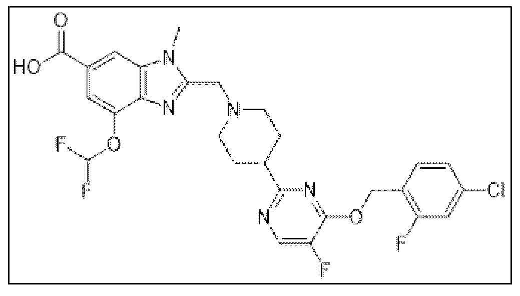 |
| 5-357 | 2-((4-(6-((4-Chloro-2-fluorobenzyl)oxy)pyridin-2-yl)piperidin-1-yl)methyl)-4-(cyclopropylmethoxy)-1-methyl-1H-benzo[d]imidazole-6-carboxylic acid | 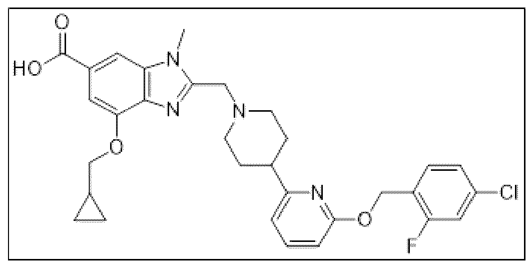 |
| 5-358 | 2-((4-(6-((4-Chloro-2-fluorobenzyl)oxy)pyridin-2-yl)piperidin-1-yl)methyl)-4-ethoxy-1-methyl-1H-benzo[d]imidazole-6-carboxylic acid | 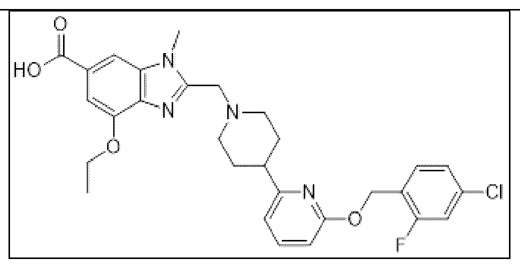 |
| 5-359 | -((4-(6-((4-Chloro-2-fluorobenzyl)oxy)pyridin-2-yl)piperidin-1-yl)methyl)-4-(1-fluoroethoxy)-1-methyl-1H-benzo[d]imidazole-6-carboxylic acid | 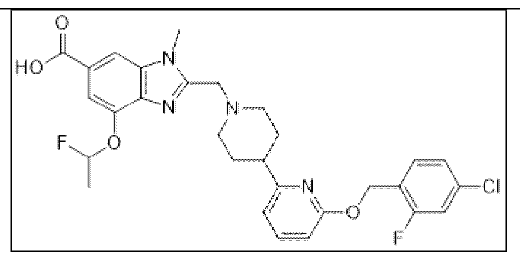 |
| 5-360 | (S)-2-((4-(6-((4-chloro-2-fluorobenzyl)oxy)pyridin-2-yl)piperidin-1-yl)methyl)-4-(1-fluoroethoxy)-1-methyl-1H-benzo[d]imidazole-6-carboxylic acid | 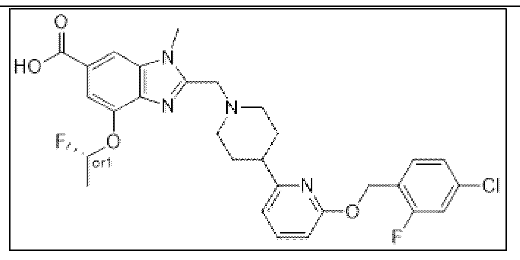 |
| 5-361 | (R)-2-((4-(6-((4-Chloro-2-fluorobenzyl)oxy)pyridin-2-yl)piperidin-1-yl)methyl)-4-(1-fluoroethoxy)-1-methyl-1H-benzo[d]imidazole-6-carboxylic acid | 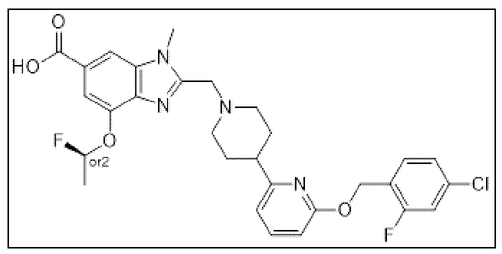 |
| 5-362 | 2-((3-(6-((4-Chloro-2-fluorophenoxy)methyl)pyridi n-2-yl)pyrrolidin-1-yl)methyl)-4-(difluoromethoxy)-1-methyl-1H-benzo[d]imidazole-6-carboxylic acid | 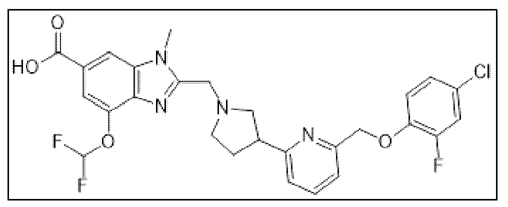 |
| 5-363 | 2-(((1r,4r)-4-(6-((4-Chloro-2-fluorobenzyl)oxy)pyridin-2-yl)cyclohexyl)(methyl)amino )-4-methoxy-1-methyl-1H-benzo[d]imidazole-6-carboxylic acid | 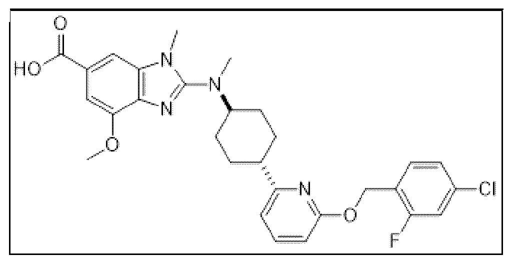 |
| 5-364 | 2-((4-(2-((4-Chloro-2-fluorobenzyl)oxy)pyridin-3-yl)piperidin-1-yl)methyl)-4-(difluoromethoxy)-1-methyl-1H-benzo[d]imidazole-6-carboxylic acid | 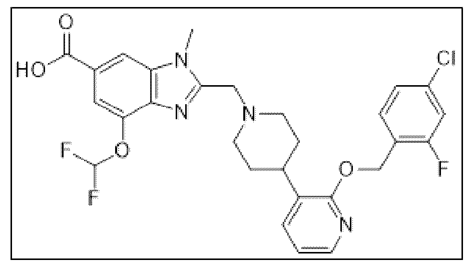 |
| 5-365 | 2-((4-(6-((4-Cyclopropyl-2-fluorobenzyl)oxy)pyridin-2-yl)piperidin-1-yl)methyl)-4-(difluoromethoxy)-1-methyl-1H-benzo[d]imidazole-6-carboxylic acid | 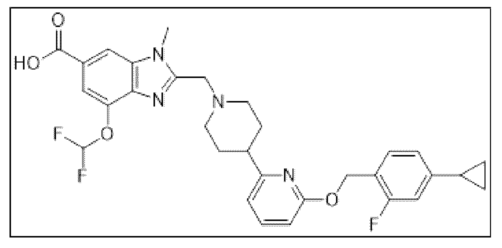 |
| 5-366 | 2-((4-(6-((4-Cyano-2,5-difluorobenzyl)oxy)pyridin-2-yl)piperidin-1-yl)methyl)-4-(difluoromethoxy)-1-methyl-1H-benzo[d]imidazole-6-carboxylic acid | 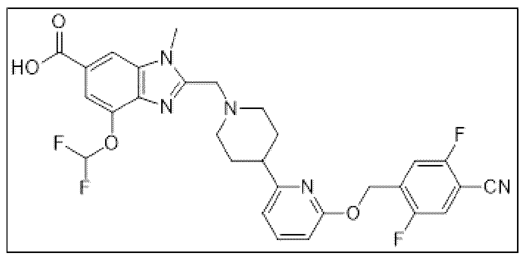 |
| 5-367 | 2-(4-((6-((4-chloro-2-fluorophenoxy)methyl)pyridi n-2-yl)oxy)-2-fluorobenzyl)-4-(difluoromethoxy)-1-methyl-1H-benzo[d]imidazole-6-carboxylic acid | 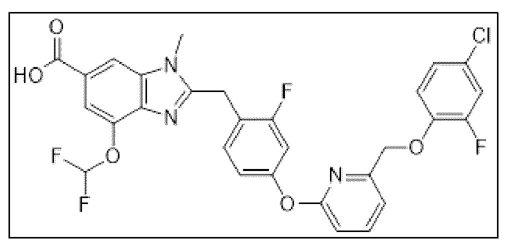 |
| 5-368 | S)-2-((4-(2-(4-Chloro-2-fluorophenyl)chroman-8-yl)piperidin-1-yl)methyl)-4-(difluoromethoxy)-1-methyl-1H-benzo[d]imidazole-6-carboxylic acid | 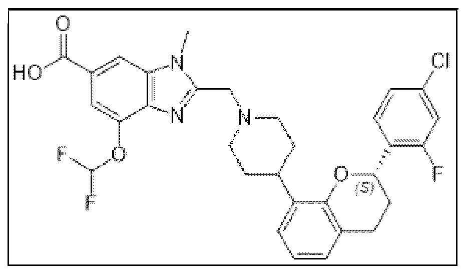 |
| 5-369 | (R)-2-((4-(2-(4-Chloro-2-fluorophenyl)chroman-8-yl)piperidin-1-yl)methyl)-4-(difluoromethoxy)-1-methyl-1H-benzo[d]imidazole-6-carboxylic acid | 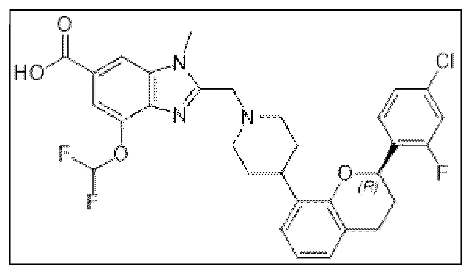 |
| 5-370 | 3-(2-((4-(6-((4-Chloro-2-fluorobenzyl)oxy)pyridin-2-yl)piperidin-1-yl)methyl)-4-(difluoromethoxy)-1-methyl-1H-benzo[d]imidazol-6-yl)-1,2,4-thiadiazol-5(4H)-one | 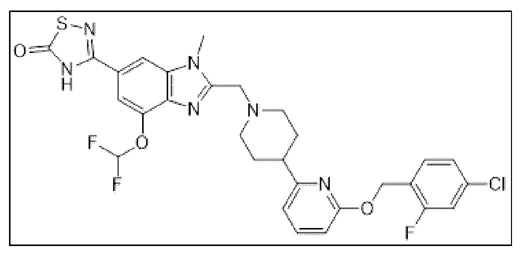 |
| 5-371 | 2-(((1r,4r)-4-(6-((4-Chloro-2-fluorobenzyl)oxy)pyridin-2-yl)cyclohexyl)amino)-4-(difluoromethoxy)-1-methyl-1H-benzo[d]imidazole-6-carboxylic acid | 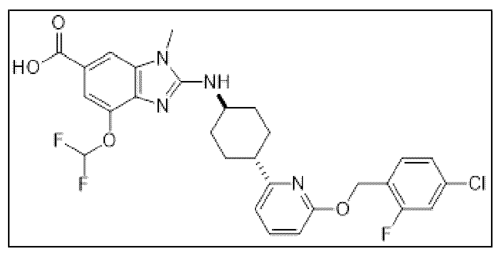 |
| 5-372 | 2-(((1r,4r)-4-(6-((4-Chloro-2-fluorobenzyl)oyridinedin-2-yl)cyclohexyl)(methyl-d3)amino)-4-(difluoromethoxy)-1-methyl-1H-benzo[d]imidazole-6-carboxylic acid | 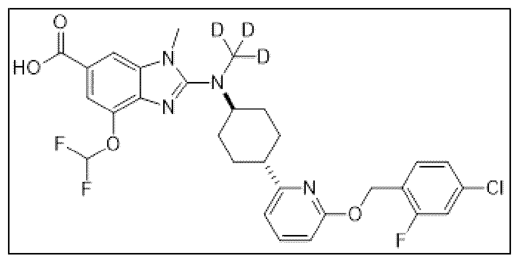 |
| 5-373 | 2-((4-(4-(4-Chloro-2-fluorobenzyl)-3,4-dihydro-2H-benzo[b][1,4]oxazin-6-yl)piperidin-1-yl)methyl)-4-(difluoromethoxy)-1-methyl-1H-benzo[d]imidazole-6-carboxylic acid | 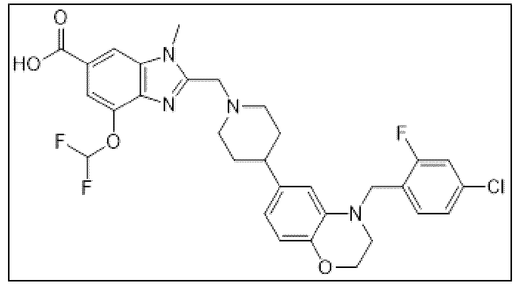 |
| 5-374 | 2-((4-(6-((4-Chloro-2-fluorobenzyl)oxy)pyrazin-2-yl)piperidin-1-yl)methyl)-4-(difluoromethoxy)-1-methyl-1H-benzo[d]imidazole-6-carboxylic acid | 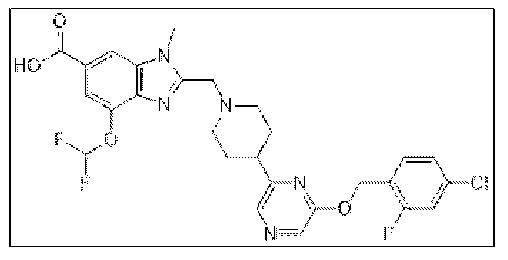 |
| 5-375 | 2-((4-(2-((4-Chloro-2-fluorobenzyl)oxy)-5-fluoropyrimidin-4-yl)piperidin-1-yl)methyl)-4-(difluoromethoxy)-1-methyl-1H-benzo[d]imidazole-6-carboxylic acid | 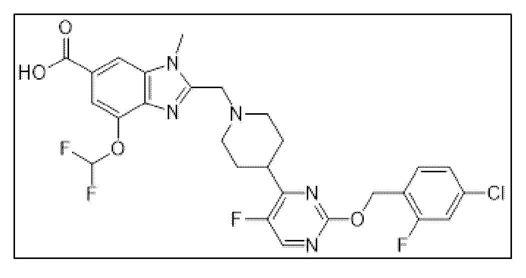 |
| 5-376 | (S)-2-((4-(6-((4-Cyano-2-fluorobenzyl)oxy)pyridin-2-yl)piperidin-1-yl)methyl)-4-hydroxy-1-(oxetan-2-ylmethyl)-1H-benzo[d]imidazole-5-carboxylic acid | 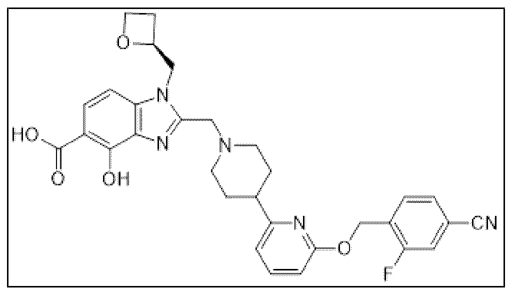 |
| 5-377 | (S)-2-((4-(6-((4-Cyano-2-fluorobenzyl)oxy)pyridin-2-yl)piperidin-1-yl)methyl)-7-hydroxy-1-(oxetan-2-ylmethyl)-1H-benzo[d]imidazole-6-carboxylic acid | 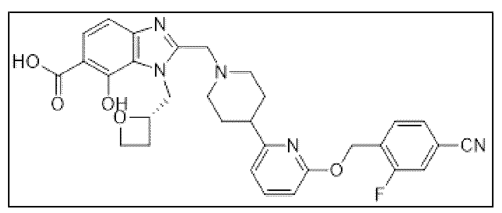 |
| 5-378 | (S)-2-((4-(6-((4-Cyano-2-fluorobenzyl)oxy)pyridin-2-yl)piperidin-1-yl)methyl)-1-(oxetan-2-ylmethyl)-1H-imidazo[4,5-c]pyridine-6-carboxylic acid | 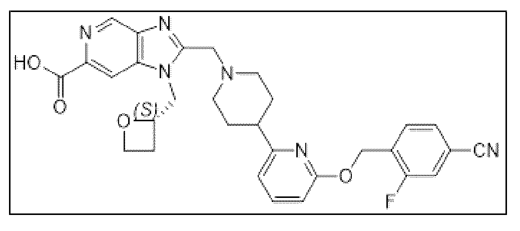 |
| 5-379 | 2-((4-(6-((4-Chloro-2-fluorobenzyl)oxy)pyridin-2-yl)piperidin-1-yl)methyl)-4-((1s,3s)-3-fluorocyclobutoxy)-1-methyl-1H-benzo[d]imidazole-6-carboxylic acid | 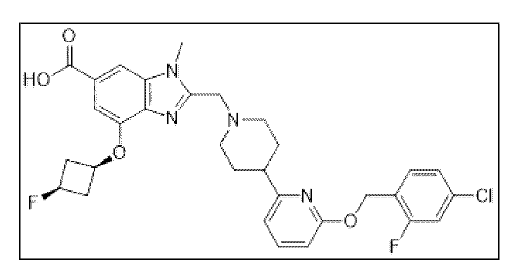 |
| 5-380 | 2-((4-(6-((4-Chloro-2-fluorobenzyl)oxy)pyridin-2-yl)piperidin-1-yl)methyl)-4-methoxy-1-methyl-1H-imidazo[4,5-c]pyridine-6-carboxylic acid | 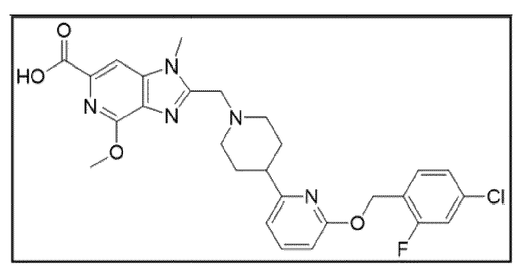 |
| 5-381 | 2-((4-(6-((4-Chloro-2-fluorophenyl)methoxy-d2)pyridin-2-yl)piperidin-1-yl)methyl-d2)-4-(ethoxy-1,1-d2)-1-methyl-1H-benzo[d]imidazole-6-carboxylic acid | 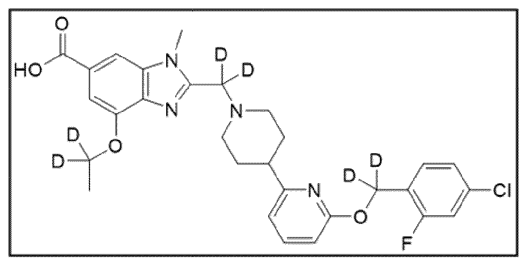 |
| 5-382 | (S)-5-(2-((4-(6-((4-Chloro-2-fluorobenzyl)oxy)pyridin-2-yl)piperidin-1-yl)methyl)-4-methoxy-1-(oxetan-2-ylmethyl)-1H-benzo[d]imidazol-6-yl)isoxazol-3(2H)-one | 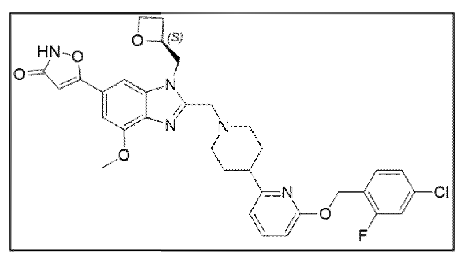 |
| 5-383 | 2-((4-(6-((4-Chloro-2-fluorobenzyl)oxy)pyridin-2-yl)piperazin-1-yl)methyl)-4-methoxy-1-methyl-1H-benzo[d]imidazole-6-carboxylic acid | 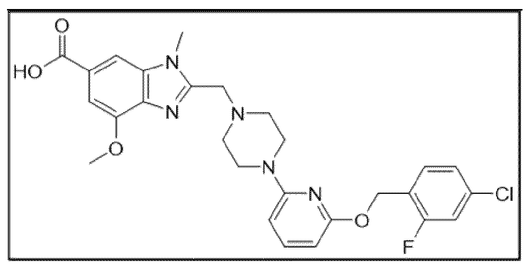 |
| 5-384 | 4-(((6-(1-((4-Ethoxy-1-methyl-6-(3-oxo-2,3-dihydroisoxazol-5-yl)-1H-benzo[d]imidazol-2-yl)methyl)piperidin-4-yl)pyridin-2-yl)oxy)methyl)-3-fluorobenzonitrile | 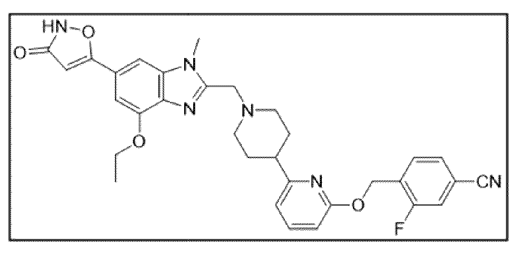 |
| 5-385 | 4-(Difluoromethoxy)-1-methyl-2-((4-(6-((4-(trifluoromethyl)benzyl)oxy) pyridin-2-yl)piperidin-1-yl)methyl)-1H-benzo[d]imidazole-6-carboxylic acid | 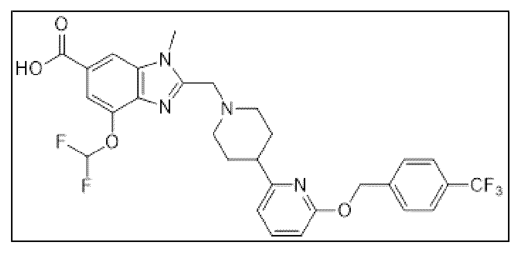 |
| 5-386 | 2-((4-((S)-2-(5-Chloropyridin-2-yl)-2-methylbenzo[d][1,3]dioxol-4-yl)piperidin-1-yl)methyl)-4-(1,1-difluoroethyl)-1-(((S)-oxetan-2-yl)methyl)-1H-benzo[d]imidazole-6-carboxylic acid | 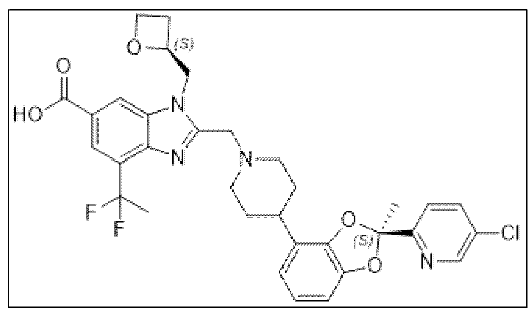 |
| 5-387 | 2-(((1S,5R,6S)-5-(6-((4-cyano-2-fluorobenzyl)oxy)pyridin-2-yl)-2-azabicyclo[4.1.0]heptan-2-yl)methyl)-4-methoxy-1-(((S)-oxetan-2-yl)methyl)-1H-benzo[d]imidazole-6-carboxylic acid | 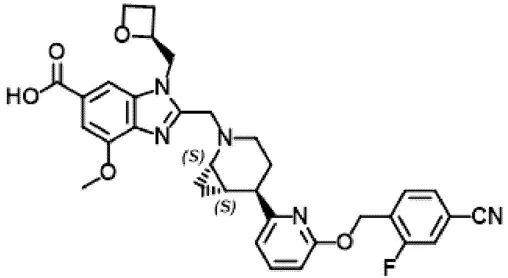 |

### THRβ Agonists

In some embodiments, the THRβ agonist administered to the patient in need thereof is resmetirom (MGL-3196, Madrigal Therapeutics). In some embodiments, the THRβ agonist administered to the patient in need thereof is ALG-055009 (Aligo).

In some embodiments, the THRβ agonist is: 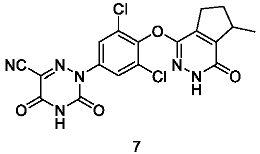 (Compound 7) or a pharmaceutically acceptable salt thereof.

In some embodiments, the THRβ agonist is 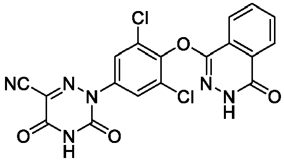 (Compound 9) which is 2-(3,5-dichloro-4-((4-oxo-3,4-dihydrophthalazin-1-yl)oxy)phenyl)-3,5-dioxo-2,3,4,5-tetrahydro-1,2,4-triazine-6-carbonitrile, or a pharmaceutically acceptable salt thereof. In some embodiments, the THRβ agonist is 2-(3,5-dichloro-4-((4-oxo-3,4-dihydrophthalazin-1-yl)oxy)phenyl)-3,5-dioxo-2,3,4,5-tetrahydro-1,2,4-triazine-6-carbonitrile potassium salt. In some embodiments, the THRβ agonist is 2-(3,5-dichloro-4-((4-oxo-3,4-dihydrophthalazin-1-yl)oxy)phenyl)-3,5-dioxo-2,3,4,5-tetrahydro-1,2,4-triazine-6-carbonitrile sodium salt.

### Pharmaceutically Acceptable Compositions and Formulations

Pharmaceutically acceptable compositions or simply "pharmaceutical compositions" of any of the compounds detailed herein are embraced by this invention. Thus, the invention includes pharmaceutical compositions comprising an GLP-1R agonist (such as the compound of Formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I''), (I‴*), (I‴), (I*), (I'), (I), (I-P01), (II**), or (II*), or a pharmaceutically acceptable salt thereof), the THRβ agonist, and a pharmaceutically acceptable carrier or excipient. In some embodiments, the pharmaceutically acceptable salt is an acid addition salt, such as a salt formed with an inorganic or organic acid. Pharmaceutical compositions according to the invention may take a form suitable for oral, buccal, parenteral, nasal, topical or rectal administration or a form suitable for administration by inhalation.

A compound as detailed herein may in one aspect be in a purified form and compositions comprising a compound in purified forms are detailed herein. Compositions comprising a compound as detailed herein or a salt thereof are provided, such as compositions of substantially pure compounds. In some embodiments, a composition containing a compound as detailed herein or a salt thereof is in substantially pure form. In some embodiments, "substantially pure" intends a composition that contains no more than 35% impurity, wherein the impurity denotes a compound other than the compound comprising the majority of the composition or a salt thereof. For example, a composition of a substantially pure compound intends a composition that contains no more than 35% impurity, wherein the impurity denotes a compound other than the compound or a salt thereof. In some embodiments, a composition of substantially pure compound or a salt thereof is provided wherein the composition contains no more than 25% impurity. In some embodiments, a composition of substantially pure compound or a salt thereof is provided wherein the composition contains or no more than 20% impurity. In some embodiments, a composition of substantially pure compound or a salt thereof is provided wherein the composition contains or no more than 10% impurity. In some embodiments, a composition of substantially pure compound or a salt thereof is provided wherein the composition contains or no more than 5% impurity. In some embodiments, a composition of substantially pure compound or a salt thereof is provided wherein the composition contains or no more than 3% impurity. In some embodiments, a composition of substantially pure compound or a salt thereof is provided wherein the composition contains or no more than 1% impurity. In some embodiments, a composition of substantially pure compound or a salt thereof is provided wherein the composition contains or no more than 0.5% impurity. In some embodiments, a composition of substantially pure compound means that the composition contains no more than 15%, in some embodiments no more than 10%, in some embodiments no more than 5% , in some embodiments no more than 3% and, in some embodiments no more than 1% impurity, which impurity may be the compound in a different stereochemical form.

In some embodiments, the compounds herein are synthetic compounds prepared for administration to a subject such as a human. In some embodiments, compositions are provided containing a compound in substantially pure form. In some embodiments, the invention embraces pharmaceutical compositions comprising a compound detailed herein and a pharmaceutically acceptable carrier or excipient. In some embodiments, methods of administering a compound are provided. The purified forms, pharmaceutical compositions and methods of administering the compounds are suitable for any compound or form thereof detailed herein.

The compounds may be formulated for any available delivery route, including an oral, mucosal (e.g., nasal, sublingual, vaginal, buccal or rectal), parenteral (e.g., intramuscular, subcutaneous or intravenous), topical or transdermal delivery form. A compound may be formulated with suitable carriers to provide delivery forms that include, but are not limited to, tablets, caplets, capsules (such as hard gelatin capsules or soft elastic gelatin capsules), cachets, troches, lozenges, gums, dispersions, suppositories, ointments, cataplasms (poultices), pastes, powders, dressings, creams, solutions, patches, aerosols (e.g., nasal spray or inhalers), gels, suspensions (e.g., aqueous or non-aqueous liquid suspensions, oil-in-water emulsions or water-in-oil liquid emulsions), solutions and elixirs.

Compounds described herein can be used in the preparation of a formulation, such as a pharmaceutical formulation, by combining the compounds as active ingredients with a pharmaceutically acceptable carrier, such as those mentioned above. Depending on the therapeutic form of the system (e.g., transdermal patch vs. oral tablet), the carrier may be in various forms. In addition, pharmaceutical formulations may contain preservatives, solubilizers, stabilizers, re-wetting agents, emulgators, sweeteners, dyes, adjusters, and salts for the adjustment of osmotic pressure, buffers, coating agents or antioxidants. Formulations comprising the compound may also contain other substances which have valuable therapeutic properties. Pharmaceutical formulations may be prepared by known pharmaceutical methods. Suitable formulations can be found, e.g., in Remington: The Science and Practice of Pharmacy, Lippincott Williams & Wilkins, 21st ed. (2005).

Compounds as described herein may be administered to a subject (e.g., a human) in a form of generally accepted oral compositions, such as tablets, coated tablets, and gel capsules in a hard or in soft shell, emulsions or suspensions. Examples of carriers, which may be used for the preparation of such compositions, are lactose, corn starch or its derivatives, talc, stearate or its salts, etc. Acceptable carriers for gel capsules with soft shell are, for instance, plant oils, wax, fats, semisolid and liquid polyols, and so on. In addition, pharmaceutical formulations may contain preservatives, solubilizers, stabilizers, re-wetting agents, emulgators, sweeteners, dyes, adjusters, and salts for the adjustment of osmotic pressure, buffers, coating agents or antioxidants.

### Methods of Use and Uses

In some embodiments, the present disclosure is directed to co-administration of a GLP-1R agonist and THRβ agonist to a subject resulting in a loss in fat mass in the subject with little or no reduction in lean mass in the subject.

In one embodiment the combinations of the present disclosure are directed to the prevention or treatment of obesity, for reducing body weight and/or food intake, or for inducing satiety in a subject in need thereof.

In some embodiments, the combinations of the present disclosure are directed to treating or preventing obesity, reducing body fat percentage, increasing lean mass percentage of total weight, or for inducing satiety in a subject in need thereof.

In some embodiments, the combinations of the present disclosure are directed to treating or preventing obesity, reducing body fat percentage, increasing lean mass percentage of total weight, or for inducing satiety in a subject in need thereof.

In some embodiments, the combinations of the present disclosure are directed to treating obesity in a subject in need thereof.

In some embodiments, the combinations of the present disclosure are directed to treating or preventing obesity in a subject in need thereof.

In some embodiments, the combinations of the present disclosure are directed to reducing body fat, increasing lean mass percentage of total weight, or inducing satiety in a subject in need thereof.

In some embodiments, the present disclosure provides a combination for use in treating or preventing obesity, for reducing body fat, and/or for increasing lean mass percentage of total weight, in a subject in need thereof comprising the administration of a THR-B agonist (e.g., Compound 9) and a GLP-1R agonist which results in a synergistic effect compared to the administration of a GLP-1R agonist alone.

In some embodiments, the present disclosure provides a combination for use in treating obesity, for reducing body fat, and/or for increasing lean mass percentage of total weight, in a subject in need thereof comprising the administration of a THR-B agonist (e.g., Compound 9) and a GLP-1R agonist which results in a synergistic effect compared to the administration of a GLP-1R agonist alone.

In some embodiments, the present disclosure provides a combination for use in decreasing food intake in a subject in need thereof. In some embodiments, administration of a compound disclosed herein causes the subject's food intake to be reduced at least 10%, e.g., at least 20%, at least 30%, at least 40%, at least 50%, at least 60%, at least 70%, at least 75%, at least 80%, at least 85%, at least 90%, or at least 95% relative to the subject's food intake in the absence of a compound disclosed herein. In some embodiments, the subject's food intake is reduced, e.g., reduced by at least 10%, at least 20%, at least 30%, at least 40%, at least 50%, at least 60%, at least 70%, at least 75%, at least 80%, at least 85%, at least 90%, or at least 95%, for at least 1 hour following administration, e.g., at least 1 hour, at least 2 hours, at least 3 hours, at least 4 hours, at least 5 hours, at least 6 hours, at least 12 hours, at least 1 day, or at least 2 days following administration.

In accordance with the present application, a compound described herein, or a pharmaceutically acceptable salt thereof, can be administered by any suitable route in the form of a pharmaceutical composition adapted to such a route, and in a dose effective for the treatment intended. In some embodiments, it is a compound of any embodiment of Formula (I) or selected from the compounds of Table 1, or a pharmaceutically acceptable salt thereof. The compounds and/or compositions described herein may be administered orally, rectally, vaginally, parenterally, or topically.

In some embodiments, the compounds and/or compositions may be administered orally. Oral administration may involve swallowing, so that the compound enters the gastrointestinal tract, or buccal or sublingual administration may be employed by which the compound enters the bloodstream directly from the mouth.

In some embodiments, the compounds and/or compositions may be administered directly into the bloodstream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

In some embodiments, the compounds and/or compositions may be administered topically to the skin or mucosa, that is, dermally or transdermally. In some embodiments, the compounds and/or compositions may be administered intranasally or by inhalation. In some embodiments, the compounds and/or compositions may be administered rectally or vaginally. In some embodiments, the compounds and/or compositions may be administered directly to the eye or ear.

The dosage regimen for the compounds and/or compositions described herein is based on a variety of factors, including the type, age, weight, sex and medical condition of the patient; the severity of the condition; the route of administration; and the activity of the particular compound employed. Thus, the dosage regimen may vary widely. In some embodiments, the total daily dose of the compounds of the present application is typically from about 0.001 to about 100 mg/kg (i.e., mg compound per kg body weight) for the treatment of the indicated conditions discussed herein. In one embodiment, total daily dose of the compounds of the present application is from about 0.01 to about 30 mg/kg, and in another embodiment, from about 0.03 to about 10 mg/kg, and in yet another embodiment, from about 0.1 to about 3. It is not uncommon that the administration of the compounds of the present application will be repeated a plurality of times in a day (typically no greater than 4 times). Multiple doses per day typically may be used to increase the total daily dose, if desired.

Suitable dosages of the THRβ agonists described herein are also described in, e.g., PCT Pub. Nos. WO2021231646, WO2023086561, and WO2023083288.

In some embodiments, about 0.5 mg to about 100 mg of the THRβ agonist is administered to the subject. In some embodiments, about 0.5 mg to about 90 mg of the compound is administered to the subject. In some embodiments, about 1 mg to about 90 mg of the compound is administered to the subject. In some embodiments, about 3 mg to about 90 mg of the compound is administered to the subject. In some embodiments, about 0.5 mg to about 30 mg of the compound is administered to the subject. In some embodiments, about 1 mg to about 30 mg of the compound is administered to the subject. In some embodiments, about 3 mg to about 90 mg of the compound is administered to the subject. In some embodiments, about 1 mg to about 5 mg of the compound is administered to the subject. In some embodiments about 1 mg to about 3 mg of the compound is administered to the subject. In some embodiments about 5 mg to about 10 mg of the compound is administered to the subject. In some embodiments, about 10 mg to about 15 mg of the compound is administered to the subject. In some embodiments, about 15 mg to about 20 mg of the compound is administered to the subject. In some embodiments, about 20 mg to about 25 mg of the compound is administered to the subject. In some embodiments, about 25 mg to about 30 mg of the compound is administered to the subject. In some embodiments, about 1 mg of the compound is administered to the subject. In some embodiments, about 2 mg of the compound is administered to the subject. In some embodiments, about 3 mg of the compound is administered to the subject. In some embodiments, about 4 mg of the compound is administered to the subject. In some embodiments, about 5 mg of the compound is administered to the subject. In some embodiments, about 6 mg of the compound is administered to the subject. In some embodiments, about 7 mg of the compound is administered to the subject. In some embodiments, about 8 mg of the compound is administered to the subject. In some embodiments, about 9 mg of the compound is administered to the subject. In some embodiments, about 10 mg of the compound is administered to the subject. In some embodiments, about 15 mg of the compound is administered to the subject. In some embodiments, about 20 mg of the compound is administered to the subject. In some embodiments, about 25 mg of the compound is administered to the subject. In some embodiments, about 30 mg of the compound is administered to the subject. In some embodiments, the compound is Compound 9 as described herein.

### Combinations

In further embodiments, the pharmaceutical compositions provided herein may be co-formulated with other active ingredients which do not impair the desired therapeutic action, or with substances that supplement the desired action. In some embodiments, the GLP-1R agonist and the THRβ agonist are administered in temporal proximity (e.g., the GLP-1R agonist and the THRβ agonist can be administered simultaneously). Accordingly, the present disclosure provides a the combination for use in treating obesity or effectuating weight loss comprising administering the GLP-1R agonist and the THRβ agonist in temporal proximity.

In some embodiments, a GLP-1R agonist of Formula (I-1), (I-1a), (I-5) (I**), or (II*), or a pharmaceutically acceptable salt thereof is administered in temporal proximity with the THRβ agonist or a pharmaceutically acceptable salt thereof.

In some embodiments, a GLP-1R agonist of Formula (I-1), (I-1a), (I-5), (I**), or (II*), or a pharmaceutically acceptable salt thereof is administered in temporal proximity with the THRβ agonist or a pharmaceutically acceptable salt thereof.

some embodiments, a GLP-1R agonist of Formula (II**), or a pharmaceutically acceptable salt thereof is administered in temporal proximity with the THRβ agonist or a pharmaceutically acceptable salt thereof.

In some embodiments, a GLP-1R agonist of Formula (II**), or a pharmaceutically acceptable salt thereof is administered in temporal proximity with the THRβ agonist.

In some embodiments, the THRβ agonist, or a pharmaceutically acceptable salt thereof, is administered in temporal proximity with a GLP-1R agonist (such as the compound of Formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I"), (I‴*), (I‴), (I*), (I'), (I), (I-P01), (II**), or (II*), or a pharmaceutically acceptable salt thereof).

In some embodiments, "temporal proximity" means that administration of one therapeutic agent occurs within a time period before or after the administration of another therapeutic agent, such that the therapeutic effect of the one therapeutic agent overlaps with the therapeutic effect of the another therapeutic agent. In some embodiments, the therapeutic effect of the one therapeutic agent completely overlaps with the therapeutic effect of the other therapeutic agent. In some embodiments, "temporal proximity" means that administration of one therapeutic agent occurs within a time period before or after the administration of another therapeutic agent, such that there is a synergistic effect between the one therapeutic agent and the another therapeutic agent. "Temporal proximity" may vary according to various factors, including but not limited to, the age, gender, weight, genetic background, medical condition, disease history, and treatment history of the subject to which the therapeutic agents are to be administered; the disease or condition to be treated or ameliorated; the therapeutic outcome to be achieved; the dosage, dosing frequency, and dosing duration of the therapeutic agents; the pharmacokinetics and pharmacodynamics of the therapeutic agents; and the route(s) through which the therapeutic agents are administered. In some embodiments, "temporal proximity" means within 15 minutes, within 30 minutes, within an hour, within two hours, within four hours, within six hours, within eight hours, within 12 hours, within 18 hours, within 24 hours, within 36 hours, within 2 days, within 3 days, within 4 days, within 5 days, within 6 days, within a week, within 2 weeks, within 3 weeks, within 4 weeks, with 6 weeks, or within 8 weeks. In some embodiments, multiple administration of one therapeutic agent can occur in temporal proximity to a single administration of another therapeutic agent. In some embodiments, temporal proximity may change during a treatment cycle or within a dosing regimen.

In some embodiments, the THRβ agonist is administered to the patient prior to the GLP-1R agonist. In some embodiments, the THRβ agonist is administered to the patient 0-1 hours, 1-2 hours, 2-3 hours, 3-4 hours, 4-5 hours, or 5-6 hours prior to the GLP-1R agonist. In some embodiments, the THRβ agonist is administered to the patient 0-1 days, 1-2 days, 2-3 days, 3-4 days, 4-5 days, or 5-6 daysprior to the GLP-1R agonist. In some embodiments, the THRβ agonist reaches a steady state in a patient prior to the administration of the GLP-1R agonist. In some embodiments, the GLP-1R agonist is administered to the patient prior to the THRβ agonist. In some embodiments, the GLP-1R agonist is administered to the patient 0-1 days, 1-2 days, 2-3 days, 3-4 days, 4-5 days, or 5-6 days prior to the THRβ agonist. In some embodiments, the GLP-1R agonist reaches a steady state in a patient prior to the administration of the THRβ agonist. In some embodiments, the THR- β agonist is administered to the patient at substantially the same time as the GLP-1R agonist.

### Articles of Manufacture and Kits

The present disclosure further provides articles of manufacture comprising a compound described herein, or a salt thereof, a composition described herein, or one or more unit dosages described herein in suitable packaging. In certain embodiments, the article of manufacture is for use in any of the methods described herein. Suitable packaging (e.g., containers) is known in the art and includes, for example, vials, vessels, ampules, bottles, jars, flexible packaging and the like. An article of manufacture may further be sterilized and/or sealed.

The present disclosure further provides kits for carrying out the methods of the present disclosure, which comprises at least two compounds described herein, or a pharmaceutically acceptable salt thereof, or a composition comprising a compound described herein, or a pharmaceutically acceptable salt thereof. The kits may employ any of the compounds disclosed herein or a pharmaceutically acceptable salt thereof. In some embodiments, the kit employs an GLP-1R agonist (such as the compound of Formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I''), (I‴*), (I‴), (I*), (I'), (I), (I-P01), (II**), or (II*), or a pharmaceutically acceptable salt thereof) and a THRβ agonist described herein. The kits may be used for any one or more of the uses described herein, and, accordingly, may contain instructions for the treatment as described herein.

Kits generally comprise suitable packaging. The kits may comprise one or more containers comprising any compound described herein or a pharmaceutically acceptable salt thereof. Each component can be packaged in separate containers or some components can be combined in one container where cross-reactivity and shelf life permit. In some embodiments, the kit includes a container comprising the GLP-1R agonist (such as the compound of Formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I''), (I‴*), (I‴), (I*), (I'), (I), (I-P01), (II**), or (II*), or a pharmaceutically acceptable salt thereof) and THRβ agonist. In other embodiments, the kit includes a first container comprising GLP-1R agonist (such as the compound of Formula (I-1), (I-1a), (I-2), (I-3), (I-4), (I-5), (I-5a), (I-5b), (I-5c), (I-5d), (I-5e), (I-5f), (I-5g), (I-5h), (I-5i), (I-5j), (I-5k), (I-5l), (I-5m), (I-5n), (I-5o), (I-5p), (IQ), (IR), (IS), (IT), (IU), (IX), (IY), (IZ), (IAA), (IAB), (IAC), (IAD), (IAE), (IAG), (IAH), (IAI), (IAJ), (I**), (I"), (I‴*), (I‴), (I*), (I'), (I), (I-P01), (II**), or (II*), or a pharmaceutically acceptable salt thereof) and a second container comprising the THRβ agonist.

The kits may be in unit dosage forms, bulk packages (e.g., multi-dose packages) or subunit doses. For example, kits may be provided that contain sufficient dosages of a compound as disclosed herein (e.g., Compound 9), or a pharmaceutically acceptable salt thereof, and/or an additional pharmaceutically active compound useful for a disease detailed herein to provide effective treatment of a subject for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the compounds and instructions for use and be packaged in quantities sufficient for storage and use in pharmacies (*e.g*., hospital pharmacies and compounding pharmacies).

The kits may optionally include a set of instructions, generally written instructions, although electronic storage media (*e.g*., magnetic diskette or optical disk) containing instructions are also acceptable, relating to the use of component(s) of the methods of the present disclosure. The instructions included with the kit generally include information as to the components and their administration to a subject.

### General Biological Protocols

### Non-alcoholic steatohepatitis (NASH) mouse model

This mouse model is fed a high fat diet that results in nonalcoholic steatohepatis (NASH) disease and increased weight.

### Liver biopsy

Mice are anesthetized and a small abdominal incision is made in the midline and the left lateral lobe of the liver is exposed. A cone shaped wedge of liver tissue is excised from the distal portion of the lobe and fixated in 10% neutral buffered formalin for histology. The cut surface of the liver is instantly electrocoagulated using bipolar coagulation.

### Blood sampling and plasma preparation

*In vivo* blood samples: Samples are collected at week 8, 30 minutes post-dose, 1 hour post-dose, 2 hours post-dose, 4 hours post-dose, 6 hours post-dose, and 24 hours post-dose (before the next dose). Tail-vein, tongue or cheek blood is collected in a Microvette tube of appropriate dimensions with anticoagulant and mixed. Blood is centrifuged. The plasma supernatants are transferred to new tubes and immediately frozen on dry ice. Termination blood samples: During anesthesia, the abdominal cavity is opened, and cardiac blood is drawn with a syringe with anticoagulant and mixed. Blood is centrifuged and plasma supernatants are transferred to new tubes and immediately frozen on dry ice.

### Tissue sampling

### Liver (NASH)

After the animal has been terminated, the liver is collected and weighed.

The liver is divided into left lateral lobe, medial lobe, right lateral lobe, and caudate lobe.

The *Post-biopsy* piece is cut from the left lateral lobe, 4 mm from the prebiopsy site with an edge. The tissue is collected in paraformaldehyde.
The *Liver Sponsor* piece is dissected from the left medial lobe, put in a tube and placed directly into liquid nitrogen.

The *Liver RNA Sequencing (RNAseq)* piece is dissected from the center of the left lateral lobe, snap frozen (put directly in liquid nitrogen in Nunc filter) and collected in a pre-cooled tube which is put in liquid nitrogen.

The *Liver TG TC* piece is dissected from the right medial lobe, with no edges, on the opposite side of the sponsor piece. The samples are weighed individually and collected in tubes before placed in liquid nitrogen.

The *Liver Extra* piece is dissected from the right lateral lobe and collected in tubes before placed in liquid nitrogen, to have as backup tissue from the study and can be used for re-analysis if necessary.

### Biopsy processing

### Microtome sectioning of Formalin fixed paraffin-embedded (FFPE) biopsies

FFPE biopsies are placed in 10% neutral buffered formalin then transferred to 70% EtOH. The FFPE biopsies are then placed in the Histokinette to infiltrate prior to embedding in blocks. Biopsy tissues are cut at 3µm on a microtome and the sections are mounted on Superfrost Plus slides.

### NAFLD Activity Score (NAS) and Fibrosis stage

Liver samples stained with Hematoxylin and Eosin (H&E) or Picro Sirius Red (PSR) are given a score for NAS and fibrosis stage respectively using the clinical criteria outlined by Kleiner et al. 2005. Total NAS represents the sum of scores for steatosis, inflammation, and ballooning, and ranges from 0-8. Adapted from: Design and validation of a histological scoring system for nonalcoholic fatty liver disease, Kleiner et al., Hepatology 41; 2005

### Histological staining procedures

Slides with paraffin embedded sections are deparaffinated in xylene and rehydrated in series of graded ethanol.

Hematoxylin & Eosin (H&E) staining: Slides are incubated in Mayer's Hematoxylin (Dako), washed in tap water, stained in Eosin Y solution (Sigma-Aldrich), dehydrated and cover slipped.

Sirius red (PSR) staining: Slides are incubated in Weigert's iron hematoxylin (Sigma-Aldrich), washed in tap water, stained in Picro-sirius red (Sigma-Aldrich) and washed twice in acidified water. Excess water is removed by shaking the slides and the slides are then dehydrated in three changes of 100% ethanol, cleared in xylene and cover slipped.

Immunohistochemistry using single chromogen: IHC is performed using standard procedures. Briefly, after antigen retrieval and blocking of endogenous peroxidase activity, slides are incubated with primary antibody. The primary antibody is detected using a polymeric HRP-linker antibody conjugate. Next, the primary antibody is visualized with DAB as chromogen. Finally, sections are counterstained in hematoxylin and cover slipped. Slides are scanned under a 20X objective in a ScanScope AT slide scanner (Aperio).

### Gene expression analysis using RNAseq

*RNA isolation:* Tissue is collected and snap-frozen in liquid nitrogen. Samples are stored at -70°C until processing. RNA is isolated using the NucleoSpin^{®} kit (MACHEREY-NAGEL).

*Library preparation and sequencing:* A total of 10 ng-1 µg purified RNA from each sample is used to generate a cDNA library using the NEBNext^{®} Ultra^{™} II Directional RNA Library Prep Kit for Illumina (New England Biolabs). cDNA libraries are then sequenced on a NextSeq 500 using NextSeq 500/550 High Output Kit V2 (Illumina).

*Data analysis:* The sequencing data is aligned to the genome of the animal species obtained from the Ensembl database using the Spliced Transcripts Alignment to a Reference (STAR) software. For the bioinformatic analysis, the quality of the data is evaluated using the standard RNA-sequencing quality control parameters, the inter- and intra-group variability is evaluated using principal component analysis and hierarchical clustering and the differentially expressed genes are identified using the R-package DESeq2. Downstream analyses such as pathway analysis or target identification are performed as agreed with the sponsor.

### Blood and plasma assays

Alanine transaminase (ALT), Aspartate transaminase (AST), Triglycerides (TG) and Total Cholesterol (TC): Blood samples are collected in heparinized tubes and plasma is separated and stored at -70°C until analysis. Samples are measured using commercial kits.

Cytokeratin 18 (CK18-M30): Blood samples are collected in EDTA tubes and plasma is separated and stored at -70°C until analysis. CK18 is measured using a commercial ELISA kit.

Insulin: Blood samples are collected in heparinized tubes and plasma is separated and stored at -70°C until analysis. Insulin is measured using a commercial MSD platform.

TIMP-1: Blood samples are collected in EDTA tubes and plasma is separated and stored at -70°C until analysis. TIMP-1 is measured using a commercial ELISA kit.

PIIINP: Blood samples are collected in EDTA tubes and plasma is separated and stored at -70°C until analysis. PIIINP is measured using a commercial ELISA kit.

### Tissue assays

Triglycerides (TG) and Total cholesterol (TC): Liver samples are homogenized and TG and TC is extracted. The samples are centrifuged and the TG and TC content is measured in the supernatant using commercial kits.

### Oral Glucose Tolerance Test (OGTT)

On the day of the OGTT, random blood glucose and body weight of the mice are measured, mice are dosed with corresponding compounds or vehicle in the morning before fasting, then the mice are fasted for 6 hours, basal fasting glucose is measured by tail vein nick thereafter. Glucose is orally gavaged with 2 g/kg at a dose volume of 5 mL/kg. Blood glucose levels are measured at 0 (pre-dose), 15-, 30-, 60-, and 120-min post glucose dosing. The second dose is administrated after OGTT treatment.

### Terminal plasma and tissue collection

The body weight and food intake of all mice is measured and then fasted for 6 hours, all mice are euthanized with CO₂ and bled via cardiac puncture. Blood samples will be processed for serum by centrifugation and stored for TC/TG/LDL/HDL/AST/ALT analysis. Blood samples are mixed with K₂EDTA anticoagulant and processed for plasma by centrifugation stored for insulin and other possible analysis.

The weight of whole liver and epididymal fat are weighed and recorded. Left lobule of liver is fixed in formalin. The remaining liver is flash frozen and stored. Pancreas is collected and fixed in formalin. Brown adipose, subcutaneous white adipose, and epididymal white adipose tissues and the brain hypothalamus region are frozen with liquid nitrogen and preserved and stored for optional qPCR analysis.

### Hypothalmus collection procedure

The brain tissue is placed in the mold, and the olfactory bulb is placed in the center of the groove. The brain tissue is sliced into 8 coronal sections evenly spaced at 2 mm intervals. The third section (6-8mm) of the coronal brain tissue is taken out, and the section is cut along the boundary of the purple part with a scalpel to separate the cerebral cortex. The remaining tissue is hypothalamus.

### Weight loss analysis

Body weight, food and water intake are measured daily. Body composition is measured using EchoMRI. Subcutaneous fat is collected and weighed at study termination. Subcutaneous fat tissue samples are assessed for UCP-1 expression by qPCR and Western blot analyses.

### EXAMPLES

The combination treatment provided herein can be tested by administering the combination of the agents to a well-known mouse model and evaluating the results. Methods of such testing can be adapted from those known.

### Example 1. Evaluating THR-B agonist Compound 9 in combination with a GLP-1R agonist (e.g., Semaglutide)

To evaluate the effect of 12 weeks of treatment with Compound 9 alone and in combination with semaglutide on metabolic parameters, hepatic pathology and NAFLD Activity Score including Fibrosis Stage in male biopsy-confirmed GAN DIO-NASH mice are determined.

### Sample Formulation

| Compound | Frequency of preparation | Dosing solution Stored at 4°C | | | Detailed instruction on compound preparation |
|---|---|---|---|---|---|
| | | Conc. | Storage [temp] | pH | |
| Vehicle (Compound 9) | Weekly | NA | 4°C | 8 | 1% HP-β-CD (Cyclodextrin) and 90% 50 mM Tris buffer in reverse osmosis water (pH 8.0 ± 0.2 with NaOH or HCl) |
| Vehicle (Semaglutide) | Weekly | NA | 4°C | 7.4 | PBS + 0.1% BSA |
| Compound 9 | Daily | Gr. 3 and 6: 1.2 mg/ml | 4°C | 8 | Weigh out compound in amber colored vial, add vehicle and pH adjust to 8.0 with NaOH or HCL; stir and sonicate (if needed) for ~20 minutes. Solution may be slightly opaque. Dilute with vehicle to achieve the low concentration. Always protect from light. |
| | | Gr. 4 and 7: 0.6 mg/ml | | | |
| Semaglutide | Weekly | 6 nmol/ml* | 4°C | 7.4 | Dilute in PBS + 0.1% BSA |

| | | | | | |
|---|---|---|---|---|---|
| * Titration: 0.12 nmol/ml day 1; 0.24 nmol/ml day 2; 0.48 nmol/ml day 3: 0.96 nmol/ml day 4; 2.4 nmol/ml day 5, 6 nmol/ml day 6 | | | | | |

### Groups of the study

| Group | | Compound | n | Model | Dosing conc. | Dosing 5ml/kg, QD, AM |
|---|---|---|---|---|---|---|
| # | Name | | | | | Route |
| 1 | Lean-chow Vehicle | Vehicle (Compound 9) | 10 | Lean chow | NA | PO |
| | | Vehicle (Semaglutide) | | | NA | SC |
| 2 | DIO-NASH Vehicle | Vehicle (Compound 9) | 18 | DIO-NASH | NA | PO |
| | | Vehicle (Semaglutide) | | | NA | SC |
| 3 | Compound 9 (6 mg/kg) | Compound 9 | 18 | DIO-NASH | 6 mg/kg | PO |
| | | Vehicle (Semaglutide) | | | NA | SC |
| 4 | Compound 9 (3 mg/kg) | Compound 9 | 18 | DIO-NASH | 3 mg/kg | PO |
| | | Vehicle (Semaglutide) | | | NA | SC |
| 5 | Semaglutide | Semaglutide* | 18 | DIO-NASH | 30 nmol/kg | SC |
| | | Vehicle (Compound 9) | | | NA | PO |
| 6 | Compound 9 (6 mg/kg)+ Semaglutide | Compound 9 | 18 | DIO-NASH | 6 mg/kg | PO |
| | | Semaglutide* | | | 30 nmol/kg | SC |
| 7 | Compound 9 (3 mg/kg)+ Semaglutide | Compound 9 | 18 | DIO-NASH | 3 mg/kg | PO |
| | | Semaglutide* | | | 30 nmol/kg | SC |

| | | | | | | |
|---|---|---|---|---|---|---|
| *Titration: 0.6 nmol/kg day 1; 1.2 nmol/kg day 2; 2.4 nmol/kg day 3: 4.8 nmol/kg day 4; 12 nmol/kg day 5, 30 nmol/kg day 6. | | | | | | |

### Measurements (In vivo pharmacology)

| Measurement Name | Groups | Sample Name | Periods or frequency | Comments |
|---|---|---|---|---|
| Body weight | All | NA | QD from day -3 to day 85/86 | NA |
| Food intake | All | NA | QW from week 1 to week 12 | NA |
| Fasting BG | All | 4h Fasting blood glucose | Week 10 | NA |
| EchoMRI | All | NA | Week 12 | Whole body lean/fat tissue mass |
| Liver weight | All | Liver weight | Termination | NA |

### Measurements (Histology)

| Measurement Name | Sample Name | Comments |
|---|---|---|
| Fibrosis quantification for randomization | Liver pre-biopsy Liver post biopsy | PSR staining |
| NAFLD Activity Score | Liver pre-biopsy Liver post biopsy | HE staining |
| Fibrosis stage | Liver pre-biopsy Liver post biopsy | PSR staining |
| Steatosis quantification | Liver post biopsy | HE staining |
| Fibrosis (PSR) quantification | Liver post biopsy | PSR staining |
| Gal-3 quantification | Liver post biopsy | IHC staining |
| Col1a1 quantification | Liver post biopsy | IHC staining |
| α-SMA quantification | Liver post biopsy | IHC staining |

### Measurements (Assays)

| Measurement | Sample Type |
|---|---|
| Plasma insulin | 4h Fasting plasma insulin |
| Plasma ALT | Plasma ALT/AST/TG/TC |
| Plasma AST | Plasma ALT/AST/TG/TC |
| Plasma TG | Plasma ALT/AST/TG/TC |
| Plasma TC | Plasma ALT/AST/TG/TC |
| Plasma TIMP-1 | Plasma TIMP-1 |
| Plasma PIIINP | Plasma PIIINP |
| Plasma CK18-M30 | Plasma CK18- M30 |
| Liver TG | Liver TG/TC |
| Liver TC | Liver TG/TC |
| Liver RNAseq | Liver RNAseq n=10 |

### Non-alcoholic steatohepatitis (NASH) mouse model

This mouse model is fed a high fat diet that results in nonalcoholic steatohepatis (NASH) disease. The NASH mice are based on male C57BL/6JRj mice fed a 40% fat, 22% fructose and 2% cholesterol diet (D09100310, SSNIFF, Germany) for at least 28 weeks, respectively, prior to experiment.

### Liver biopsy

Mice are anesthetized by inhalation anesthesia using isoflurane (2-3%). A small abdominal incision is made in the midline and the left lateral lobe of the liver is exposed. A cone shaped wedge of liver tissue (approximately 50 mg) is excised from the distal portion of the lobe and fixated in 10% neutral buffered formalin (10% NBF) for histology. The cut surface of the liver is instantly electrocoagulated using bipolar coagulation (ERBE VIO 100 electrosurgical unit). The liver is returned to the abdominal cavity, the abdominal wall is sutured and the skin is closed with staplers. For post-operative recovery mice receive carprofen (5 mg/kg) administered subcutaneously on OP day and post-OP day 1 and 2.

### Blood sampling and plasma preparation

*In vivo* blood samples: Samples are collected at week 8, 30 minutes post-dose, 1 hour post-dose, 2 hours post-dose, 4 hours post-dose, 6 hours post-dose, and 24 hours post-dose (before the next dose). Tail-vein, tongue or cheek blood is collected in a Microvette tube of appropriate dimensions with anticoagulant and mixed by inversion 5 times. Blood is placed at 4°C until it is centrifuged at 3000 g for 10 minutes. The plasma supernatants are transferred to new tubes and immediately frozen on dry ice. The samples are stored at -70°C.

Termination blood samples: During anesthesia with isoflurane, the abdominal cavity is opened, and cardiac blood is drawn with a syringe into a Microvette/Vacuette of appropriate dimensions with anticoagulant and mixed by inversion 5 times. Blood is placed at 4°C until it is centrifuged at 3000 g for 10 minutes. The plasma supernatants are transferred to new tubes and immediately frozen on dry ice. The samples are stored at -70°C.

### Tissue sampling

### Liver (NASH)

After the animal has been terminated by heart puncture, the liver is collected and weighed. Specific liver samples and biopsies are dissected and processed as specified in table "Termination samples" and further described below.

The liver is divided into left lateral lobe, medial lobe, right lateral lobe, and caudate lobe. The remaining lobes are not used unless specified in the protocol.

The *Post-biopsy* piece (~200 mg, less than 0.7 x 0.5 cm) is cut from the left lateral lobe, 4 mm from the prebiopsy site with an edge. The tissue is collected in paraformaldehyde.

The *Liver Sponsor* piece (~150 mg) is dissected from the left medial lobe, put in a tube and placed directly into liquid nitrogen. The samples are stored at -70°C. Please note that the size of the sponsor piece varies according to the size of the left medial lobe.

The *Liver RNA Sequencing (RNAseq)* piece (20±10 mg) is dissected from the center of the left lateral lobe, snap frozen (put directly in liquid nitrogen in Nunc filter) and collected in a pre-cooled tube which is put in liquid nitrogen. The samples are stored at -70°C.

The *Liver TG TC* piece (25±5 mg) is dissected from the right medial lobe, with no edges, on the opposite side of the sponsor piece. The samples are weighed individually and collected in tubes before placed in liquid nitrogen. The samples are stored at -70°C.

The *Liver Extra* piece (~100-300 mg) is dissected from the right lateral lobe and collected in tubes before placed in liquid nitrogen, to have as backup tissue from the study and can be used for re-analysis if necessary. The samples are stored at -70°C.

### Biopsy processing

### Microtome sectioning of Formalin fixed paraffin-embedded (FFPE) biopsies

FFPE biopsies are placed in 10% neutral buffered formalin (10% NBF) for approximately 24h and then transferred to 70% EtOH and stored at 4°C. The FFPE biopsies are then placed in the Histokinette to infiltrate prior to embedding in blocks. Biopsy tissues are cut at 3µm on a microtome and the sections are mounted on Superfrost Plus slides and stored at 4°C.

### NAFLD Activity Score (NAS) and Fibrosis stage

Liver samples stained with Hematoxylin and Eosin (H&E) or Picro Sirius Red (PSR) are given a score for NAS and fibrosis stage respectively using the clinical criteria outlined by Kleiner et al. 2005. Total NAS represents the sum of scores for steatosis, inflammation, and ballooning, and ranges from 0-8. Adapted from: Design and validation of a histological scoring system for nonalcoholic fatty liver disease, Kleiner et al., Hepatology 41; 2005

### Histological staining procedures

In brief, slides with paraffin embedded sections are deparaffinated in xylene and rehydrated in series of graded ethanol.

Hematoxylin & Eosin (H&E) staining: Slides are incubated in Mayer's Hematoxylin (Dako), washed in tap water, stained in Eosin Y solution (Sigma-Aldrich), dehydrated and cover slipped.

Sirius red (PSR) staining: Slides are incubated in Weigert's iron hematoxylin (Sigma-Aldrich), washed in tap water, stained in Picro-sirius red (Sigma-Aldrich) and washed twice in acidified water. Excess water is removed by shaking the slides and the slides are then dehydrated in three changes of 100% ethanol, cleared in xylene and cover slipped.

Immunohistochemistry using single chromogen: IHC is performed using standard procedures. Briefly, after antigen retrieval and blocking of endogenous peroxidase activity, slides are incubated with primary antibody. The primary antibody is detected using a polymeric HRP-linker antibody conjugate. Next, the primary antibody is visualized with DAB as chromogen. Finally, sections are counterstained in hematoxylin and cover slipped. Slides are scanned under a 20X objective in a ScanScope AT slide scanner (Aperio).

### Gene expression analysis using RNAseq

RNA isolation: Tissue is collected and snap-frozen in liquid nitrogen. Samples are stored at -70°C until processing. RNA is isolated using the NucleoSpin^{®} kit (MACHEREY-NAGEL).

Library preparation and sequencing: A total of 10 ng-1 µg purified RNA from each sample is used to generate a cDNA library using the NEBNext^{®} Ultra^{™} II Directional RNA Library Prep Kit for Illumina (New England Biolabs). cDNA libraries are then sequenced on a NextSeq 500 using NextSeq 500/550 High Output Kit V2 (Illumina).

Data analysis: The sequencing data is aligned to the genome of the animal species obtained from the Ensembl database using the Spliced Transcripts Alignment to a Reference (STAR) software. For the bioinformatic analysis, the quality of the data is evaluated using the standard RNA-sequencing quality control parameters, the inter- and intra-group variability is evaluated using principal component analysis and hierarchical clustering and the differentially expressed genes are identified using the R-package DESeq2. Downstream analyses such as pathway analysis or target identification are performed as agreed with the sponsor.

### Blood and plasma assays

Alanine transaminase (ALT), Aspartate transaminase (AST), Triglycerides (TG) and Total Cholesterol (TC): Blood samples are collected in heparinized tubes and plasma is separated and stored at -70°C until analysis. Samples are measured using commercial kits (Roche Diagnostics), on the cobas c 501 autoanalyzer.

Cytokeratin 18 (CK18-M30): Blood samples are collected in EDTA tubes and plasma is separated and stored at -70°C until analysis. CK18 is measured using a commercial ELISA kit (Cusabio).

Insulin: Blood samples are collected in heparinized tubes and plasma is separated and stored at -70°C until analysis. Insulin is measured using the commercial MSD platform (Meso Scale Diagnostics).

TIMP-1: Blood samples are collected in EDTA tubes and plasma is separated and stored at -70°C until analysis. TIMP-1 is measured using a commercial ELISA kit (R&D Systems).

PIIINP: Blood samples are collected in EDTA tubes and plasma is separated and stored at -70°C until analysis. PIIINP is measured using a commercial ELISA kit (Cusabio).

### Tissue assays

Triglycerides (TG) and Total cholesterol (TC): Liver samples are homogenized and TG and TC is extracted in 5% NP-40 by heating twice to 90°C. The samples are centrifuged and the TG and TC content is measured in the supernatant using commercial kits (Roche Diagnostics) on the cobas c 501 autoanalyzer.

### Example 2: In vivo efficacy of Compound (1-2) alone and in combination with Compound 9 on weight loss in a high fat diet-induced obesity model in hGLP1R-C57BL/6J mice

hGLP1R-C57BL/6J mice on >20 weeks high fat diet to induce obesity are administered Compound (1-2) alone or in combination with Compound 9. Other groups are administered alternative GLP-1R agonists, e.g., semaglutide. Body weight and food-intake are measured daily.

### Animal Use

| **Species Level Weight** | C57BL6/J-h-GLP1-r SPF ~45 g |
|---|---|
| **Age (Order)** | 6-7 weeks |
| **Age (Start)** | 23-24 weeks |
| **Sex** | Male |
| **Method of Identification** | The mice are singly housed per cage with a unique cage |
| **Animal Number** | 127 |
| **Animal Number (used)** | 88 |

### Mouse Generation

118 h-GLP1R-C57BL/6J mice at 6-7 weeks age old are fed a high fat diet (HFD, Research Diets D12492i 60%kcal) for ~20 weeks to obtain a diet induced obese (DIO) phenotype, while n=10 h-GLP1R-C57BL/6J mice are fed regular chow diet to serve as lean controls. All animals have ad libitum access to diet and water. The animal room environment is controlled (target conditions: temperature 20 to 24°C, relative humidity 30 to 70%). Temperature and relative humidity are monitored daily. An electronic time-controlled lighting system is used to provide a 12-hour light/12-hour dark cycle. 2-3 mice are housed per plastic cage, which is in accordance with the National Research Council "Guide for the Care and Use of Laboratory Animals." Enrichment toys are provided. Before the formal experiment, the animals are housed in a single cage and adapted to the environment for at least one week. Weight is measured once a week during modeling.

### Study Procedure

### Group Design

| **Group ID** | **Treatment** | **Number** | **Dose level (mg/kg)** | **Dose volume (mL/kg)** | **Dose Method** | **Dose Frequency¹ Study Days** | **Start Dosing time** |
|---|---|---|---|---|---|---|---|
| 1 | Vehicle (Lean) | 8 | - | 5 | PO | BID*30 | 9:30am, 17:30pm |
| 2 | Vehicle (DIO) | 10 | - | 5 | PO | BID*30 | 9:30am, 17:30pm |
| 3 | Danuglipron | 10 | 10 | 5 | PO | BID*30 | 9:30am, 17:30pm |
| 4 | Compound (1-2) High | 10 | 10 | 5 | PO | BID*30 | 9:30am, 17:30pm |
| 5 | Compound (1-2) Low | 10 | 3 | 5 | PO | BID*30 | 9:30am, 17:30pm |
| 6 | Compound 9 | 10 | 3 | 5 | PO | QD*30 | 9:30am |
| | Vehicle | 10 | - | 5 | PO | QD*30 | 17:30pm |
| 7 | Compound (1-2) High+ Compound 9 | 10 | 10+3 | 5 | PO | QD*30 | 9:30am |
| | Compound (1-2) High+ Vehicle | 10 | 10 | 5 | PO | QD*30 | 17:30pm |
| 8 | Compound (1-2) Low+ Compound 9 | 10 | 3+3 | 5 | PO | QD*30 | 9:30am |
| | Compound (1-2) Low+ Vehicle | 10 | 3 | 5 | PO | QD*30 | 17:30pm |
| 9 | Semaglutide | 10 | 10 nmol/kg | 2 | SC | QD*30 | 9:30am |
| | Saline | 10 | - | 2 | SC | QD*30 | 17:30pm |

| | | | | | | | |
|---|---|---|---|---|---|---|---|
| ¹Test article is administered in the morning (9:30 to 10:30 am) for the Groups 6 and 9, and then administered a dose of vehicle (10% Solutol HS15: 90% sterile water, [v/v]) or saline (semaglutide vehicle) in the evening (17:30 to 18:30 pm) to balance treatment. For combination groups (7 and 8), Compound (1-2) and Compound 9 are administered in the morning (9:30 to 10:30 am), and then administered Compound (1-2) and vehicle in the evening (17:30 to 18:30 pm). SC = subcutaneous injection. | | | | | | | |

### Acclimation

Minus Day-14 to Day-1: After ~16 weeks of modeling to achieve an average model weight ~42 g, the mice are orally dosed with vehicle (10 % Solutol HS15: 90% sterile water, [v/v]) twice daily for 1~2 weeks to ensure a smooth dosing operation and to acclimate the mice to repeat dosing. Mice with body weights that have not stabilized by the end of the dosing acclimation are removed from the study.

### Grouping

All animals on HFD are assigned into 8 groups based on baseline body weight, 3 day's food intake and body composition (Day-4).

### Dose and Food Intake

Day1-30: Body weight and food intake are recorded daily before dosing in the morning.

BID dosing: Dosing time is 9:30 am-10:30 am and 17:30 pm-18:30 pm.

QD dosing (For group 6 and 9): The dosing time of Compound 9 (3 mg/kg, PO), or semaglutide (10 nmol/kg, SC) is between 9:30 am-10:30 am, while vehicle (10% Solutol HS15: 90% sterile water, [v/v]) or saline (semaglutide vehicle) is dosed between 17:30 pm-18:30 pm.

### Body Composition

Baseline (Day-4), Day 24: Lean and fat mass are measured by Body composition analyzer (Bruker's minispec- LF90) in the morning.

### Oral Glucose Tolerance Test (OGTT)

On day 28, random blood glucose and body weight of the mice are measured, mice are dosed with corresponding compounds or vehicle in the morning before fasting, then the mice are placed in new cages at 9:00 am and fasted for 6 hours, basal fasting glucose is measured by tail vein nick thereafter. Glucose is orally gavaged with 2 g/kg at a dose volume of 5 mL/kg. Blood glucose levels are measured at 0 (pre-dose), 15-, 30-, 60-, and 120-min post glucose dosing. The second dose is administrated after OGTT treatment.

### Terminal plasma and tissue collection

Day 31: The body weight and food intake of all mice is measured and then fasted for 6 hours, all mice are euthanized with CO₂ and bled via cardiac puncture. 500 µL blood samples will be processed for serum by centrifugation at 4°C, 3200 x g for 10 min and stored at -80°C for TC/TG/LDL/HDL/AST/ALT analysis (Analyzed by Biochemical analyzer: Beckman AU480/Hitachi 7180). 200 µL - 300 µL blood samples are mixed with K₂EDTA anticoagulant and processed for plasma by centrifugation at 4°C, 3200 x g for 10 min and stored at -80°C for insulin and other possible analysis.

The weight of whole liver and epididymal fat are weighed and recorded. Left lobule of liver is fixed in formalin. The remaining liver is flash frozen and stored at -80°C. Pancreas is collected and fixed in formalin. Brown adipose, subcutaneous white adipose, and epididymal white adipose tissues and the brain hypothalamus region are frozen with liquid nitrogen and preserved and stored at -80°C for optional qPCR analysis.

### Hypothalmus collection procedure

The brain tissue is placed in the mold, and the olfactory bulb is placed in the center of the groove. The brain tissue is sliced into 8 coronal sections evenly spaced at 2 mm intervals. The third section (6-8mm) of the coronal brain tissue is taken out, and the section is cut along the boundary of the purple part with a scalpel to separate the cerebral cortex. The remaining tissue is hypothalamus.

### Blood Collection

Day 21: Animals in all groups are divided into 3 sub-groups respectively. All mice for PK sampling, except for the pre-dose mice, receive the first dose of corresponding compound in the morning between 9:30 am to 10:30 am, 60 µL blood is collected from mice under the jaw into pre-chilled tubes containing 1.2 µL K₂ EDTA, and placed on ice. For the evening dose, the 8-hour post-1^{st}-dose samples are collected before the second dose of the day. Blood samples are processed to plasma within 1 hour of collection by centrifugation at 4°C, 3200 x g for 10 min. About 30 µL of plasma is collected and stored at -80°C for further analysis.

### Data Processing and Analsyis

The significances of the differences among groups and within groups are evaluated by one-way or two-way ANOVA using Graph Pad statistic software. A p-value of less than 0.05 are considered statistically significant.

### Example 3. Effects of 4 weeks of treatment with Semaglutide alone and in combination with Compound 9 on body weight regulation and composition in male DIO mice at thermoneutrality

### Methods

Male C57BL/6JRj mice (n=48), 5 weeks of age were fed a high fat diet (HFD) for 24 weeks before treatment starts. Mice were randomized during week -1 into 7 groups (n= 12 each group) based on body weight and fat tissue mass. The DIO mice were dosed once a day per oral (PO) and subcutaneous (SC) for 28 days.
Group 1 received Vehicle (PO + SC),
Group 2 Semaglutide (30 nmol/kg, SC) + Vehicle (PO),
Group 3 Semaglutide (30 nmol/kg, SC) + Compound 9 (6 mg/kg, PO),
Group 4 Semaglutide (30 nmol/kg, SC) Wk1-4 + Compound 9 Wk2-4 (6 mg/kg, PO). See Fig. 3.

Body weight, food intake and water intake were measured daily, EchoMRI was performed during week -1 and at week 4, and plasma samples were collected for sponsor at terminiation.

### Results

Treatment with Compound 9 in combination with Semaglutide (Semaglutide + Compound 9), induced a reduction of absolute and relative body weight, compared Semaglutide alone. See Figs. 4-5.
Semaglutide + Compound 9 potentiated semaglutide-induced weight loss and led to a specific loss of fat mass without additional lean mass loss. See Figs. 6-9.

### Example 4: Effect of 6 weeks of treatment with Compound 9 alone and in combination with Semaglutide on metabolic parameters, energy expenditure and glycemic control in male DIO mice at thermoneutrality.

### Methods

Male C57BL/6JRj mice (5 weeks of age, n=70) were fed a high fat diet (n= 60, 60% HFD, D12492, Ssniff) for 24 weeks, or a lean-chow diet (n= 10, Altromin 1324) before treatment start. HFD animals were randomized during week -1 based on body weight, glucose levels and fat tissue mass into 5 treatments groups. Lean mice (group 1) were treated with vehicle (QD + QD, PO + SC) and DIO mice were treated (QD + QD, PO + SC) as follow: group 2 received vehicle (PO + SC), group 3 received Compound 9 6 mg/kg, PO + vehicle (SC), group 4 received Semaglutide (30 nmol/kg, SC) + vehicle (PO), group 5 received Compound 9 (6 mg/kg, PO) + Semaglutide (30 nmol/kg, SC) and group 6 received Tirzepatide (30 nmol/kg, SC) + vehicle (PO). Body weight, food and water intake were measured daily from day -3, body composition (EchoMRI) was measured during week -1 and week 6; energy expenditure was assessed at study day 5-7 and at study day 34-36. Fasting blood glucose and insulin levels were measured at baseline and termination, while an oral glucose tolerance test was performed at week 4. At termination, the *M. gastrocnemius* muscle was weighted and collected for RNAseq analysis, while plasma, brain, liver, BAT, epidydimal and subcutaneous WAT depots were collected for sponsor. *See* Fig. 10

**Table 4-1 - Study Groups**

| **#** | **Group name** | ***n* in group** | **Animal model** | **Dose** | **Route of administration** | **Dosing frequency** |
|---|---|---|---|---|---|---|
| 1 | Vehicle Lean | 9 | Lean mice | NA | PO + SC | QD + QD |
| 2 | Vehicle DIO | 12 | DIO mice | NA | PO + SC | QD + QD |
| 3 | Compound 9 + Vehicle | 12 | DIO mice | 6 mg/kg + NA | PO + SC | QD + QD |
| 4 | Semaglutide + Vehicle | 12 | DIO mice | 30 nmol/kg + NA | SC + PO | QD + QD |
| 5 | Compound 9 + Semaglutide | 12 | DIO mice | 6 mg/kg + 30 nmol/kg | PO + SC | QD + QD |
| 6 | Tirzepatide + Vehicle | 12 | DIO mice | 30 nmol/kg + NA | SC + PO | QD + QD |

### Results (See Figs. 11-17)

At week 1, compared to vehicle, Compound 9 in combination with Semaglutide reduced absolute and relative body weight, together with Semaglutide and Tirzepatide alone. Heat production was increased by Compound 9 + Semaglutide treatment in the light phase, together with Semaglutide and Tirzepatide alone, while the RER was reduced compared to vehicle during both dark and light phase in these three treatment groups. Compound 9 in combination with Semaglutide increased oxygen consumption both during dark and light phase.

The OGTT test indicated an increased glucose disposal for the Compound 9 + Semaglutide treated group, as well as Semaglutide and Tirzepatide alone; after 4 weeks of treatment, the combination of Compound 9 and Semaglutide reduced absolute and relative fat tissue mass compared to the vehicle-treated group, while absolute lean tissue mass was decreased and relative tissue mass was increased, and the same changes were observed for the Semaglutide and Tirzepatide treatment alone.

The energy expenditure measurement showed that after 5 weeks of treatment, Compound 9 alone increased both dark and light-phase heat production. Moreover, the oxygen consumption was significantly higher for the Compound 9 + Semaglutide group during both dark and light phase, while only higher in the light phase for Semaglutide and Tirzepatide alone. Plasma insulin level was increased at week 6 upon Compound 9 treatment, while significantly lower in combination with Semaglutide, Semaglutide and Tirzepatide alone.

### Example 5. Evaluating THR-B agonist Compound 9 in combination with a GLP-1R agonist (e.g., orforglipron)

### Background

GLP-1R regulates post prandial blood glucose and satiety. The later effect can induce weight loss but efficacy is limited by metabolic adaptation, a compensatory process that lowers energy expenditure (EE). Moreover, the higher dose levels of GLP-1R needed to achieve clinical meaningful weight loss, are associated with more severe and frequent GI-related side effects including nausea and emesis, which can negatively impact patient compliance. Mechanisms to improve the effectiveness of GLP-1 based therapies at lower doses, could mitigate GI-related side effects and improve patient compliance and outcomes. In addition, combining these mechanisms in an all-oral medication could further improve patient compliance over injectable medications

Thyroid hormone receptor beta (THRβ) plays a central role in regulating energy metabolism. Compound 9 is an orally bioavailable, highly selective, thyroid receptor beta agonist. In preclinical models of obesity, Compound 9 enhances the weight loss efficacy of the injectable peptide GLP-1R agonist semaglutide by mitigating the lowering of EE associated with weight loss and increasing fat mass loss, while preserving relative lean mean loss. In this example, Compound 9 was tested in combination with orforglipron, an orally bioavailable GLP-1R agonist in late-stage clinical development for the treatment of type II diabetes myelitis (T2DM) and obesity. Specifically, the combination of Compound 9 with a low dose of orforglipron was tested to see if it could achieve a similar weight loss efficacy as a 10-fold higher dose of orforglipron in monotherapy. Like other small molecule GLP-1R agonists, orforglipron is inactive on rodent GLP-1R. Therefore, for the current study, diet-induced obese (DIO) transgenic mice expressing human GLP-1R (hGLP-1R) were used to assess in vivo efficacy Compound 9 in combination with orforglipron.

### Methods

DIO hGLP-1R mice were housed at room temperature (21-25°C) with a relative humidity of 40-70%. Temperature and relative humidity were monitored and recorded twice daily. An electronic time-controlled lighting system was used to provide a 12 h light/12 h dark cycle, 19:00 pm-7:00 am, lights out and enrichment toys were provided. The mice were fed high fat diet (HFD, Research Diets 12492, 60% kca, 1% fat) for >50 weeks to induce obesity. Mice were acclimated to once-daily (QD) oral dosing (per os, PO) for 1 week with body weight and food intake measured on consecutive 4 days prior to dosing start (Day 1). Body mass composition was measured by EchoMRI (Bruker Minispec LF90II) on Day 0 and mice were randomized to treatment groups (Table 5-1) based on baseline body weight, food intake, and body composition.

Test compounds were formulated in vehicle (10% [v/v] Solutol HS15 + 90% [v/v] saline) and mice were treated QD PO for 21 days with vehicle, Compound 9 (3 mg/kg), orforglipron (0.2 and 2 mg/kg), and Compound 9 (3 mg/kg) in combination with orforglipron (0.2 mg/kg). Body weight and food intake were measured daily, and mice were maintained on HFD throughout the study period. On Day 20 of the study, plasma samples were collected at predose (0), 2-, 4-, 8-, and 24-hours postdose for pharmacokinetic analyses of Compound 9, orforglipron, and combination treatment groups. Body mass composition was assessed on Day 19. Liver was collected and weighed at study termination and portions were preserved for steatosis assessment by histology. Subcutaneous fat was collected and weighed at study termination. A sample of subcutaneous fat tissue was assessed for UCP-1 expression by qPCR and Western blot analyses.

**Table 5-1: Treatment groups**

| **Group ID** | **Treatment** | **n** | **Dose (mg/kg)** | **Dose volume (mL/kg)** | **Dose Method** | **Dose Frequency** |
|---|---|---|---|---|---|---|
| I | Vehicle | 6 | - | 5 | PO | QD*21 days |
| 2 | Compound 9 | 6 | 3 | 5 | PO | QD*21 days |
| 3 | Orforglipron | 7 | 2 | 5 | PO | QD*21 days |
| 4 | Orforglipron | 7 | 0.2 | 5 | PO | QD*21 days |
| 5 | Compound 9 + Orforglipron | 7 | 0.2+3 | 5 | PO | QD*21 days |

### Results

Treatment with orforglipron (0.2 mg/kg and 2 mg/kg PO QD) resulted in dose-dependent body weight decreases over time (FIG. 18), while body weights in mice treated with vehicle (PO QD) or Compound 9 (3 mg/kg PO QD) alone remained relatively unchanged during the study period. In contrast, mice treated with the combination of Compound 9 (3 mg/kg PO QD) and orforglipron (0.2 mg/kg PO QD) showed comparable weight loss to the high dose of orforglipron (2 mg/kg PO QD) monotherapy. On Day 20 decreases in body weight were significant for mice treated with orforglipron (0.2 and 2 mg/kg) and Compound 9 in combination with orforglipron (0.2 mg/kg) (FIG. 19). Moreover, treatment with the combination resulted in significantly greater weight loss compared with the low dose of orforglipron (0.2 mg/kg) monotherapy. Daily food intake was profoundly reduced in a dose-dependent manner during the first few days in mice treated with orforglipron before steadily increasing towards vehicle control levels (FIG. 20). Daily food intake was similar between mice treated with low dose orforglipron (0.2 mg/kg PO QD) and those treated with Compound 9 (3 mg/kg PO QD) in combination with orforglipron (0.2 mg/kg PO QD). Daily food intake was similar in mice treated with vehicle and Compound 9 (3 mg/kg PO QD). All treatment groups showed acute decreases in food intake following body mass composition measurements on Day 19, suggestive of animal handling induced stress response.

Treatment with orforglipron (0.2 mg/kg and 2 mg/kg PO QD) resulted in a dose-dependent decrease in fat mass (FIG. 21A), while fat mass remained relatively unchanged in mice treated with vehicle (PO QD) or Compound 9 (3 mg/kg PO QD). In contrast, the mice treated with the combination of Compound 9 (3 mg/kg PO QD) and orforglipron (0.2 mg/kg PO QD) showed comparable fat mass loss to the high dose of orforglipron (2 mg/kg PO QD) monotherapy. Lean mass loss was statistically significant in mice treated with orforglipron (0.2 mg/kg and 2 mg/kg PO QD) compared to vehicle treatment (FIG. 21B). Mice treated Compound 9 in combination with orforglipron (0.2 mg/kg PO QD) showed a similar mean decrease in lean mass, which was not statistically significant relative to orforglipron (0.2 mg/kg PO QD) monotherapy. Lean mass loss was negligible in mice treated with vehicle or Compound 9 (3 mg/kg PO QD).

Treatment with orforglipron (0.2 mg/kg and 2 mg/kg PO QD) resulted in a dose-dependent decrease in subcutaneous fat, although only the high dose was significantly different from vehicle treatment (FIG. 22**Error! Reference source not found**.). Treatment with the c ombination of Compound 9 (3 mg/kg PO QD) and orforglipron (0.2 mg/kg PO QD) showed similar levels of subcutaneous fat compared to the high dose of orforglipron (2 mg/kg PO QD). The subcutaneous fat levels in mice treated Compound 9 (3 mg/kg PO QD) alone were not significantly different from mice treated with vehicle.

All treatment groups showed significantly lower liver weights at the end of study compared to vehicle control (FIG. 23). Mice treated with Compound 9 (3 mg/kg PO QD) in combination with orforglipron (0.2 mg/kg PO QD) showed trends towards further reductions in liver weight compared to mice treated with the low dose of orforglipron (0.2 mg/kg PO QD).

Concentration-time profiles of Compound 9 and orforglipron are shown in FIG. 24A and FIG. 24B, respectively, alone or in combination. Select derived pharmacokinetic parameters are shown in Table 5-2. Orforglipron exposure increased in a less than dose-proportional manner and exposure was largely unaffected when dosed in combination with Compound 9. Similarly, exposure to Compound 9 was unaffected when dosed in combination with orforglipron.

**Table 5-2: Derived pharmacokinetic parameters**

| **Analyte** | **Treatment group** | **AUC₀₋ₗₐₛₜ (h*ng/mL)** | **Cₘₐₓ (ng/mL)** |
|---|---|---|---|
| **Compound 9** | Compound 9 (3 mg/kg) | 19,000 (5310) | 2480 (220) |
| | Compound 9 (3 mg/kg) + orforglipron (0.2 mg/kg) | 17,600 (1810) | 2780 (379) |
| **Orforglipron** | Orforglipron (0.2 mg/kg) | 543 (34.8) | 47.4 (6.47) |
| | Orforglipron (2 mg/kg) | 2670 (428) | 251 (39.8) |
| | Compound 9 (3 mg/kg) + orforglipron (0.2 mg/kg) | 607 (23.7) | 45.1 (3.45) |

| | | | |
|---|---|---|---|
| Abbreviations: AUC₀₋ₗₐₛₜ = area under the plasma concentration time curve from time 0 to time of the last measurable concentration; Cₘₐₓ = maximum observed concentration | | | |

### Summary

Compound 9 enhanced the weight loss efficacy of a low dose of orforglipron to a level comparable to a 10-fold higher dose of orforglipron as monotherapy.

Daily doses of orforglipron at 0.2 mg/kg in mice provided equivalent unbound exposures (AUC_{24h,u} ~0.300 h.ng/mL) following daily doses of 6 mg/day in humans, while 2 mg/kg in mice provided equivalent unbound exposures (AUC_{24h,u} ~1.60 h.ng/mL) following daily doses of 45 mg/day in humans (Pratt et, al (2023). The daily dose of 45 mg orforglipron in humans is associated with the highest change in body weight at week 26 compared to 12, 24, or 36 mg/day doses, however compared to placebo, the occurrence of total adverse events was significantly higher with orforglipron 12 mg [OR 2.49 (95% CI: 1.25, 4.98), *p =* 0.01, *I*² = 12%], 24 mg [OR 2.23 (95% CI: 1.13, 4.43), *p =* 0.02, *I*² = 10%], 36 mg [OR 2.04 (95% CI: 1.08, 3.84), *p* = 0.03, *I*² = 0%] and 45 mg [OR 2.96 (95% CI: 1.41, 6.21), *p =* 0.004, *I*² = 17%] daily doses; the highest rates seen with orforglipron 45 mg. (Frias et al., Lancet. 2023;402(10400):472-483, Wharton et al., N Engl J Med. 2023;389(10):877-888, Pratt et al, Diabetes Obes Metabol. 2023;25:2642-2649).

The enhanced weight loss observed did not appear to be driven by changes in food intake. The enhanced weight loss effects of Compound 9 in combination with orforglipron were driven by larger reductions in fat mass, including subcutaneous fat, without additional decreases in lean mass. Lastly, liver weights were reduced by the combination of Compound 9 and orforglipron, suggesting enhanced anti-steatotic effects with the combination. The ability of Compound 9 to substantially enhance the efficacy of a low dose of GLP-1R agonist may ultimately translate to improved GI tolerability, leading to improved patient compliance and better outcomes.

### Example 6. Evaluating THR-B agonist Compound 9 in combination with a GLP-1R agonist (e.g., Compound 1-2)

Transgenic C57/BL6J male mice expressing the human glucagon-like peptide-1 receptor (hGLP-1R) were fed a high fat diet (HFD, Research Diets D12492i 60%kcal) for 17 weeks to obtain a diet induced obese (DIO) phenotype. Mice were randomly divided into groups (n=8-10 per group) based on baseline body weight. Mice were treated for 30 days with Compound 9 [3 mpk, PO, once daily], Compound (1-2) [10 mpk PO, twice daily], Compound 9 [3 mpk PO, once daily] + Compound (1-2) [10 mpk, twice daily], Semaglutide [10 nmol/kg SQ, once daily] or vehicle: 10% Solutol HS15: 90% sterile water, (v/v). Body weight was measured daily and the mean percent change from baseline was calculated for each treatment group (FIGs. 25 and 26). The combination of Compound 9 + Compound (1-2) exerted a greater effect on reducing body weight compared to mice treated with either Compound 9 or Compound (1-2) alone.

### EQUIVALENTS

Although the foregoing invention has been described in some detail by way of illustration and example for purposes of clarity of understanding, it is apparent to those skilled in the art that certain minor changes and modifications will be practiced in light of the above teaching. Therefore, the description and examples should not be construed as limiting the scope of the invention.

## Claims

1. A combination comprising:
(i) a THRβ agonist which is: 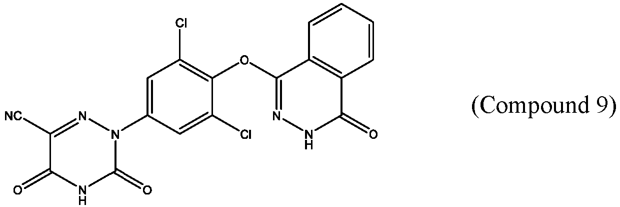 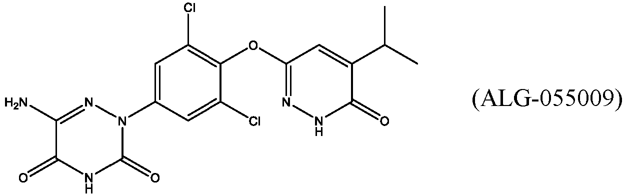 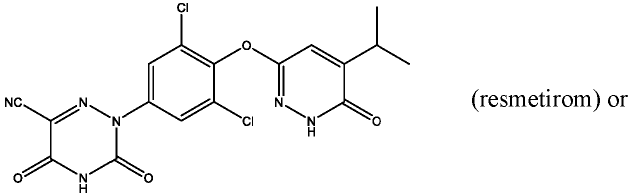 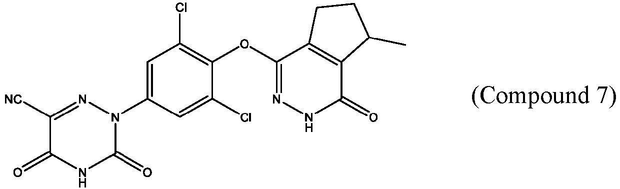 or a pharmaceutically acceptable salt thereof, and
(ii) a GLP-1R agonist, or a pharmaceutically acceptable salt thereof,
for use in
(i) effectuating weight loss in a patient having a BMI of 25 kg/m² or greater, or
(ii) the prevention or treatment of obesity.

2. The non-therapeutic use of a combination comprising:
(i) a THRβ agonist which is: 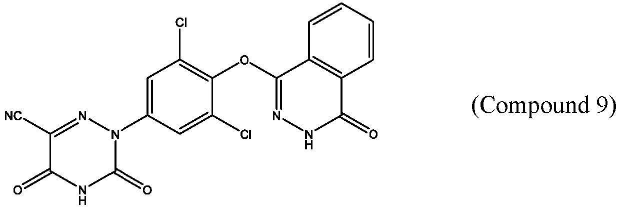 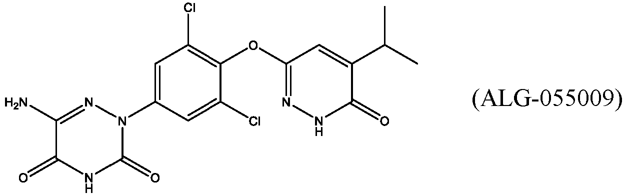 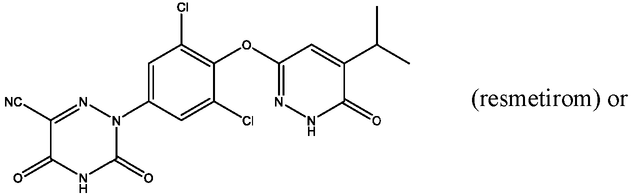 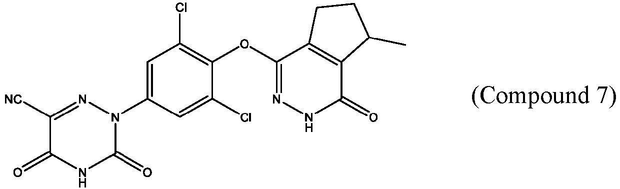 or a pharmaceutically acceptable salt thereof, and
(ii) a GLP-1R agonist, or a pharmaceutically acceptable salt thereof,
for effectuating weight loss.

3. The combination for use of claim 1 or the use of claim 2, wherein the THRβ agonist is Compound 9: 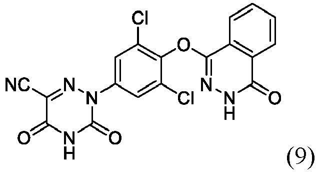 or a pharmaceutically acceptable salt thereof.

4. The combination for use or use of claim 3, wherein the THRβ agonist is a potassium salt of Compound 9.

5. The combination for use of claim 1 or the use of claim 2, wherein the THRβ agonist is: 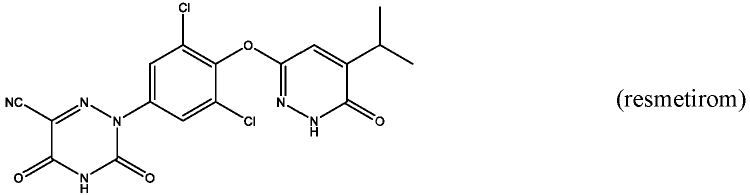 or a pharmaceutically acceptable salt thereof.

6. The combination for use or use of any one of the preceding claims, wherein the GLP-1R agonist is a compound of Formula (I-1): 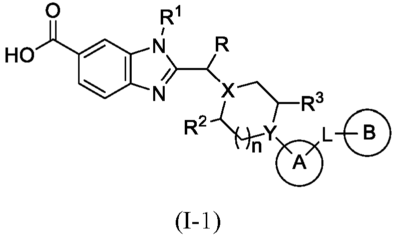 or a pharmaceutically acceptable salt thereof, wherein:
X is N or CH;
Y is N or CR⁴;
n is 0 or 1;
R is hydrogen;
R¹ is -C₁-C₆ alkylene-R⁵;
R² is hydrogen, oxo, or C₁-C₆ alkyl;
R³ is hydrogen, oxo, or C₁-C₆ alkyl and R⁴ is hydrogen, OH, or C₁-C₆ alkyl;
or R³ and R⁴ are taken together with the carbon atoms to which they are attached to form C₃-C₆ cycloalkyl optionally substituted by halo or C₁-C₃ alkyl;
R⁵ is 5-membered heterocyclyl or 5-membered heteroaryl, each of which comprises 1, 2, or 3 heteroatoms independently selected from O, N, and S, wherein at least one heteroatom of R⁵ is S, and further wherein R⁵ is optionally substituted by halo, -O-C₁₋₆ alkyl, C₁₋₆ alkyl, C₁₋₆ alkenyl, or C₁-C₆ haloalkyl;
Ring A is 5- to 12-membered heterocyclene or 5- to 12-membered heteroarylene, each of which is independently optionally substituted by halo, CN, C₃-C₆ cycloalkyl, or C₁-C₆ alkyl optionally substituted by halo or OH;
L is a bond, -O-, C₁-C₆ alkylene, *-O-C₁-C₆ alkylene-**, *-C₁-C₆ alkylene-O-**, or *-NR⁶-C₁-C₆ alkylene-**, wherein
* represents the point of attachment to ring A and ** represents the point of attachment to ring B;
when L is *-O-C₁-C₆ alkylene-**, the C₁-C₆ alkylene of L is optionally substituted by R^{L}, wherein each R^{L} is independently C₁-C₆ alkyl or halo, or two R^{L} are taken together with the carbon atom or atoms to which they are attached to form C₃-C₆ cycloalkyl or 3- to 6-membered heterocyclyl; and
when L is C₁-C₆ alkylene, the C₁-C₆ alkylene is optionally substituted by R^{L1}, wherein each R^{L1} is independently halo, OH, oxo, or C₁-C₆ alkyl, or two R^{L1} are taken together with the carbon atom or atoms to which they are attached to form C₃-C₆ cycloalkyl or 3- to 6-membered heterocyclyl;
R⁶ is hydrogen or C₁-C₆ alkyl; and
Ring B is C₃-C₁₀ cycloalkyl, C₆-C₁₄ aryl, 4- to 12-membered heterocyclyl, or 5- to 12-membered heteroaryl, each of which is independently optionally substituted by one to three substituents independently selected from the group consisting of halo, CN, oxo, C₁-C₆ alkyl, C₁-C₆ haloalkyl, -COCH₃, -CONH₂, -S(O)₂CH₃, and phenyl.

7. The combination for use or use of any one of the preceding claims, wherein the GLP-1R agonist is a compound of Formula (I-1a): 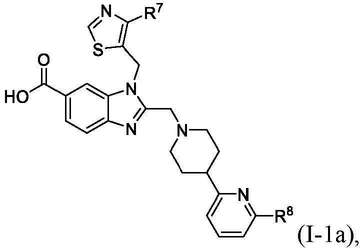 or a pharmaceutically acceptable salt thereof wherein R⁷ is hydrogen, chloro, bromo, fluoro, methyl, or vinyl; and R⁸ is

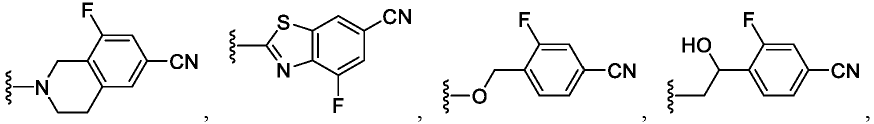

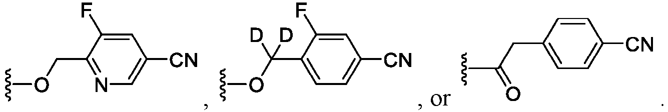

8. The combination for use or use of any one of the preceding claims, wherein the GLP-1R agonist is Compound 1-2: 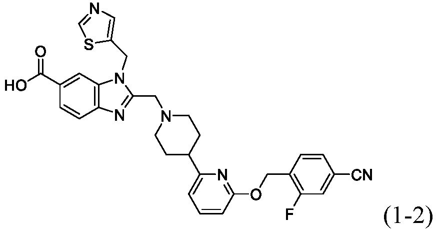 or a pharmaceutically acceptable salt thereof.

9. The combination for use or use of claim 8, wherein the GLP-1R agonist is a meglumine salt of Compound 1-2.

10. The combination for use or use of any one of claims 1 to 5, wherein the GLP-1R agonist is orforglipron, danuglipron, liraglutide, exenatide, dulaglutide, albiglutide, lixisenatide, tirzepatide, or semaglutide.

11. The combination for use of claim 1 or use of claim 2, wherein the THRβ agonist is Compound 9 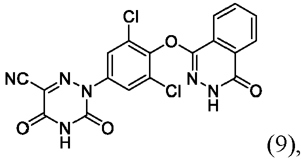 or a pharmaceutically acceptable salt thereof and the GLP-1R agonist is orforglipron, danuglipron, or semaglutide, or a pharmaceutically acceptable salt thereof.

12. The combination for use or use of any of the preceding claims, wherein the THRβ agonist or a pharmaceutically acceptable salt thereof, and the GLP-1R agonist, or a pharmaceutically acceptable salt thereof, are formulated:
• in separate pharmaceutically acceptable compositions; or
• in a single pharmaceutically acceptable composition.

13. The combination for use or use of any of the preceding claims, wherein:
• the proportion of lean body mass relative to total body mass in a patient increases; and/or
• the proportion of fat mass relative to total body mass in a patient decreases.

14. The combination for use or use of any one of the preceding claims, wherein the patient has a Body Mass Index (BMI) of 27 kg/m² or greater, preferably 30 kg/m² or greater.

15. The combination for use or use of any one of the preceding claims, wherein:
• the administration of the THRβ agonist allows for administration of a reduced dose of the GLP-1R agonist while maintaining equivalent weight loss; and/or
• the administration of the THRβ agonist increases weight loss compared to administration of a GLP-1R agonist at an equivalent dose.

## Patentansprüche

1. Kombination, umfassend:
(i) einen THRß-Agonisten, bei dem es sich um 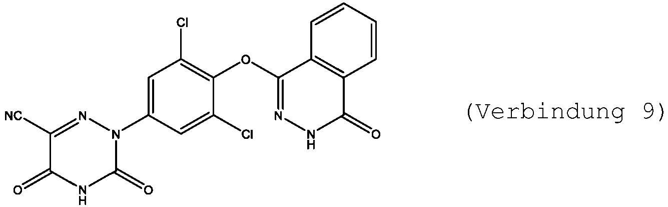 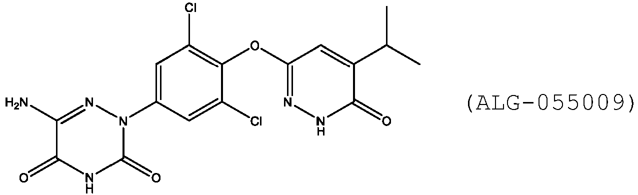 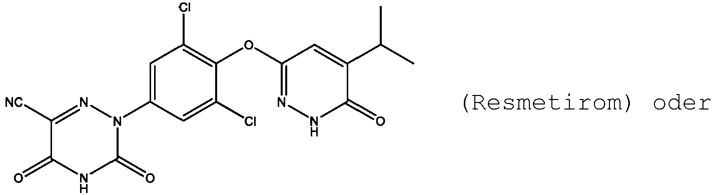 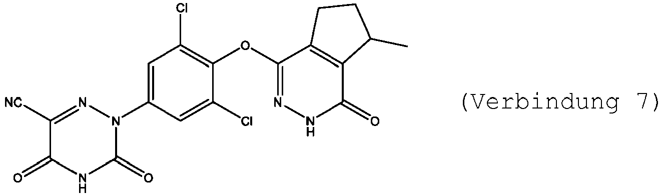 oder ein pharmazeutisch unbedenkliches Salz davon handelt, und
(ii) einen GLP-1R-Agonisten oder ein pharmazeutisch unbedenkliches Salz davon,
zur Verwendung
(i) beim Herbeiführen eines Gewichtsverlusts bei einem Patienten mit einem BMI von 25 kg/m² oder mehr, oder
(ii) bei der Prävention oder Behandlung von Übergewicht.

2. Nichttherapeutische Verwendung einer Kombination, umfassend:
(i) einen THRß-Agonisten, bei dem es sich um 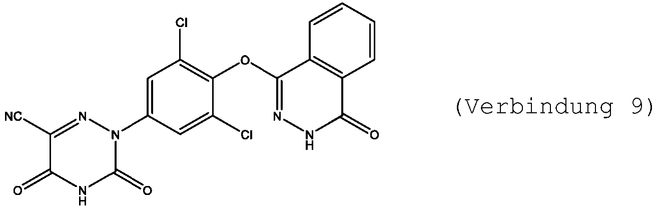 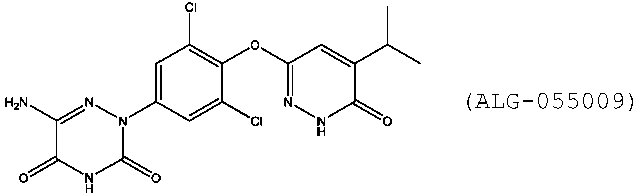 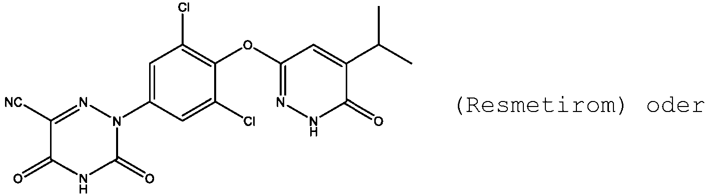 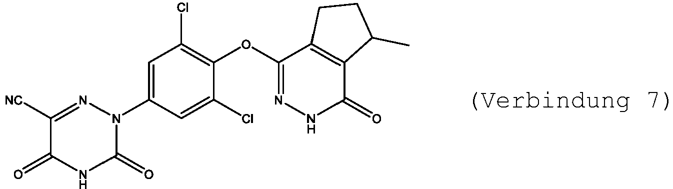 oder ein pharmazeutisch unbedenkliches Salz davon handelt, und
(ii) einen GLP-1R-Agonisten oder ein pharmazeutisch unbedenkliches Salz davon, zum Herbeiführen eines Gewichtsverlusts.

3. Kombination zur Verwendung nach Anspruch 1 oder Verwendung nach Anspruch 2, wobei es sich bei dem THRβ-Agonisten um Verbindung 9: 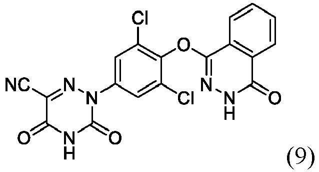 oder ein pharmazeutisch unbedenkliches Salz davon handelt.

4. Kombination zur Verwendung oder Verwendung nach Anspruch 3, wobei es sich bei dem THRß-Agonisten um ein Kaliumsalz von Verbindung 9 handelt.

5. Kombination zur Verwendung nach Anspruch 1 oder Verwendung nach Anspruch 2, wobei es sich bei dem THRβ-Agonisten um: 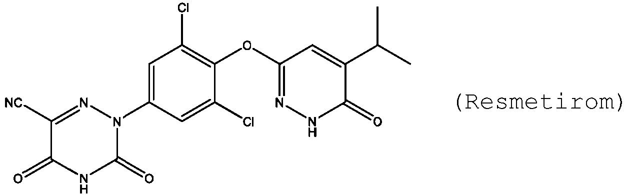 oder ein pharmazeutisch unbedenkliches Salz davon handelt.

6. Kombination zur Verwendung oder Verwendung nach einem der vorhergehenden Ansprüche, wobei es sich bei dem GLP-1R-Agonisten um eine Verbindung der Formel (I-1): 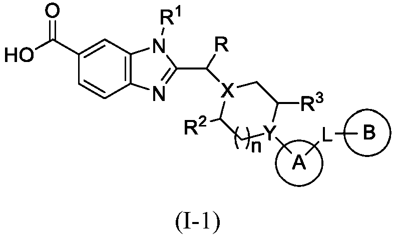 oder ein pharmazeutisch unbedenkliches Salz davon handelt, wobei:
X für N oder CH steht;
Y für N oder CR⁴ steht;
n für 0 oder 1 steht;
R für Wasserstoff steht;
R¹ für -C₁-C₆-Alkylen-R⁵ steht;
R² für Wasserstoff, Oxo oder C₁-C₆-Alkyl steht;
R³ für Wasserstoff, Oxo oder C₁-C₆-Alkyl steht und R⁴ für Wasserstoff, OH oder C₁-C₆-Alkyl steht;
oder R³ und R⁴ zusammen mit den Kohlenstoffatomen, an die sie gebunden sind, C₃-C₆-Cycloalkyl bilden, das gegebenenfalls durch Halogen oder C₁-C₃-Alkyl substituiert ist;
R⁵ für 5-gliedriges Heterocyclyl oder 5-gliedriges Heteroaryl steht, die jeweils 1, 2 oder 3 unabhängig voneinander aus O, N und S ausgewählte Heteroatome umfassen, wobei mindestens ein Heteroatom von R⁵ für S steht und wobei R⁵ weiterhin gegebenenfalls durch Halogen, -O-C₁₋₆-Alkyl, C₁₋₆-Alkyl, C₁₋₆-Alkenyl oder C₁-C₆-Halogenalkyl substituiert ist;
Ring A für 5- bis 12-gliedriges Heterocyclen oder 5- bis 12-gliedriges Heteroarylen steht, die jeweils unabhängig gegebenenfalls durch Halogen, CN, C₃-C₆-Cycloalkyl oder C₁-C₆-Alkyl substituiert sind, das gegebenenfalls durch Halogen oder OH substituiert ist;
L für eine Bindung, -O-, C₁-C₆-Alkylen, *-O-C₁-C₆-Alkylen-**, *-C₁-C₆-Alkylen-O-** oder *-NR⁶-C₁-C₆-Alkylen-** steht, wobei
* den Anknüpfpunkt am Ring A und ** den Anknüpfpunkt am Ring B kennzeichnet;
wenn L für *-O-C₁-C₆-Alkylen-** steht, das C₁-C₆-Alkylen von L gegebenenfalls durch R^{L} substituiert ist, wobei R^{L} jeweils unabhängig für C₁-C₆-Alkyl oder Halogen steht, oder zwei R^{L} zusammen mit dem/den Kohlenstoffatom(en), an das/die sie gebunden sind, C₃-C₆-Cycloalkyl oder 3- bis 6-gliedriges Heterocyclyl bilden; und
wenn L für C₁-C₆-Alkylen steht, das C₁-C₆-Alkylen gegebenenfalls durch R^{L1} substituiert ist, wobei R^{L1} jeweils unabhängig für Halogen, OH, Oxo oder C₁-C₆-Alkyl steht, oder zwei R^{L1} zusammen mit dem/den Kohlenstoffatom(en), an das/die sie gebunden sind, C₃-C₆-Cycloalkyl oder 3- bis 6-gliedriges Heterocyclyl bilden;
R⁶ für Wasserstoff oder C₁-C₆-Alkyl steht; und
Ring B für C₃-C₁₀-Cycloalkyl, C₆-C₁₄-Aryl, 4- bis 12-gliedriges Heterocyclyl oder 5- bis 12-gliedriges Heteroaryl steht, die jeweils unabhängig gegebenenfalls durch einen bis drei unabhängig aus der aus Halogen, CN, Oxo, C₁-C₆-Alkyl, C₁-C₆-Halogenalkyl, -COCH₃, -CONH₂,-S(O)₂CH₃ und Phenyl bestehenden Gruppe ausgewählte Substituenten substituiert sind.

7. Kombination zur Verwendung oder Verwendung nach einem der vorhergehenden Ansprüche, wobei es sich bei dem GLP-1R-Agonisten um eine Verbindung der Formel (I-1a): 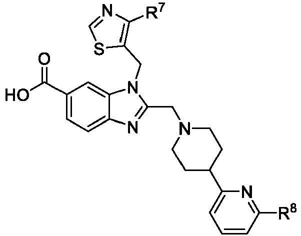 (I-1a) oder ein pharmazeutisch unbedenkliches Salz davon handelt, wobei R⁷ für Wasserstoff, Chlor, Brom, Fluor, Methyl oder Vinyl steht; und R⁸ für 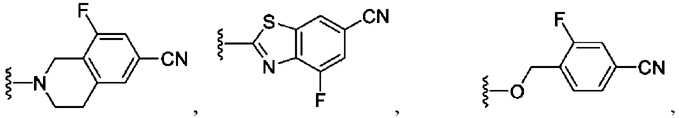 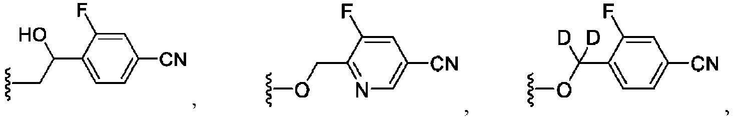 oder 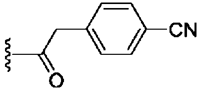 steht.

8. Kombination zur Verwendung oder Verwendung nach einem der vorhergehenden Ansprüche, wobei es sich bei dem GLP-1R-Agonisten um Verbindung 1-2: 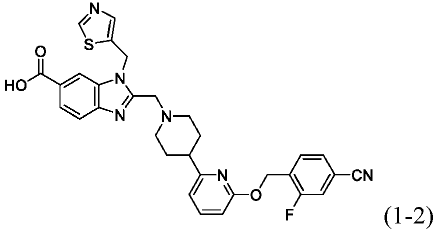 oder ein pharmazeutisch unbedenkliches Salz davon handelt.

9. Kombination zur Verwendung oder Verwendung nach Anspruch 8, wobei es sich bei dem GLP-1R-Agonisten um ein Megluminsalz von Verbindung 1-2 handelt.

10. Kombination zur Verwendung oder Verwendung nach einem der Ansprüche 1 bis 5, wobei es sich bei dem GLP-1R-Agonisten um Orforglipron, Danuglipron, Liraglutid, Exenatid, Dulaglutid, Albiglutid, Lixisenatid, Tirzepatid oder Semaglutid handelt.

11. Kombination zur Verwendung nach Anspruch 1 oder Verwendung nach Anspruch 2, wobei es sich bei dem THRß-Agonisten um Verbindung 9 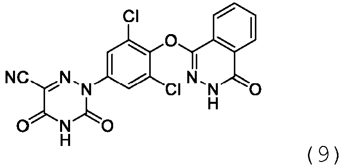 oder ein pharmazeutisch unbedenkliches Salz davon handelt und es sich bei dem GLP-1R-Agonisten um Orforglipron, Danuglipron oder Semaglutid oder ein pharmazeutisch unbedenkliches Salz davon handelt.

12. Kombination zur Verwendung oder Verwendung nach einem der vorhergehenden Ansprüche, wobei der THRβ-Agonist oder ein pharmazeutisch unbedenkliches Salz davon und der GLP-1R-Agonist oder ein pharmazeutisch unbedenkliches Salz davon wie folgt formuliert sind:
• in getrennten pharmazeutisch unbedenklichen Zusammensetzungen oder
• in einer einzigen pharmazeutisch unbedenklichen Zusammensetzung.

13. Kombination zur Verwendung oder Verwendung nach einem der vorhergehenden Ansprüche, wobei:
• der Anteil der fettfreien Körpermasse relativ zur Gesamtkörpermasse bei einem Patienten zunimmt und/oder
• der Anteil der Fettmasse an der Gesamtkörpermasse bei einem Patienten abnimmt.

14. Kombination zur Verwendung oder Verwendung nach einem der vorhergehenden Ansprüche, wobei der Patient einen Körpermasseindex (Body Mass Index, BMI) von 27 kg/m² oder mehr, vorzugsweise 30 kg/m² oder mehr, aufweist.

15. Kombination zur Verwendung oder Verwendung nach einem der vorhergehenden Ansprüche, wobei:
• die Verabreichung des THRß-Agonisten die Verabreichung einer reduzierten Dosis des GLP-1R-Agonisten unter Beibehaltung eines äquivalenten Gewichtsverlusts ermöglicht und/oder
• die Verabreichung des THRß-Agonisten den Gewichtsverlust im Vergleich zur Verabreichung eines GLP-1R-Agonisten in einer äquivalenten Dosis erhöht.

## Revendications

1. Combinaison comprenant :
(i) un agoniste de THRβ qui est : 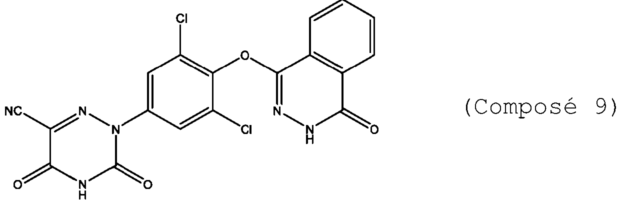 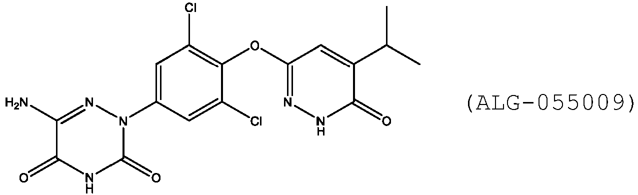 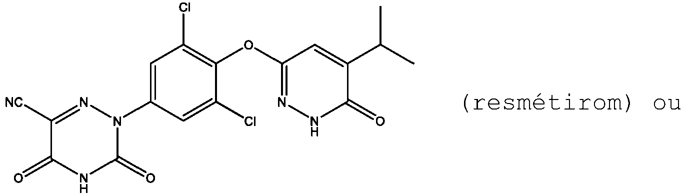 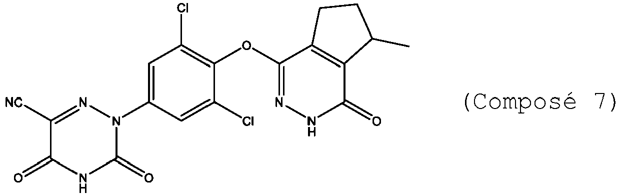 ou un sel pharmaceutiquement acceptable de celui-ci, et
(ii) un agoniste de GLP-1R, ou un sel pharmaceutiquement acceptable de celui-ci,
pour une utilisation dans
(i) la perte de poids chez un patient ayant un IMC de 25 kg/m² ou plus, ou
(ii) la prévention ou le traitement de l'obésité.

2. Utilisation non thérapeutique d'une combinaison comprenant :
(i) un agoniste de THRβ qui est : 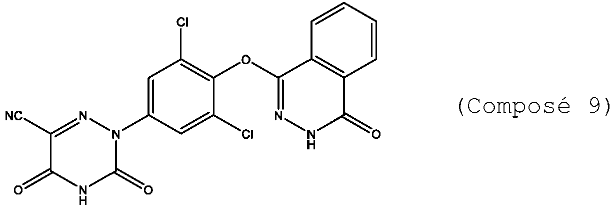 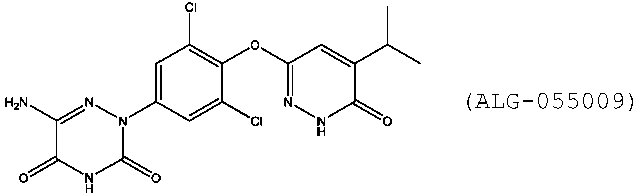 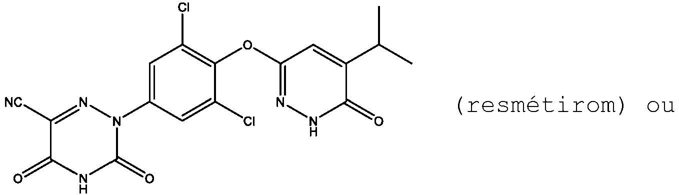 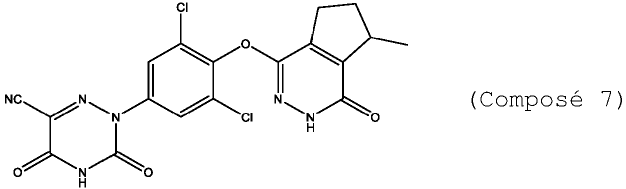 ou un sel pharmaceutiquement acceptable de celui-ci, et
(ii) un agoniste de GLP-1R, ou un sel pharmaceutiquement acceptable de celui-ci,
pour réaliser une perte de poids.

3. Combinaison pour utilisation selon la revendication 1 ou utilisation selon la revendication 2, dans laquelle l'agoniste de THRβ est le composé 9 : 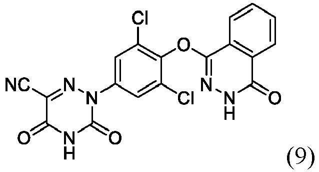 ou un sel pharmaceutiquement acceptable de celui-ci.

4. Combinaison pour utilisation ou utilisation selon la revendication 3, dans laquelle l'agoniste de THRβ est un sel de potassium du composé 9.

5. Combinaison pour utilisation selon la revendication 1 ou utilisation selon la revendication 2, dans laquelle l'agoniste de THRβ est : 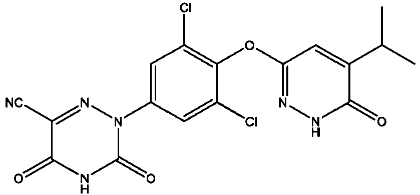 (resmétirom) ou un sel pharmaceutiquement acceptable de celui-ci.

6. Combinaison pour utilisation ou utilisation selon l'une quelconque des revendications précédentes, dans laquelle l'agoniste de GLP-1R est un composé de la formule (I-1) : 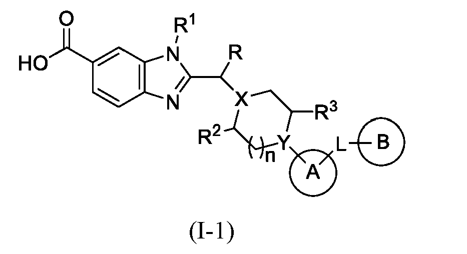 ou un sel pharmaceutiquement acceptable de celui-ci, dans laquelle :
X est N ou CH ;
Y est N ou CR⁴ ;
n est 0 ou 1 ;
R est hydrogène ;
R¹ est -C₁-C₆ alkylène-R⁵ ;
R² est hydrogène, oxo, ou C₁-C₆ alkyle ;
R³ est hydrogène, oxo, ou C₁-C₆ alkyle et R⁴ est hydrogène, OH, ou C₁-C₆ alkyle ;
ou R³ et R⁴ sont pris ensemble avec les atomes de carbone auxquels ils sont fixés pour former C₃-C₆ cycloalkyle éventuellement substitué par halogéno ou C₁-C₃ alkyle ;
R⁵ est hétérocyclyle à 5 chaînons ou hétéroaryle à 5 chaînons, chacun desquels comprenant 1, 2, ou 3 hétéroatomes indépendamment choisis parmi O, N, et S, dans laquelle au moins un hétéroatome parmi R⁵ est S, et en outre dans laquelle R⁵ est éventuellement substitué par halogéno, -O-C₁₋₆ alkyle, C₁₋₆ alkyle, C₁₋₄ alcényle ou C₁-C₆ halogénoalkyle ;
le cycle A est hétérocyclène à 5 à 12 chaînons ou hétéroarylène à 5 à 12 chaînons, chacun desquels étant indépendamment éventuellement substitué par halogéno, CN, C₃-C₆ cycloalkyle ou C₁-C₆ alkyle éventuellement substitué par halogéno ou OH ;
L est une liaison, -O-, C₁-C₆ alkylène, *-O-C₁-C₆ alkylène-**, *-C₁-C₆ alkylène-O-** ou *-NR⁶-C₁-C₆ alkylène-**, dans laquelle
* représente le point de fixation au cycle A et ** représente le point de fixation au cycle B ;
lorsque L est *-O-C₁-C₆ alkylène-**, le C₁-C₆ alkylène de L est éventuellement substitué par R^{L}, dans laquelle chaque R^{L} est indépendamment C₁-C₆ alkyle ou halogéno, ou deux R^{L} sont pris ensemble avec l'atome ou les atomes de carbone auxquels ils sont fixés pour former C₃-C₆ cycloalkyle ou hétérocyclyle à 3 à 6 chaînons ; et
lorsque L est C₁-C₆ alkylène, le C₁-C₆ alkylène est éventuellement substitué par R^{L1}, dans laquelle chaque R^{L1} est indépendamment halogéno, OH, oxo ou C₁-C₆ alkyle, ou deux R^{L1} sont pris ensemble avec l'atome ou les atomes de carbone auxquels ils sont fixés pour former C₃-C₆ cycloalkyle ou hétérocyclyle à 3 à 6 chaînons ;
R⁶ est hydrogène ou C₁-C₆ alkyle ; et
le cycle B est C₃-C₁₀ cycloalkyle, C₆-C₁₄ aryle, hétérocyclyle à 4 à 12 chaînons ou hétéroaryle à 5 à 12 chaînons, chacun desquels étant indépendamment éventuellement substitué par un à trois substituants indépendamment choisis dans un groupe constitué d'halogéno, CN, oxo, C₁-C₆ alkyle, C₁-C₆ halogénoalkyle, -COCH₃, -CONH₂, -S(O)₂CH₃ et phényle.

7. Combinaison pour utilisation ou utilisation selon l'une quelconque des revendications précédentes, dans laquelle l'agoniste de GLP-1R est un composé de la formule (I-1a) : 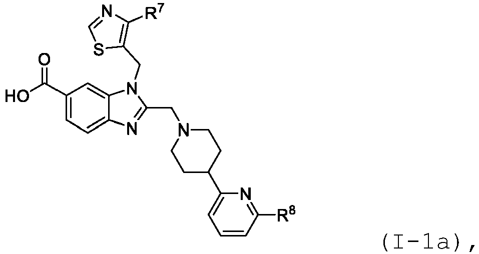 ou un sel pharmaceutiquement acceptable de celui-ci, dans laquelle R⁷ est hydrogène, chloro, bromo, fluoro, méthyle ou vinyle ; et R⁸ est 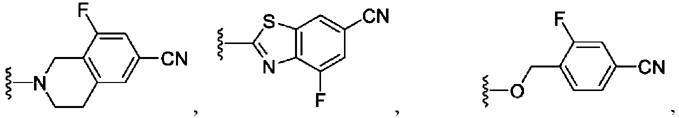 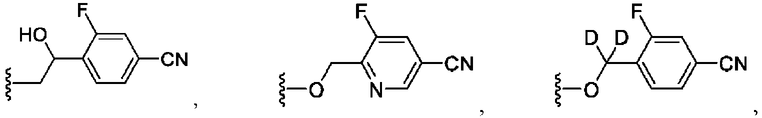 ou

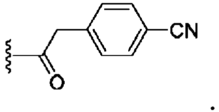

8. Combinaison pour utilisation ou utilisation selon l'une quelconque des revendications précédentes, dans laquelle l'agoniste de GLP-1R est le composé 1-2 : 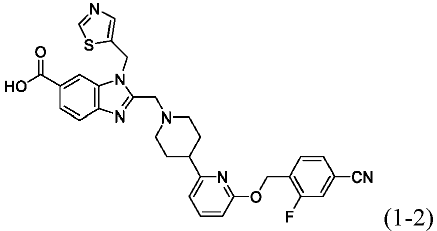 ou un sel pharmaceutiquement acceptable de celui-ci.

9. Combinaison pour utilisation ou utilisation selon la revendication 8, dans laquelle l'agoniste de GLP-1R est un sel de méglumine du composé 1-2.

10. Combinaison pour utilisation ou utilisation selon l'une quelconque des revendications 1 à 5, dans laquelle l'agoniste de GLP-1R est l'orforglipron, le danuglipron, le liraglutide, l'exénatide, le dulaglutide, l'albiglutide, le lixisenatide, le tirzépatide ou le sémaglutide.

11. Combinaison pour utilisation selon la revendication 1 ou utilisation selon la revendication 2, dans laquelle l'agoniste de THRβ est le composé 9 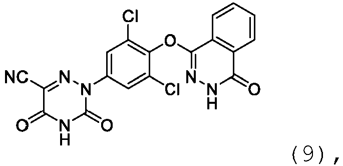 ou un sel pharmaceutiquement acceptable de celui-ci, et l'agoniste de GLP-1R est l'orforglipron, le danuglipron ou le sémaglutide, ou un sel pharmaceutiquement acceptable de celui-ci.

12. Combinaison pour utilisation ou utilisation selon l'une quelconque des revendications précédentes, dans laquelle l'agoniste de THRβ ou un sel pharmaceutiquement acceptable de celui-ci, et l'agoniste de GLP-1R, ou un sel pharmaceutiquement acceptable de celui-ci, sont formulés :
• dans des compositions pharmaceutiquement acceptables distinctes ; ou
• dans une composition pharmaceutiquement acceptable unique.

13. Combinaison pour utilisation ou utilisation selon l'une quelconque des revendications précédentes, dans laquelle :
• la proportion de masse corporelle maigre par rapport à la masse corporelle totale chez un patient augmente ; et/ou
• la proportion de masse graisseuse par rapport à la masse corporelle totale chez un patient diminue.

14. Combinaison pour utilisation ou utilisation selon l'une quelconque des revendications précédentes, dans laquelle le patient a un indice de masse corporelle (IMC) de 27 kg/m² ou plus, de préférence de 30 kg/m² ou plus.

15. Combinaison pour utilisation ou utilisation selon l'une quelconque des revendications précédentes, dans laquelle :
• l'administration de l'agoniste de THRβ permet l'administration d'une dose réduite de l'agoniste de GLP-1R tout en maintenant une perte de poids équivalente ; et/ou
• l'administration de l'agoniste de THRβ augmente la perte de poids par rapport à l'administration d'un agoniste de GLP-1R à une dose équivalente.
